(19) Europäisches Patentamt / European Patent Office / Office européen des brevets

(11) **EP 3 498 829 A1**

(12) **EUROPEAN PATENT APPLICATION**

(43) Date of publication:
**19.06.2019 Bulletin 2019/25**

(51) Int Cl.:
***C12N 9/04*** *(2006.01)* ***C12N 9/02*** *(2006.01)*
***C12P 7/10*** *(2006.01)*

(21) Application number: **18171345.4**

(22) Date of filing: **08.11.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2012 US 201261724831 P**
**15.03.2013 US 201361793716 P**

(62) Document number(s) of the earlier application(s) in accordance with Art. 76 EPC:
**13812217.1 / 2 917 343**

(71) Applicant: **Lallemand Hungary Liquidity Management LLC**
**1138 Budapest (HU)**

(72) Inventors:
- **ZELLE, Rintze Meindert**
  **Hanover, NH 03755 (US)**
- **SHAW, Arthur J., IV**
  **Grantham, NH 03753 (US)**
- **VAN DIJKEN, Johannes Pieter**
  **3115 HG Schiedam (NL)**

(74) Representative: **Pawlyn, Anthony Neil**
**Urquhart-Dykes & Lord LLP**
**7th Floor, Churchill House**
**Churchill Way**
**Cardiff CF10 2HH (GB)**

Remarks:
This application was filed on 08-05-2018 as a divisional application to the application mentioned under INID code 62.

(54) **METHOD FOR ACETATE CONSUMPTION DURING ETHANOLIC FERMENTATION OF CELLULOSIC FEEDSTOCKS**

(57) The present invention provides for novel metabolic pathways to detoxify biomass-derived acetate via metabolic conversion to ethanol, acetone, or isopropanol. More specifically, the invention provides for a recombinant microorganism comprising one or more native and/or heterologous enzymes that function in one or more first engineered metabolic pathways to achieve: (1) conversion of acetate to ethanol; (2) conversion of acetate to acetone; or (3) conversion of acetate to isopropanol; and one or more native and/or heterologous enzymes that function in one or more second engineered metabolic pathways to produce an electron donor used in the conversion of acetate to less inhibitory compounds; wherein the one or more native and/or heterologous enzymes is activated, upregulated, or downregulated.

Figure 1

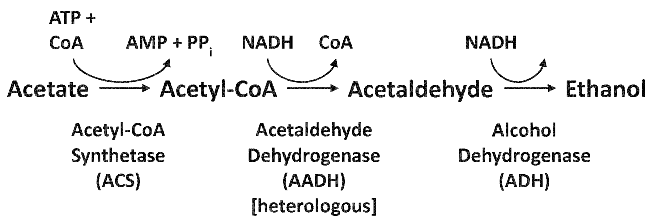


**Net reaction**

acetate + 2 ATP + 2 NADH → ethanol + 2 ADP + 2 $P_i$

EP 3 498 829 A1

SEQUENCE LISTING

<110> MASCOMA CORPORATION
ZELLE, RINTZE MEINDERT
SHAW, ARTHUR J., IV
VAN DIJKEN, JOHANNES PIETER

<120> METHOD FOR ACETATE CONSUMPTION DURING ETHANOLIC FERMENTATION OF CELLULOSIC FEEDSTOCKS

<130> 2608.067PC02

<140> To Be Assigned
<141> Herewith

<150> US 61/724,831
<151> 2012-11-09

<150> US 61/793,716
<151> 2013-03-15

<160> 62

<170> PatentIn version 3.5

<210> 1
<211> 3279
<212> DNA
<213> Saccharomyces cerevisiae

<220>
<221> misc_feature
<222> (1)..(3279)
<223> GDH2

<400> 1
atgcttttg ataacaaaaa tcgcggtgct ttaaactcac tgaacacacc agatattgct 60

tctttatcaa tatcatccat gtcggactat cacgtgtttg attttcccgg taaggacctg 120

cagagagagg aagtgataga tttgctagat cagcaagggt ttattcccga cgatttgatc 180

gaacaagaag tagattggtt ttataactca ttgggtattg acgatttgtt cttctcgaga 240

gaatctcccc aattaatctc gaatatcata cattctttgt atgcttcaaa gctagatttc 300

tttgcgaagt ccaaattcaa cggaattcag ccaaggctat tcagcattaa aaacaaaatt 360

ataactaatg ataatcatgc catctttatg gaatctaata ctggtgtcag cataagcgat 420

tctcagcaaa aaaactttaa atttgctagt gacgccgtcg gaaacgatac tttggagcat 480

ggtaaggata ccatcaaaaa aaataggatt gaaatggatg attcttgtcc accttatgaa 540

ttagattccg aaattgatga ccttttcctg gataacaagt ctcaaaaaaa ctgcagatta 600

gtttcttttt gggctccaga aagcgaatta aagctaactt ttgtttatga gagtgtttac 660

cctaatgatg atccagccgg cgtagatatt tcctctcagg atttgctgaa aggtgatatt 720

gaatcgatta gtgataagac catgtacaaa gtttcgtcga acgaaaataa aaaactatac 780

ggtctcttac ttaagttggt taaagaaaga gaaggtcctg tcattaagac tactcgctcc 840
gtagaaaata aggatgaaat taggttatta gtcgcttaca agcgattcac cactaagcgt 900
tattactctg ctttgaactc tttgttccac tattacaagt tgaaaccttc taagttctat 960
ttagagtcgt ttaatgttaa ggatgatgac atcattatct tttccgttta tttgaacgag 1020
aaccagcaat tggaagatgt tctacttcac gatgtggagg cagcattgaa acaggttgaa 1080
agagaagctt cattgctata cgctatccca aacaattctt tccatgaggt ttaccagaga 1140
cgtcaattct cgcccaaaga agctatatat gctcatattg gtgctatatt cattaaccat 1200
tttgttaatc gtttaggctc tgattatcaa aaccttttat ctcaaatcac cattaagcgt 1260
aatgatacta ctcttttgga gattgtagaa aacctaaaaa gaaagttaag aaatgaaacc 1320
ttaactcagc aaactattat caacatcatg tcgaagcatt acactataat ttccaagttg 1380
tataaaaatt ttgctcaaat tcactattat cataatagta ctaaagatat ggagaagaca 1440
ttatcttttc aaagactgga aaaagtggag ccttttaaga atgaccaaga gttcgaagct 1500
tacttgaata aattcattcc aaatgattca cctgatttgt tgatcctgaa aacactgaac 1560
atcttcaaca agtctatttt gaagacaaat ttctttatta caagaaaagt agcaatatca 1620
ttcagattag atccttccct ggtgatgaca aaattcgaat atccagagac accctatggt 1680
atatttttg tcgttggtaa tactttcaaa gggttccata tcaggttcag agatatcgca 1740
aggggcggta ttcgtatagt ctgttccagg aatcaggata tttatgattt gaattccaag 1800
aacgttattg atgagaacta tcaattggcc tctactcagc aacgtaaaaa taaggatatt 1860
ccagagggtg gctctaaagg tgtcatctta ttgaacccag gattggtaga acatgaccag 1920
acatttgtcg ccttttccca atatgtggat gcaatgattg acattctaat caacgatcca 1980
ttaaaggaaa actatgtcaa ccttttacca aaggaggaaa tattattttt tggcccagat 2040
gaaggaactg ctggtttcgt ggattgggca actaaccatg ctcgtgtgag gaactgccca 2100
tggtggaaat catttttgac tggaaaatcc ccatctttgg gtggtattcc ccatgacgaa 2160
tatggtatga cttctctggg tgttcgtgct tatgttaata aaatttacga aactttaaac 2220
ttgacaaatt ctactgttta caaattccaa actggtggtc cggatggtga tttgggatcc 2280
aatgaaattc ttttatcttc gccaaacgaa tgttatttgg caattctgga cggttcaggt 2340
gtcctgtgtg atcctaaagg tttagataaa gatgaattat gccgcttggc acatgaaagg 2400
aaaatgattt ccgatttcga cacttccaaa ttatcaaaca acggattttt tgtttctgtg 2460
gatgcaatgg atatcatgct accaaatggt acaattgtag ctaacggcac aaccttcaga 2520
aacacctttc atactcaaat tttcaaattt gtggatcatg tcgacatttt tgttccatgc 2580
ggtggtagac caaactcaat tactctaaat aatctacatt attttgttga cgaaaagact 2640
gggaaatgta aaattccata tattgtggag ggtgccaatc tatttataac gcaacctgct 2700

aaaaatgctt tggaggaaca tggctgtatt ctgttcaaag atgcttctgc aaacaaaggt 2760

ggtgtcacat cttcatcaat ggaagtgttg gcctcactag cgcttaacga taacgacttc 2820

gtgcacaaat ttattggaga tgttagtggt gagaggtctg cgttgtacaa gtcgtacgtt 2880

gtagaagtgc agtcaagaat tcagaaaaat gctgaattag agtttggtca gttatggaat 2940

ttgaatcaac taaatggaac ccacatttca gaaatttcaa accaattgtc cttcactata 3000

aacaaattga acgacgatct agttgcttct caagagttgt ggctcaatga tctaaaatta 3060

agaaactacc tattgttgga taaaataatt ccaaaaattc tgattgatgt tgctgggcct 3120

cagtccgtat tggaaaacat tccagagagc tatttgaaag ttcttctgtc gagttactta 3180

tcaagcactt ttgtttacca gaacggtatc gatgttaaca ttggaaaatt cttggaattt 3240

attggtgggt taaaaagaga agcggaggca agtgcttga 3279

<210> 2
<211> 1092
<212> PRT
<213> Saccharomyces cerevisiae

<220>
<221> misc_feature
<222> (1)..(1092)
<223> GDH2

<400> 2

Met Leu Phe Asp Asn Lys Asn Arg Gly Ala Leu Asn Ser Leu Asn Thr
1               5                   10                  15

Pro Asp Ile Ala Ser Leu Ser Ile Ser Ser Met Ser Asp Tyr His Val
            20                  25                  30

Phe Asp Phe Pro Gly Lys Asp Leu Gln Arg Glu Glu Val Ile Asp Leu
        35                  40                  45

Leu Asp Gln Gln Gly Phe Ile Pro Asp Asp Leu Ile Glu Gln Glu Val
    50                  55                  60

Asp Trp Phe Tyr Asn Ser Leu Gly Ile Asp Asp Leu Phe Phe Ser Arg
65                  70                  75                  80

Glu Ser Pro Gln Leu Ile Ser Asn Ile Ile His Ser Leu Tyr Ala Ser
                85                  90                  95

Lys Leu Asp Phe Phe Ala Lys Ser Lys Phe Asn Gly Ile Gln Pro Arg
            100                 105                 110

```
Leu Phe Ser Ile Lys Asn Lys Ile Ile Thr Asn Asp Asn His Ala Ile
        115                 120                 125

Phe Met Glu Ser Asn Thr Gly Val Ser Ile Ser Asp Ser Gln Gln Lys
    130                 135                 140

Asn Phe Lys Phe Ala Ser Asp Ala Val Gly Asn Asp Thr Leu Glu His
145                 150                 155                 160

Gly Lys Asp Thr Ile Lys Lys Asn Arg Ile Glu Met Asp Asp Ser Cys
                165                 170                 175

Pro Pro Tyr Glu Leu Asp Ser Glu Ile Asp Asp Leu Phe Leu Asp Asn
            180                 185                 190

Lys Ser Gln Lys Asn Cys Arg Leu Val Ser Phe Trp Ala Pro Glu Ser
        195                 200                 205

Glu Leu Lys Leu Thr Phe Val Tyr Glu Ser Val Tyr Pro Asn Asp Asp
    210                 215                 220

Pro Ala Gly Val Asp Ile Ser Ser Gln Asp Leu Leu Lys Gly Asp Ile
225                 230                 235                 240

Glu Ser Ile Ser Asp Lys Thr Met Tyr Lys Val Ser Ser Asn Glu Asn
                245                 250                 255

Lys Lys Leu Tyr Gly Leu Leu Leu Lys Leu Val Lys Glu Arg Glu Gly
            260                 265                 270

Pro Val Ile Lys Thr Thr Arg Ser Val Glu Asn Lys Asp Glu Ile Arg
        275                 280                 285

Leu Leu Val Ala Tyr Lys Arg Phe Thr Thr Lys Arg Tyr Tyr Ser Ala
    290                 295                 300

Leu Asn Ser Leu Phe His Tyr Tyr Lys Leu Lys Pro Ser Lys Phe Tyr
305                 310                 315                 320

Leu Glu Ser Phe Asn Val Lys Asp Asp Asp Ile Ile Ile Phe Ser Val
                325                 330                 335

Tyr Leu Asn Glu Asn Gln Gln Leu Glu Asp Val Leu Leu His Asp Val
            340                 345                 350

Glu Ala Ala Leu Lys Gln Val Glu Arg Glu Ala Ser Leu Leu Tyr Ala
        355                 360                 365
```

```
Ile Pro Asn Asn Ser Phe His Glu Val Tyr Gln Arg Arg Gln Phe Ser
    370                 375                 380

Pro Lys Glu Ala Ile Tyr Ala His Ile Gly Ala Ile Phe Ile Asn His
385                 390                 395                 400

Phe Val Asn Arg Leu Gly Ser Asp Tyr Gln Asn Leu Leu Ser Gln Ile
                405                 410                 415

Thr Ile Lys Arg Asn Asp Thr Thr Leu Leu Glu Ile Val Glu Asn Leu
            420                 425                 430

Lys Arg Lys Leu Arg Asn Glu Thr Leu Thr Gln Gln Thr Ile Ile Asn
        435                 440                 445

Ile Met Ser Lys His Tyr Thr Ile Ile Ser Lys Leu Tyr Lys Asn Phe
    450                 455                 460

Ala Gln Ile His Tyr Tyr His Asn Ser Thr Lys Asp Met Glu Lys Thr
465                 470                 475                 480

Leu Ser Phe Gln Arg Leu Glu Lys Val Glu Pro Phe Lys Asn Asp Gln
                485                 490                 495

Glu Phe Glu Ala Tyr Leu Asn Lys Phe Ile Pro Asn Asp Ser Pro Asp
            500                 505                 510

Leu Leu Ile Leu Lys Thr Leu Asn Ile Phe Asn Lys Ser Ile Leu Lys
        515                 520                 525

Thr Asn Phe Phe Ile Thr Arg Lys Val Ala Ile Ser Phe Arg Leu Asp
    530                 535                 540

Pro Ser Leu Val Met Thr Lys Phe Glu Tyr Pro Glu Thr Pro Tyr Gly
545                 550                 555                 560

Ile Phe Phe Val Val Gly Asn Thr Phe Lys Gly Phe His Ile Arg Phe
                565                 570                 575

Arg Asp Ile Ala Arg Gly Gly Ile Arg Ile Val Cys Ser Arg Asn Gln
            580                 585                 590

Asp Ile Tyr Asp Leu Asn Ser Lys Asn Val Ile Asp Glu Asn Tyr Gln
        595                 600                 605

Leu Ala Ser Thr Gln Gln Arg Lys Asn Lys Asp Ile Pro Glu Gly Gly
    610                 615                 620
```

Ser Lys Gly Val Ile Leu Leu Asn Pro Gly Leu Val Glu His Asp Gln
625 630 635 640

Thr Phe Val Ala Phe Ser Gln Tyr Val Asp Ala Met Ile Asp Ile Leu
645 650 655

Ile Asn Asp Pro Leu Lys Glu Asn Tyr Val Asn Leu Leu Pro Lys Glu
660 665 670

Glu Ile Leu Phe Phe Gly Pro Asp Glu Gly Thr Ala Gly Phe Val Asp
675 680 685

Trp Ala Thr Asn His Ala Arg Val Arg Asn Cys Pro Trp Trp Lys Ser
690 695 700

Phe Leu Thr Gly Lys Ser Pro Ser Leu Gly Gly Ile Pro His Asp Glu
705 710 715 720

Tyr Gly Met Thr Ser Leu Gly Val Arg Ala Tyr Val Asn Lys Ile Tyr
725 730 735

Glu Thr Leu Asn Leu Thr Asn Ser Thr Val Tyr Lys Phe Gln Thr Gly
740 745 750

Gly Pro Asp Gly Asp Leu Gly Ser Asn Glu Ile Leu Leu Ser Ser Pro
755 760 765

Asn Glu Cys Tyr Leu Ala Ile Leu Asp Gly Ser Gly Val Leu Cys Asp
770 775 780

Pro Lys Gly Leu Asp Lys Asp Glu Leu Cys Arg Leu Ala His Glu Arg
785 790 795 800

Lys Met Ile Ser Asp Phe Asp Thr Ser Lys Leu Ser Asn Asn Gly Phe
805 810 815

Phe Val Ser Val Asp Ala Met Asp Ile Met Leu Pro Asn Gly Thr Ile
820 825 830

Val Ala Asn Gly Thr Thr Phe Arg Asn Thr Phe His Thr Gln Ile Phe
835 840 845

Lys Phe Val Asp His Val Asp Ile Phe Val Pro Cys Gly Gly Arg Pro
850 855 860

Asn Ser Ile Thr Leu Asn Asn Leu His Tyr Phe Val Asp Glu Lys Thr

```
865                 870                 875                 880

Gly Lys Cys Lys Ile Pro Tyr Ile Val Glu Gly Ala Asn Leu Phe Ile
                885                 890                 895

Thr Gln Pro Ala Lys Asn Ala Leu Glu Glu His Gly Cys Ile Leu Phe
            900                 905                 910

Lys Asp Ala Ser Ala Asn Lys Gly Gly Val Thr Ser Ser Ser Met Glu
        915                 920                 925

Val Leu Ala Ser Leu Ala Leu Asn Asp Asn Asp Phe Val His Lys Phe
    930                 935                 940

Ile Gly Asp Val Ser Gly Glu Arg Ser Ala Leu Tyr Lys Ser Tyr Val
945                 950                 955                 960

Val Glu Val Gln Ser Arg Ile Gln Lys Asn Ala Glu Leu Glu Phe Gly
                965                 970                 975

Gln Leu Trp Asn Leu Asn Gln Leu Asn Gly Thr His Ile Ser Glu Ile
            980                 985                 990

Ser Asn Gln Leu Ser Phe Thr Ile  Asn Lys Leu Asn Asp  Asp Leu Val
        995                 1000                1005

Ala Ser  Gln Glu Leu Trp Leu  Asn Asp Leu Lys Leu  Arg Asn Tyr
    1010                1015                1020

Leu Leu  Leu Asp Lys Ile Ile  Pro Lys Ile Leu Ile  Asp Val Ala
    1025                1030                1035

Gly Pro  Gln Ser Val Leu Glu  Asn Ile Pro Glu Ser  Tyr Leu Lys
    1040                1045                1050

Val Leu  Leu Ser Ser Tyr Leu  Ser Ser Thr Phe Val  Tyr Gln Asn
    1055                1060                1065

Gly Ile  Asp Val Asn Ile Gly  Lys Phe Leu Glu Phe  Ile Gly Gly
    1070                1075                1080

Leu Lys  Arg Glu Ala Glu Ala  Ser Ala
    1085                1090
```

<210> 3
<211> 450
<212> DNA
<213> Saccharomyces cerevisiae

<220>
<221> misc_feature
<222> (1)..(450)
<223> promoter pTPI1

<400> 3
ctacttattc ccttcgagat tatatctagg aacccatcag gttggtggaa gattacccgt 60

tctaagactt ttcagcttcc tctattgatg ttacacctgg acaccccttt tctggcatcc 120

agtttttaat cttcagtggc atgtgagatt ctccgaaatt aattaaagca atcacacaat 180

tctctcggat accacctcgg ttgaaactga caggtggttt gttacgcatg ctaatgcaaa 240

ggagcctata tacctttggc tcggctgctg taacagggaa tataaagggc agcataattt 300

aggagtttag tgaacttgca acatttacta ttttcccttc ttacgtaaat atttttcttt 360

ttaattctaa atcaatcttt ttcaattttt tgtttgtatt cttttcttgc ttaaatctat 420

aactacaaaa aacacataca taaactaaaa 450

<210> 4
<211> 376
<212> PRT
<213> Artificial sequence

<220>
<221> misc_feature
<222> (1)..(376)
<223> thymidine kinase gene from Herpes Simplex Virus Type 1

<400> 4

Met Ala Ser Tyr Pro Cys His Gln His Ala Ser Ala Phe Asp Gln Ala
1 5 10 15

Ala Arg Ser Arg Gly His Ser Asn Arg Arg Thr Ala Leu Arg Pro Arg
20 25 30

Arg Gln Gln Glu Ala Thr Glu Val Arg Leu Glu Gln Lys Met Pro Thr
35 40 45

Leu Leu Arg Val Tyr Ile Asp Gly Pro His Gly Met Gly Lys Thr Thr
50 55 60

Thr Thr Gln Leu Leu Val Ala Leu Gly Ser Arg Asp Asp Ile Val Tyr
65 70 75 80

Val Pro Glu Pro Met Thr Tyr Trp Gln Val Leu Gly Ala Ser Glu Thr
85 90 95

Ile Ala Asn Ile Tyr Thr Thr Gln His Arg Leu Asp Gln Gly Glu Ile

```
            100                 105                 110

Ser Ala Gly Asp Ala Ala Val Val Met Thr Ser Ala Gln Ile Thr Met
        115                 120                 125

Gly Met Pro Tyr Ala Val Thr Asp Ala Val Leu Ala Pro His Val Gly
    130                 135                 140

Gly Glu Ala Gly Ser Ser His Ala Pro Pro Pro Ala Leu Thr Leu Ile
145                 150                 155                 160

Phe Asp Arg His Pro Ile Ala Ala Leu Leu Cys Tyr Pro Ala Ala Arg
                165                 170                 175

Tyr Leu Met Gly Ser Met Thr Pro Gln Ala Val Leu Ala Phe Val Ala
            180                 185                 190

Leu Ile Pro Pro Thr Leu Pro Gly Thr Asn Ile Val Leu Gly Ala Leu
        195                 200                 205

Pro Glu Asp Arg His Ile Asp Arg Leu Ala Lys Arg Gln Arg Pro Gly
    210                 215                 220

Glu Arg Leu Asp Leu Ala Met Leu Ala Ala Ile Arg Arg Val Tyr Gly
225                 230                 235                 240

Leu Leu Ala Asn Thr Val Arg Tyr Leu Gln Gly Gly Gly Ser Trp Trp
                245                 250                 255

Glu Asp Trp Gly Gln Leu Ser Gly Thr Ala Val Pro Pro Gln Gly Ala
            260                 265                 270

Glu Pro Gln Ser Asn Ala Gly Pro Arg Pro His Ile Gly Asp Thr Leu
        275                 280                 285

Phe Thr Leu Phe Arg Ala Pro Glu Leu Leu Ala Pro Asn Gly Asp Leu
    290                 295                 300

Tyr Asn Val Phe Ala Trp Ala Leu Asp Val Leu Ala Lys Arg Leu Arg
305                 310                 315                 320

Pro Met His Val Phe Ile Leu Asp Tyr Asp Gln Ser Pro Ala Gly Cys
                325                 330                 335

Arg Asp Ala Leu Leu Gln Leu Thr Ser Gly Met Val Gln Thr His Val
            340                 345                 350
```

Thr Thr Pro Gly Ser Ile Pro Thr Ile Cys Asp Leu Ala Arg Thr Phe
355 360 365

Ala Arg Glu Met Gly Glu Ala Asn
370 375

<210> 5
<211> 957
<212> DNA
<213> Scheffersomyces stipitis

<220>
<221> misc_feature
<222> (1)..(957)
<223> XYL1

<400> 5
atgccttcta ttaagttgaa ctctggttac gacatgccag ccgtcggttt cggctgttgg 60

aaagtcgacg tcgacacctg ttctgaacag atctaccgtg ctatcaagac cggttacaga 120

ttgttcgacg gtgccgaaga ttacgccaac gaaaagttag ttggtgccgg tgtcaagaag 180

gccattgacg aaggtatcgt caagcgtgaa gacttgttcc ttacctccaa gttgtggaac 240

aactaccacc acccagacaa cgtcgaaaag gccttgaaca gaaccctttc tgacttgcaa 300

gttgactacg ttgacttgtt cttgatccac ttcccagtca ccttcaagtt cgttccatta 360

gaagaaaagt acccaccagg attctactgt ggtaagggtg acaacttcga ctacgaagat 420

gttccaattt tagagacctg gaaggctctt gaaaagttgg tcaaggccgg taagatcaga 480

tctatcggtg tttctaactt cccaggtgct ttgctcttgg acttgttgag aggtgctacc 540

atcaagccat ctgtcttgca agttgaacac cacccatact tgcaacaacc aagattgatc 600

gaattcgctc aatcccgtgg tattgctgtc accgcttact cttcgttcgg tcctcaatct 660

ttcgttgaat tgaaccaagg tagagctttg aacacttctc cattgttcga gaacgaaact 720

atcaaggcta tcgctgctaa gcacggtaag tctccagctc aagtcttgtt gagatggtct 780

tcccaaagag gcattgccat cattccaaag tccaacactg tcccaagatt gttggaaaac 840

aaggacgtca acagcttcga cttggacgaa caagatttcg ctgacattgc caagttggac 900

atcaacttga gattcaacga cccatgggac tgggacaaga ttcctatctt cgtctaa 957

<210> 6
<211> 966
<212> DNA
<213> Candida boidinii

<220>
<221> misc_feature
<222> (1)..(966)
<223> Aldolase Reductase

<400> 6
atgtcaagcc cacttttaac tttaaacaat ggcttaaaga tgccacaaat cggttttggt 60

tgttggaaag tcgacaatgc cacttgtgcc gaaactattt atgaagccat taaagtcggt 120

tacagattat tcgatggtgc tatggattac ggtaatgaaa aagaagttgg tgaaggtgtt 180

aacaaagcga tcaaagatgg tttagttaaa agagaagaat tattcattgt ttcaaaatta 240

tggaacaatt tccatcatcc agattcagtt aaactagcaa tcaaaaaagt tctatctgat 300

ttaaatttag aatacattga tttattctat atgcatttcc caattgctca aaaatttgtt 360

ccaattgaaa agaaatatcc accaaatttt tattgtggtg atggtgataa atggagtttt 420

gaagatgtcc cacttttaac aacttggaga gctatggaag aattggttga agaaggttta 480

gttaaatcaa ttggtatctc taactttgtc ggtgctttga ttcaagattt attaagaggt 540

tgtaaaatta gaccagcagt tttagaaatt gaacatcacc catatttagt tcaaccaaga 600

ttaattgaat acgctaaaac tgaaggtatt cacgttaccg catactcttc atttggtcca 660

caatcatttg ttgaattaga ccatcctaaa gttaaagact gtaccactct attcaaacat 720

gaaacaatta cttcaattgc ttcagctcat gacgtccctc cagctaaagt cttattgaga 780

tgggctactc aaagaggttt agcagttatc ccaaaatcta ataaaaagga aagattatta 840

ggtaatttga aaattaatga ttttgattta actgaagctg aacttgaaaa aattgaagca 900

ttagatattg gtttaagatt taatgatcca tggacttggg gttacaatat tccaacattt 960

atttaa 966

<210> 7
<211> 969
<212> DNA
<213> Neurospora crassa

<220>
<221> misc_feature
<222> (1)..(969)
<223> Xylose Reductase

<400> 7
atggttcctg ccatcaaact gaactctggc ttcgatatgc ctcaagttgg ttttggtttg 60

tggaaagtgg atggatcaat cgcctcagat gtggtctata atgcaatcaa agccggctat 120

agactgtttg acggtgcttg tgactatgga aacgaagtag aatgcggcca aggagtagcc 180

agggcaatta aggaaggaat agtgaaaaga gaggaattgt tcattgtctc aaagctatgg 240

aatacatttc acgatgggga cagagtagag cctatcgtta ggaagcaatt agctgattgg 300

ggtttggaat actttgactt atacttaatt catttcccag tagcgttaga atacgttgac 360

ccttctgtta gatacccacc tggctggcat ttcgatggta aaagtgaaat tagaccatca 420

aaagccacaa tccaggaaac atggaccgca atggaatccc ttgttgaaaa gggactatcc 480

aaatcaatag gtgtctctaa tttccaagct caattgcttt acgatcttct aagatacgct 540

aaagtcagac cagcaacttt acagattgaa catcacccat acttggtgca acaaaaccta 600

ctgaatttgg ccaaagcgga gggtatcgct gttactgctt actcttcatt tggcccagct 660

tcctttagag agtttaacat ggaacatgca cagaagttac aaccactgct cgaagatcca 720

actataaagg caatcggtga taagtacaat aaggaccctg ctcaagtttt gttgcgttgg 780

gcaacgcaac gagggcttgc gataattcca aaatctagta gagaagctac catgaaatct 840

aatttgaact ctttagactt tgatctaagc gaggaggata tcaaaacaat cagtgggttt 900

gatagaggta ttagattcaa tcaaccaact aactattttt ctgctgaaaa tctctggatt 960

ttcggttaa 969

<210> 8
<211> 1092
<212> DNA
<213> Scheffersomyces stipitis

<220>
<221> misc_feature
<222> (1)..(1092)
<223> XYL2

<400> 8
atgactgcta acccttcctt ggtgttgaac aagatcgacg acatttcgtt cgaaacttac 60

gatgccccag aaatctctga acctaccgat gtcctcgtcc aggtcaagaa aaccggtatc 120

tgtggttccg acatccactt ctacgcccat ggtagaatcg gtaacttcgt tttgaccaag 180

ccaatggtct tgggtcacga atccgccggt actgttgtcc aggttggtaa gggtgtcacc 240

tctcttaagg ttggtgacaa cgtcgctatc gaaccaggta ttccatccag attctccgac 300

gaatacaaga gcggtcacta caacttgtgt cctcacatgg ccttcgccgc tactcctaac 360

tccaaggaag gcgaaccaaa cccaccaggt accttatgta agtacttcaa gtcgccagaa 420

gacttcttgg tcaagttgcc agaccacgtc agcttggaac tcggtgctct tgttgagcca 480

ttgtctgttg gtgtccacgc ctctaagttg ggttccgttg ctttcggcga ctacgttgcc 540

gtctttggtg ctggtcctgt tggtcttttg gctgctgctg tcgccaagac cttcggtgct 600

aagggtgtca tcgtcgttga cattttcgac aacaagttga agatggccaa ggacattggt 660

gctgctactc acaccttcaa ctccaagacc ggtggttctg aagaattgat caaggctttc 720

ggtggtaacg tgccaaacgt cgttttggaa tgtactggtg ctgaaccttg tatcaagttg 780

ggtgttgacg ccattgcccc aggtggtcgt ttcgttcaag tcggtaacgc tgctggtcca 840

gtcagcttcc caatcaccgt tttcgccatg aaggaattga ctttgttcgg ttctttcaga 900

tacggattca acgactacaa gactgctgtt ggaatctttg acactaacta ccaaaacggt 960

agagaaaatg ctccaattga ctttgaacaa ttgatcaccc acagatacaa gttcaaggac 1020

gctattgaag cctacgactt ggtcagagcc ggtaagggtg ctgtcaagtg tctcattgac 1080

ggccctgagt aa 1092

<210> 9
<211> 3024
<212> DNA
<213> Piromyces sp.

<220>
<221> misc_feature
<222> (1)..(3024)
<223> adhE

<400> 9
acgctactta tttttataac atcgttgtct aaaaaaaaat tataatttat taattttttt 60

ttattaagta aaatatattt tttgagaata tacattttat ttaataaaaa acttaataaa 120

acaaaaaagc tataatacta taatatcatt gaatattata aaattttttt atatttttaa 180

tatctatttc acccaatttt attaattttt taataaaata aaataatata atcaaaatgt 240

ccggattaca aatgttccaa aacctttctc tttacggtag tctcgccgaa atcgatacta 300

gcgaaaagct taacgaagct atggacaaat taactgctgc ccaagaacaa ttcagagaat 360

acaaccaaga acaagttgac aaaatcttca aggctgttgc tttagctgct tctcaaaacc 420

gtgttgcttt cgctaagtac gcacacgaag aaacccaaaa gggtgttttc gaagataagg 480

ttatcaagaa cgaattcgct gctgattaca tttaccacaa gtactgcaat gacaagaccg 540

ccggtatcat tgaatatgat gaagccaatg gtcttatgga aattgctgaa ccagttggtc 600

cagttgttgg tattgctcca gttactaacc caacttctac tatcatctac aagtctttaa 660

ttgccttaaa gacccgtaac tgtattatct tctcaccaca tccaggagct cacaaggcct 720

ctgttttcgt tgttaaggtc ttacaccaag ctgctgttaa ggctggtgcc ccagaaaact 780

gtattcaaat catcttccca aagatggatt taactactga attattacac caccaaaaga 840

ctcgtttcat ttgggctact ggtggtccag gtttagttca cgcctcttac acttctggta 900

agccagctct tggtggtggt ccaggtaatg ctccagctct tattgatgaa acttgtgata 960

tgaacgaagc tgttggttct atcgttgttt ctaagacttt cgattgtggt atgatctgtg 1020

ccactgaaaa cgctgttgtc gttgtcgaat ctgtctacga aaacttcgtt gctaccatga 1080

agaagcgtgg tgcctacttc atgactccag aagaaaccaa gaaggcttct aaccttcttt 1140

tcggagaagg tatgagatta aatgctaagg ctgttggtca aactgccaag actttagctg 1200

aaatggccgg tttcgaagtc ccagaaaaca ccgttgttct ctgtggtgaa gcttctgaag 1260

ttaaattcga agaaccaatg gctcacgaaa agttaactac tatcctcggt atctacaagg 1320

ctaaggactt tgacgatggt gtcagattat gtaaggaatt agttactttc ggtggtaagg 1380

gtcacactgc tgttctctac accaaccaaa acaacaagga ccgtattgaa aagtaccaaa 1440

acgaagttcc agccttccac atcttagttg acatgccatc ttccctcggt tgtattggtg 1500

atatgtacaa cttccgtctt gctccagctc ttaccattac ttgtggtact atgggtggtg 1560

gttcctcctc tgataacatt ggtccaaagc acttacttaa catcaagcgt gttggtatga 1620

gacgcgaaaa catgctttgg ttcaagattc caaagtctgt ctacttcaag cgtgctatcc 1680

tttctgaagc tttatctgac ttacgtgaca cccacaagcg tgctatcatt attaccgata 1740

gaactatgac tatgttaggt caaactgaca agatcattaa ggcttgtgaa ggtcatggta 1800

tggtctgcac tgtctacgat aaggttgtcc cagatccaac tatcaagtgt attatggaag 1860

gtgttaatga aatgaacgtc ttcaagccag atttagctat tgctcttggt ggtggttctg 1920

ctatggatgc cgctaagatg atgcgtttat tctacgaata cccagaccaa gacttacaag 1980

atattgctac tcgtttcgtc gatatccgta agcgtgttgt tggttgtcca aagcttggta 2040

gacttattaa gactcttgtc tgtatcccaa ctacctctgg tactggtgcc gaagttactc 2100

cattcgctgt cgttacctct gaagaaggtc gtaagtaccc attagtcgac tacgaactta 2160

ctccagatat ggctattgtt gatccagaat tcgctgttgg tatgccaaag cgtttaactt 2220

cttggactgg tattgatgct cttacccacg ccattgaatc ttacgtttct attatggcta 2280

ctgacttcac tagaccatac tctctccgtg ctgttggtct tatcttcgaa tccctttccc 2340

ttgcttacaa caacggtaag gatattgaag ctcgtgaaaa gatgcacaat gcttctgcta 2400

ttgctggtat ggcctttgcc aacgctttcc ttggttgttg tcactctgtt gctcaccaac 2460

ttggttccgt ctaccacatt ccacacggtc ttgccaacgc tttaatgctt tctcacatca 2520

ttaagtacaa cgctactgac tctccagtta agatgggtac cttcccacaa tacaagtacc 2580

cacaagctat gcgtcactac gctgaaattg ctgaactctt attaccacca actcaagttg 2640

ttaagatgac tgatgttgat aaggttcaat acttaattga ccgtgttgaa caattaaagg 2700

ctgacgttgg tattccaaag tctattaagg aaactggaat ggttactgaa gaagacttct 2760

tcaacaaggt tgaccaagtt gctatcatgg ccttcgatga ccaatgtact ggtgctaacc 2820

cacgttaccc attagtttct gaattaaaac aattaatgat tgatgcctgg aacggtgttg 2880

tcccaaagct ctaaattaat cgtttaaatg aaagaaacaa gaaaaattaa atcattgaat 2940

tttaaaaaag aagtgatacc cagaagcaaa agttcaaaag gttcttgcct tcctttcgtg 3000

aaggttgttt aataatgaaa aaaa 3024

<210> 10
<211> 713

<212> PRT
<213> Entamoeba histolytica

<220>
<221> misc_feature
<222> (1)..(713)
<223> Acetyl-CoA synthetase

<400> 10

```
Met Gln Phe Glu Pro Leu Phe Asn Pro Lys Ser Val Pro Val Ile Gly
1               5                   10                  15

Ala Ser Asp Arg Lys Glu Ser Val Gly Tyr Ala Val Met Asn Asn Met
            20                  25                  30

Ile Lys Gly Gly Tyr Lys Gly Asn Leu Tyr Pro Val Gly Arg Lys Pro
        35                  40                  45

Glu Leu Phe Gly Lys Lys Cys Tyr Ala Lys Ile Gly Lys Ile Glu Glu
    50                  55                  60

Lys Val Asp Leu Ala Val Ile Ala Ile Pro Ala Lys Phe Val Pro Gly
65                  70                  75                  80

Val Cys Ile Glu Cys Gly Glu Ala Gly Val Lys Gly Leu Ile Ile Ile
                85                  90                  95

Thr Ala Gly Phe Ala Glu Ala Gly Glu Glu Gly Lys Lys Met Cys Ile
            100                 105                 110

Glu Ile Gln Ala Thr Cys Gln Lys Tyr Asn Met Arg Met Ile Gly Pro
        115                 120                 125

Asn Cys Leu Gly Ile Ile Asn Pro Arg Asp Gly Val Asn Ala Ser Phe
    130                 135                 140

Ala Ser Val Met Pro Glu Ala Gly Gly Val Ala Phe Ile Ser Gln Ser
145                 150                 155                 160

Gly Ala Leu Cys Thr Ala Ile Leu Asp Trp Ala Ala Asn Gln His Val
                165                 170                 175

Gly Phe Ser Tyr Phe Val Ser Ile Gly Ser Ser Ile Asp Thr Asp Tyr
            180                 185                 190

Ala Asp Leu Phe Glu Phe Phe Ala Lys Asp Pro Lys Val Thr Ser Ile
        195                 200                 205
```

```
Leu Met Tyr Ile Glu Ser Ile Lys Asp Ala Lys Lys Phe Val Leu Arg
    210                 215                 220

Ala Arg Glu Phe Ala Ala Asp Lys Pro Ile Ile Leu Leu Lys Ala Gly
225                 230                 235                 240

Lys Ser Ser Glu Gly Ala Ala Ala Ala Met Ser His Thr Gly Ser Leu
                245                 250                 255

Ala Gly Asn Asp Ala Val Tyr Asp Ala Val Phe Asp Arg Cys Gly Cys
            260                 265                 270

Ile Arg Val Asp Ser Ile Cys Asp Leu Trp Asp Cys Ala His Val Leu
        275                 280                 285

Ala Thr Gln Asn Ile Pro Gln Asn Asn Arg Leu Cys Ile Ile Thr Asn
    290                 295                 300

Ala Gly Gly Pro Gly Val Ile Ser Thr Asp Arg Leu Val Ser Val His
305                 310                 315                 320

Gly His Leu Ala Lys Leu Ser Glu Ser Thr Met Asn Glu Leu Asn Ala
                325                 330                 335

Phe Leu Ser Pro Phe Trp Ser His Ser Asn Pro Val Asp Val Leu Gly
            340                 345                 350

Asp Ala Thr Ala Gly Val Tyr Gln Lys Thr Leu Asp Ile Val Ile Lys
        355                 360                 365

Asp Pro Gln Ile Asp Gly Val Val Val Val Leu Thr Pro Gln Ala Met
    370                 375                 380

Thr Asp Pro Val Ala Val Ala Lys Ser Leu Val Glu His Gly Pro Tyr
385                 390                 395                 400

Gln Asn Gln Ser Leu Pro His Gly Trp Val Asn Gln Lys Ser Glu Ala
                405                 410                 415

Gly Val Lys Ile Leu Glu Glu Gly Lys Ile Pro Asn Phe Glu Thr Pro
            420                 425                 430

Glu Arg Ala Val Thr Ala Phe Gly Tyr Ile Met Arg His Pro Asp Ile
        435                 440                 445

Ala Ala Lys Leu Lys Glu Ile Pro Lys Tyr Leu Asp Val Gln Val Asp
    450                 455                 460
```

```
Tyr Glu Gly Ala Lys Lys Leu Ile Ala Asp Val Val Ala Asp Gly Arg
465                 470                 475                 480

Thr Thr Phe Thr Glu Tyr Glu Gly Lys Met Met Phe Ser Lys Tyr Gly
                485                 490                 495

Ile Pro Ile Lys Gly Met Ala Lys Ala Ser Thr Glu Asp Glu Ala Val
            500                 505                 510

Ala Glu Ala Met Lys Ile Gly Thr Pro Val Val Met Lys Ile Leu Ser
        515                 520                 525

Pro Asp Ile Met His Lys Thr Asp Val Gly Gly Val Lys Val Lys Leu
    530                 535                 540

Thr Thr Glu Glu Glu Ile Arg Lys Ala Tyr Arg Asp Ile Met Thr Ser
545                 550                 555                 560

Val Lys Glu Lys Lys Pro Glu Ala Arg Ile His Gly Val Leu Leu Glu
                565                 570                 575

Lys Met Val Gly Phe Lys Tyr Glu Cys Ile Ile Gly Cys Lys Lys Asp
            580                 585                 590

Pro Leu Phe Gly Pro Val Ile Val Phe Gly Met Gly Gly Val Thr Val
        595                 600                 605

Glu Leu Tyr Lys Asp Thr Asn Ile Ala Leu Pro Pro Ile Gly Leu Gln
    610                 615                 620

Glu Ala Asp Arg Leu Ile Asp Gly Thr Lys Ile Ser Lys Leu Leu Arg
625                 630                 635                 640

Gly Tyr Arg Gly Met Pro Ala Cys Asp Val Glu Gly Leu Lys Lys Ile
                645                 650                 655

Leu Val Gln Phe Ser Lys Met Ile Met Asp Phe Pro Glu Ile Ser Glu
            660                 665                 670

Val Asp Ile Asn Pro Leu Ala Val Ser Tyr Glu Glu Phe Leu Val Leu
        675                 680                 685

Asp Ala Lys Ile Val Leu Asp Lys Asn Met Ile Gly Lys Glu Val Pro
    690                 695                 700

Lys Tyr Ser His Leu Val Ile Gln Pro
705                 710
```

<210> 11
<211> 726
<212> PRT
<213> Giardia intestinalis

<220>
<221> misc_feature
<222> (1)..(726)
<223> Acetyl-CoA synthetase

<400> 11

Met Arg Gln Asn Tyr Ser Thr Lys Tyr Lys Lys Met Gly Lys Leu Ser
1 5 10 15

Phe Leu Thr Asn Pro Ala Ser Val Ala Val Ile Gly Ala Ser Pro Asn
20 25 30

Ala Gly Lys Val Gly Asn Thr Val Val Thr Asn Ile Lys Glu Ser Gly
35 40 45

Tyr Thr Gly Lys Val Tyr Pro Ile Asn Pro Thr Ala Thr Glu Ile Leu
50 55 60

Gly Tyr Lys Thr Tyr Lys Ser Val Leu Asp Val Pro Asp Ser Ile Asp
65 70 75 80

Val Val Ile Val Val Ile Pro Ser Lys Ala Val Leu Ala Ala Ala Lys
85 90 95

Glu Cys Ala Gln Lys Lys Val Lys Ser Leu Val Val Ile Thr Ala Gly
100 105 110

Phe Lys Glu Ile Gly Gly Glu Gly Val Gln Met Glu Gln Asp Leu Thr
115 120 125

Lys Ile Cys Lys Asp Ala Gly Ile Arg Leu Val Gly Pro Asn Cys Leu
130 135 140

Gly Ile Val Thr Pro Asn Leu Asn Cys Thr Phe Ala Ser Ala Lys Pro
145 150 155 160

Ser Lys Gly Ser Ile Ala Phe Leu Ser Gln Ser Gly Ala Met Leu Thr
165 170 175

Ser Ile Leu Asp Trp Ala Leu Thr Asn Gly Ile Gly Phe Ser Asn Phe
180 185 190

```
Ile Ser Leu Gly Asn Lys Ala Asp Val Asp Glu Val Asp Leu Ile Met
        195                 200                 205

Glu Val Ala Glu Asp Pro Asn Thr Asp Ile Ile Leu Leu Tyr Leu Glu
    210                 215                 220

Ser Ile Val Asp Gly Arg Lys Phe Leu Glu Gln Ile Pro Thr Cys Val
225                 230                 235                 240

His Lys Lys Pro Val Ile Ile Leu Lys Ser Gly Thr Ser Ala Ala Gly
                245                 250                 255

Ala Ala Ala Ala Ser Ser His Thr Gly Ala Leu Ala Gly Asn Asp Ile
            260                 265                 270

Ala Phe Asp Leu Ala Phe Glu Lys Ala Gly Val Leu Arg Ala Ala Thr
        275                 280                 285

Met Ser Asp Leu Phe Asp Leu Gly Arg Leu Phe Val Ser His Arg Leu
    290                 295                 300

Pro Lys Gly Asp Asn Phe Val Ile Val Thr Asn Ala Gly Gly Pro Gly
305                 310                 315                 320

Ile Val Thr Thr Asp Ala Phe Glu Thr Tyr His Val Gly Met Ala Ala
                325                 330                 335

Leu Ser Asp Lys Thr Lys Glu Ala Leu Ala Lys Val Leu Pro Gly Glu
            340                 345                 350

Ala Ser Val Lys Asn Pro Val Asp Ile Val Gly Asp Ala Pro Pro Lys
        355                 360                 365

Arg Tyr Glu Asp Ala Leu Glu Ile Cys Phe Lys Glu Pro Pro Glu Thr
    370                 375                 380

Val Ala Gly Ala Val Ile Leu Val Thr Pro Gln Gly Gln Thr Lys Pro
385                 390                 395                 400

Cys Glu Val Ala Glu Leu Cys Thr Arg Met Tyr Ala Lys Tyr Pro Asp
                405                 410                 415

Arg Leu Val Val Ser Ala Phe Met Gly Gly Leu Thr Met Gln Glu Pro
            420                 425                 430

Ser Lys Ile Leu Asn Asn Ala Lys Met Pro Val Phe Pro Phe Pro Glu
        435                 440                 445
```

```
Pro Ala Ile His Ala Thr Gly Ala Val Leu Lys Tyr Arg Lys Ile Lys
    450                 455                 460

Asn Arg Lys Thr Leu Ala Glu Lys Lys Val Glu Val Phe Lys Val Asp
465                 470                 475                 480

Asn Glu Arg Ile Lys Lys Ile Ile Ala Gly Ala Arg Ala Asp Gly Arg
                485                 490                 495

Thr Val Leu Leu Ser His Glu Thr Ser Glu Ile Phe Thr Leu Tyr Gly
            500                 505                 510

Val Asn Ala Pro Lys Thr Lys Leu Ala Thr Asn Glu Ala Glu Ala Ala
        515                 520                 525

Thr Phe Ala Lys Glu Val Thr Phe Pro Val Val Met Lys Ile Val Ser
    530                 535                 540

Pro Gln Ile Ile His Lys Ser Asp Cys Gly Gly Val Lys Leu Asn Ile
545                 550                 555                 560

Lys Thr Glu Ala Glu Ala Thr Ala Ala Phe Lys Glu Ile Met Ala Asn
                565                 570                 575

Ala Ala Lys Asn Gly Pro Lys Gly Ala Val Leu Lys Gly Val Glu Ile
            580                 585                 590

Gln Gln Met Val Asp Phe Ser Lys Tyr Gln Lys Thr Thr Glu Met Ile
        595                 600                 605

Val Gly Val Asn Arg Asp Pro Thr Trp Gly Pro Met Ile Met Val Gly
    610                 615                 620

Gln Gly Gly Ile Tyr Ala Asn Tyr Ile Lys Asp Val Ala Phe Asp Leu
625                 630                 635                 640

Ala Tyr Lys Tyr Asp Arg Glu Asp Ala Glu Ala Gln Leu Lys Lys Thr
                645                 650                 655

Lys Ile Tyr Glu Ile Leu Asn Gly Val Arg Gly Gln Pro Arg Ser Asp
            660                 665                 670

Ile Lys Gly Leu Leu Asp Thr Met Val Lys Leu Ala Gln Leu Val Asn
        675                 680                 685

Asp Phe Ser Glu Ile Thr Glu Leu Asp Met Asn Pro Leu Leu Val Phe
    690                 695                 700
```

```
Glu Glu Gln Lys Glu Gly Lys Asn Pro Gly Ile Ala Ala Val Asp Val
705                 710                 715                 720

Lys Ile Thr Leu Ser His
                725

<210>  12
<211>  462
<212>  PRT
<213>  Pyrococcus furiosus

<220>
<221>  misc_feature
<222>  (1)..(462)
<223>  Acetyl-CoA synthetase

<400>  12

Met Ser Leu Glu Ala Leu Phe Asn Pro Lys Ser Val Ala Val Ile Gly
1               5                   10                  15

Ala Ser Ala Lys Pro Gly Lys Ile Gly Tyr Ala Ile Met Lys Asn Leu
            20                  25                  30

Ile Glu Tyr Gly Tyr Glu Gly Lys Ile Tyr Pro Val Asn Ile Lys Gly
        35                  40                  45

Gly Glu Ile Glu Ile Asn Gly Arg Lys Phe Lys Val Tyr Lys Ser Val
    50                  55                  60

Leu Glu Ile Pro Asp Glu Val Asp Met Ala Val Ile Val Val Pro Ala
65                  70                  75                  80

Lys Phe Val Pro Gln Val Leu Glu Glu Cys Gly Gln Lys Gly Val Lys
                85                  90                  95

Val Val Pro Ile Ile Ser Ser Gly Phe Gly Glu Leu Gly Glu Glu Gly
            100                 105                 110

Lys Lys Val Glu Gln Gln Leu Val Glu Thr Ala Arg Lys Tyr Gly Met
        115                 120                 125

Arg Ile Leu Gly Pro Asn Ile Phe Gly Val Val Tyr Thr Pro Ala Lys
    130                 135                 140

Leu Asn Ala Thr Phe Gly Pro Thr Asp Val Leu Pro Gly Pro Leu Ala
145                 150                 155                 160
```

```
Leu Ile Ser Gln Ser Gly Ala Leu Gly Ile Ala Leu Met Gly Trp Thr
                165                 170                 175

Ile Leu Glu Lys Ile Gly Leu Ser Ala Val Val Ser Val Gly Asn Lys
            180                 185                 190

Ala Asp Ile Asp Asp Ala Asp Leu Leu Glu Phe Phe Lys Asp Asp Glu
        195                 200                 205

Asn Thr Arg Ala Ile Leu Ile Tyr Met Glu Gly Val Lys Asp Gly Arg
    210                 215                 220

Arg Phe Met Glu Val Ala Lys Glu Val Ser Lys Lys Lys Pro Ile Ile
225                 230                 235                 240

Val Ile Lys Ala Gly Arg Ser Glu Arg Gly Ala Lys Ala Ala Ala Ser
                245                 250                 255

His Thr Gly Ser Leu Ala Gly Ser Asp Lys Val Tyr Ser Ala Ala Phe
            260                 265                 270

Lys Gln Ser Gly Val Leu Arg Ala Tyr Thr Ile Gly Glu Ala Phe Asp
        275                 280                 285

Trp Ala Arg Ala Leu Ser Asn Leu Pro Glu Pro Gln Gly Asp Asn Val
    290                 295                 300

Val Ile Ile Thr Asn Gly Gly Gly Ile Gly Val Met Ala Thr Asp Ala
305                 310                 315                 320

Ala Glu Glu Glu Gly Leu His Leu Tyr Asp Asn Leu Glu Glu Leu Lys
                325                 330                 335

Ile Phe Ala Asn His Met Pro Pro Phe Gly Ser Tyr Lys Asn Pro Val
            340                 345                 350

Asp Leu Thr Gly Met Ala Asp Gly Lys Ser Tyr Glu Gly Ala Ile Arg
        355                 360                 365

Asp Ala Leu Ala His Pro Glu Met His Ser Ile Ala Val Leu Tyr Cys
    370                 375                 380

Gln Thr Ala Val Leu Asp Pro Arg Glu Leu Ala Glu Ile Val Ile Arg
385                 390                 395                 400

Glu Tyr Asn Glu Ser Gly Arg Lys Lys Pro Leu Val Val Ala Ile Val
                405                 410                 415
```

Gly Gly Ile Glu Ala Lys Glu Ala Ile Asp Met Leu Asn Glu Asn Gly
420 425 430

Ile Pro Ala Tyr Pro Glu Pro Glu Arg Ala Ile Lys Ala Leu Ser Ala
435 440 445

Leu Tyr Lys Trp Ser Lys Trp Lys Ala Lys His Lys Glu Lys
450 455 460

<210> 13
<211> 232
<212> PRT
<213> Pyrococcus furiosus

<220>
<221> misc_feature
<222> (1)..(232)
<223> Acetyl-CoA synthetase

<400> 13

Met Asp Arg Val Ala Lys Ala Arg Glu Ile Ile Glu Lys Ala Lys Ala
1 5 10 15

Glu Asn Arg Pro Leu Val Glu Pro Glu Ala Lys Glu Ile Leu Lys Leu
20 25 30

Tyr Gly Ile Pro Val Pro Glu Phe Lys Val Ala Arg Asn Glu Glu Glu
35 40 45

Ala Val Lys Phe Ser Gly Glu Ile Gly Tyr Pro Val Val Met Lys Ile
50 55 60

Val Ser Pro Gln Ile Ile His Lys Ser Asp Ala Gly Gly Val Lys Ile
65 70 75 80

Asn Ile Lys Asn Asp Glu Glu Ala Arg Glu Ala Phe Arg Thr Ile Met
85 90 95

Gln Asn Ala Arg Asn Tyr Lys Pro Asp Ala Asp Leu Trp Gly Val Ile
100 105 110

Ile Tyr Arg Met Leu Pro Leu Gly Arg Glu Val Ile Val Gly Met Ile
115 120 125

Arg Asp Pro Gln Phe Gly Pro Ala Val Met Phe Gly Leu Gly Gly Ile
130 135 140

Phe Val Glu Ile Leu Lys Asp Val Ser Phe Arg Val Ala Pro Ile Thr

145 150 155 160

Lys Glu Asp Ala Leu Glu Met Ile Arg Glu Ile Lys Ala Tyr Pro Ile
165 170 175

Leu Ala Gly Ala Arg Gly Glu Lys Pro Val Asn Ile Glu Ala Leu Ala
180 185 190

Asp Ile Ile Val Lys Val Gly Glu Leu Ala Leu Glu Leu Pro Glu Ile
195 200 205

Lys Glu Ile Asp Ile Asn Pro Ile Phe Ala Tyr Glu Asp Ser Ala Ile
210 215 220

Ala Val Asp Ala Arg Met Ile Leu
225 230

<210> 14
<211> 462
<212> PRT
<213> Pyrococcus furiosus

<220>
<221> misc_feature
<222> (1)..(462)
<223> Acetyl-CoA synthetase

<400> 14

Met Ser Leu Glu Ala Leu Phe Asn Pro Lys Ser Val Ala Val Ile Gly
1 5 10 15

Ala Ser Ala Lys Pro Gly Lys Ile Gly Tyr Ala Ile Met Lys Asn Leu
20 25 30

Ile Glu Tyr Gly Tyr Glu Gly Lys Ile Tyr Pro Val Asn Ile Lys Gly
35 40 45

Gly Glu Ile Glu Ile Asn Gly Arg Lys Phe Lys Val Tyr Lys Ser Val
50 55 60

Leu Glu Ile Pro Asp Glu Val Asp Met Ala Val Ile Val Val Pro Ala
65 70 75 80

Lys Phe Val Pro Gln Val Leu Glu Glu Cys Gly Gln Lys Gly Val Lys
85 90 95

Val Val Pro Ile Ile Ser Ser Gly Phe Gly Glu Leu Gly Glu Glu Gly
100 105 110

Lys Lys Val Glu Gln Gln Leu Val Glu Thr Ala Arg Lys Tyr Gly Met
115 120 125

Arg Ile Leu Gly Pro Asn Ile Phe Gly Val Val Tyr Thr Pro Ala Lys
130 135 140

Leu Asn Ala Thr Phe Gly Pro Thr Asp Val Leu Pro Gly Pro Leu Ala
145 150 155 160

Leu Ile Ser Gln Ser Gly Ala Leu Gly Ile Ala Leu Met Gly Trp Thr
165 170 175

Ile Leu Glu Lys Ile Gly Leu Ser Ala Val Val Ser Val Gly Asn Lys
180 185 190

Ala Asp Ile Asp Asp Ala Asp Leu Leu Glu Phe Phe Lys Asp Asp Glu
195 200 205

Asn Thr Arg Ala Ile Leu Ile Tyr Met Glu Gly Val Lys Asp Gly Arg
210 215 220

Arg Phe Met Glu Val Ala Lys Glu Val Ser Lys Lys Lys Pro Ile Ile
225 230 235 240

Val Ile Lys Ala Gly Arg Ser Glu Arg Gly Ala Lys Ala Ala Ala Ser
245 250 255

His Thr Gly Ser Leu Ala Gly Ser Asp Lys Val Tyr Ser Ala Ala Phe
260 265 270

Lys Gln Ser Gly Val Leu Arg Ala Tyr Thr Ile Gly Glu Ala Phe Asp
275 280 285

Trp Ala Arg Ala Leu Ser Asn Leu Pro Glu Pro Gln Gly Asp Asn Val
290 295 300

Val Ile Ile Thr Asn Gly Gly Gly Ile Gly Val Met Ala Thr Asp Ala
305 310 315 320

Ala Glu Glu Glu Gly Leu His Leu Tyr Asp Asn Leu Glu Glu Leu Lys
325 330 335

Ile Phe Ala Asn His Met Pro Pro Phe Gly Ser Tyr Lys Asn Pro Val
340 345 350

Asp Leu Thr Gly Met Ala Asp Gly Lys Ser Tyr Glu Gly Ala Ile Arg
355 360 365

Asp Ala Leu Ala His Pro Glu Met His Ser Ile Ala Val Leu Tyr Cys
370 375 380

Gln Thr Ala Val Leu Asp Pro Arg Glu Leu Ala Glu Ile Val Ile Arg
385 390 395 400

Glu Tyr Asn Glu Ser Gly Arg Lys Lys Pro Leu Val Val Ala Ile Val
405 410 415

Gly Gly Ile Glu Ala Lys Glu Ala Ile Asp Met Leu Asn Glu Asn Gly
420 425 430

Ile Pro Ala Tyr Pro Glu Pro Glu Arg Ala Ile Lys Ala Leu Ser Ala
435 440 445

Leu Tyr Lys Trp Ser Lys Trp Lys Ala Lys His Lys Glu Lys
450 455 460

<210> 15
<211> 232
<212> PRT
<213> Pyrococcus furiosus

<220>
<221> misc_feature
<222> (1)..(232)
<223> Acetyl-CoA synthetase

<400> 15

Met Asp Arg Val Ala Lys Ala Arg Glu Ile Ile Glu Lys Ala Lys Ala
1 5 10 15

Glu Asn Arg Pro Leu Val Glu Pro Glu Ala Lys Glu Ile Leu Lys Leu
20 25 30

Tyr Gly Ile Pro Val Pro Glu Phe Lys Val Ala Arg Asn Glu Glu Glu
35 40 45

Ala Val Lys Phe Ser Gly Glu Ile Gly Tyr Pro Val Val Met Lys Ile
50 55 60

Val Ser Pro Gln Ile Ile His Lys Ser Asp Ala Gly Gly Val Lys Ile
65 70 75 80

Asn Ile Lys Asn Asp Glu Glu Ala Arg Glu Ala Phe Arg Thr Ile Met
85 90 95

Gln Asn Ala Arg Asn Tyr Lys Pro Asp Ala Asp Leu Trp Gly Val Ile
100 105 110

Ile Tyr Arg Met Leu Pro Leu Gly Arg Glu Val Ile Val Gly Met Ile
115 120 125

Arg Asp Pro Gln Phe Gly Pro Ala Val Met Phe Gly Leu Gly Gly Ile
130 135 140

Phe Val Glu Ile Leu Lys Asp Val Ser Phe Arg Val Ala Pro Ile Thr
145 150 155 160

Lys Glu Asp Ala Leu Glu Met Ile Arg Glu Ile Lys Ala Tyr Pro Ile
165 170 175

Leu Ala Gly Ala Arg Gly Glu Lys Pro Val Asn Ile Glu Ala Leu Ala
180 185 190

Asp Ile Ile Val Lys Val Gly Glu Leu Ala Leu Glu Leu Pro Glu Ile
195 200 205

Lys Glu Ile Asp Ile Asn Pro Ile Phe Ala Tyr Glu Asp Ser Ala Ile
210 215 220

Ala Val Asp Ala Arg Met Ile Leu
225 230

<210> 16
<211> 335
<212> PRT
<213> Saccharomyces cerevisiae

<220>
<221> misc_feature
<222> (1)..(335)
<223> TAL1

<400> 16

Met Ser Glu Pro Ala Gln Lys Lys Gln Lys Val Ala Asn Asn Ser Leu
1 5 10 15

Glu Gln Leu Lys Ala Ser Gly Thr Val Val Val Ala Asp Thr Gly Asp
20 25 30

Phe Gly Ser Ile Ala Lys Phe Gln Pro Gln Asp Ser Thr Thr Asn Pro
35 40 45

Ser Leu Ile Leu Ala Ala Ala Lys Gln Pro Thr Tyr Ala Lys Leu Ile
50 55 60

Asp Val Ala Val Glu Tyr Gly Lys Lys His Gly Lys Thr Thr Glu Glu
65 70 75 80

Gln Val Glu Asn Ala Val Asp Arg Leu Leu Val Glu Phe Gly Lys Glu
85 90 95

Ile Leu Lys Ile Val Pro Gly Arg Val Ser Thr Glu Val Asp Ala Arg
100 105 110

Leu Ser Phe Asp Thr Gln Ala Thr Ile Glu Lys Ala Arg His Ile Ile
115 120 125

Lys Leu Phe Glu Gln Glu Gly Val Ser Lys Glu Arg Val Leu Ile Lys
130 135 140

Ile Ala Ser Thr Trp Glu Gly Ile Gln Ala Ala Lys Glu Leu Glu Glu
145 150 155 160

Lys Asp Gly Ile His Cys Asn Leu Thr Leu Leu Phe Ser Phe Val Gln
165 170 175

Ala Val Ala Cys Ala Glu Ala Gln Val Thr Leu Ile Ser Pro Phe Val
180 185 190

Gly Arg Ile Leu Asp Trp Tyr Lys Ser Ser Thr Gly Lys Asp Tyr Lys
195 200 205

Gly Glu Ala Asp Pro Gly Val Ile Ser Val Lys Lys Ile Tyr Asn Tyr
210 215 220

Tyr Lys Lys Tyr Gly Tyr Lys Thr Ile Val Met Gly Ala Ser Phe Arg
225 230 235 240

Ser Thr Asp Glu Ile Lys Asn Leu Ala Gly Val Asp Tyr Leu Thr Ile
245 250 255

Ser Pro Ala Leu Leu Asp Lys Leu Met Asn Ser Thr Glu Pro Phe Pro
260 265 270

Arg Val Leu Asp Pro Val Ser Ala Lys Lys Glu Ala Gly Asp Lys Ile
275 280 285

Ser Tyr Ile Ser Asp Glu Ser Lys Phe Arg Phe Asp Leu Asn Glu Asp
290 295 300

Ala Met Ala Thr Glu Lys Leu Ser Glu Gly Ile Arg Lys Phe Ser Ala

305 310 315 320

Asp Ile Val Thr Leu Phe Asp Leu Ile Glu Lys Lys Val Thr Ala
325 330 335

<210> 17
<211> 600
<212> PRT
<213> Saccharomyces cerevisiae

<220>
<221> misc_feature
<222> (1)..(600)
<223> XKS1

<400> 17

Met Leu Cys Ser Val Ile Gln Arg Gln Thr Arg Glu Val Ser Asn Thr
1 5 10 15

Met Ser Leu Asp Ser Tyr Tyr Leu Gly Phe Asp Leu Ser Thr Gln Gln
20 25 30

Leu Lys Cys Leu Ala Ile Asn Gln Asp Leu Lys Ile Val His Ser Glu
35 40 45

Thr Val Glu Phe Glu Lys Asp Leu Pro His Tyr His Thr Lys Lys Gly
50 55 60

Val Tyr Ile His Gly Asp Thr Ile Glu Cys Pro Val Ala Met Trp Leu
65 70 75 80

Glu Ala Leu Asp Leu Val Leu Ser Lys Tyr Arg Glu Ala Lys Phe Pro
85 90 95

Leu Asn Lys Val Met Ala Val Ser Gly Ser Cys Gln Gln His Gly Ser
100 105 110

Val Tyr Trp Ser Ser Gln Ala Glu Ser Leu Leu Glu Gln Leu Asn Lys
115 120 125

Lys Pro Glu Lys Asp Leu Leu His Tyr Val Ser Ser Val Ala Phe Ala
130 135 140

Arg Gln Thr Ala Pro Asn Trp Gln Asp His Ser Thr Ala Lys Gln Cys
145 150 155 160

Gln Glu Phe Glu Glu Cys Ile Gly Gly Pro Glu Lys Met Ala Gln Leu
165 170 175

Thr Gly Ser Arg Ala His Phe Arg Phe Thr Gly Pro Gln Ile Leu Lys
180 185 190

Ile Ala Gln Leu Glu Pro Glu Ala Tyr Glu Lys Thr Lys Thr Ile Ser
195 200 205

Leu Val Ser Asn Phe Leu Thr Ser Ile Leu Val Gly His Leu Val Glu
210 215 220

Leu Glu Glu Ala Asp Ala Cys Gly Met Asn Leu Tyr Asp Ile Arg Glu
225 230 235 240

Arg Lys Phe Ser Asp Glu Leu Leu His Leu Ile Asp Ser Ser Ser Lys
245 250 255

Asp Lys Thr Ile Arg Gln Lys Leu Met Arg Ala Pro Met Lys Asn Leu
260 265 270

Ile Ala Gly Thr Ile Cys Lys Tyr Phe Ile Glu Lys Tyr Gly Phe Asn
275 280 285

Thr Asn Cys Lys Val Ser Pro Met Thr Gly Asp Asn Leu Ala Thr Ile
290 295 300

Cys Ser Leu Pro Leu Arg Lys Asn Asp Val Leu Val Ser Leu Gly Thr
305 310 315 320

Ser Thr Thr Val Leu Leu Val Thr Asp Lys Tyr His Pro Ser Pro Asn
325 330 335

Tyr His Leu Phe Ile His Pro Thr Leu Pro Asn His Tyr Met Gly Met
340 345 350

Ile Cys Tyr Cys Asn Gly Ser Leu Ala Arg Glu Arg Ile Arg Asp Glu
355 360 365

Leu Asn Lys Glu Arg Glu Asn Asn Tyr Glu Lys Thr Asn Asp Trp Thr
370 375 380

Leu Phe Asn Gln Ala Val Leu Asp Asp Ser Glu Ser Ser Glu Asn Glu
385 390 395 400

Leu Gly Val Tyr Phe Pro Leu Gly Glu Ile Val Pro Ser Val Lys Ala
405 410 415

Ile Asn Lys Arg Val Ile Phe Asn Pro Lys Thr Gly Met Ile Glu Arg
420 425 430

Glu Val Ala Lys Phe Lys Asp Lys Arg His Asp Ala Lys Asn Ile Val
435 440 445

Glu Ser Gln Ala Leu Ser Cys Arg Val Arg Ile Ser Pro Leu Leu Ser
450 455 460

Asp Ser Asn Ala Ser Ser Gln Gln Arg Leu Asn Glu Asp Thr Ile Val
465 470 475 480

Lys Phe Asp Tyr Asp Glu Ser Pro Leu Arg Asp Tyr Leu Asn Lys Arg
485 490 495

Pro Glu Arg Thr Phe Phe Val Gly Gly Ala Ser Lys Asn Asp Ala Ile
500 505 510

Val Lys Lys Phe Ala Gln Val Ile Gly Ala Thr Lys Gly Asn Phe Arg
515 520 525

Leu Glu Thr Pro Asn Ser Cys Ala Leu Gly Gly Cys Tyr Lys Ala Met
530 535 540

Trp Ser Leu Leu Tyr Asp Ser Asn Lys Ile Ala Val Pro Phe Asp Lys
545 550 555 560

Phe Leu Asn Asp Asn Phe Pro Trp His Val Met Glu Ser Ile Ser Asp
565 570 575

Val Asp Asn Glu Asn Trp Asp Arg Tyr Asn Ser Lys Ile Val Pro Leu
580 585 590

Ser Glu Leu Glu Lys Thr Leu Ile
595 600

<210> 18
<211> 680
<212> PRT
<213> Saccharomyces cerevisiae

<220>
<221> misc_feature
<222> (1)..(680)
<223> TKL1

<400> 18

Met Thr Gln Phe Thr Asp Ile Asp Lys Leu Ala Val Ser Thr Ile Arg
1 5 10 15

```
Ile Leu Ala Val Asp Thr Val Ser Lys Ala Asn Ser Gly His Pro Gly
            20                  25                  30

Ala Pro Leu Gly Met Ala Pro Ala Ala His Val Leu Trp Ser Gln Met
        35                  40                  45

Arg Met Asn Pro Thr Asn Pro Asp Trp Ile Asn Arg Asp Arg Phe Val
    50                  55                  60

Leu Ser Asn Gly His Ala Val Ala Leu Leu Tyr Ser Met Leu His Leu
65                  70                  75                  80

Thr Gly Tyr Asp Leu Ser Ile Glu Asp Leu Lys Gln Phe Arg Gln Leu
                85                  90                  95

Gly Ser Arg Thr Pro Gly His Pro Glu Phe Glu Leu Pro Gly Val Glu
            100                 105                 110

Val Thr Thr Gly Pro Leu Gly Gln Gly Ile Ser Asn Ala Val Gly Met
        115                 120                 125

Ala Met Ala Gln Ala Asn Leu Ala Ala Thr Tyr Asn Lys Pro Gly Phe
    130                 135                 140

Thr Leu Ser Asp Asn Tyr Thr Tyr Val Phe Leu Gly Asp Gly Cys Leu
145                 150                 155                 160

Gln Glu Gly Ile Ser Ser Glu Ala Ser Ser Leu Ala Gly His Leu Lys
                165                 170                 175

Leu Gly Asn Leu Ile Ala Ile Tyr Asp Asp Asn Lys Ile Thr Ile Asp
            180                 185                 190

Gly Ala Thr Ser Ile Ser Phe Asp Glu Asp Val Ala Lys Arg Tyr Glu
        195                 200                 205

Ala Tyr Gly Trp Glu Val Leu Tyr Val Glu Asn Gly Asn Glu Asp Leu
    210                 215                 220

Ala Gly Ile Ala Lys Ala Ile Ala Gln Ala Lys Leu Ser Lys Asp Lys
225                 230                 235                 240

Pro Thr Leu Ile Lys Met Thr Thr Thr Ile Gly Tyr Gly Ser Leu His
                245                 250                 255

Ala Gly Ser His Ser Val His Gly Ala Pro Leu Lys Ala Asp Asp Val
            260                 265                 270
```

```
Lys Gln Leu Lys Ser Lys Phe Gly Phe Asn Pro Asp Lys Ser Phe Val
        275                 280                 285

Val Pro Gln Glu Val Tyr Asp His Tyr Gln Lys Thr Ile Leu Lys Pro
    290                 295                 300

Gly Val Glu Ala Asn Asn Lys Trp Asn Lys Leu Phe Ser Glu Tyr Gln
305                 310                 315                 320

Lys Lys Phe Pro Glu Leu Gly Ala Glu Leu Ala Arg Arg Leu Ser Gly
                325                 330                 335

Gln Leu Pro Ala Asn Trp Glu Ser Lys Leu Pro Thr Tyr Thr Ala Lys
            340                 345                 350

Asp Ser Ala Val Ala Thr Arg Lys Leu Ser Glu Thr Val Leu Glu Asp
        355                 360                 365

Val Tyr Asn Gln Leu Pro Glu Leu Ile Gly Gly Ser Ala Asp Leu Thr
    370                 375                 380

Pro Ser Asn Leu Thr Arg Trp Lys Glu Ala Leu Asp Phe Gln Pro Pro
385                 390                 395                 400

Ser Ser Gly Ser Gly Asn Tyr Ser Gly Arg Tyr Ile Arg Tyr Gly Ile
                405                 410                 415

Arg Glu His Ala Met Gly Ala Ile Met Asn Gly Ile Ser Ala Phe Gly
            420                 425                 430

Ala Asn Tyr Lys Pro Tyr Gly Gly Thr Phe Leu Asn Phe Val Ser Tyr
        435                 440                 445

Ala Ala Gly Ala Val Arg Leu Ser Ala Leu Ser Gly His Pro Val Ile
    450                 455                 460

Trp Val Ala Thr His Asp Ser Ile Gly Val Gly Glu Asp Gly Pro Thr
465                 470                 475                 480

His Gln Pro Ile Glu Thr Leu Ala His Phe Arg Ser Leu Pro Asn Ile
                485                 490                 495

Gln Val Trp Arg Pro Ala Asp Gly Asn Glu Val Ser Ala Ala Tyr Lys
            500                 505                 510

Asn Ser Leu Glu Ser Lys His Thr Pro Ser Ile Ile Ala Leu Ser Arg
        515                 520                 525
```

Gln Asn Leu Pro Gln Leu Glu Gly Ser Ser Ile Glu Ser Ala Ser Lys
    530                 535                 540

Gly Gly Tyr Val Leu Gln Asp Val Ala Asn Pro Asp Ile Ile Leu Val
545                 550                 555                 560

Ala Thr Gly Ser Glu Val Ser Leu Ser Val Glu Ala Ala Lys Thr Leu
                565                 570                 575

Ala Ala Lys Asn Ile Lys Ala Arg Val Val Ser Leu Pro Asp Phe Phe
            580                 585                 590

Thr Phe Asp Lys Gln Pro Leu Glu Tyr Arg Leu Ser Val Leu Pro Asp
        595                 600                 605

Asn Val Pro Ile Met Ser Val Glu Val Leu Ala Thr Thr Cys Trp Gly
    610                 615                 620

Lys Tyr Ala His Gln Ser Phe Gly Ile Asp Arg Phe Gly Ala Ser Gly
625                 630                 635                 640

Lys Ala Pro Glu Val Phe Lys Phe Phe Gly Phe Thr Pro Glu Gly Val
                645                 650                 655

Ala Glu Arg Ala Gln Lys Thr Ile Ala Phe Tyr Lys Gly Asp Lys Leu
            660                 665                 670

Ile Ser Pro Leu Lys Lys Ala Phe
        675                 680

<210>  19
<211>  238
<212>  PRT
<213>  Saccharomyces cerevisiae

<220>
<221>  misc_feature
<222>  (1)..(238)
<223>  RPE1

<400>  19

Met Val Lys Pro Ile Ile Ala Pro Ser Ile Leu Ala Ser Asp Phe Ala
1               5                   10                  15

Asn Leu Gly Cys Glu Cys His Lys Val Ile Asn Ala Gly Ala Asp Trp
            20                  25                  30

Leu His Ile Asp Val Met Asp Gly His Phe Val Pro Asn Ile Thr Leu
35 40 45

Gly Gln Pro Ile Val Thr Ser Leu Arg Arg Ser Val Pro Arg Pro Gly
50 55 60

Asp Ala Ser Asn Thr Glu Lys Lys Pro Thr Ala Phe Phe Asp Cys His
65 70 75 80

Met Met Val Glu Asn Pro Glu Lys Trp Val Asp Asp Phe Ala Lys Cys
85 90 95

Gly Ala Asp Gln Phe Thr Phe His Tyr Glu Ala Thr Gln Asp Pro Leu
100 105 110

His Leu Val Lys Leu Ile Lys Ser Lys Gly Ile Lys Ala Ala Cys Ala
115 120 125

Ile Lys Pro Gly Thr Ser Val Asp Val Leu Phe Glu Leu Ala Pro His
130 135 140

Leu Asp Met Ala Leu Val Met Thr Val Glu Pro Gly Phe Gly Gly Gln
145 150 155 160

Lys Phe Met Glu Asp Met Met Pro Lys Val Glu Thr Leu Arg Ala Lys
165 170 175

Phe Pro His Leu Asn Ile Gln Val Asp Gly Gly Leu Gly Lys Glu Thr
180 185 190

Ile Pro Lys Ala Ala Lys Ala Gly Ala Asn Val Ile Val Ala Gly Thr
195 200 205

Ser Val Phe Thr Ala Ala Asp Pro His Asp Val Ile Ser Phe Met Lys
210 215 220

Glu Glu Val Ser Lys Glu Leu Arg Ser Arg Asp Leu Leu Asp
225 230 235

<210> 20
<211> 258
<212> PRT
<213> Saccharomyces cerevisiae

<220>
<221> misc_feature
<222> (1)..(258)
<223> RKI1

<400> 20

```
Met Ala Ala Gly Val Pro Lys Ile Asp Ala Leu Glu Ser Leu Gly Asn
1               5                   10                  15

Pro Leu Glu Asp Ala Lys Arg Ala Ala Ala Tyr Arg Ala Val Asp Glu
            20                  25                  30

Asn Leu Lys Phe Asp Asp His Lys Ile Ile Gly Ile Gly Ser Gly Ser
        35                  40                  45

Thr Val Val Tyr Val Ala Glu Arg Ile Gly Gln Tyr Leu His Asp Pro
    50                  55                  60

Lys Phe Tyr Glu Val Ala Ser Lys Phe Ile Cys Ile Pro Thr Gly Phe
65                  70                  75                  80

Gln Ser Arg Asn Leu Ile Leu Asp Asn Lys Leu Gln Leu Gly Ser Ile
                85                  90                  95

Glu Gln Tyr Pro Arg Ile Asp Ile Ala Phe Asp Gly Ala Asp Glu Val
            100                 105                 110

Asp Glu Asn Leu Gln Leu Ile Lys Gly Gly Gly Ala Cys Leu Phe Gln
        115                 120                 125

Glu Lys Leu Val Ser Thr Ser Ala Lys Thr Phe Ile Val Val Ala Asp
    130                 135                 140

Ser Arg Lys Lys Ser Pro Lys His Leu Gly Lys Asn Trp Arg Gln Gly
145                 150                 155                 160

Val Pro Ile Glu Ile Val Pro Ser Ser Tyr Val Arg Val Lys Asn Asp
                165                 170                 175

Leu Leu Glu Gln Leu His Ala Glu Lys Val Asp Ile Arg Gln Gly Gly
            180                 185                 190

Ser Ala Lys Ala Gly Pro Val Val Thr Asp Asn Asn Asn Phe Ile Ile
        195                 200                 205

Asp Ala Asp Phe Gly Glu Ile Ser Asp Pro Arg Lys Leu His Arg Glu
    210                 215                 220

Ile Lys Leu Leu Val Gly Val Val Glu Thr Gly Leu Phe Ile Asp Asn
225                 230                 235                 240

Ala Ser Lys Ala Tyr Phe Gly Asn Ser Asp Gly Ser Val Glu Val Thr
```

245 250 255

Glu Lys

<210> 21
<211> 2122
<212> DNA
<213> Saccharomyces cerevisiae

<220>
<221> misc_feature
<222> (1)..(2122)
<223> GRE3

<400> 21
acagacttcg taaagagcaa tcggagatga atttaagaaa tacaatcaaa agaaaagaga 60

aattttatga tagtcaagaa caaattcttg agttacaaga gggagacgtt gatgattcgt 120

tgatttggaa cgttcctatg gcatcattat ctactaattc atttctagcg tctgctaagc 180

ccgatgatat gaataacttg gctggcaaga atgacttatc agaatatacc ggaggtttgg 240

taaatgataa ctctgaaatt tcttatacaa aacaaaatca taggtactcg aacatctctt 300

ttgcgagtac aacatcaaac gcctcattat tggactttaa tgagatgcct acgtctccga 360

ttccaggttt gaacaaagta actgattttc agttcattca agacacaacc aagagtctag 420

cctctgttta tttgcattct tccaataggc tttcaagatc taagctgtcc gaaagaacaa 480

agtcttccga tttcttgcca attgaactaa aagaagctca aaatcaaggc atggaagatt 540

tgatacttgt ctcggagaac aaactagatg tggtcagcca ttcaagaccg agttggttac 600

cacccaagga tcgccaggaa aaaaagcttc atgaaaggca aattaacaaa agcatgagtg 660

ttgcttccct tgaccaacta ggaaaaaata aagacagaga agaaaagttg attagagatg 720

aaacaaatag gcaaaaatat gtgttattat tggacagaga tataactaga aactcctcct 780

tacaaagcct aagtaaaatg gtttgggaca ctccatttag tgacgaaact aggtcaacaa 840

tttacagcga aattttacag agcaagacta ggtttattac caaaaactat attcaaccat 900

ttcatgagct acaggagctt ttaacaaaaa tgggagactt tcctaaaaac aaggaaattg 960

aaatatcgca gctaatcgaa acaagtttga ggcgaaaagt gagcggttta catgatatat 1020

gtcctgattt gatgctttta ttgaagataa aatctatctc atcacagggt atagtcaccg 1080

gtgatgaact ccttttccat catttcttgg tgagtgaatc atttcagaac ctggggctaa 1140

acgagatttg gaatattgtt aatttagtac aaatgacgtg ttttaatgat ctttgtaaag 1200

aaaagttcga tgcaaaggtt ttagaacgta agggtgtcgt agccggttat ttatcgcaaa 1260

acgaggagtt caaggatgaa tttaatacgg agtgtataaa ctctaccacc tggtggaaca 1320

tcctagaacg tattgatcat aagcttttta tgtggatcat ggatattata gtagtcaaca 1380

attcccagag ctacaaaaat agcccaatca acgaagatga gtttgttaac aaggattggg 1440

aatattaccg ctcgaagaaa gtggtaataa actacaagat cttgatttca tttgcattaa 1500

atgtattgtt aaattaccac tttggattca ctgatttaag aagtctttgt aacgtgaatg 1560

accagagatt ttgcattcca gtattcatca atgatgaatt cgtagacgca gatactgtaa 1620

atgccgtgtt catcaagaaa tgggcgcatt actacaagaa gttttgatat tttttgtaac 1680

tgtaatttca ctcatgcaca agaaaaaaaa aactggatta aaagggagcc caaggaaaac 1740

tcctcagcat atatttagaa gtctcctcag catatagttg tttgttttct ttacacattc 1800

actgtttaat aaaactttta taatatttca ttatcggaac tctagattct atacttgttt 1860

cccaattgtt gctggtagta aacgtatacg tcataaaagg gaaaagccac atgcggaaga 1920

attttatgga aaaaaaaaaa acctcgaagt tactacttct aggggcccta tcaagtaaat 1980

tactcctggt acactgaagt atataaggga tatagaagca aatagttgtc agtgcaatcc 2040

ttcaagacga ttgggaaaat actgtaatat aaatcgtaaa ggaaaattgg aaattttttta 2100

aagatgtctt cactggttac tc 2122

<210> 22
<211> 2160
<212> DNA
<213> Saccharomyces cerevisiae

<220>
<221> misc_feature
<222> (1)..(2160)
<223> downstream sequence to delete GRE3

<400> 22
gcctgatcca gccagtaaaa tccatactca acgacgatat gaacaaattt ccctcattcc 60

gatgctgtat atgtgtataa atttttacat gctcttctgt ttagacacag aacagcttta 120

aataaaatgt tggatatact ttttctgcct gtggtgtcat ccacgctttt aattcatctc 180

ttgtatggtt gacaatttgg ctatttttta acagaaccca acggtaattg aaattaaaag 240

ggaaacgagt gggggcgatg agtgagtgat actaaaatag acaccaagag agcaaagcgg 300

tcccaaaatc atttgagtaa ccggatatct atcgggatat taatagcagc ttccatttca 360

actaaaacaa cagcaagata tgagcgacaa gatatccttt ctacctcccg aacccatcca 420

actacttgac gaagactcca cggagcctga actcgacatt gactcacaac aagaaaatga 480

gggacccatc agtgcgtcaa acagcaatga tagcactagc catagtaatg attgcggtgc 540

cacaattacc agaacaagac ctagacgaag cagttctatc aatgcaaact ttagttttca 600

aaaggctcat gtcagcgatt gcaccatagt caatggcgac catggaacaa agtttgctgt 660

ctggagaatt accgtatttc ttgaacccaa cttgaaggct tttgcggcca agagggaaag 720

ctataaaatc caaacctata aacgatactc cgatttcgtc agattacgag agaatttgct 780

cacaagaatc aagacagcga aacctgagaa acttaactgt ttgcagattc cacaccttcc 840

cccttcagtg cagtggtaca gttcttggaa atatcaagaa gtgaatctga acaaggactg 900

gctggcaaaa agacagagag ggctcgagta cttcctcaat cacatcatcc ttaacagcag 960

cctcgtagaa atgaccaaag atatactcat acagtttcta gagccttcaa aacgagttgc 1020

atagctcacc atccctatcc aaccgactat tcttctcatc gactactact atcccattta 1080

actcgggcgc gttgttaatt aatcactcga tggggaatgc cttgagctga ccgcaatgaa 1140

aacttttagg ggatcgtcca acattaaagg aagaacgaaa cggactccac agtttctaat 1200

ataaataaac aatgataaaa catatagttt cgccattcag gacgaatttt gttggcatca 1260

gcaagtccgt gctgtcaagg atgattcatc acaaggttac aatcataggt tctggccccg 1320

ctgcccacac cgctgctata tacttggcaa gagcagagat gaagcccaca ttatatgagg 1380

gaatgatggc caacggaatt gctgctggtg gccaattgac aacaaccacc gatatcgaaa 1440

atttcccagg gtttcctgaa tcgttgagtg gcagtgaact gatggagagg atgaggaaac 1500

aatctgccaa gtttggcact aacataatta ccgagactgt ctctaaagtc gatttatctt 1560

caaaaccatt cagattatgg accgaattta atgaggatgc agagcctgtg accactgatg 1620

ctataatctt ggccacgggt gcttccgcta agagaatgca tttaccaggg gaggaaacct 1680

actggcagca gggaatatct gcctgtgctg tatgtgatgg tgcagtccct atctttagaa 1740

acaagccatt ggccgttatt ggtggtggtg actctgcgtg tgaggaagcg gaatttctta 1800

cgaagtatgc gtcgaaagta tatatattag taagaaagga tcattttcgt gcatctgtaa 1860

taatgcagag acgaattgag aaaaatccaa acatcattgt tttgttcaac acagttgcat 1920

tagaagctaa gggtgatggt aagttattga atatgttgag aattaagaat actaaaagta 1980

atgtggagaa cgatttagaa gtaaatggac tattttacgc aataggtcac agccctgcca 2040

cagatatagt taaaggacaa gtagatgaag aagagacggg gtatataaaa actgtgcctg 2100

gatcgtctct gacttctgtg ccaggttttt ttgctgcagg tgacgttcag gactctaggt 2160

<210> 23
<211> 1518
<212> DNA
<213> Saccharomyces cerevisiae

<220>
<221> misc_feature
<222> (1)..(1518)
<223> zwf1

<400> 23

```
atgagtgaag gccccgtcaa attcgaaaaa aataccgtca tatctgtctt tggtgcgtca      60
ggtgatctgg caaagaagaa gacttttccc gccttatttg ggcttttcag agaaggttac     120
cttgatccat ctaccaagat cttcggttat gcccggtcca aattgtccat ggaggaggac     180
ctgaagtccc gtgtcctacc ccacttgaaa aaacctcacg gtgaagccga tgactctaag     240
gtcgaacagt tcttcaagat ggtcagctac atttcgggaa attacgacac agatgaaggc     300
ttcgacgaat taagaacgca gatcgagaaa ttcgagaaaa gtgccaacgt cgatgtccca     360
caccgtctct tctatctggc cttgccgcca agcgtttttt tgacggtggc caagcagatc     420
aagagtcgtg tgtacgcaga gaatggcatc acccgtgtaa tcgtagagaa acctttcggc     480
cacgacctgg cctctgccag ggagctgcaa aaaaacctgg ggcccctctt taaagaagaa     540
gagttgtaca gaattgacca ttacttgggt aaagagttgg tcaagaatct tttagtcttg     600
aggttcggta accagttttt gaatgcctcg tggaatagag acaacattca aagcgttcag     660
atttcgttta aagagaggtt cggcaccgaa ggccgtggcg gctatttcga ctctataggc     720
ataatcagag acgtgatgca gaaccatctg ttacaaatca tgactctctt gactatggaa     780
agaccggtgt cttttgaccc ggaatctatt cgtgacgaaa aggttaaggt tctaaaggcc     840
gtggccccca tcgacacgga cgacgtcctc ttgggccagt acggtaaatc tgaggacggg     900
tctaagcccg cctacgtgga tgatgacact gtagacaagg actctaaatg tgtcactttt     960
gcagcaatga ctttcaacat cgaaaacgag cgttgggagg gcgtccccat catgatgcgt    1020
gccggtaagg ctttgaatga gtccaaggtg gagatcagac tgcagtacaa agcggtcgca    1080
tcgggtgtct tcaaagacat tccaaataac gaactggtca tcagagtgca gcccgatgcc    1140
gctgtgtacc taaagtttaa tgctaagacc cctggtctgt caaatgctac ccaagtcaca    1200
gatctgaatc taacttacgc aagcaggtac caagactttt ggattccaga ggcttacgag    1260
gtgttgataa gagacgccct actgggtgac cattccaact ttgtcagaga tgacgaattg    1320
gatatcagtt ggggcatatt caccccatta ctgaagcaca tagagcgtcc ggacggtcca    1380
acaccggaaa tttaccccta cggatcaaga ggtccaaagg gattgaagga atatatgcaa    1440
aaacacaagt atgttatgcc cgaaaagcac ccttacgctt ggcccgtgac taagccagaa    1500
gatacgaagg ataattag                                                   1518
```

<210> 24
<211> 2232
<212> DNA
<213> Saccharomyces cerevisiae

<220>
<221> misc_feature
<222> (1)..(2232)
<223> stb5

<400> 24

atggatggtc ccaattttgc acatcaaggc gggagatcac aacgtactac tgaattgtat 60

tcgtgcgcac gatgcagaaa attaaagaag aagtgtggta aacaaatacc gacatgtgca 120

aactgcgata aaaatggggc acactgttca tatccaggta gagccccaag acgtaccaag 180

aaggagttag cggatgctat gctacgaggg gaatatgttc cagtgaaaag gaacaagaag 240

gtaggaaaaa gcccattgag cactaagagc atgccaaact cttctagtcc gctatccgca 300

aatggcgcta taactcccgg gttttcgcct tacgaaaacg atgatgcaca taagatgaaa 360

cagctaaaac cgtcagatcc aataaatctt gtcatggggg caagtccaaa ttctagcgaa 420

ggtgtctcat cgctaatttc ggtgctaaca tcgctgaatg ataattctaa tccttcttcg 480

cacttatcct ctaatgaaaa ttccatgatt ccttcccgat cattgccagc ttccgtgcag 540

caaagttcga caacttcatc attcggagga tataacacgc cttcaccact aattagcagt 600

catgtgcctg cgaacgccca agccgtaccg ctacaaaaca acaatcgcaa tactagcaac 660

ggggataacg gcagtaatgt taaccacgat aataacaatg gcagtaccaa cacaccgcaa 720

ttgagtctta ccccatatgc aaacaattca gcccccaatg ggaaattcga ttctgtgccg 780

gttgatgcat cctcgatcga atttgaaact atgtcctgtt gctttaaagg tggtagaaca 840

acatcgtggg tcagagagga tggctcgttc aagtcaattg atagatcctt actggacagg 900

ttcattgccg catacttcaa acacaatcac cgtctatttc ccatgattga taaaatagca 960

ttcctaaatg acgccgcgac aattactgat ttcgaaaggt tatatgacaa caaaaactac 1020

cctgacagct ttgttttcaa agtatacatg atcatggcta ttggttgtac aactttacag 1080

cgtgctggta tggtttctca ggacgaagag tgtctgagtg aacatttggc tttttttggcc 1140

atgaaaaaat ttcgtagtgt tataatttta caagatatcg aaactgtacg atgcctattg 1200

ttgttgggta tttattcgtt ttttgagcca aagggctcct cgtcatggac aattagtggt 1260

atcatcatgc gattgaccat aggattaggt ttaaatagag agttaactgc caaaaaactc 1320

aagagcatgt ctgctttaga agcagaggca agatatagag tgttttggag tgcttactgc 1380

tttgaaaggc tagtatgcac ctcgttgggc cgtatatccg ggatcgacga cgaagacatc 1440

actgtgccac taccgagggc gttgtatgtg gatgaaagag acgatttaga gatgaccaag 1500

ttgatgatat cattaaggaa aatgggcggt cgcatttata aacaagtcca ctctgtaagt 1560

gcagggcgac aaaagttaac catcgaacaa aagcaggaaa tcataagtgg attacgcaag 1620

gagctagacg aaatttattc tcgagaatca gaaagaagga aactgaaaaa atctcaaatg 1680

gatcaggtgg agagggagaa caattctact acaaatgtaa tatccttcca tagttctgag 1740

atttggctag caatgagata ctcccagttg caaatcttac tatacagacc atctgcattg 1800

atgccaaaac cgcccattga ctcactatcc actctaggag agttttgctt gcaagcctgg 1860

aaacatactt atacactgta caagaagcgg ttattaccct tgaactggat aacccttttc 1920

agaacattaa ccatttgtaa cactatctta tactgtcttt gccagtggag catcgacctc 1980

attgaaagta aaatcgaaat tcaacagtgt gtggaaatac taagacattt cggtgaaaga 2040

tggatttttg ctatgagatg cgcggatgtt ttccaaaaca ttagcaacac aattctcgat 2100

attagtttaa gccatggtaa agttcctaat atggaccaat taacaagaga gttatttggc 2160

gccagcgatt catatcaaga catattagac gaaaacaatg ttgacgtctc ttgggttgat 2220

aaacttgtat ga 2232

<210> 25
<211> 910
<212> PRT
<213> Bifidobacterium adolescentis

<220>
<221> misc_feature
<222> (1)..(910)
<223> adhE

<400> 25

Met Ala Asp Ala Lys Lys Lys Glu Glu Pro Thr Lys Pro Thr Pro Glu
1 5 10 15

Glu Lys Leu Ala Ala Ala Glu Ala Glu Val Asp Ala Leu Val Lys Lys
20 25 30

Gly Leu Lys Ala Leu Asp Glu Phe Glu Lys Leu Asp Gln Lys Gln Val
35 40 45

Asp His Ile Val Ala Lys Ala Ser Val Ala Ala Leu Asn Lys His Leu
50 55 60

Val Leu Ala Lys Met Ala Val Glu Glu Thr His Arg Gly Leu Val Glu
65 70 75 80

Asp Lys Ala Thr Lys Asn Ile Phe Ala Cys Glu His Val Thr Asn Tyr
85 90 95

Leu Ala Gly Gln Lys Thr Val Gly Ile Ile Arg Glu Asp Asp Val Leu
100 105 110

Gly Ile Asp Glu Ile Ala Glu Pro Val Gly Val Val Ala Gly Val Thr
115 120 125

Pro Val Thr Asn Pro Thr Ser Thr Ala Ile Phe Lys Ser Leu Ile Ala
130 135 140

```
Leu Lys Thr Arg Cys Pro Ile Ile Phe Gly Phe His Pro Gly Ala Gln
145                 150                 155                 160

Asn Cys Ser Val Ala Ala Ala Lys Ile Val Arg Asp Ala Ala Ile Ala
                165                 170                 175

Ala Gly Ala Pro Glu Asn Cys Ile Gln Trp Ile Glu His Pro Ser Ile
            180                 185                 190

Glu Ala Thr Gly Ala Leu Met Lys His Asp Gly Val Ala Thr Ile Leu
        195                 200                 205

Ala Thr Gly Gly Pro Gly Met Val Lys Ala Ala Tyr Ser Ser Gly Lys
    210                 215                 220

Pro Ala Leu Gly Val Gly Ala Gly Asn Ala Pro Ala Tyr Val Asp Lys
225                 230                 235                 240

Asn Val Asp Val Val Arg Ala Ala Asn Asp Leu Ile Leu Ser Lys His
                245                 250                 255

Phe Asp Tyr Gly Met Ile Cys Ala Thr Glu Gln Ala Ile Ile Ala Asp
            260                 265                 270

Lys Asp Ile Tyr Ala Pro Leu Val Lys Glu Leu Lys Arg Arg Lys Ala
        275                 280                 285

Tyr Phe Val Asn Ala Asp Glu Lys Ala Lys Leu Glu Gln Tyr Met Phe
    290                 295                 300

Gly Cys Thr Ala Tyr Ser Gly Gln Thr Pro Lys Leu Asn Ser Val Val
305                 310                 315                 320

Pro Gly Lys Ser Pro Gln Tyr Ile Ala Lys Ala Ala Gly Phe Glu Ile
                325                 330                 335

Pro Glu Asp Ala Thr Ile Leu Ala Ala Glu Cys Lys Glu Val Gly Glu
            340                 345                 350

Asn Glu Pro Leu Thr Met Glu Lys Leu Ala Pro Val Gln Ala Val Leu
        355                 360                 365

Lys Ser Asp Asn Lys Glu Gln Ala Phe Glu Met Cys Glu Ala Met Leu
    370                 375                 380

Lys His Gly Ala Gly His Thr Ala Ala Ile His Thr Asn Asp Arg Asp
```

```
385                 390                 395                 400

Leu Val Arg Glu Tyr Gly Gln Arg Met His Ala Cys Arg Ile Ile Trp
                405                 410                 415

Asn Ser Pro Ser Ser Leu Gly Gly Val Gly Asp Ile Tyr Asn Ala Ile
            420                 425                 430

Ala Pro Ser Leu Thr Leu Gly Cys Gly Ser Tyr Gly Gly Asn Ser Val
        435                 440                 445

Ser Gly Asn Val Gln Ala Val Asn Leu Ile Asn Ile Lys Arg Ile Ala
    450                 455                 460

Arg Arg Asn Asn Asn Met Gln Trp Phe Lys Ile Pro Ala Lys Thr Tyr
465                 470                 475                 480

Phe Glu Pro Asn Ala Ile Lys Tyr Leu Arg Asp Met Tyr Gly Ile Glu
                485                 490                 495

Lys Ala Val Ile Val Cys Asp Lys Val Met Glu Gln Leu Gly Ile Val
            500                 505                 510

Asp Lys Ile Ile Asp Gln Leu Arg Ala Arg Ser Asn Arg Val Thr Phe
        515                 520                 525

Arg Ile Ile Asp Tyr Val Glu Pro Glu Pro Ser Val Glu Thr Val Glu
    530                 535                 540

Arg Gly Ala Ala Met Met Arg Glu Glu Phe Glu Pro Asp Thr Ile Ile
545                 550                 555                 560

Ala Val Gly Gly Gly Ser Pro Met Asp Ala Ser Lys Ile Met Trp Leu
                565                 570                 575

Leu Tyr Glu His Pro Glu Ile Ser Phe Ser Asp Val Arg Glu Lys Phe
            580                 585                 590

Phe Asp Ile Arg Lys Arg Ala Phe Lys Ile Pro Pro Leu Gly Lys Lys
        595                 600                 605

Ala Lys Leu Val Cys Ile Pro Thr Ser Ser Gly Thr Gly Ser Glu Val
    610                 615                 620

Thr Pro Phe Ala Val Ile Thr Asp His Lys Thr Gly Tyr Lys Tyr Pro
625                 630                 635                 640
```

Ile Thr Asp Tyr Ala Leu Thr Pro Ser Val Ala Ile Val Asp Pro Val
645 650 655

Leu Ala Arg Thr Gln Pro Arg Lys Leu Ala Ser Asp Ala Gly Phe Asp
660 665 670

Ala Leu Thr His Ala Phe Glu Ala Tyr Val Ser Val Tyr Ala Asn Asp
675 680 685

Phe Thr Asp Gly Met Ala Leu His Ala Ala Lys Leu Val Trp Asp Asn
690 695 700

Leu Ala Glu Ser Val Asn Gly Glu Pro Gly Glu Glu Lys Thr Arg Ala
705 710 715 720

Gln Glu Lys Met His Asn Ala Ala Thr Met Ala Gly Met Ala Phe Gly
725 730 735

Ser Ala Phe Leu Gly Met Cys His Gly Met Ala His Thr Ile Gly Ala
740 745 750

Leu Cys His Val Ala His Gly Arg Thr Asn Ser Ile Leu Leu Pro Tyr
755 760 765

Val Ile Arg Tyr Asn Gly Ser Val Pro Glu Glu Pro Thr Ser Trp Pro
770 775 780

Lys Tyr Asn Lys Tyr Ile Ala Pro Glu Arg Tyr Gln Glu Ile Ala Lys
785 790 795 800

Asn Leu Gly Val Asn Pro Gly Lys Thr Pro Glu Glu Gly Val Glu Asn
805 810 815

Leu Ala Lys Ala Val Glu Asp Tyr Arg Asp Asn Lys Leu Gly Met Asn
820 825 830

Lys Ser Phe Gln Glu Cys Gly Val Asp Glu Asp Tyr Tyr Trp Ser Ile
835 840 845

Ile Asp Gln Ile Gly Met Arg Ala Tyr Glu Asp Gln Cys Ala Pro Ala
850 855 860

Asn Pro Arg Ile Pro Gln Ile Glu Asp Met Lys Asp Ile Ala Ile Ala
865 870 875 880

Ala Tyr Tyr Gly Val Ser Gln Ala Glu Gly His Lys Leu Arg Val Gln
885 890 895

```
Arg Gln Gly Glu Ala Ala Thr Glu Glu Ala Ser Glu Arg Ala
            900                 905                 910


<210>  26
<211>  1882
<212>  DNA
<213>  Saccharomyces cerevisiae


<220>
<221>  misc_feature
<222>  (1)..(1882)
<223>  upstream sequence to delete Gpd1

<400>  26
aagcctacag gcgcaagata acacatcacc gctctccccc ctctcatgaa aagtcatcgc      60

taaagaggaa cactgaaggt tcccgtaggt tgtctttggc acaaggtagt acatggtaaa     120

aactcaggat ggaataattc aaattcacca atttcaacgt cccttgttta aaaagaaaag     180

aatttttctc tttaaggtag cactaatgca ttatcgatga tgtaaccatt cacacaggtt     240

atttagcttt tgatccttga accattaatt aacccagaaa tagaaattac ccaagtgggg     300

ctctccaaca caatgagagg aaaggtgact tttaaggggg gccagaccct gttaaaaacc     360

tttgatggct atgtaataat agtaaattaa gtgcaaacat gtaagaaaga ttctcggtaa     420

cgaccataca aatattgggc gtgtggcgta gtcggtagcg cgctccctta gcatgggaga     480

ggtctccggt tcgattccgg actcgtccaa attatttttt actttccgcg gtgccgagat     540

gcagacgtgg ccaactgtgt ctgccgtcgc aaaatgattt gaattttgcg tcgcgcacgt     600

ttctcacgta cataataagt attttcatac agttctagca agacgaggtg gtcaaaatag     660

aagcgtccta tgttttacag tacaagacag tccatactga aatgacaacg tacttgactt     720

ttcagtattt tctttttctc acagtctggt tatttttgaa agcgcacgaa atatatgtag     780

gcaagcattt tctgagtctg ctgacctcta aaattaatgc tattgtgcac cttagtaacc     840

caaggcagga cagttacctt gcgtggtgtt actatggccg gaagcccgaa agagttatcg     900

ttactccgat tattttgtac agctgatggg accttgccgt cttcattttt tttttttttc     960

acctatagag ccgggcagag ctgcccggct taactaaggg ccggaaaaaa aacggaaaaa    1020

agaaagccaa gcgtgtagac gtagtataac agtatatctg acacgcacgt gatgaccacg    1080

taatcgcatc gcccctcacc tctcacctct caccgctgac tcagcttcac taaaaaggaa    1140

aatatatact ctttcccagg caaggtgaca gcggtccccg tctcctccac aaaggcctct    1200

cctggggttt gagcaagtct aagtttacgt agcataaaaa ttctcggatt gcgtcaaata    1260

ataaaaaaag taaccccact tctacttcta catcggaaaa acattccatt cacatatcgt    1320

ctttggccta tcttgttttg tcctcggtag atcaggtcag tacaaacgca acacgaaaga    1380
```

acaaaaaaag aagaaaacag aaggccaaga cagggtcaat gagactgttg tcctcctact 1440

gtccctatgt ctctggccga tcacgcgcca ttgtccctca gaaacaaatc aaacacccac 1500

accccgggca cccaaagtcc ccacccacac caccaatacg taaacggggc gccccctgca 1560

ggccctcctg cgcgcggcct cccgccttgc ttctctcccc ttccttttct ttttccagtt 1620

ttccctattt tgtccctttt tccgcacaac aagtatcaga atgggttcat caaatctatc 1680

caacctaatt cgcacgtaga ctggcttggt attggcagtt tcgtagttat atatatacta 1740

ccatgagtga aactgttacg ttaccttaaa ttctttctcc ctttaatttt cttttatctt 1800

actctcctac ataagacatc aagaaacaat tgtatattgt acaccccccc cctccacaaa 1860

cacaaatatt gataatataa ag 1882

<210> 27
<211> 1852
<212> DNA
<213> Saccharomyces cerevisiae

<220>
<221> misc_feature
<222> (1)..(1852)
<223> downstream sequence to delete Gpdl

<400> 27
atttattgga gaaagataac atatcatact ttcccccact tttttcgagg ctcttctata 60

tcatattcat aaattagcat tatgtcattt ctcataacta ctttatcacg ttagaaatta 120

cttattatta ttaaattaat acaaaattta gtaaccaaat aaatataaat aaatatgtat 180

atttaaattt taaaaaaaaa atcctataga gcaaaaggat tttccattat aatattagct 240

gtacacctct tccgcatttt ttgagggtgg ttacaacacc actcattcag aggctgtcgg 300

cacagttgct tctagcatct ggcgtccgta tgtatgggtg tattttaaat aataaacaaa 360

gtgccacacc ttcaccaatt atgtctttaa gaaatggaca agttccaaag agcttgccca 420

aggctcgaca aggatgtact ttggaatatc tatattcaag tacgtggcgc gcatatgttt 480

gagtgtgcac acaataaagg tttttagata ttttgcggcg tcctaagaaa ataaggggtt 540

tcttaaaaaa taacaatagc aaacaaagtt ccttacgatg atttcagatg tgaatagcat 600

ggtcatgatg agtatatacg tttttataaa taattaaaag ttttcctctt gtctgttttt 660

ttgttggctc gtggttgttc tcgaaaaagg agagttttca ttttcgaaat aggtgattat 720

catcatgttg ttatcacccc acgacgaaga taatacggag ctcaccgttt tctttttttt 780

tccctttggc tgaaatttcc caccagaaca aacgtgacaa aattatcttt gaatccaaag 840

tagcttatat atatacgtag aagtgtttcg agacacacat ccaaatacga ggttgttcaa 900

tttaaaccca agaatacata aaaaaatat agatatatta acttagtaaa caatgactgc 960

aagcacacca tccaatgtca tgacattgtt cttgttaagg catggacaaa gtgaattgaa 1020

tcacgagaat atattctgtg gttggattga cgctaagcta accgaaaaag gtaaagaaca 1080

agctcgtcat tctgccgagc taatcgaaca atattgtaaa gctaataatt tgagattacc 1140

ccagattggt tacacctcac gtttaattag gacccaacag accatagaaa cgatgtgtga 1200

agaatttaag ttaaagccac aactgcaggt tgtttacgac tttaataaaa tcaaacttgg 1260

agacgaattt ggcagtgatg acaaggataa tatgaaaatc ccgattcttc aaacttggag 1320

gctaaatgaa cgtcattacg gttcctggca gggccagagg aaaccgaatg ttttaaaaga 1380

atatggtaag gataaatata tgttcattag gagagattac gagggtaagc caccacctgt 1440

agatcttgac cgtgagatga ttcaacaaga aaatgagaag ggctcttcta ctgggtacga 1500

attcaaggag ccaaacagac aaataaaata tgaattggaa tgcagcaatc atgacattgt 1560

attaccggat tccgaatctc ttcgtgaagt ggtttataga ttgaatcctt ttctacaaaa 1620

tgtcatatta aaattagcca atcaatatga tgaatcttca tgcctgattg tgggccatgg 1680

aagttcagtg agatcgctac tgaaaattct ggagggtata tcagatgatg acatcaagaa 1740

tgttgatatt ccaaatggta tccccttagt cgttgaatta gataagaata atggtcttaa 1800

gtttatcaga aaattctacc tagatcctga atctgctaag atcaatgctg ag 1852

<210> 28
<211> 540
<212> DNA
<213> Saccharomyces cerevisiae

<220>
<221> misc_feature
<222> (1)..(540)
<223> Gpd2 promtor region

<400> 28
caaggaatta ccatcaccgt caccatcacc atcatatcgc cttagcctct agccatagcc 60

atcatgcaag cgtgtatctt ctaagattca gtcatcatca ttaccgagtt tgttttcctt 120

cacatgatga agaaggtttg agtatgctcg aaacaataag acgacgatgg ctctgccatt 180

gttatattac gcttttgcgg cgaggtgccg atgggttgct gaggggaaga gtgtttagct 240

tacggaccta ttgccattgt tattccgatt aatctattgt tcagcagctc ttctctaccc 300

tgtcattcta gtattttttt tttttttttt tggttttact tttttttctt cttgcctttt 360

tttcttgtta cttttttttct agtttttttt ccttccacta agctttttcc ttgatttatc 420

cttgggttct tctttctact cctttagatt tttttttttat atattaattt ttaagtttat 480

gtattttggt agattcaatt ctctttccct ttccttttcc ttcgctcccc ttccttatca 540

<210> 29

<211> 2074
<212> DNA
<213> Saccharomyces cerevisiae

<220>
<221> misc_feature
<222> (1)..(2074)
<223> downstream sequence to delete Gpd2

<400> 29
tctgatcttt cctgttgcct ctttttcccc caaccaattt atcattatac acaagttcta 60

caactactac tagtaacatt actacagtta ttataatttt ctattctctt tttctttaag 120

aatctatcat taacgttaat ttctatatat acataactac cattatacac gctattatcg 180

tttacatatc acatcaccgt taatgaaaga tacgacaccc tgtacactaa cacaattaaa 240

taatcgccat aacctttttct gttatctata gcccttaaag ctgtttcttc gagctttttc 300

actgcagtaa ttctccacat gggcccagcc actgagataa gagcgctatg ttagtcacta 360

ctgacggctc tccagtcatt tatgtgattt tttagtgact catgtcgcat ttggcccgtt 420

tttttccgct gtcgcaacct atttccatta acggtgccgt atggaagagt catttaaagg 480

caggagagag agattactca tcttcattgg atcagattga tgactgcgta cggcagatag 540

tgtaatctga gcagttgcga gacccagact ggcactgtct caatagtata ttaatgggca 600

tacattcgta ctcccttgtt cttgcccaca gttctctctc tctttacttc ttgtatcttg 660

tctccccatt gtgcagcgat aaggaacatt gttctaatat acacggatac aaaagaaata 720

cacataattg cataaaataa tgtctaaggg aaaagtttgt ttggcttatt ctggtggttt 780

agatacctcc gtcattttgg cttggctact agaccaaggc tacgaagttg tagctttcat 840

ggctaatgta gggcaagaag aagatttcga tgccgccaag gaaaaggcct tgaagatcgg 900

tgcctgcaag ttcgtttgtg tggattgtcg tgaagatttt gtcaaggata ttctattccc 960

agctgtacag gtcaacgctg tgtacgaaga cgtttatctg ttgggtacct ctttggcaag 1020

acctgttatt gccaaagccc aaattgacgt cgctaaacag gagggctgtt tcgcggtctc 1080

tcatggttgt accggtaaag gtaatgatca aatcagattc gaattgtcat tttacgctct 1140

gaagccagac gttaagtgta ttacaccatg gagaatgcct gaatttttcg aaagatttgc 1200

tggcagaaag gatttgttag actatgctgc acaaaagggt attcccgtcg cccaaaccaa 1260

ggccaagcca tggtctactg acgaaaacca agcccacatt tcttacgagg caggtatctt 1320

ggaagaccca gataccaccc caccaaagga catgtggaaa ttgatcgtcg atccaatgga 1380

tgctccggac caaccacaag atttgaccat tgactttgaa cgtggtcttc cagtcaagtt 1440

gacctacacc gacaacaaga cttccaagga agtttccgtt accaagcctt tggatgtttt 1500

cttggccgca tccaacttag caagggccaa cggtgttggt agaatcgata ttgtagaaga 1560

```
tcgttacatt aacttgaaat ccagaggttg ttacgaacag gctccattga ctgttttgag     1620

aaaagctcat gttgatttgg aaggtttgac tttagacaaa gaagtccgtc aattgagaga     1680

ctcattcgtc acaccaaact actccagatt gatatataac ggtttcctac ttcacccaga     1740

gtgtgagtac atcagatcta tgatccaacc atcccaaaat agcgttaacg gtactgtcag     1800

ggttagactg tataagggta acgtcatcat tctgggcaga tctacaaaga ctgaaaagtt     1860

gtacgatccg acagaatcct ctatggatga gttgaccggt ttcttaccta ccgataccac     1920

cggtttcatt gccatccagg ccattagaat taaaaaatac ggtgaatcca aaaaaaccaa     1980

aggtgaagag ttgactttgt aagtccgcta gttcatcgcc tcaagataga taacgatctc     2040

ttcctccacc tcctatttct gcacactctt gtga                                 2074
```

```
<210>  30
<211>  1630
<212>  DNA
<213>  Thermoanaerobacter pseudethanolicus


<220>
<221>  misc_feature
<222>  (1)..(1630)
<223>  adhB

<400>  30
tgaacaatag acaacccctt tctgtgatct tgtttttttgc aaatgctatt ttatcacaag      60

agatttctct agttcttttt tacttaaaaa aaccctacga aattttaaac tatgtcgaat      120

aaattattga taatttttaa ctatgtgcta ttatattatt gcaaaaaatt taacaatcat      180

cgcgtaagct agttttcaca ttaatgactt acccagtatt ttaggaggtg tttaatgatg      240

aaaggttttg caatgctcag tatcggtaaa gttggctgga ttgagaagga aaagcctgct      300

cctggcccat ttgatgctat tgtaagacct ctagctgtgg ccccttgcac ttcggacatt      360

cataccgttt ttgaaggagc cattggcgaa agacataaca tgatactcgg tcacgaagct      420

gtaggtgaag tagttgaagt aggtagtgag gtaaaagatt ttaaacctgg tgatcgcgtt      480

gttgtgccag ctattacccc tgattggtgg acctctgaag tacaaagagg atatcaccag      540

cactccggtg gaatgctggc aggctggaaa ttttcgaatg taaaagatgg tgtttttggt      600

gaatttttttc atgtgaatga tgctgatatg aatttagcac atctgcctaa agaaattcca      660

ttggaagctg cagttatgat tcccgatatg atgaccactg gttttcacgg agctgaactg      720

gcagatatag aattaggtgc gacggtagca gttttgggta ttggcccagt aggtcttatg      780

gcagtcgctg gtgccaaatt gcgtggagcc ggaagaatta ttgccgtagg cagtagacca      840

gtttgtgtag atgctgcaaa atactatgga gctactgata ttgtaaacta taaagatggt      900

cctatcgaaa gtcagattat gaatctaact gaaggcaaag gtgtcgatgc tgccatcatc      960
```

```
gctggaggaa atgctgacat tatggctaca gcagttaaga ttgttaaacc tggtggcacc     1020

atcgctaatg taaattattt tggcgaagga gaggttttgc ctgttcctcg tcttgaatgg     1080

ggttgcggca tggctcataa aactataaaa ggcgggctat gccccggtgg acgtctaaga     1140

atggaaagac tgattgacct tgtttttat aagcctgtcg atccttctaa gctcgtcact      1200

cacgttttcc agggatttga caatattgaa aaagccttta tgttgatgaa agacaaacca     1260

aaagacctaa tcaaacctgt tgtaatatta gcataaaaat ggggacttag tccattttta     1320

tgctaataag gctaaataca ctggtttttt tatatgacac atcggccagt aaactcttgg     1380

taaaaaaata acaaaaaata gttattttct taacattttt acgccattaa cacttgataa     1440

catcatcgaa gaagtaaata aacaactatt aaataaaaga agaaggagga ttatcatgtt     1500

caaaatttta gaaaaaagag aattggcacc ttccatcaag ttgtttgtaa tagaggcacc     1560

actagtagcc aaaaaagcaa ggccaggcca attcgttatg ctaaggataa aagaaggagg     1620

agaaagaatt                                                            1630
```

<210> 31
<211> 351
<212> PRT
<213> Clostridium beijerinckii

<220>
<221> misc_feature
<222> (1)..(351)
<223> secondary alcohol dehydrogenases

<400> 31

```
Met Lys Gly Phe Ala Met Leu Gly Ile Asn Lys Leu Gly Trp Ile Glu
1               5                   10                  15

Lys Glu Arg Pro Val Ala Gly Ser Tyr Asp Ala Ile Val Arg Pro Leu
            20                  25                  30

Ala Val Ser Pro Cys Thr Ser Asp Ile His Thr Val Phe Glu Gly Ala
        35                  40                  45

Leu Gly Asp Arg Lys Asn Met Ile Leu Gly His Glu Ala Val Gly Glu
    50                  55                  60

Val Val Glu Val Gly Ser Glu Val Lys Asp Phe Lys Pro Gly Asp Arg
65                  70                  75                  80

Val Ile Val Pro Cys Thr Thr Pro Asp Trp Arg Ser Leu Glu Val Gln
                85                  90                  95

Ala Gly Phe Gln Gln His Ser Asn Gly Met Leu Ala Gly Trp Lys Phe
```

```
            100                 105                 110

Ser Asn Phe Lys Asp Gly Val Phe Gly Glu Tyr Phe His Val Asn Asp
        115                 120                 125

Ala Asp Met Asn Leu Ala Ile Leu Pro Lys Asp Met Pro Leu Glu Asn
    130                 135                 140

Ala Val Met Ile Thr Asp Met Met Thr Thr Gly Phe His Gly Ala Glu
145                 150                 155                 160

Leu Ala Asp Ile Gln Met Gly Ser Ser Val Val Val Ile Gly Ile Gly
                165                 170                 175

Ala Val Gly Leu Met Gly Ile Ala Gly Ala Lys Leu Arg Gly Ala Gly
            180                 185                 190

Arg Ile Ile Gly Val Gly Ser Arg Pro Ile Cys Val Glu Ala Ala Lys
        195                 200                 205

Phe Tyr Gly Ala Thr Asp Ile Leu Asn Tyr Lys Asn Gly His Ile Val
    210                 215                 220

Asp Gln Val Met Lys Leu Thr Asn Gly Lys Gly Val Asp Arg Val Ile
225                 230                 235                 240

Met Ala Gly Gly Gly Ser Glu Thr Leu Ser Gln Ala Val Ser Met Val
                245                 250                 255

Lys Pro Gly Gly Ile Ile Ser Asn Ile Asn Tyr His Gly Ser Gly Asp
            260                 265                 270

Ala Leu Leu Ile Pro Arg Val Glu Trp Gly Cys Gly Met Ala His Lys
        275                 280                 285

Thr Ile Lys Gly Gly Leu Cys Pro Gly Gly Arg Leu Arg Ala Glu Met
    290                 295                 300

Leu Arg Asp Met Val Val Tyr Asn Arg Val Asp Leu Ser Lys Leu Val
305                 310                 315                 320

Thr His Val Tyr His Gly Phe Asp His Ile Glu Glu Ala Leu Leu Leu
                325                 330                 335

Met Lys Asp Lys Pro Lys Asp Leu Ile Lys Ala Val Val Ile Leu
            340                 345                 350
```

<210> 32
<211> 1056
<212> DNA
<213> Clostridium beijerinckii

<220>
<221> misc_feature
<222> (1)..(1056)
<223> codon-optimized secondary alcohol dehydrogenase

<400> 32
atgaaagggt ttgctatgtt gggtattaac aaattaggtt ggatcgaaaa ggaaagacct 60

gttgccgggt cctacgatgc catagttcgt ccattagcag tgtctccatg cactagtgat 120

attcataccg tttttgaagg tgctttaggc gacagaaaga atatgatttt gggtcatgag 180

gcggttggcg aagtagttga agtcggctca gaagttaaag atttcaaacc aggtgatagg 240

gtaattgtac catgtacaac gcctgattgg agatctcttg aagtccaagc aggattccaa 300

caacactcca atgggatgct ggcagggtgg aaattttcta acttcaagga tggagttttc 360

ggcgaatact tccacgtgaa tgatgctgat atgaatttgg ctatcttacc aaaggacatg 420

cctctcgaaa acgcagtgat gatcacagac atgatgacaa caggttttca tggcgctgaa 480

ttagccgaca ttcaaatggg atcttcagta gtcgttatcg gtataggcgc tgtgggatta 540

atgggaatcg caggggctaa actaagaggt gccggtagaa ttattggagt tggtagtagg 600

ccaatctgcg tagaagcagc gaagttttac ggtgccaccg atattctgaa ttacaaaaat 660

ggacacatag tagaccaagt aatgaaacta acaaacggaa aaggtgttga tagagtgatc 720

atggcaggtg gtggttctga gactttgtca caggctgtgt caatggtcaa acctggtggt 780

atcatttcaa atatcaatta ccatggcagc ggtgatgctc tacttatacc tagagtcgaa 840

tggggatgtg ggatggctca taaaactatc aagggcggat tgtgtccagg cggtagactg 900

cgagcggaga tgctcagaga tatggtcgtt tataacagag tcgatctttc taagcttgtt 960

actcatgttt atcacggctt tgatcatatc gaggaggcct tattgttgat gaaagacaag 1020

ccaaaggact tgattaaagc agttgtgata ctataa 1056

<210> 33
<211> 1824
<212> DNA
<213> Saccharomyces cerevisiae

<220>
<221> misc_feature
<222> (1)..(1824)
<223> ARI1

<400> 33
gtgaaagctc ggaatacata tttatgacgg aagaagacag aaatctacgg ggcagttgga 60

tcggtgagcc aaaagagtgt tttaccttag accttgcaac atcttctata taccgaagaa 120
ggaagaacat gatattcttc tggtaaactc ttatccagtg gaaaacgcac ccagcatatt 180
cgaaaatata tcaactatct cccctttca tactaataat taatagcatt catattgaaa 240
taataaaaaa gatacgttta atacttacgc cagctctcta gttacagttt cctaacgcat 300
acgtcatcaa tttgttaaga tcggcttcgc tctataaaaa tgtcggccga atttctataa 360
attcggccga aattagcaca ggattttccg cggttccgac ccctatccta gaaacacgga 420
aaaacttgct aataattccg gaatttattc tatgcaactt tatgaagaca aattactata 480
aatgaaccgc tcattcagaa aaactatgtc tcgagctcaa tggatcttac tacatagttt 540
ataaaaacag taattgtgca ttgtacaact gtgctaaaca aacttaaaaa agtaataatt 600
atgaccactg ataccactgt tttcgtttct ggcgcaaccg gtttcattgc tctacacatt 660
gtgaacgatc tgttgaaagc tggctataca gtcatcggct caggtagatc tcaagaaaaa 720
aatgatggct tgctcaaaaa atttaataac aatcccaaac tatcgatgga aattgtggaa 780
gatattgctg ctccaaacgc ctttgatgaa gttttcaaaa aacatggtaa ggaaattaag 840
attgtgctac acactgcctc cccattccat tttgaaacta ccaattttga aaaggattta 900
ctaacccctg cagtgaacgg tacaaaatct atcttggaag cgattaaaaa atatgctgca 960
gacactgttg aaaaagttat tgttacttcg tctactgctg ctctggtgac acctacagac 1020
atgaacaaag aagatttggt gatcacggag gagagttgga ataaggatac atgggacagt 1080
tgtcaagcca acgccgttgc cgcatattgt ggctcgaaaa agtttgctga aaaaactgct 1140
tgggaatttc ttaaagaaaa caagtctagt gtcaaattca cactatccac tatcaatccg 1200
ggattcgttt ttggtcctca aatgtttgca gattcgctaa aacatggcat aaatacctcc 1260
tcaggtatcg tatctgagtt aattcattcc aaggtaggtg gagaatttta taattactgt 1320
ggcccattta ttgacgtgcg tgacgtttct aaagcccacc tagttgcaat tgaaaaacca 1380
gaatgtaccg gccaaagatt agtattgagt gaaggtttat tctgctgtca agaaatcgtt 1440
gacatcttga acgaggaatt ccctcaatta aagggcaaga tagctacagg tgaacctgcg 1500
accggtccaa gctttttaga aaaaaactct tgcaagtttg acaattctaa gacaaaaaaa 1560
ctactgggat tccagtttta caatttaaag gattgcatag ttgacaccgc ggcgcaaatg 1620
tcagaagttc aaaatgaagc ctaagtatca cgctaattga agttttttt gatcactcca 1680
ataggcaaat ctatagatat ataaaaaata tagacaagac ttttttttta cattgccagt 1740
tttcttttt ccttttagt atctattcaa atgggcgacc ctattgtctg atttcattag 1800
cttcatcaca caaaagtgcc acga 1824

<210> 34
<211> 347

<212> PRT
<213> Saccharomyces cerevisiae

<220>
<221> misc_feature
<222> (1)..(347)
<223> ARI1

<400> 34

```
Met Thr Thr Asp Thr Thr Val Phe Val Ser Gly Ala Thr Gly Phe Ile
1               5                   10                  15

Ala Leu His Ile Val Asn Asp Leu Leu Lys Ala Gly Tyr Thr Val Ile
            20                  25                  30

Gly Ser Gly Arg Ser Gln Glu Lys Asn Asp Gly Leu Leu Lys Lys Phe
        35                  40                  45

Asn Asn Asn Pro Lys Leu Ser Met Glu Ile Val Glu Asp Ile Ala Ala
    50                  55                  60

Pro Asn Ala Phe Asp Glu Val Phe Lys Lys His Gly Lys Glu Ile Lys
65                  70                  75                  80

Ile Val Leu His Thr Ala Ser Pro Phe His Phe Glu Thr Thr Asn Phe
                85                  90                  95

Glu Lys Asp Leu Leu Thr Pro Ala Val Asn Gly Thr Lys Ser Ile Leu
            100                 105                 110

Glu Ala Ile Lys Lys Tyr Ala Ala Asp Thr Val Glu Lys Val Ile Val
        115                 120                 125

Thr Ser Ser Thr Ala Ala Leu Val Thr Pro Thr Asp Met Asn Lys Glu
    130                 135                 140

Asp Leu Val Ile Thr Glu Glu Ser Trp Asn Lys Asp Thr Trp Asp Ser
145                 150                 155                 160

Cys Gln Ala Asn Ala Val Ala Ala Tyr Cys Gly Ser Lys Lys Phe Ala
                165                 170                 175

Glu Lys Thr Ala Trp Glu Phe Leu Lys Glu Asn Lys Ser Ser Val Lys
            180                 185                 190

Phe Thr Leu Ser Thr Ile Asn Pro Gly Phe Val Phe Gly Pro Gln Met
        195                 200                 205
```

```
Phe Ala Asp Ser Leu Lys His Gly Ile Asn Thr Ser Ser Gly Ile Val
    210                 215                 220

Ser Glu Leu Ile His Ser Lys Val Gly Gly Glu Phe Tyr Asn Tyr Cys
225                 230                 235                 240

Gly Pro Phe Ile Asp Val Arg Asp Val Ser Lys Ala His Leu Val Ala
                245                 250                 255

Ile Glu Lys Pro Glu Cys Thr Gly Gln Arg Leu Val Leu Ser Glu Gly
            260                 265                 270

Leu Phe Cys Cys Gln Glu Ile Val Asp Ile Leu Asn Glu Glu Phe Pro
        275                 280                 285

Gln Leu Lys Gly Lys Ile Ala Thr Gly Glu Pro Ala Thr Gly Pro Ser
    290                 295                 300

Phe Leu Glu Lys Asn Ser Cys Lys Phe Asp Asn Ser Lys Thr Lys Lys
305                 310                 315                 320

Leu Leu Gly Phe Gln Phe Tyr Asn Leu Lys Asp Cys Ile Val Asp Thr
                325                 330                 335

Ala Ala Gln Met Ser Glu Val Gln Asn Glu Ala
            340                 345


<210>  35
<211>  1125
<212>  DNA
<213>  Entamoeba histolytica


<220>
<221>  misc_feature
<222>  (1)..(1125)
<223>  ADH1

<400>  35
gcacgaggaa aaaccacaat gaaaggactt gctatgcttg gaattggaag aattggatgg      60

attgaaaaga aaatcccaga atgtggacca cttgatgcat tagttagacc attagcactt     120

gcaccatgta catcagatac acataccgtt tgggcaggag ctattggaga tagacatgat     180

atgattcttg gacatgaagc ggttggacaa attgttaaag ttggatcatt agttaagaga     240

ttaaaagttg gagataaagt tattgtacca gctattacac cagattgggg agaagaagaa     300

tcgcaaagag gatatccaat gcattcagga ggaatgcttg gaggatggaa attctcaaat     360

ttcaaggatg gagtttttc agaagttttc catgttaatg aagcagatgc caatcttgca      420

cttcttccaa gagatattaa accagaagat gcagttatgt tatcagatat ggtaactact     480
```

ggattccatg gagcagaatt agctaatatt aaacttggag atactgtttg tgttattggt 540

attggaccag ttggattaat gtcagttgca ggagcaaacc atcttggagc aggaagaatc 600

tttgcagtag gatcaagaaa acattgttgt gatattgcat tggaatatgg agcaacagat 660

attattaatt ataaaaatgg agatattgta gaacaaattc ttaaagctac agacggcaaa 720

ggagttgata aagtcgttat tgcaggaggt gatgttcata catttgcaca agcagtcaaa 780

atgattaaac caggatcaga tattggaaat gttaattatc ttggagaagg agataatatt 840

gatattccaa gaagtgaatg gggagttgga atgggtcata aacacattca tggaggttta 900

accccaggtg gaagagtcag aatggaaaaa ttagcatcac ttatttcaac tggtaaatta 960

gatacttcta aacttattac acatagattt gaaggattag aaaaagttga agatgcatta 1020

atgttaatga agaataaacc agcagacctt atcaaaccag ttgtcagaat tcattatgat 1080

gatgaagata ctcttcatta aattcattaa ttcaaagtat taaac 1125

<210> 36
<211> 1502
<212> DNA
<213> Cucumis melo

<220>
<221> misc_feature
<222> (1)..(1502)
<223> ADH1

<400> 36
tcccaaaatt caaatccttt tacctataaa tactctcact cacttcttcc ttcatcatca 60

tcatcgtcat ttctctttct aacccaaatc aaattttgtt tctctctctc tctctctctc 120

ttcaaatccc tttcaccata acccacaact atgtccactg ccggtcaggt catcaaatgc 180

aaagctgctg tggctcggga ggccggaaag ccacttgtca ttgaaaaagt tgaagtggca 240

ccaccgcaag ctaatgaagt ccgattgaag atccttttca cttctctctg tcataccgat 300

gtttatttct gggaagccaa gggacaaacc ccattgtttc ctcgtatttt tggacataag 360

gctggaggaa ttgttgagag tgttggagaa ggagtgaaag atcttcaacc aggagatcat 420

gttcttccta ttttcactgg tgaatgtggg gattgtagtc attgtcaatc tgaagaaagc 480

aatatgtgtg atcttcttcg aatcaatacc gatcgtggag ttatgatcaa tgatggcaaa 540

actagattct ccaaaaatgg acaacccatt catcattttg ttggaacctc cacttttagt 600

gaatacactg ttgttcatgt tggttgcttg gctaagatca accctgctgc ccctcttgac 660

aaagtttgtg ttcttagctg cggcatttcc acaggccttg gtgccacttt gaatgttgca 720

aagcctaaaa agggtcaatc tgttgcgatc tttggacttg gagttgttgg acttgctgct 780

gctgaaggag caagaattgc tggtgcatct aggatcattg gtgttgacct gaacccggct 840

cgattcgaag aagcaaagaa atttggttgc aacgaatttg tgaatccaaa ggatcacaac 900

aagccagttc aagaggtgat tgctgagatg acgaacggag gagttgaccg aagcgtcgag 960

tgtacgggaa gcatccaagc aatgatcgca gcatttgaat gcgttcacga tgggtggggt 1020

gttgctgttc ttgtgggagt cccaaacaaa gacgatgcat tcaaaactca tcctatgaat 1080

ttccttaacg aaagaactct aaagggtaca ttcttcggca actacaaacc ccgaaccgac 1140

attccggggg tggtcgagaa gtacttgagc aaggagctgg aattggagaa gttcattaca 1200

catacagtgt cattttctga gatcaacaag gcgtttgatt acatgctgaa aggggagtcg 1260

attcgatgca ttattagaat ggataattga aataataact gtgggatgag atgaaaataa 1320

gggaataaga ttatgtggtg attgaaagag gctggagagt tctggttttc cttatttctt 1380

tctaagtttg tgtttaatgt tttctgagag tggaatgttc gcgatagtgt tatcgccttt 1440

ttgtcaattt catccaactc ttgaaatatt gtagtcatat tataatcgaa aaaaaaaaaa 1500

aa 1502

<210> 37
<211> 2142
<212> DNA
<213> Saccharomyces cerevisiae

<220>
<221> misc_feature
<222> (1)..(2142)
<223> acs1

<400> 37
atgtcgccct ctgccgtaca atcatcaaaa ctagaagaac agtcaagtga aattgacaag 60

ttgaaagcaa aaatgtccca gtctgccgcc actgcgcagc agaagaagga acatgagtat 120

gaacatttga cttcggtcaa gatcgtgcca caacggccca tctcagatag actgcagccc 180

gcaattgcta cccactattc tccacacttg gacgggttgc aggactatca gcgcttgcac 240

aaggagtcta ttgaagaccc tgctaagttc ttcggttcta aagctaccca atttttaaac 300

tggtctaagc cattcgataa ggtgttcatc ccagacccta aaacgggcag gccctccttc 360

cagaacaatg catggttcct caacggccaa ttaaacgcct gttacaactg tgttgacaga 420

catgccttga agactcctaa caagaaagcc attattttcg aaggtgacga gcctggccaa 480

ggctattcca ttacctacaa ggaactactt gaagaagttt gtcaagtggc acaagtgctg 540

acttactcta tgggcgttcg caagggcgat actgttgccg tgtacatgcc tatggtccca 600

gaagcaatca taaccttgtt ggccatttcc cgtatcggtg ccattcactc cgtagtcttt 660

gccgggtttt cttccaactc cttgagagat cgtatcaacg atggggactc taaagttgtc 720

atcactacag atgaatccaa cagaggtggt aaagtcattg agactaaaag aattgttgat 780

```
gacgcgctaa gagagacccc aggcgtgaga cacgtcttgg tttatagaaa gaccaacaat       840
ccatctgttg ctttccatgc ccccagagat ttggattggg caacagaaaa gaagaaatac       900
aagacctact atccatgcac acccgttgat tctgaggatc cattattctt gttgtatacg       960
tctggttcta ctggtgcccc caagggtgtt caacattcta ccgcaggtta cttgctggga      1020
gctttgttga ccatgcgcta cacttttgac actcaccaag aagacgtttt cttcacagct      1080
ggagacattg gctggattac aggccacact tatgtggttt atggtccctt actatatggt      1140
tgtgccactt tggtctttga agggactcct gcgtacccaa attactcccg ttattgggat      1200
attattgatg aacacaaagt cacccaattt tatgttgcgc caactgcttt gcgtttgttg      1260
aaaagagctg gtgattccta catcgaaaat cattccttaa aatctttgcg ttgcttgggt      1320
tcggtcggtg agccaattgc tgctgaagtt tgggagtggt actctgaaaa aataggtaaa      1380
aatgaaatcc ccattgtaga cacctactgg caaacagaat ctggttcgca tctggtcacc      1440
ccgctggctg gtggtgttac accaatgaaa ccgggttctg cctcattccc cttcttcggt      1500
attgatgcag ttgttcttga ccctaacact ggtgaagaac ttaacaccag ccacgcagag      1560
ggtgtccttg ccgtcaaagc tgcatggcca tcatttgcaa gaactatttg gaaaaatcat      1620
gataggtatc tagacactta tttgaaccct taccctggct actatttcac tggtgatggt      1680
gctgcaaagg ataaggatgg ttatatctgg attttgggtc gtgtagacga tgtggtgaac      1740
gtctctggtc accgtctgtc taccgctgaa attgaggctg ctattatcga agatccaatt      1800
gtggccgagt gtgctgttgt cggattcaac gatgacttga ctggtcaagc agttgctgca      1860
tttgtggtgt tgaaaaacaa atctagttgg tccaccgcaa cagatgatga attacaagat      1920
atcaagaagc atttggtctt tactgttaga aaagacatcg ggccatttgc cgcaccaaaa      1980
ttgatcattt tagtggatga cttgcccaag acaagatccg gcaaaattat gagacgtatt      2040
ttaagaaaaa tcctagcagg agaaagtgac caactaggcg acgtttctac attgtcaaac      2100
cctggcattg ttagacatct aattgattcg gtcaagttgt aa                         2142
```

<210> 38
<211> 2127
<212> DNA
<213> Saccharomyces kluyveri

<220>
<221> misc_feature
<222> (1)..(2127)
<223> acs1

<400> 38

```
atgtcacccg ctgtcgtcaa agtaggacag gcagaagatt cgcaatcgga tgttatccag        60
aagctgaagg ctcagaacaa gagtggcgaa gctgcacact tggagtacga gcatttgact       120
```

agtgttcctg tgatcgagca gaagccggtt accgatcggt tggctccaga gttacaacag 180
cactacaagc ctcatttgtc tggtcttgat gagtacaagc aactgtataa ggaatcgttg 240
gagaatccag ggaaattttt tggtgagcgt gccagcacgt tgttggactg ggtcaaaccg 300
tttgaccagg tttttatggc tgatgatgag ggcaaaccgg cgtttgacaa caacgcgtgg 360
tttaccaacg gtcagttgaa cgcctgttac aacatggttg atagacatgc tattaaaact 420
ccaaacaaag ccgctattat ttatgaagcc gacgaaccgg gcgaaggtta cattttgact 480
tatagagagt tgttggaaca ggtctgcaga gttgcacagg tattgacaca ttccatgggg 540
gttcgcaagg gggacaccgt tgccgtttac atgcccatga ttccccaggc cttggtcacc 600
ttgttggcta tctcccgtat cggtgccatc cactctgtcg tgtttgccgg gttcagttcc 660
aattccctac gtgaccgtat caacgacgcc tactcgaaag tcgtgattac cactgacgaa 720
tccaagagag gcggaaaagt gattgaaacg aaaaggattg tggacgatgc tctaaaggaa 780
acacctcagg tggaacacgt tcttgtctac aaacgtacgc acagtccaaa ggtcaacttc 840
catgccccaa gagatttgga ctgggacgtt gaagtcaaga agtacaaggc ttactctcct 900
atcgaaccgg ttgattcgga acatcccttg tttttgttgt acacctccgg ttctacaggt 960
gctccaaagg gtgttcaaca ctcaaccgct ggatatctat tgcaggcaat gctatccatg 1020
cgctatacct ttgataccca caaggaggat atcttcttca ccgcgggtga cattggttgg 1080
atcactggac acacctatgt cgtttatggt ccgttgttga ccggttgtac cactatggtt 1140
tttgaaggca ctcctgcata ccctaactac tcgaggtatt gggaaattgt tgacaagtac 1200
aaggttaccc agttctacgt tgctccaacc gccttgcgtt tgttgaagag ggctggtgat 1260
tctttcacag agggctactc tttgaaatcc ttgcgttgtc taggtaccgt tggtgaaccc 1320
attgctgcag aagtttggga gtggtattcc gaaaagattg gtcgcaatga aatacccatc 1380
attgacactt actggcagac ggaatctggt tctcatctag tcaccccaat ggctggcggt 1440
gttacaccaa tgaagccagg ttctgcttct ttcccattct ttggtatcga gttggccgtg 1500
ttggacccgg ccagtggcga agagttgaag ggtgaacccg ttgaaggtgt cttggctatc 1560
aaaaaaccat ggccatcttt tgctaggacc atctggaaaa accatgacag atatctggat 1620
acttacttga acccttaccc aggctactac ttcactggtg acggtgctgc ccgtgacaag 1680
gatgggttta tttggatttt gggacgtgtc gatgacgttg taaacgtttc gggccaccgt 1740
ctatccactg ctgaaatcga agctgcaatc atcgaagatg acatggttgc cgaatgtgcc 1800
gttgtcggct atgcagatga cttgactggt caagcggttg ccgcctttgt tgtgttgaag 1860
aataagaaca gctgggccac tgcgagcgaa gatgagttac aaagcatcaa gaagcacttg 1920
attctaactg tcagaaagga tattggccca ttcgcggcac caaaattaat tgtgttggtt 1980

gacgacttgc caaagactag atccggtaaa atcatgagac gtattctaag aaagattcta 2040

tccggtgaag ccgatcagct cggtgatgtt tccactttgt cgaacccagg catcgtcaag 2100

catttgatcg attctgtgaa attttga 2127

<210> 39
<211> 2052
<212> DNA
<213> Saccharomyces cerevisiae

<220>
<221> misc_feature
<222> (1)..(2052)
<223> acs2

<400> 39
atgacaatca aggaacataa agtagtttat gaagctcaca acgtaaaggc tcttaaggct 60

cctcaacatt tttacaacag ccaacccggc aagggttacg ttactgatat gcaacattat 120

caagaaatgt atcaacaatc tatcaatgag ccagaaaaat tctttgataa gatggctaag 180

gaatacttgc attgggatgc tccatacacc aaagttcaat ctggttcatt gaacaatggt 240

gatgttgcat ggtttttgaa cggtaaattg aatgcatcat acaattgtgt tgacagacat 300

gcctttgcta atcccgacaa gccagctttg atctatgaag ctgatgacga atccgacaac 360

aaaatcatca catttggtga attactcaga aaagtttccc aaatcgctgg tgtcttaaaa 420

agctggggcg ttaagaaagg tgacacagtg gctatctatt tgccaatgat tccagaagcg 480

gtcattgcta tgttggctgt ggctcgtatt ggtgctattc actctgttgt ctttgctggg 540

ttctccgctg gttcgttgaa agatcgtgtc gttgacgcta attctaaagt ggtcatcact 600

tgtgatgaag gtaaaagagg tggtaagacc atcaacacta aaaaaattgt tgacgaaggt 660

ttgaacggag tcgatttggt ttcccgtatc ttggttttcc aaagaactgg tactgaaggt 720

attccaatga aggccggtag agattactgg tggcatgagg aggccgctaa gcagagaact 780

tacctacctc ctgtttcatg tgacgctgaa gatcctctat ttttattata cacttccggt 840

tccactggtt ctccaaaggg tgtcgttcac actacaggtg gttatttatt aggtgccgct 900

ttaacaacta gatacgtttt tgatattcac ccagaagatg ttctcttcac tgccggtgac 960

gtcggctgga tcacgggtca cacctatgct ctatatggtc cattaacctt gggtaccgcc 1020

tcaataattt tcgaatccac tcctgcctac ccagattatg gtagatattg gagaattatc 1080

caacgtcaca aggctaccca tttctatgtg gctccaactg ctttaagatt aatcaaacgt 1140

gtaggtgaag ccgaaattgc caaatatgac acttcctcat tacgtgtctt gggttccgtc 1200

ggtgaaccaa tctctccaga cttatgggaa tggtatcatg aaaaagtggg taacaaaaac 1260

tgtgtcattt gtgacactat gtggcaaaca gagtctggtt ctcatttaat tgctcctttg 1320

gcaggtgctg tcccaacaaa acctggttct gctaccgtgc cattctttgg tattaacgct 1380

tgtatcattg accctgttac aggtgtggaa ttagaaggta atgatgtcga aggtgtcctt 1440

gccgttaaat caccatggcc atcaatggct agatctgttt ggaaccacca cgaccgttac 1500

atggatactt acttgaaacc ttatcctggt cactatttca caggtgatgg tgctggtaga 1560

gatcatgatg gttactactg gatcaggggt agagttgacg acgttgtaaa tgtttccggt 1620

catagattat ccacatcaga aattgaagca tctatctcaa atcacgaaaa cgtctcggaa 1680

gctgctgttg tcggtattcc agatgaattg accggtcaaa ccgtcgttgc atatgtttcc 1740

ctaaaagatg gttatctaca aaacaacgct actgaaggtg atgcagaaca catcacacca 1800

gataatttac gtagagaatt gatcttacaa gttaggggtg agattggtcc tttcgcctca 1860

ccaaaaacca ttattctagt tagagatcta ccaagaacaa ggtcaggaaa gattatgaga 1920

agagttctaa gaaaggttgc ttctaacgaa gccgaacagc taggtgacct aactactttg 1980

gccaacccag aagttgtacc tgccatcatt tctgctgtag agaaccaatt tttctctcaa 2040

aaaaagaaat aa 2052

<210> 40
<211> 2052
<212> DNA
<213> Saccharomyces kluyveri

<220>
<221> misc_feature
<222> (1)..(2052)
<223> acs2

<400> 40
atgtctgcta aagaacacaa agttgtccat gaggctcaca acgtcgagcc tcgttatgct 60

ccagaacatt tctacaagag tcaaccagga aagggatatg tcaatgactt gacccattac 120

cgtcagatgt acgagcaatc cattaacgac ccagaaggtt tttttggccc attggcccaa 180

gaatatttgc attgggacag accgtttact aaggttcaat cgggttccct agaaaacggc 240

gatgttgcct ggtttttaaa cggtgaatta aatgcttctt acaactgtgt tgatagacat 300

gcttttgcca acccatctaa gcctgctatc atttacgaag ccgacgatga aaaggaaaat 360

agagttatca cctttggtga attgttgaga caagtctccg aagttgccgg tgtgttgaag 420

agctggggtg ttaaaaaagg tgacacagtt gccgtttaca tgccaatgat tccagaagct 480

gttgttgcta tgttagcagt tgctcgtttg ggtgctatcc actctgttat ctttgctggt 540

ttctcatccg gttctctaaa agagcgtgtt gttgatgctg gttgtaaagt tgtcattacc 600

tgtgatgaag gccgtagagg tggtaagact gttcacgcca agaagatcgt cgacgaaggt 660

ttgtctggtg ttgactctgt gtcccacatt ttggttttcc aaagaactgg ttctcaaggt 720

atcccaatga aaccaggcag agatttctgg tggcacgaag aatccgaaaa gcacaggggc 780

tatttgccac ctgtcccagt caactctgaa gatccattat tcctattgta tacctcaggt 840

tctaccggat ctccaaaagg tgtcgtccac acaactggtg gttacttgtt gggtgctgcc 900

ttgaccacta gatacgtttt cgacattcat ccagaagatg ttttgttcac tgcgggtgat 960

gtgggttgga ttactgggca cacctatgcc ttgtacggtc cactagcttt gggtactgct 1020

accattatct ttgaatctac tccagcttat ccagactacg gtagatactg gagaatcatt 1080

gaacgtcata aggccactca cttctacgtt gctccaactg ctttgagatt gatcaagcgt 1140

gtgggtgaag ctgaaattgg taaatatgat atctcgtccc taagagttct aggttctgtc 1200

ggtgaaccga tctctccaga tttatgggaa tggtatcacg aaaagattgg taacaagaac 1260

tgtgttatct gtgacactat gtggcaaaca gaatctggtt ctcatctgat tgccccactg 1320

gcaggtgccg ttccaaccaa gccaggttct gctactgttc catttttggg cgttaacacc 1380

tgtatcattg atccagtttc cggtgaggaa ttaaagggca atgatgttga aggtgtcttg 1440

gctgttaaag ctccatggcc atccatggct agatctgtct ggaacaacca ctcccgttac 1500

ttcgaaacct atatgaagcc atatccaggc tactacttta ctggtgatgg tgctggtagg 1560

gatcacgatg gttactactg gattaggggt agagttgacg atgttgttaa cgtttctggt 1620

cacagattat ccaccgctga aatcgaagct gctttggtgg aacacgaagg cgtctctgaa 1680

tctgccgttg tcggtatcac cgatgaatta actggtcaag ctgttgttgc ttttgtctct 1740

ttgaaggacg gttatttgca agaaaacgct gccgaagggg atgctgctca cattactcca 1800

gataacttgc gtcgtgaact aattttgcaa gttagaggtg agattggtcc attcgctgcc 1860

cccaagaccg ttatcgttgt taaggacttg ccaaagacta gatctggtaa gatcatgagg 1920

agaatcttga gaaagattgc ctccaacgaa gctgagcaat taggcgattt gtctactttg 1980

gccaaccaag atgttgttcc atcaattatc tatgctgtcg aaaaccaatt ttttgctcaa 2040

aagaagaaat aa 2052

<210> 41
<211> 1047
<212> DNA
<213> Saccharomyces cerevisiae

<220>
<221> misc_feature
<222> (1)..(1047)
<223> ADH1

<400> 41
atgtctatcc cagaaactca aaaaggtgtt atcttctacg aatcccacgg taagttggaa 60

tacaaagata ttccagttcc aaagccaaag gccaacgaat tgttgatcaa cgttaaatac 120

```
tctggtgtct gtcacactga cttgcacgct tggcacggtg actggccatt gccagttaag      180

ctaccattag tcggtggtca cgaaggtgcc ggtgtcgttg tcggcatggg tgaaaacgtt      240

aagggctgga agatcggtga ctacgccggt atcaaatggt tgaacggttc ttgtatggcc      300

tgtgaatact gtgaattggg taacgaatcc aactgtcctc acgctgactt gtctggttac      360

acccacgacg gttctttcca acaatacgct accgctgacg ctgttcaagc cgctcacatt      420

cctcaaggta ccgacttggc ccaagtcgcc cccatcttgt gtgctggtat caccgtctac      480

aaggctttga agtctgctaa cttgatggcc ggtcactggg ttgctatctc cggtgctgct      540

ggtggtctag gttctttggc tgttcaatac gccaaggcta tgggttacag agtcttgggt      600

attgacggtg gtgaaggtaa ggaagaatta ttcagatcca tcggtggtga agtcttcatt      660

gacttcacta aggaaaagga cattgtcggt gctgttctaa aggccactga cggtggtgct      720

cacggtgtca tcaacgtttc cgtttccgaa gccgctattg aagcttctac cagatacgtt      780

agagctaacg gtaccaccgt tttggtcggt atgccagctg gtgccaagtg ttgttctgat      840

gtcttcaacc aagtcgtcaa gtccatctct attgttggtt cttacgtcgg taacagagct      900

gacaccagag aagctttgga cttcttcgcc agaggtttgg tcaagtctcc aatcaaggtt      960

gtcggcttgt ctaccttgcc agaaatttac gaaaagatgg aaaagggtca aatcgttggt     1020

agatacgttg ttgacacttc taaataa                                         1047
```

<210> 42
<211> 199
<212> PRT
<213> Mycobacterium gastri

<220>
<221> misc_feature
<222> (1)..(199)
<223> rmpB (PHI)

<400> 42

```
Met Thr Gln Ala Ala Glu Ala Asp Gly Ala Val Lys Val Val Gly Asp
1               5                   10                  15

Asp Ile Thr Asn Asn Leu Ser Leu Val Arg Asp Glu Val Ala Asp Thr
            20                  25                  30

Ala Ala Lys Val Asp Pro Glu Gln Val Ala Val Leu Ala Arg Gln Ile
        35                  40                  45

Val Gln Pro Gly Arg Val Phe Val Ala Gly Ala Gly Arg Ser Gly Leu
    50                  55                  60

Val Leu Arg Met Ala Ala Met Arg Leu Met His Phe Gly Leu Thr Val
```

```
65                  70                  75                  80

His Val Ala Gly Asp Thr Thr Thr Pro Ala Ile Ser Ala Gly Asp Leu
                85                  90                  95

Leu Leu Val Ala Ser Gly Ser Gly Thr Thr Ser Gly Val Val Lys Ser
            100                 105                 110

Ala Glu Thr Ala Lys Lys Ala Gly Ala Arg Ile Ala Ala Phe Thr Thr
        115                 120                 125

Asn Pro Asp Ser Pro Leu Ala Gly Leu Ala Asp Ala Val Val Ile Ile
    130                 135                 140

Pro Ala Ala Gln Lys Thr Asp His Gly Ser His Ile Ser Arg Gln Tyr
145                 150                 155                 160

Ala Gly Ser Leu Phe Glu Gln Val Leu Phe Val Val Thr Glu Ala Val
                165                 170                 175

Phe Gln Ser Leu Trp Asp His Thr Glu Val Glu Ala Glu Glu Leu Trp
            180                 185                 190

Thr Arg His Ala Asn Leu Glu
        195

<210>  43
<211>  207
<212>  PRT
<213>  Mycobacterium gastri

<220>
<221>  misc_feature
<222>  (1)..(207)
<223>  rmpA (HPS)

<400>  43

Met Lys Leu Gln Val Ala Ile Asp Leu Leu Ser Thr Glu Ala Ala Leu
1               5                   10                  15

Glu Leu Ala Gly Lys Val Ala Glu Tyr Val Asp Ile Ile Glu Leu Gly
            20                  25                  30

Thr Pro Leu Ile Glu Ala Glu Gly Leu Ser Val Ile Thr Ala Val Lys
        35                  40                  45

Lys Ala His Pro Asp Lys Ile Val Phe Ala Asp Met Lys Thr Met Asp
    50                  55                  60
```

```
Ala Gly Glu Leu Glu Ala Asp Ile Ala Phe Lys Ala Gly Ala Asp Leu
65                  70                  75                  80

Val Thr Val Leu Gly Ser Ala Asp Asp Ser Thr Ile Ala Gly Ala Val
                85                  90                  95

Lys Ala Ala Gln Ala His Asn Lys Gly Val Val Val Asp Leu Ile Gly
            100                 105                 110

Ile Glu Asp Lys Ala Thr Arg Ala Gln Glu Val Arg Ala Leu Gly Ala
        115                 120                 125

Lys Phe Val Glu Met His Ala Gly Leu Asp Glu Gln Ala Lys Pro Gly
    130                 135                 140

Phe Asp Leu Asn Gly Leu Leu Ala Ala Gly Glu Lys Ala Arg Val Pro
145                 150                 155                 160

Phe Ser Val Ala Gly Gly Val Lys Val Ala Thr Ile Pro Ala Val Gln
                165                 170                 175

Lys Ala Gly Ala Glu Val Ala Val Ala Gly Gly Ala Ile Tyr Gly Ala
            180                 185                 190

Ala Asp Pro Ala Ala Ala Ala Lys Glu Leu Arg Ala Ala Ile Ala
        195                 200                 205
```

<210> 44
<211> 1161
<212> DNA
<213> Saccharomyces cerevisiae

<220>
<221> misc_feature
<222> (1)..(1161)
<223> SFA1

<400> 44

```
atgtccgccg ctactgttgg taaacctatt aagtgcattg ctgctgttgc gtatgatgcg      60

aagaaaccat taagtgttga agaaatcacg gtagacgccc caaaagcgca cgaagtacgt     120

atcaaaattg aatatactgc tgtatgccac actgatgcgt acactttatc aggctctgat     180

ccagaaggac ttttcccttg cgttctgggc cacgaaggag ccggtatcgt agaatctgta     240

ggcgatgatg tcataacagt taagcctggt gatcatgtta ttgctttgta cactgctgag     300

tgtggcaaat gtaagttctg tacttccggt aaaaccaact tatgtggtgc tgttagagct     360

actcaaggga aaggtgtaat gcctgatggg accacaagat ttcataatgc gaaaggtgaa     420
```

gatatatacc atttcatggg ttgctctact ttttccgaat atactgtggt ggcagatgtc 480

tctgtggttg ccatcgatcc aaaagctccc ttggatgctg cctgtttact gggttgtggt 540

gttactactg gttttggggc ggctcttaag acagctaatg tgcaaaaagg cgataccgtt 600

gcagtatttg gctgcgggac tgtaggactc tccgttatcc aaggtgcaaa gttaaggggc 660

gcttccaaga tcattgccat tgacattaac aataagaaaa aacaatattg ttctcaattt 720

ggtgccacgg attttgttaa tcccaaggaa gatttggcca aagatcaaac tatcgttgaa 780

aagttaattg aaatgactga tggggtctg gattttactt ttgactgtac tggtaatacc 840

aaaattatga gagatgcttt ggaagcctgt cataaaggtt ggggtcaatc tattatcatt 900

ggtgtggctg ccgctggtga agaaatttct acaaggccgt tccagctggt cactggtaga 960

gtgtggaaag gctctgcttt tggtggcatc aaaggtagat ctgaaatggg cggtttaatt 1020

aaagactatc aaaaaggtgc cttaaaagtc gaagaattta tcactcacag gagaccattc 1080

aaagaaatca atcaagcctt tgaagatttg cataacggtg attgcttaag aaccgtcttg 1140

aagtctgatg aaataaaata g 1161

<210> 45
<211> 900
<212> DNA
<213> Saccharomyces cerevisiae

<220>
<221> misc_feature
<222> (1)..(900)
<223> YJL068C

<400> 45
atgaaggttg ttaaggaatt tagtgtctgt ggtggcagat tgatcaagtt gtcacataac 60

tcgaactcta ccaagaccag catgaacgtc aatatctatt tgcctaagca ctattacgcc 120

caagattttc caagaaataa gcgtatccca actgtgtttt acctttctgg cttgacgtgc 180

acgccagaca acgcctctga gaaggctttt tggcagtttc aagctgacaa gtacggattt 240

gcaatagtct ttccggatac gtccccacgt ggtgacgaag tagccaatga tcctgagggc 300

tcctgggatt ttggacaggg cgccggattc tatctaaatg ccacccaaga accatacgcc 360

caacattacc agatgtacga ctacattcac aaagaactcc cacaaacatt agattctcat 420

tttaacaaga acggtgacgt aaagctggac ttcttggaca atgttgccat cacaggccat 480

tcgatggggg gatatggtgc aatttgtggg tatttgaagg gctattccgg aaagagatac 540

aaatcttgtt ctgccttcgc ccctatcgtg aacccttcca acgttccctg ggggtcaaaaa 600

gcgtttaaag gttatctggg cgaagaaaaa gcccagtggg aagcgtacga cccatgttta 660

ttaatcaaga atattagaca tgtgggcgac gacagaattt tgatccatgt aggagactcc 720

gatccctttt tggaagaaca cttgaaaccg gaattactac ttgaggcggt gaaagccact 780

tcatggcagg actacgtgga aataaaaaaa gttcacggct ttgatcactc ctattacttt 840

gtcagcactt tcgttccaga acatgctgaa tttcatgcgc gaaacttggg tttgatttga 900

<210> 46
<211> 1131
<212> DNA
<213> Saccharomyces cerevisiae

<220>
<221> misc_feature
<222> (1)..(1131)
<223> FDH1

<400> 46
atgtcgaagg gaaaggtttt gctggttctt tacgaaggtg gtaagcatgc tgaagagcag 60

gaaaagttat tggggtgtat tgaaaatgaa cttggtatca gaaatttcat tgaagaacag 120

ggatacgagt tggttactac cattgacaag gaccctgagc caacctcaac ggtagacagg 180

gagttgaaag acgctgaaat tgtcattact acgccctttt tccccgccta catctcgaga 240

aacaggattg cagaagctcc taacctgaag ctctgtgtaa ccgctggcgt cggttcagac 300

catgtcgatt tagaagctgc aaatgaacgg aaaatcacgg tcaccgaagt tactggttct 360

aacgtcgttt ctgtcgcaga gcacgttatg gccacaattt tggttttgat aagaaactat 420

aatggtggtc atcaacaagc aattaatggt gagtgggata ttgccggcgt ggctaaaaat 480

gagtatgatc tggaagacaa aataatttca acggtaggtg ccggtagaat tggatatagg 540

gttctggaaa gattggtcgc atttaatccg aagaagttac tgtactacga ctaccaggaa 600

ctacctgcgg aagcaatcaa tagattgaac gaggccagca agcttttcaa tggcagaggt 660

gatattgttc agagagtaga gaaattggag gatatggttg ctcagtcaga tgttgttacc 720

atcaactgtc cattgcacaa ggactcaagg ggtttattca ataaaaagct tatttcccac 780

atgaaagatg gtgcatactt ggtgaatacc gctagaggtg ctatttgtgt cgcagaagat 840

gttgccgagg cagtcaagtc tggtaaattg gctggctatg gtggtgatgt ctgggataag 900

caaccagcac caaaagacca tccctggagg actatggaca ataaggacca cgtgggaaac 960

gcaatgactg ttcatatcag tggcacatct ctggatgctc aaaagaggta cgctcaggga 1020

gtaaagaaca tcctaaatag ttacttttcc aaaaagtttg attaccgtcc acaggatatt 1080

attgtgcaga atggttctta tgccaccaga gcttatggac agaagaaata a 1131

<210> 47
<211> 1095
<212> DNA
<213> Candida boidinii

<220>
<221> misc_feature
<222> (1)..(1095)
<223> FDH3

<400> 47
atgaagattg tcttagttct ttatgatgct ggtaagcacg ctgctgatga agaaaaatta 60

tatggttgta ctgaaaataa attaggtatt gccaattggt taaaagatca aggtcatgaa 120

ctaattacta cttctgataa agaaggtgaa acaagcgaat tggataaaca tatcccagat 180

gctgatatta tcatcaccac tcctttccat cctgcttata tcactaagga aagacttgac 240

aaggctaaga acttaaaatt agtcgttgtc gctggtgttg gttctgatca cattgattta 300

gattatatta atcaaacagg taagaaaatc tcagtcctgg aagttacagg ttctaatgtt 360

gtctctgttg ctgaacacgt tgtcatgacc atgcttgtct tggttagaaa tttcgttcca 420

gcacatgaac aaattattaa ccacgattgg gaggttgctg ctatcgctaa ggatgcttac 480

gatatcgaag gtaaaactat cgctaccatt ggtgctggta gaattggtta cagagtcttg 540

gaaagattac tcccatttaa tccaaaagaa ttattatact acgattatca agctttacca 600

aaagaagctg aagaaaaagt tggtgctaga agagttgaaa atattgaaga attagttgct 660

caagctgata tcgttacagt taatgctcca ttacacgcag gtacaaaagg tttaattaat 720

aaggaattat tatctaaatt taaaaaaggt gcttggttag tcaataccgc aagaggtgct 780

atttgtgttg ctgaagatgt tgcagcagct ttagaatctg gtcaattaag aggttacggt 840

ggtgatgttt ggttcccaca accagctcca aaggatcacc catggagaga tatgagaaat 900

aaatatggtg ctggtaatgc catgactcct cactactctg gtactacttt agacgctcaa 960

acaagatacg ctgaaggtac taaaaatatt ttggaatcat tctttaccgg taaatttgat 1020

tacagaccac aagatattat cttattaaat ggggaatacg ttactaaagc ttacggtaaa 1080

cacgataaga aatag 1095

<210> 48
<211> 380
<212> PRT
<213> Ogataea polymorpha

<220>
<221> misc_feature
<222> (1)..(380)
<223> Glycerol dehydrogenase

<400> 48

Met Lys Gly Leu Leu Tyr Tyr Gly Thr Asn Asp Ile Arg Tyr Ser Glu
1 5 10 15

Thr Val Pro Glu Pro Glu Ile Lys Asn Pro Asn Asp Val Lys Ile Lys

20 25 30

Val Ser Tyr Cys Gly Ile Cys Gly Thr Asp Leu Lys Glu Phe Thr Tyr
35 40 45

Ser Gly Gly Pro Val Phe Phe Pro Lys Gln Gly Thr Lys Asp Lys Ile
50 55 60

Ser Gly Tyr Glu Leu Pro Leu Cys Pro Gly His Glu Phe Ser Gly Thr
65 70 75 80

Val Val Glu Val Gly Ser Gly Val Thr Ser Val Lys Pro Gly Asp Arg
85 90 95

Val Ala Val Glu Ala Thr Ser His Cys Ser Asp Arg Ser Arg Tyr Lys
100 105 110

Asp Thr Val Ala Gln Asp Leu Gly Leu Cys Met Ala Cys Gln Ser Gly
115 120 125

Ser Pro Asn Cys Cys Ala Ser Leu Ser Phe Cys Gly Leu Gly Gly Ala
130 135 140

Ser Gly Gly Phe Ala Glu Tyr Val Val Tyr Gly Glu Asp His Met Val
145 150 155 160

Lys Leu Pro Asp Ser Ile Pro Asp Asp Ile Gly Ala Leu Val Glu Pro
165 170 175

Ile Ser Val Ala Trp His Ala Val Glu Arg Ala Arg Phe Gln Pro Gly
180 185 190

Gln Thr Ala Leu Val Leu Gly Gly Gly Pro Ile Gly Leu Ala Thr Ile
195 200 205

Leu Ala Leu Gln Gly His His Ala Gly Lys Ile Val Cys Ser Glu Pro
210 215 220

Ala Leu Ile Arg Arg Gln Phe Ala Lys Glu Leu Gly Ala Glu Val Phe
225 230 235 240

Asp Pro Ser Thr Cys Asp Asp Ala Asn Ala Val Leu Lys Ala Met Val
245 250 255

Pro Glu Asn Glu Gly Phe His Ala Ala Phe Asp Cys Ser Gly Val Pro
260 265 270

Gln Thr Phe Thr Thr Ser Ile Val Ala Thr Gly Pro Ser Gly Ile Ala
275 280 285

Val Asn Val Ala Val Trp Gly Asp His Pro Ile Gly Phe Met Pro Met
290 295 300

Ser Leu Thr Tyr Gln Glu Lys Tyr Ala Thr Gly Ser Met Cys Tyr Thr
305 310 315 320

Val Lys Asp Phe Gln Glu Val Val Lys Ala Leu Glu Asp Gly Leu Ile
325 330 335

Ser Leu Asp Lys Ala Arg Lys Met Ile Thr Gly Lys Val His Leu Lys
340 345 350

Asp Gly Val Glu Lys Gly Phe Lys Gln Leu Ile Glu His Lys Glu Asn
355 360 365

Asn Val Lys Ile Leu Val Thr Pro Asn Glu Val Ser
370 375 380

<210> 49
<211> 380
<212> PRT
<213> Ogataea polymorpha

<220>
<221> misc_feature
<222> (1)..(380)
<223> Formaldehyde dehydrogenase FLD1

<400> 49

Met Ser Thr Val Gly Lys Thr Ile Thr Cys Lys Ala Ala Val Ala Trp
1 5 10 15

Glu Ala Gly Lys Asp Leu Thr Ile Glu Thr Ile Glu Val Ala Pro Pro
20 25 30

Lys Ala His Glu Val Arg Val Lys Ile Ala Tyr Thr Gly Val Cys His
35 40 45

Thr Asp Gly Tyr Thr Leu Ser Gly Asn Asp Pro Glu Gly Gln Phe Pro
50 55 60

Val Ile Phe Gly His Glu Gly Ala Gly Val Val Glu Ser Val Gly Glu
65 70 75 80

Gly Val Thr Ser Val Lys Val Gly Asp His Val Val Cys Leu Tyr Thr
85 90 95

```
Pro Glu Cys Arg Glu Cys Lys Phe Cys Lys Ser Gly Lys Thr Asn Leu
            100                 105                 110

Cys Gly Lys Ile Arg Ala Thr Gln Gly Lys Gly Val Met Pro Asp Gly
        115                 120                 125

Thr Ser Arg Phe Thr Cys Lys Gly Lys Thr Leu Leu His Tyr Met Gly
    130                 135                 140

Cys Ser Thr Phe Ser Gln Tyr Thr Val Leu Ala Asp Ile Ser Val Val
145                 150                 155                 160

Ala Val Asp Pro Lys Ala Pro Met Asp Arg Thr Cys Leu Leu Gly Cys
                165                 170                 175

Gly Ile Thr Thr Gly Tyr Gly Ala Ala Ile Asn Thr Ala Lys Ile Ser
            180                 185                 190

Glu Gly Asp Asn Ile Gly Val Phe Gly Ala Gly Cys Ile Gly Leu Ser
        195                 200                 205

Val Ile Gln Gly Ala Val Lys Lys Lys Ala Gly Lys Ile Ile Val Ile
    210                 215                 220

Asp Val Asn Asp Ala Lys Lys Asp Trp Ala Phe Lys Phe Gly Ala Thr
225                 230                 235                 240

Asp Phe Val Asn Pro Thr Lys Leu Pro Glu Gly Gln Ser Ile Val Asp
                245                 250                 255

Lys Leu Ile Glu Met Thr Asp Gly Gly Cys Asp Phe Thr Phe Asp Cys
            260                 265                 270

Thr Gly Asn Val Gln Val Met Arg Asn Ala Leu Glu Ala Cys His Lys
        275                 280                 285

Gly Trp Gly Glu Ser Ile Ile Ile Gly Val Ala Pro Ala Gly Lys Glu
    290                 295                 300

Ile Ser Thr Arg Pro Phe Gln Leu Val Thr Gly Arg Val Trp Arg Gly
305                 310                 315                 320

Cys Ala Phe Gly Gly Ile Lys Gly Arg Thr Gln Met Pro Asp Leu Val
                325                 330                 335

Gln Asp Tyr Met Asp Gly Glu Ile Lys Val Asp Glu Phe Ile Thr His
```

340 345 350

Arg His Pro Leu Asn Asp Ile Asn Gln Ala Phe His Asp Met His Lys
355 360 365

Gly Asp Cys Ile Arg Ala Val Val Thr Met Asp Glu
370 375 380

<210> 50
<211> 362
<212> PRT
<213> Ogataea polymorpha

<220>
<221> misc_feature
<222> (1)..(362)
<223> Formate dehydrogenase

<400> 50

Met Lys Val Val Leu Val Leu Tyr Asp Ala Gly Lys His Ala Gln Asp
1 5 10 15

Glu Glu Arg Leu Tyr Gly Cys Thr Glu Asn Ala Leu Gly Ile Arg Asp
20 25 30

Trp Leu Glu Lys Gln Gly His Glu Leu Val Val Thr Ser Asp Lys Glu
35 40 45

Gly Gln Asn Ser Val Leu Glu Lys Asn Ile Ser Asp Ala Asp Val Ile
50 55 60

Ile Ser Thr Pro Phe His Pro Ala Tyr Ile Thr Lys Glu Arg Ile Asp
65 70 75 80

Lys Ala Lys Lys Leu Lys Leu Leu Val Val Ala Gly Val Gly Ser Asp
85 90 95

His Ile Asp Leu Asp Tyr Ile Asn Gln Ser Gly Arg Asp Ile Ser Val
100 105 110

Leu Glu Val Thr Gly Ser Asn Val Val Ser Val Ala Glu His Val Val
115 120 125

Met Thr Met Leu Val Leu Val Arg Asn Phe Val Pro Ala His Glu Gln
130 135 140

Ile Ile Ser Gly Gly Trp Asn Val Ala Glu Ile Ala Lys Asp Ser Phe
145 150 155 160

Asp Ile Glu Gly Lys Val Ile Ala Thr Ile Gly Ala Gly Arg Ile Gly
165 170 175

Tyr Arg Val Leu Glu Arg Leu Val Ala Phe Asn Pro Lys Glu Leu Leu
180 185 190

Tyr Tyr Asp Tyr Gln Ser Leu Ser Lys Glu Ala Glu Glu Lys Val Gly
195 200 205

Ala Arg Arg Val His Asp Ile Lys Glu Leu Val Ala Gln Ala Asp Ile
210 215 220

Val Thr Ile Asn Cys Pro Leu His Ala Gly Ser Lys Gly Leu Val Asn
225 230 235 240

Ala Glu Leu Leu Lys His Phe Lys Lys Gly Ala Trp Leu Val Asn Thr
245 250 255

Ala Arg Gly Ala Ile Cys Val Ala Glu Asp Val Ala Ala Ala Val Lys
260 265 270

Ser Gly Gln Leu Arg Gly Tyr Gly Gly Asp Val Trp Phe Pro Gln Pro
275 280 285

Ala Pro Lys Asp His Pro Trp Arg Ser Met Ala Asn Lys Tyr Gly Ala
290 295 300

Gly Asn Ala Met Thr Pro His Tyr Ser Gly Ser Val Ile Asp Ala Gln
305 310 315 320

Val Arg Tyr Ala Gln Gly Thr Lys Asn Ile Leu Glu Ser Phe Phe Thr
325 330 335

Gln Lys Phe Asp Tyr Arg Pro Gln Asp Ile Ile Leu Leu Asn Gly Lys
340 345 350

Tyr Lys Thr Lys Ser Tyr Gly Ala Asp Lys
355 360

<210> 51
<211> 1755
<212> DNA
<213> Saccharomyces cerevisiae

<220>
<221> misc_feature
<222> (1)..(1755)
<223> dihydroxyacetone kinase DAK1

<400> 51
atgtccgcta aatcgtttga agtcacagat ccagtcaatt caagtctcaa agggtttgcc 60
cttgctaacc cctccattac gctggtccct gaagaaaaaa ttctcttcag aaagaccgat 120
tccgacaaga tcgcattaat ttctggtggt ggtagtggac atgaacctac acacgccggt 180
ttcattggta agggtatgtt gagtggcgcc gtggttggcg aaatttttgc atccccttca 240
acaaaacaga ttttaaatgc aatccgttta gtcaatgaaa atgcgtctgg cgttttattg 300
attgtgaaga actacacagg tgatgttttg cattttggtc tgtccgctga gagagcaaga 360
gccttgggta ttaactgccg cgttgctgtc ataggtgatg atgttgcagt tggcagagaa 420
aagggtggta tggttggtag aagagcattg gcaggtaccg ttttggttca taagattgta 480
ggtgccttcg cagaagaata ttctagtaag tatggcttag acggtacagc taaagtggct 540
aaaattatca acgacaattt ggtgaccatt ggatcttctt tagaccattg taaagttcct 600
ggcaggaaat tcgaaagtga attaaacgaa aaacaaatgg aattgggtat gggtattcat 660
aacgaacctg gtgtgaaagt tttagaccct attccttcta ccgaagactt gatctccaag 720
tatatgctac caaaactatt ggatccaaac gataaggata gagcttttgt aaagtttgat 780
gaagatgatg aagttgtctt gttagttaac aatctcggcg gtgtttctaa ttttgttatt 840
agttctatca cttccaaaac tacggatttc ttaaaggaaa attacaacat aaccccggtt 900
caaacaattg ctggcacatt gatgacctcc ttcaatggta atgggttcag tatcacatta 960
ctaaacgcca ctaaggctac aaaggctttg caatctgatt ttgaggagat caaatcagta 1020
ctagacttgt tgaacgcatt tacgaacgca ccgggctggc caattgcaga ttttgaaaag 1080
acttctgccc catctgttaa cgatgacttg ttacataatg aagtaacagc aaaggccgtc 1140
ggtacctatg actttgacaa gtttgctgag tggatgaaga gtggtgctga acaagttatc 1200
aagagcgaac cgcacattac ggaactagac aatcaagttg gtgatggtga ttgtggttac 1260
actttagtgg caggagttaa aggcatcacc gaaaaccttg acaagctgtc gaaggactca 1320
ttatctcagg cggttgccca aatttcagat ttcattgaag gctcaatggg aggtacttct 1380
ggtggtttat attctattct tttgtcgggt ttttcacacg gattaattca ggtttgtaaa 1440
tcaaaggatg aacccgtcac taaggaaatt gtggctaagt cactcggaat tgcattggat 1500
actttataca aatatacaaa ggcaaggaag ggatcatcca ccatgattga tgctttagaa 1560
ccattcgtta aagaatttac tgcatctaag gatttcaata aggcggtaaa agctgcagag 1620
gaaggtgcta aatccactgc tacattcgag gccaaatttg gcagagcttc gtatgtcggc 1680
gattcatctc aagtagaaga tcctggtgca gtaggcctat gtgagttttt gaaggggtt 1740
caaagcgcct tgtaa 1755

<210> 52
<211> 1776
<212> DNA
<213> Saccharomyces cerevisiae

<220>
<221> misc_feature
<222> (1)..(1776)
<223> dihydroxyacetone kinase DAK2

<400> 52
atgtctcaca aacaattcaa atcagatgga aacatcgtta ctccctacct acttggcctt 60

gctcgaagca atcccggcct tacagtgatt aagcacgaca gagtggtttt ccggactgcg 120

tcagctccta attcagggaa ccctcctaaa gtttcattgg tttctggagg tggcagtggt 180

catgagccaa cgcatgccgg ttttgttggt gaaggtgcct tagatgcgat tgcagcaggt 240

gccatttttg cttctccttc aactaaacag atctattctg ctattaaagc tgttgaatct 300

cctaagggta ccttgatcat tgtaaaaaat tacaccggtg atattataca ttttggtctc 360

gctgctgaaa gagctaaagc tgctggaatg aaagtcgaac tggttgctgt aggagatgat 420

gtctctgtcg gtaagaagaa aggttcttta gtcgggcgtc gaggtctcgg agccaccgta 480

ttggtgcata aaattgctgg ggcagccgct tctcatggac tggagttggc agaagttgcc 540

gaagttgctc agtcagtagt tgacaatagt gtcacaattg cggcatctct tgatcactgc 600

acggttcctg gccacaaacc tgaagccatt ttgggcgaga atgagtatga aatcggtatg 660

ggtattcata acgagtctgg tacctataag tcttctccgc tgccatcgat ttctgagctc 720

gtttcccaga tgcttcctct tcttctcgat gaggatgaag accgttctta tgtgaagttt 780

gagcccaaag aggacgtagt tcttatggtt aacaacatgg gtggtatgtc taatctagaa 840

ttgggttatg ctgcagaggt catttctgaa caattgattg ataagtatca aattgtgccc 900

aagagaacga ttactggagc attcattact gcattgaatg gtcctgggtt tggtatcact 960

cttatgaacg cttcaaaagc tggtggcgat atccttaagt atttcgatta tcctaccaca 1020

gcgagtggat ggaatcaaat gtaccattct gccaaagatt gggaggtact tgccaaaggg 1080

caggttccca ccgcccctc tttaaagaca ttgaggaatg aaaaaggttc gggtgtgaaa 1140

gctgattatg acacttttgc taaaattttg cttgctggga ttgcaaaaat taacgaggtt 1200

gaaccaaagg ttacttggta cgataccatt gcaggagatg gtgattgcgg aactactctt 1260

gtgtccggtg gtgaagcatt ggaagaagct attaaaaacc atacgttgcg cctcgaggat 1320

gctgctcttg gtatcgaaga tattgcgtat atggttgagg attctatggg tggtacgtcc 1380

ggtggtctgt actctatcta tctttctgct ctcgcacaag gagttaggga ttctggggac 1440

aaggaactta ctgcggaaac tttcaaaaag gcatcaaacg ttgcactaga tgctttgtat 1500

aagtatacga gagcccgtcc tggttacagg actctgatcg atgctctgca accttttgtc 1560

gaagcgctga aagccgggaa gggtcccaga gccgccgccc aagctgctta tgatggtgcc 1620

gaaaagacaa ggaagatgga tgcccttgtt gggcgtgctt cttacgtggc taaggaggag 1680

ctgagaaaac tcgacagcga aggtggatta ccagatccag gagcagttgg tcttgctgca 1740

ctactcgatg gatttgttac agctgctggg tactag 1776

<210> 53
<211> 2999
<212> DNA
<213> Saccharomyces cerevisiae

<220>
<221> misc_feature
<222> (1)..(2999)
<223> upstream sequence of DAK1

<400> 53
ctttctagtt cctctgggaa aggaatctct aagtcatttg tatcatacaa gaagcttttc 60

agaaagacat tcaaggacat gtagacatgt tccctgagga cttcaatttt cctcttaacc 120

atcatttcaa ttaaagattc attagaagac tcaacgtcca aaggatatga tgctatttca 180

tcggtaatga atttgtatct catggctaaa aacacactta ataatgattt attagtcttt 240

ccatcaaatg gtggaattga attcaagtac tgtagtattt tctttagtga gtttactgat 300

acattagtag acagtaattt taccagcccg gataacatgg tagtactaat ttcgtttaaa 360

actttttcgc aaacttcatc cacaatggtt gaaccaggga atcttgacct cagggaagta 420

gtgtgtgtgt atagcatgac ggcctcctgg taatgacctg ttctgataca tgtgcgggcc 480

aaaaatggta gttccatcaa atccgtaatg ctatccaaat tttctagtac agtgactaat 540

gtgtcagatc taatgtcgtc cttgtcatct tctttagttg aaattctgtt tcttaaccta 600

cttaatgcct tgtgaaactc atcctccttc ttctttcttg ccagattgtt actctcagtc 660

gtcatgatgc gacctgtgtc gttgtcttct ttatcgtctt ccaaaaagtc atcaatagaa 720

acgctttcat tgtttatatc gtcatttgta acgtttcgat cggctgcctt attaatatta 780

gtatccaatt cccataattg ctccaaagac ttggctatat catccaattg agccctatca 840

tcgttttcaa gaatatttcc gataatctga gacgtattat ctagtagtgt tttccttatc 900

tttctatcaa gggcagataa ttcagcatca atttctgcta tgtcttcagt tatgctacca 960

ggaacagccc tagatgaaaa ataactttca tagtctttcg tgttagattg taggatatcc 1020

tgtaggaaat caagacttaa ccgtttttgt tcctctgtta aatcatcaga aatgaggctg 1080

tttagtatca gctccatttt actcaatgat tatgtttatt gttgaaatat gttccctcaa 1140

atgtcctaac acttctatga ttattttttc tgtgcttctc ttttcagtat gttactacgc 1200

tatattttta gacattgaag ccatgatcgc gagatcgatc taatgtacgt ataaaaagaa 1260

aatggacttc aagagtacaa ctaactaaag gaaaatccaa ctcttcctat aaatttagaa 1320

ttagcatatt caaaaaagaa gaaacaaagc actcacgatg ggggtcgaac ccataatctt 1380

ctgattagaa gtcagacgcg ttgccattac gccacgcgag ctaaatttct tgaattgttg 1440

ggtaaacaaa tattactaat acaaatgtta ttagaaacca aaaatgcact tttccggggt 1500

taatatatat gattgagtga tggatgaaga atgagataat tgtttaaatt ctatagttgt 1560

caagcgctgt gataccagat acacaaaatg tactagaggt tctcctcgag aatatgaaaa 1620

tccacaatag agaaccgata tttctgtgta ggaatattat tatttcttct ttcattttgt 1680

atgttctcgt tcattttcct agcacattat caaccctttc atttcaattt ccattaaaat 1740

tggtgactgt ttctcaatat ttatttgacc gtcttatacc caatatggta atataccagt 1800

aatataaata ctagtcgtta gatgatagtt gcttcttatt ccgaaaatga gtatggaagt 1860

gttgcatatg atagggcggc tacagtgatg gtaaacataa gatactttag cgggaaatta 1920

gcaactggaa gttaaattat ctagacataa gtgtggcggt cacgctgaac gcaggagatc 1980

ggatagattg ataagctgat caagaacatt gatcggtttg ttgtttaaag aatggttttt 2040

gaaaacgttt gaccagttgc ttctcccaga cgcttaccga tatgatgata aagataatat 2100

cttcaattga ataccccgtg gatcagcacg aataacagaa aaaaagggtg aaattcaccg 2160

taagcatgat acgcactacg ttcttcttac ctttgccaac gtgttgtctt tgacgtacgt 2220

aattatggga gatcgttgat gattagcccc agctcacttt cttcttaatg actgacccgc 2280

tactatcaaa attaaggtgt caaatatcat gatgaatgag gtctctaggc gactcaatta 2340

tacatctttt agagattttt ttactacttg cagataattt ctcaagggat tagattcaaa 2400

tctggcttgt caattacgcc cttttcaagc tcatcaaatt gcgtatgtca ttcatgcttc 2460

cattaggaac catagaagca tggctgaaat ggcaatatac ggcttcccaa tttcaactct 2520

aaagtaatgg cggtcgaatt taatctatat tttacagttt tatacgtact ttaaaagcaa 2580

tcagtaaaca cctctggtgc tattcaaggg ttttttgcct ttatttgtta ctgtcaattg 2640

tctggcgctg tgataaaaaa caaggcataa agctccccg tcatgaacat taagactcgc 2700

tagacgagag agtgaaatat aatgcatttc ctgatttaaa tgcgctacaa acatggtgta 2760

aatctggccc ggagtgagtg cttgccaatt tggcttctaa gggagaaaga tcaaaccact 2820

cccaattgcg tcattttgaa agagtggcca cctcgcgagc gtctgtcgaa ctaactgatg 2880

aataaatata taaggagaaa atcacttcaa cttcgctaca agtagtcact atttgtagca 2940

actgtaaacg aacacatcaa agaataagat tacattctat atctaagact aaattttaa 2999

<210> 54
<211> 3000
<212> DNA

<213> Saccharomyces cerevisiae

<220>
<221> misc_feature
<222> (1)..(3000)
<223> downstream sequence of DAK1

<400> 54

```
gtacttggct cacgaataca tatcaagata cttatgatat atatatatag aaaaagctta      60
cttttcttgg agttattgtt attatcatcg cgaagaacga ttgtataacc cggttcaacg     120
cgaaacgaat cgttaaactg gtgaaatgtt aacgcgagtg tcagagatat acatagtatg     180
agagtagcta gatgttgaat cggtggtaag aacaagaagg aaataccgtt aacaagtgaa     240
ggaacaatct agtattgttg aacaagaatt atgagtaccg actttgatag aatttacttg     300
aaccaatcta aatttagcgg tagattccgt attgctgatt ctgggttagg gtggaaaatt     360
agtaccagtg gtggctctgc agcaaatcag gcaagaaaac catttttatt accagccaca     420
gaattatcta ccgtccaatg gagtaggggc tgcaggggtt acgacttgaa gataaatacc     480
aaaaatcaag gtgttatcca actagatgga ttttctcagg atgactataa cttaatcaag     540
aatgatttcc atcgccgttt taatattcag gtagagcaaa gagaacattc cttacgtggt     600
tggaactggg gtaagacaga ccttgccagg aatgaaatgg tttttgcttt aaatggtaaa     660
ccaacttttg aaattcctta tgctagaata aataatacaa atttgacctc taaaaatgaa     720
gtaggaatag aatttaatat tcaagatgaa gagtaccaac cagccggtga cgaattggta     780
gagatgaggt tctatattcc tggtgttatt caaacaaacg tcgatgaaaa catgaccaaa     840
aaggaagagt caagcaacga ggtcgtacca aagaaagaag atggtgctga aggagaagat     900
gtacaaatgg cagtagagga aaagagtatg gcagaagcat tctatgaaga actaaaggaa     960
aaggcagaca tcggggaagt cgctggtgat gcaatagttt ccttccaaga cgtctttttt    1020
accacgccaa gaggtcgtta tgatatcgat atttacaaga actccattag actcaggggt    1080
aagacctatg aatacaaatt gcaacatcgt caaatacaaa gaattgtttc gttaccaaag    1140
gcagatgata tccatcactt attggttttg gcaattgaac ctcctttacg tcaaggacag    1200
accacctacc cctttcttgt cttacaattt cagaaagatg aggaaacaga agtgcaattg    1260
aatctagaag atgaagatta tgaggaaaat tataaggata aattgaaaaa acaatatgat    1320
gctaaaactc atatagtttt aagtcatgta ttaaaaggtc tgactgaccg tagagtcatt    1380
gttcctggag aatataaatc caaatatgat cagtgtgcag tttcatgttc tttcaaagca    1440
aacgaaggtt atttgtatcc attagataac gctttcttct ttttaactaa gccaactttg    1500
tacataccat tcagtgatgt tagcatggta aacatttcaa gagcaggaca aacttctacg    1560
tcatcgagga cgtttgattt ggaagtggta ctgcgttcaa atagaggttc taccactttt    1620
```

gccaacatca gtaaggaaga gcagcaatta ttggaacaat tcctaaagtc taaaaaccta 1680

agggtgaaga atgaagatag agaggtacaa gaaaggttac aaaccgcttt aggttcagac 1740

agtgacgaag aggatattaa tatgggttcc gctggtgaag atgatgaatc agtagatgag 1800

gattttcagg tcagctctga taatgacgca gacgaagttg cagaagagtt tgattcagat 1860

gcggctttaa gtgatgctga gggggtagc gacgaagaaa ggccttcgaa gaagcctaag 1920

gtagaatagt aataatttta gactgtataa gttaaattta ttgatattgt gtaaaaacta 1980

actaatatat tttgccaatt gatattatca tgacatggtg agtgtaagac accacctctt 2040

aattactggt gttattctat acatttattt gaaattggtt ttgttttgca aaatatttat 2100

gttttgttaa tctcctctac cctttcaatg cttgaaaaat actttcaact tttcgattgg 2160

gtgatgaaaa aaagacaaat agtgtaaagg gttcaaaaat aaataacaag caagagaaag 2220

ggactttgct tttctcattt agtcaccagt aagttatgtc atggtgtaga ataacgaatt 2280

acagaaaact aatataactg atgaaagacc agggagtaaa atggctttga ctcagtttga 2340

aaatgatttg gaaatattaa gagatatgta cccagaactg gaaatgaaat cggtaaaagt 2400

agaggaggaa ggtgaattcc ctcaaagaat taacggaaag ttactgttca agatatcact 2460

attggccgat gtaaatattg agttcggcga gcaacatatg ttactttcaa acttatctaa 2520

tgaatgcgtg gagttcacca tatatagctg tcattatccg gacattcgac ggtgtgttgt 2580

tatggatatc aaatccttat ggatatcaac agatgaaaag aagatgttaa ttgacaaagc 2640

gctgagactc gttgaagaaa ctgtagatat gagtattgag ttcgcggatt cgtttacctc 2700

catccttatc ctcatctttg ggtttcttat agatgataca gctatattac tattccctaa 2760

tggaataaga aagtgcctga cacaggatca gtatgacttg tttaagcaga taagtgagga 2820

agccaccctc caaaaagtga gcagatctaa ctaccattgt tgtatttgta tggaaatgga 2880

aaagggtgtt agaatgatca aattgccatg tgaaaatgcg aatgtagaac actatctttg 2940

cagaggatgc gccaaatctt atttcactgc aatgattcag gaaaaccgaa tatccagtgt 3000

<210> 55
<211> 3000
<212> DNA
<213> Saccharomyces cerevisiae

<220>
<221> misc_feature
<222> (1)..(3000)
<223> upstream sequence of DAK2

<400> 55
gataatgaca ataattttct ttgagcacaa ttagtttatt tggcaacttg ctctttatta 60

ttagtaaata tagcacatat tcatataaaa cacatcttca gtgggattac ctagttgatg 120

gtgccaggaa ttttcctatc gtcaacgagc tgaaaatttt attattttta ttaataacat 180

aatgtgagac tcctggtttg acatttaatt ttacgtatta gtcgaatttt gttcttgcct 240

acaataaaaa gacaattaag ccgcaggcag tctcattctt tattacaaaa acaaaacgat 300

agaatttaga gcacaagtaa gagatggtaa caaagtcacg gctcccggat gtagtatgtc 360

gtcaaataat aagttcgtga aattaataat taggttataa atcgtaaaaa attgaaaata 420

ttaattatga cgaagtaggc acagatttct tgctgccagt gttgctgttg ctgttaacac 480

cagattcatc tgagacagtg ccatcattct ggtggaactc cgcatgtaaa agtttaccta 540

ccctattctc aatataaccg ttgtctgggt agttgactgg ggattcgcat ccagtaaaaa 600

tgaaaatgtc atatatgagt gctccagcaa taccgccggc aattggacca ccccaggctc 660

cccatgtcca ccaccaatgt gtgagatgaa aagcatgtgg accatagcca atcatggaag 720

caaatatgcg aggaccgaga tctcttgcag gattgattgt gaaacttgtt tgatatccaa 780

gggccatacc aattgcagcg actaagaatc caataattaa tgcggtcata ccattgccag 840

gtggagcatt actatcatcc aatagcgcca tcaaacaacc cacaagtata gaggctccta 900

tgaattcgtc aaagaaggca tttctccacg tgacgtaaga ctttggatca gtaaacaaac 960

acgcaccggt cgccgttgtt cttatgtgcg gacctccctc aaattctgtg atagagctcc 1020

aaaaataacc ataagccata gctcctccaa aatatgcacc gataatctga gcaacaatat 1080

atacgggcac ctttttccag ggaattttc gaaaaattgc cattgaaatc gtaacagcag 1140

ggttaatatg accaccacta ataccgcctg cgacgtaaac accaagcata caaccgaacc 1200

cccatgcaaa tgatagggat tcataggaac caccactacc ttttgttaca gttgcttgaa 1260

gattaccacc aacaccaaaa atgacaagaa ctagtgtccc gagaaactcc gcaaacggtt 1320

ctcgcatatg atagcgaatt tttgcccaaa agttaggaaa tgtcattata tccgcgtctt 1380

catcttctga ggcaccaatt tcattaccat ctaatgcact cgtactttta ttttcctctt 1440

caataagttc ttcaggaagc ttcgtataaa ccggagtaga accatcgagg gtttgcatgt 1500

tctttaattt tctggattct gcatcggcta tggaggatgc gtagctttca tcgcctaatg 1560

aaaaattgac attatgcgaa gtacgctttt tcttacgtga aacattctca atatgcgtgt 1620

tagagggctt ttgtgggttt tcaggctcta acttagtagg tttcacatct gcaccaacag 1680

tattatcgcg ttgcgtttga gcttcgatgt caccaagctt tttacccggt gctgcacctc 1740

taaggagcct tttccagtct ttcactgacg tagtagaacc ccccttgaa atatcggtcg 1800

accctcgtct cgatgatgcg cgggaccttg catccatatt cttttgtatc atctttgctg 1860

ccaagcctcg atccaataca tgcggtaacg gctgatttaa actccaaaca ggcctgttcc 1920

tgctactacc catagttgga tttaattgcc gatatagtgg gtctacataa ttattactgt 1980

tgccttgatt tacccccctg gatagataat cttgcattat acgtgcttgc tcagcatctg 2040

aaaaactgtt taaagtcttc aaattaggca aagaataagt aaaacccatg gaagggacct 2100

gtggttgtac cgaatccttc ctctcgtcaa ccactttttt caagggtgtc aaagcagaaa 2160

gtttatttct cgatgatcct ccagtttcaa ttgcttttcg agtctcacct agctttcgcc 2220

tcaagagagt gctgtcattt tcgttatttt ccctagattt tttgacactt tcttcccaag 2280

ctattttacc attaggttct tcttttagcg ttggtggccg tgtactctcg gaagaggagg 2340

atgacctccc agattcgtaa ctcattactt gggtctagat catatatcag aggagcgtta 2400

tactgtgcga ttatacgctt ctttttatat gaataagggg gagacatggt gaaaaggtac 2460

cagaactttt gatcgaccaa gactaggtaa agctcaaaca acgtttataa ctcaaatttc 2520

cggggtaagt ggggtaccgg aaaattatga tattccggag cggagttatc aacggagaaa 2580

actaggcctt ctgatggaac ttaatttaaa aaattaatca caacctatgc atattattcc 2640

cgcagagggt gattgtgagt aaatccctgc acagaaacaa ttcccgccag gccataacta 2700

gattctaaat tatttaactc ataatttcat gaaatcgtat cgtagtacca aatagggaga 2760

tattgagcca agtaaattct tacgtcacca tagttggata attaagtact tgatattgta 2820

taaggatctc aacaatacga gaaggggaaa ataccgcaat gtgtgattga attttcaaac 2880

tttggatcat taaatatata taaatgaacc cagatcagcc cttttttttt ctagtattgt 2940

ctgtaaagtg tattttacct caaaatctga caaaacccaa ctacaattga ctaaataatc 3000

<210> 56
<211> 3000
<212> DNA
<213> Saccharomyces cerevisiae

<220>
<221> misc_feature
<222> (1)..(3000)
<223> downstream sequence of DAK2

<400> 56
aattgctcgt acacactaga agccaaacat aacagcttta aaggctttca tttttgaact 60

ttttaaaaaa ttgaatactc caactgaagg tgaactagtt gtgtctctga atatattttt 120

atagatatac gaattgatga agtaccgcaa attaagctaa aaagtaatgc ttcttgcagc 180

ttttaattgt tctttctgca atctacaatt acttttcttg attccttctc cgttcccctg 240

tgttgtctgg aagtataatt tgtccaggaa gattttttga atagccattg ttttctttaa 300

attaaatcgg agtgtttaaa tccattccaa tctctttttt ctcgcaagtc aacaaacagg 360

tgttaacttt cttttccccg ctgttttctt acctatgaat agtctcaatt cctttttaga 420

agatctgcac attctctgat actatgaaca agttctagga tagcaatcta agttttatga 480

ttctcttatt tcggattcga tttcaataaa gatcgtagta ttagaagtat agaatgtatt 540

gtaatttttt ttcctaatct tattaattca tggaaggcat tgaactcaac agcatatttt 600

aaatgtttgt atcttgtttt ctctttcaaa aaaaaaatgg tgtcattcat tattttatgg 660

tcaaccctat acatcaattt ttctctgaaa atattgacaa ataaagtagt tgattcttgt 720

tctaccaatt agtgatatta tgcatgactg ttaacaactt tttgactaat ctctgaaatc 780

atatgaagat cttgctgcat ttcatgcatc taagaaatca acctatatca acagatttca 840

ataattactc taaacttatg ctgtaactta gaaagtaacc agcctgtgtt gactgattga 900

gttgcgtatt aactgcgcct agtcatttca acacttataa tttgcttcag cttaagtgtg 960

gttcatcttt ttttttctgg aaactttgca tgccctcaaa gcatgagtag ttagttatct 1020

ttttgacaat gatctctttt gaaaatatct actgtagatt tgcatggacg cacgtcgccc 1080

atacgccaaa ctttggcaat gatactcgtt attcgtaata tcagtccgtc aaggtgctgt 1140

gatttctcta ttttatattg cctattattt tttcaaatga tttgagccgt tttaaattga 1200

gtatgcaatg agtcttttga atcaaccgta aggcagttcc ataaccactg ccacgaatac 1260

gtttcactac cttgaagaat ctctaatgta ggccgtattc ttcgcactta gttctgacga 1320

tgtagacatc tcattatata agagcataag cgcctgtttc tagaatcatt tcttcgtgac 1380

ccagcttttt gagttatttc gcggtatttt gaaacatttc tcgagcttga cgtgaacatc 1440

cttatatttc atgacaaact cgatcattgg aacatccctg cctcgatttt agagctagta 1500

tcaaatttca atctctttgt gatggagccc cgctcctatt tcaaaagaga agtttcttgt 1560

atgcatatgt tattgaagtc tgattatagc aagtgcaatg tcgtctcaat tattttaact 1620

atttttagcc atacatgtta gttatcctca aagagagcct ccagactggg aagcagtgtt 1680

tgtcatttca aataagtaga tttcacagtt tgtatgattt tcgaagccag gattcattgg 1740

gctttgagta aagagaagcc gcgtattacg aacagcttac gatattgtaa aatattccct 1800

tattgtggtg ccccaatgga tacatgccag agaaatgtct gtgaaattga acaattacaa 1860

tgacgagagc aagtaatccg gcggccttgt ctctctttca ctagtaccgt ctatatctct 1920

tgagcgccaa tatgcgaaag ctttcacaag gttgatgttc atggtattcg gcgtcgatag 1980

cgaattgctt actaagaaac attagggtgc agtacagcct tgtttttcca gttcgactaa 2040

ccttttttctt ggcagtatgg agactgacta ggtctcccaa acattcattg taactgctgt 2100

ttaaagattt tgttctaacc taaattcaag tgagaagctg aacatgtgtc tctacttatg 2160

atatcacgac agcaaatact aatcttgcca taaatagtct agcgttttgc aacttacctc 2220

tagatatatt ttatttcttg aggaaccgtt ttcgtcggta ataacaaaat actactgaaa 2280

cgccacagca ttgagagaat acgttatcga ttacggcttt cttctcgctc cagatgtcgc 2340

gggtaagata ttcacctcaa acttttcttg ttgagtgtcg tcacaaatct agaacctaca 2400

tgccatctca acgatttttc tggagaaagg cctcactccg ttccgtacgt aatgcataga 2460

taaagtatca ggatcttcac gatgctcgag agttacttag tagtctgagt ttatgcgaaa 2520

aaaactccgc cgttgtaata atcgggaata cacagaagta gtactgcact atcactggga 2580

tactcaaaaa ccttcttttt aacttttcta tcccacaaat agaacatagg aaagaacatt 2640

gactcctcca cttgaagtta aattacagga acaaacgcct aactataatt tcgacattgt 2700

tgcatcaacg aatcgaccga aagaaaaatc tggagttgca gttatcactt gtatgtgcac 2760

taagatttat atttttactc ctgagatctg ccaaatcggt agcttattga actgcgttcc 2820

tttttcccct gagttctcga ggtacctgcg gctttgtctg tgccatctcc cccactttaa 2880

agtaccccac gttactaccg cgtttttccc cacccccggc ttaataaatt agctatatct 2940

tgttgactta aatacggaga aaagaagaaa accttcaaga aatgcttcat tgtcttgtca 3000

<210> 57
<211> 2028
<212> DNA
<213> Zygosaccharomyces bailii

<220>
<221> misc_feature
<222> (1)..(2028)
<223> ACS

<400> 57
atgacagtca aagaacacaa ggtagtgcac gaggcacaaa acgtagaagc gctgcatgcg 60

ccagagcatt tttacaagtc acaaccaggc cccagctaca tcaaggacat gaagcagtac 120

aaggagatgt acaaacaatc tgtggaagac ccagaaaact tctttggtga aaaggctagg 180

gagctgctgg attgggacag acctttttacg agaagcaagt acggttcgtt ggaaaatggc 240

gatgtcacgt ggtttttaaa tggtgaattg aacgcagcct acaactgtgt tgacaggcac 300

gcttttgcaa acccagacaa gcccgcattg atctacgagg ctgacgagga ggctgacaac 360

aggatgataa ccttcagcga gctgctgaga caggtttcgc gggtcgctgg ggttctacaa 420

agctggggag tgaaaaaggg cgacactgtg gcagtgtact tgcccatgat tcctgaagcg 480

gtggtggcca tgttggccat tgcaagactt ggtgccatcc actctgtggt gttcgctggc 540

ttctctgctg gctctttgaa agaccgtgtg gtagatgctg gttgtaaagt ggtaatcacg 600

tgcgacgaag gtaagagagg cggtaagaca gttcacacta aaaagatcgt ggacgaaggt 660

ttgaacggta tcagccttgt ctctcacatt cttgtcttcc agagaaccgg gagcgaaggt 720

atccccatga ccgccggtag ggattactgg tggcatgagg agaccgccaa gcagagaagt 780

tacttgcctc ctgtgccttg caattccgaa gatccattgt tcttgctata cacttctggg 840

tctacgggct cccctaaagg tgttgtccat tctaccgccg gttacctttt gggtgccgct 900

atgaccacca gatatgtctt cgacatccat ccagaagacg ttctctttac cgccggtgac 960

gttggctgga tcactggcca cacctatgct ctatatggcc cattggttct cggtacggcc 1020

agtatcatct ttgaatctac ccctgcctac ccagattatg gtaggtattg gagaattatc 1080

cagcgtcaca aggcaacaca tttctatgtg gctcctacag ctttgagact catcaaacgt 1140

gttggtgaag ctgaaatccc caaatacgac atctcgtcgc ttcgtgtgct tgggtctgtt 1200

ggtgagccca tctccccaga gctttgggag tggtactatg aaaaagttgg taacaaaaac 1260

tgtgtcattt gcgatacgat gtggcagaca gaatctggct ctcatttgat cgcccctcaa 1320

gctggtgcag ttccaacgaa accaggttcc gccactgtac ctttctttgg tgtggacgct 1380

tgcatcatcg atcctgttac tggtattgag ttgcaaggca acgatgtgga aggtgtccta 1440

gcggtcaaat cttcctggcc atcaatggct cgttctgtct ggcaaaatca tcaccgttac 1500

gtcgacacat atttgaagcc atacccaggt tattacttta caggtgatgg tgccgggagg 1560

gaccacgatg gctactactg gattagaggc agagtggacg acgtggtaaa tgtctcaggt 1620

cacagacttt ctacagctga gatcgaagcc tctttgacca atcatgataa tgtctctgag 1680

tctgctgtag tcggcattgc tgatgaattg acaggtcagt cagttattgc ctttgtctct 1740

ttgaaggacg gttcttccag ggaatcttct gccgtcgtag ctatgcgtcg cgaattggtt 1800

ctccaggtta gaggtgaaat tggtcccttc gcagccccta agtgtgtcat tttggtcaag 1860

gacttgccca aaaccagatc aggcaaaatt atgagaagag ttctaaggaa agtggcctct 1920

aacgaagcgg accagttggg tgatctatct accatggcga actccgaggt tgttccatct 1980

atcattgccg ctgtagatga acaattcttt gctgagaaaa agaaataa 2028

<210> 58
<211> 1773
<212> DNA
<213> Acetobacter aceti

<220>
<221> misc_feature
<222> (1)..(1773)
<223> ACS

<400> 58
atgcttccat ggacgacata cgaggcgatg tatgacgcag ccctgaacca gccagagcag 60

ttctggctgg ctgcggcaca gcgcgtcaca tggaagcagg cccctgtgac cgcatgcagg 120

acacggtcgg atggctggca tgactggttt cccgacgcca cgctcaatac ctgccataac 180

gccgtggacc ggcatgtgga gaatgggcgc ggagggcagg cggcattgat ctggcattcc 240

tgcgccacca gggaacgtca ggttataacc tacagggagt tgcagagcag ggttgccgga 300

tttgccggtg gtctgcgctc gttgggggtg gagaaaggcg agcgtgtcct gatcgccatg 360

ccgaccatga tcgagacggt catcgccatg ctggcctgtg cacggatcgg cgctgtacat 420

gtcgtggtct ttgccggtta cgctgggcct gaactggcgc gacggatcga tgatgcggca 480

ccgaaagtca tcatcatcgc cagttgcagc tttcaggggc agacgcccgt tccgtccgtg 540

cccgccctga acgaggcgct ggctgcggcg acgcactgcc cacaggcctg cgtgatcgtg 600

cagcgcgaag cgtgcccggt ttcgcttcta ccggtgcggg atcatgattt tcacacgctg 660

gaacagtccg caccagcaga gccgctcatg ctgcgctccg aagatcccct gtatattctt 720

cacacgtccg gcacgacggg caatgcgaag ggcattgtgc gtgacaatgg tggccatgct 780

gtcgctctcg ccttgtccat ggatctgatc tacggctgca aacccggtga taccttcttc 840

acgacatcgg atctgggttg ggtggtcggc cattcctatg gcgtctatgc gccgctgatc 900

agcggctgca ccagcgtgat tgtggaaggc ggtgcttcag cttctgcgat ccgcatgctc 960

tgtcacgaac acgcagtgaa atgcctgttc accacaccaa cacagatgcg gctgatgcga 1020

caggagagtc gccatctgtc aggggcgata ctgcccgcgc tggcccgaat cttcgtggcc 1080

ggggagtatg ctgacccaac attgctggag tggacgcggt cctatttccg caaacccgta 1140

gtcaatcact ggtggcagac tgaaaccgga tggagcatca ccgcgcattt ttttggtctg 1200

cccgagcgtg agccggtctc gctcatgaat gacatcgggc ggcctgcacc gggattctgt 1260

ccggccattg tgccgtccat agccgatgag cagtatgggg agatcgtcct ttctttgccg 1320

ttgccacctg gttgtctcgc tgggatgtgg aaggatggtg ctatccgcct tccgtccact 1380

tatcttgatg aaataggtag atattaccgc acctttgatg aaggtatgat cgaggccaac 1440

cgcgccgtgc atatgctcgg gcgttctgac gatgttatca aggtcgcagg ccggaggatt 1500

tccggcgtac agatcgaaaa gatcattgcc acccatccag ccgttcatac ctgcgccgtg 1560

gtcgcgatcc ccgatgaact gcgaggccag cgacctgtcg cctatgtggt cgttgaccct 1620

gaggcctcct gcgaaccatc ttctgaggaa atcgtcgtgc tggtcaacga agtcctcggg 1680

cgttgggttg gtctgaagga agtccgtttc atcaggcatc taccgaccac ggtatctggc 1740

aagatcacaa ggaaacgtct gctggtgtcc tga 1773

<210> 59
<211> 1401
<212> DNA
<213> Escherichia coli

<220>
<221> misc_feature
<222> (1)..(1401)
<223> udhA

<400> 59
atgcctcaca gttatgacta cgacgctatt gttataggtt caggaccagg tggagaaggt 60

gcagccatgg gattagttaa acaaggggcc agagttgcgg tgatcgaaag ataccagaac 120

gttggaggtg gctgtaccca ttgggggacc atcccttcta aggctctgag acatgctgtc 180

agtagaataa tcgagtttaa tcaaaatcct ctttactcag atcattctcg actactaaga 240

tcttcatttg cagacatcct gaatcacgca gataacgtaa tcaatcaaca aactaggatg 300

agacaaggtt tttacgaacg taatcattgc gaaattctac aagggaatgc tagatttgtg 360

gatgaacaca ctctggcgtt agattgtcca gacggtagtg tcgaaactct tacagcagaa 420

aaattcgtca tagcctgtgg ttcaagacct taccatccaa cagatgttga tttcacacat 480

cctagaatct acgactccga ttctattctg tcaatgcacc atgaaccaag gcacgtattg 540

atatatggtg ctggagtcat tggttgtgaa tacgcaagca tctttagagg catggatgtt 600

aaagtagact tgattaatac aagagacaga ctccttgcgt ttttagatca ggagatgtct 660

gattccctct cataccactt ctggaactct ggtgtagtga taagacataa cgaggaatac 720

gaaaagattg agggttgcga cgatggtgta atcatgcatc ttaagtctgg caaaaagttg 780

aaagcagatt gcttattgta cgctaatggc agaactggca acacagactc tttagcatta 840

caaaatatcg gcttggagac tgattctcgt gggcaactaa aggttaattc aatgtaccaa 900

acagcccagc cacatgttta cgcagttggt gatgttattg gctatccaag cttagcatcc 960

gcagcttacg atcagggtag aatagctgcc caagccctag ttaagggcga agctacagca 1020

cacttaattg aagatatccc aaccggaatc tacacaattc cagaaatttc ctctgtagga 1080

aaaactgaac aacagcttac ggctatgaaa gtcccttatg aagtgggtag ggcccaattc 1140

aaacatttgg caagagccca aatagtcggg atgaacgtgg gaacattgaa aatcttgttt 1200

cacagagaaa ctaaagagat tttgggcatt cattgttttg gagaaagagc tgctgaaatc 1260

atccatattg gacaagccat catggagcaa aagggcggtg gtaatactat cgaatacttc 1320

gttaacacca cattcaatta tccaacgatg gctgaggctt atagagtggc tgctctaaac 1380

ggtttgaacc gactgtttta a 1401

<210> 60
<211> 466
<212> PRT
<213> Escherichia coli

<220>
<221> misc_feature
<222> (1)..(466)
<223> udhA

<400> 60

Met Pro His Ser Tyr Asp Tyr Asp Ala Ile Val Ile Gly Ser Gly Pro
1 5 10 15

```
Gly Gly Glu Gly Ala Ala Met Gly Leu Val Lys Gln Gly Ala Arg Val
            20                  25                  30

Ala Val Ile Glu Arg Tyr Gln Asn Val Gly Gly Gly Cys Thr His Trp
        35                  40                  45

Gly Thr Ile Pro Ser Lys Ala Leu Arg His Ala Val Ser Arg Ile Ile
    50                  55                  60

Glu Phe Asn Gln Asn Pro Leu Tyr Ser Asp His Ser Arg Leu Leu Arg
65                  70                  75                  80

Ser Ser Phe Ala Asp Ile Leu Asn His Ala Asp Asn Val Ile Asn Gln
                85                  90                  95

Gln Thr Arg Met Arg Gln Gly Phe Tyr Glu Arg Asn His Cys Glu Ile
            100                 105                 110

Leu Gln Gly Asn Ala Arg Phe Val Asp Glu His Thr Leu Ala Leu Asp
        115                 120                 125

Cys Pro Asp Gly Ser Val Glu Thr Leu Thr Ala Glu Lys Phe Val Ile
    130                 135                 140

Ala Cys Gly Ser Arg Pro Tyr His Pro Thr Asp Val Asp Phe Thr His
145                 150                 155                 160

Pro Arg Ile Tyr Asp Ser Asp Ser Ile Leu Ser Met His His Glu Pro
                165                 170                 175

Arg His Val Leu Ile Tyr Gly Ala Gly Val Ile Gly Cys Glu Tyr Ala
            180                 185                 190

Ser Ile Phe Arg Gly Met Asp Val Lys Val Asp Leu Ile Asn Thr Arg
        195                 200                 205

Asp Arg Leu Leu Ala Phe Leu Asp Gln Glu Met Ser Asp Ser Leu Ser
    210                 215                 220

Tyr His Phe Trp Asn Ser Gly Val Val Ile Arg His Asn Glu Glu Tyr
225                 230                 235                 240

Glu Lys Ile Glu Gly Cys Asp Asp Gly Val Ile Met His Leu Lys Ser
                245                 250                 255

Gly Lys Lys Leu Lys Ala Asp Cys Leu Leu Tyr Ala Asn Gly Arg Thr
            260                 265                 270
```

Gly Asn Thr Asp Ser Leu Ala Leu Gln Asn Ile Gly Leu Glu Thr Asp
275 280 285

Ser Arg Gly Gln Leu Lys Val Asn Ser Met Tyr Gln Thr Ala Gln Pro
290 295 300

His Val Tyr Ala Val Gly Asp Val Ile Gly Tyr Pro Ser Leu Ala Ser
305 310 315 320

Ala Ala Tyr Asp Gln Gly Arg Ile Ala Ala Gln Ala Leu Val Lys Gly
325 330 335

Glu Ala Thr Ala His Leu Ile Glu Asp Ile Pro Thr Gly Ile Tyr Thr
340 345 350

Ile Pro Glu Ile Ser Ser Val Gly Lys Thr Glu Gln Gln Leu Thr Ala
355 360 365

Met Lys Val Pro Tyr Glu Val Gly Arg Ala Gln Phe Lys His Leu Ala
370 375 380

Arg Ala Gln Ile Val Gly Met Asn Val Gly Thr Leu Lys Ile Leu Phe
385 390 395 400

His Arg Glu Thr Lys Glu Ile Leu Gly Ile His Cys Phe Gly Glu Arg
405 410 415

Ala Ala Glu Ile Ile His Ile Gly Gln Ala Ile Met Glu Gln Lys Gly
420 425 430

Gly Gly Asn Thr Ile Glu Tyr Phe Val Asn Thr Thr Phe Asn Tyr Pro
435 440 445

Thr Met Ala Glu Ala Tyr Arg Val Ala Ala Leu Asn Gly Leu Asn Arg
450 455 460

Leu Phe
465

<210> 61
<211> 1395
<212> DNA
<213> Azotobacter vinelandii

<220>
<221> misc_feature
<222> (1)..(1395)

<223> codon-optimized sthA

<400> 61
atggcagtgt acaattatga tgtcgttgtt ataggaacag gtcctgctgg agaaggagca 60

gctatgaatg cagtaaaagc aggtagaaaa gttgctgttg tggacgaccg accacaggtt 120

ggtggtaact gcactcatct agggactatt ccatctaaag cacttagaca ttctgtcaga 180

cagattatgc aatacaataa caatccattg tttagacaaa taggtgaacc aagatggttc 240

tctttcgctg atgtgttgaa gtcagcggaa caagtgatcg ccaagcaagt ctcatccaga 300

actgggtatt acgcaaggaa tagaattgat accttttttcg gtacagcttc attttgtgac 360

gagcatacaa ttgaagtggt tcatttgaat gggatggttg aaactcttgt tgccaaacag 420

tttgtaatag ccactggctc cagaccttat agaccagctg atgttgattt tacacaccca 480

aggatatacg attcagatac cattttatcc ttagggcata cacctagaag gctcattatc 540

tacggagccg gtgttatagg ttgtgaatac gccagcatct tttctggctt aggagtgctc 600

gtcgatctaa tcgacaatag agatcaattg ttgtcttttc tggatgatga aatctctgac 660

tctctatcat accatttgag aaacaataat gtcctaattc gtcacaacga ggagtatgaa 720

agagtcgaag gtctggataa cggtgtaatc cttcacctga agagcggtaa aaagatcaaa 780

gctgatgcgt tcctatggtc aaatggcaga actggtaata ctgacaaact aggcttggag 840

aacattggtt taaaggccaa cggaagaggg cagatccaag tagacgaaca ctatcgtaca 900

gaggtttcta acatatacgc agctggcgat gtaatcggct ggccatcttt agctagtgcc 960

gcctacgacc aagggcgatc agctgctgga agtattaccg aaaatgactc ctggagattc 1020

gtagatgatg tccctacagg tatctacacc attcctgaaa tttcatctgt cggcaagacg 1080

gaaagagaat taacacaagc taaagttcca tacgaagtgg gtaaagcatt ctttaaaggt 1140

atggccagag cacaaatcgc tgtagagaag gctggaatgc tgaagatctt gttccataga 1200

gaaacgttag agatactcgg cgttcattgt tttggttatc aagcaagtga aatcgttcac 1260

attggccaag ctattatgaa tcaaaaaggt gaagcaaaca cattgaaata cttcatcaat 1320

actactttta actacccaac aatggccgag gcttacagag tagcagcgta cgatggactt 1380

aatcgtttgt tttaa 1395

<210> 62
<211> 464
<212> PRT
<213> Azotobacter vinelandii

<220>
<221> misc_feature
<222> (1)..(464)
<223> codon-optimized sthA

<400> 62

```
Met Ala Val Tyr Asn Tyr Asp Val Val Val Ile Gly Thr Gly Pro Ala
1               5                   10                  15

Gly Glu Gly Ala Ala Met Asn Ala Val Lys Ala Gly Arg Lys Val Ala
            20                  25                  30

Val Val Asp Asp Arg Pro Gln Val Gly Gly Asn Cys Thr His Leu Gly
        35                  40                  45

Thr Ile Pro Ser Lys Ala Leu Arg His Ser Val Arg Gln Ile Met Gln
    50                  55                  60

Tyr Asn Asn Asn Pro Leu Phe Arg Gln Ile Gly Glu Pro Arg Trp Phe
65                  70                  75                  80

Ser Phe Ala Asp Val Leu Lys Ser Ala Glu Gln Val Ile Ala Lys Gln
                85                  90                  95

Val Ser Ser Arg Thr Gly Tyr Tyr Ala Arg Asn Arg Ile Asp Thr Phe
            100                 105                 110

Phe Gly Thr Ala Ser Phe Cys Asp Glu His Thr Ile Glu Val Val His
        115                 120                 125

Leu Asn Gly Met Val Glu Thr Leu Val Ala Lys Gln Phe Val Ile Ala
    130                 135                 140

Thr Gly Ser Arg Pro Tyr Arg Pro Ala Asp Val Asp Phe Thr His Pro
145                 150                 155                 160

Arg Ile Tyr Asp Ser Asp Thr Ile Leu Ser Leu Gly His Thr Pro Arg
                165                 170                 175

Arg Leu Ile Ile Tyr Gly Ala Gly Val Ile Gly Cys Glu Tyr Ala Ser
            180                 185                 190

Ile Phe Ser Gly Leu Gly Val Leu Val Asp Leu Ile Asp Asn Arg Asp
        195                 200                 205

Gln Leu Leu Ser Phe Leu Asp Asp Glu Ile Ser Asp Ser Leu Ser Tyr
    210                 215                 220

His Leu Arg Asn Asn Asn Val Leu Ile Arg His Asn Glu Glu Tyr Glu
225                 230                 235                 240

Arg Val Glu Gly Leu Asp Asn Gly Val Ile Leu His Leu Lys Ser Gly
```

245 250 255

Lys Lys Ile Lys Ala Asp Ala Phe Leu Trp Ser Asn Gly Arg Thr Gly
260 265 270

Asn Thr Asp Lys Leu Gly Leu Glu Asn Ile Gly Leu Lys Ala Asn Gly
275 280 285

Arg Gly Gln Ile Gln Val Asp Glu His Tyr Arg Thr Glu Val Ser Asn
290 295 300

Ile Tyr Ala Ala Gly Asp Val Ile Gly Trp Pro Ser Leu Ala Ser Ala
305 310 315 320

Ala Tyr Asp Gln Gly Arg Ser Ala Ala Gly Ser Ile Thr Glu Asn Asp
325 330 335

Ser Trp Arg Phe Val Asp Asp Val Pro Thr Gly Ile Tyr Thr Ile Pro
340 345 350

Glu Ile Ser Ser Val Gly Lys Thr Glu Arg Glu Leu Thr Gln Ala Lys
355 360 365

Val Pro Tyr Glu Val Gly Lys Ala Phe Phe Lys Gly Met Ala Arg Ala
370 375 380

Gln Ile Ala Val Glu Lys Ala Gly Met Leu Lys Ile Leu Phe His Arg
385 390 395 400

Glu Thr Leu Glu Ile Leu Gly Val His Cys Phe Gly Tyr Gln Ala Ser
405 410 415

Glu Ile Val His Ile Gly Gln Ala Ile Met Asn Gln Lys Gly Glu Ala
420 425 430

Asn Thr Leu Lys Tyr Phe Ile Asn Thr Thr Phe Asn Tyr Pro Thr Met
435 440 445

Ala Glu Ala Tyr Arg Val Ala Ala Tyr Asp Gly Leu Asn Arg Leu Phe
450 455 460

## Description

BACKGROUND OF THE INVENTION

Energy conversion, utilization and access underlie many of the great challenges of our time, including those associated with sustainability, environmental quality, security, and poverty. New applications of emerging technologies are required to respond to these challenges. Biotechnology, one of the most powerful of the emerging technologies, can give rise to important new energy conversion processes. Plant biomass and derivatives thereof are a resource for the biological conversion of energy to forms useful to humanity.

Among forms of plant biomass, lignocellulosic biomass ("biomass") is particularly well-suited for energy applications because of its large-scale availability, low cost, and environmentally benign production. In particular, many energy production and utilization cycles based on cellulosic biomass have near-zero greenhouse gas emissions on a life-cycle basis. The primary obstacle impeding the more widespread production of energy from biomass feedstocks is the general absence of low-cost technology for overcoming the recalcitrance of these materials to conversion into useful products. Lignocellulosic biomass contains carbohydrate fractions (e.g., cellulose and hemicellulose) that can be converted into ethanol or other products such as lactic acid and acetic acid. In order to convert these fractions, the cellulose and hemicellulose must ultimately be converted or hydrolyzed into monosaccharides; it is the hydrolysis that has historically proven to be problematic.

Biologically mediated processes are promising for energy conversion. Biomass processing schemes involving enzymatic or microbial hydrolysis commonly involve four biologically mediated transformations: (1) the production of saccharolytic enzymes (cellulases and hemicellulases); (2) the hydrolysis of carbohydrate components present in pretreated biomass to sugars; (3) the fermentation of hexose sugars *(e.g.,* glucose, mannose, and galactose); and (4) the fermentation of pentose sugars (*e.g.,* xylose and arabinose). These four transformations occur in a single step in a process configuration called consolidated bioprocessing (CBP), which is distinguished from other less highly integrated configurations in that it does not involve a dedicated process step for cellulase and/or hemicellulase production.

CBP offers the potential for lower cost and higher efficiency than processes featuring dedicated cellulase production. The benefits result in part from avoided capital costs, substrate and other raw materials, and utilities associated with cellulase production. In addition, several factors support the realization of higher rates of hydrolysis, and hence reduced reactor volume and capital investment using CBP, including enzyme-microbe synergy and the use of thermophilic organisms and/or complexed cellulase systems. Moreover, cellulose-adherent cellulolytic microorganisms are likely to compete successfully for products of cellulose hydrolysis with non-adhered microbes, *e.g.*, contaminants, which could increase the stability of industrial processes based on microbial cellulose utilization. Progress in developing CBP-enabling microorganisms is being made through two strategies: engineering naturally occurring cellulolytic microorganisms to improve product-related properties, such as yield and titer; and engineering non-cellulolytic organisms that exhibit high product yields and titers to express a heterologous cellulase and hemicellulase system enabling cellulose and hemicellulose utilization.

Biological conversion of lignocellulosic biomass to ethanol or other chemicals requires a microbial catalyst to be metabolically active during the extent of the conversion. For CBP, a further requirement is placed on the microbial catalyst - it must also grow and produce sufficient cellulolytic and other hydrolytic enzymes in addition to metabolic products. A significant challenge for a CBP process occurs when the lignocellulosic biomass contains compounds inhibitory to microbial growth, which is common in natural lignocellulosic feedstocks. Arguably the most important inhibitory compound is acetic acid (acetate), which is released during deacetylation of polymeric substrates. Acetate is particularly inhibitory for CBP processes, as cells must constantly expend energy to export acetate anions, which then freely diffuse back into the cell as acetic acid. This phenomena, combined with the typically low sugar release and energy availability during the fermentation, limits the cellular energy that can be directed towards cell mass generation and enzyme production, which further lowers sugar release.

Removal of acetate prior to fermentation would significantly improve CBP dynamics; however, chemical and physical removal systems are typically too expensive or impractical for industrial application. Thus, there is a need for an alternate acetate removal system for CBP that does not suffer from the same problems associated with these chemical and physical removal systems. As a novel alternative, this invention describes the metabolic conversion of acetate to a less inhibitory compound, such as a non-charged solvent, including but not limited to, acetone, isopropanol, or ethanol. The metabolic conversion of acetate requires the input of electrons. Under anaerobic conditions, the surplus of NADH that is generated during biomass formation is reoxidized via glycerol formation. While the electrons from the surplus NADH can be used for acetate conversion when glycerol production is reduced, the amount of NADH available is limited and is insufficient to completely consume acetate in high concentrations. The present invention combines the metabolic conversion of acetate with processes that produce surplus electron donors, including, but not limited to, processes involved in xylose fermentation and the oxidative branch of the phosphate pentose pathway, to free up more electrons for efficient acetate consumption. In addition, the improved conversion of acetate also results in several process benefits described below.

BRIEF SUMMARY OF THE INVENTION

The invention is generally directed to the improved reduction or removal of acetate from biomass processing such as the CBP processing of lignocellulosic biomass. The invention is also generally directed to the adaptation of CBP organisms to growth in the presence of inhibitory compounds, including, but not limited to, acetate.

One aspect of the invention relates to a recombinant microorganism comprising one or more native and/or heterologous enzymes that function in one or more first engineered metabolic pathways to convert acetate to an alcohol, wherein said one or more native and/or heterologous enzymes is activated, upregulated or downregulated; and one or more native and/or heterologous enzymes that function in one or more second engineered metabolic pathways to produce an electron donor used in the conversion of acetate to an alcohol, wherein said one or more native and/or heterologous enzymes is activated, upregulated or downregulated. In certain embodiments, the acetate is produced as a by-product of biomass processing. In certain emodiments, the recombinant microorganism produces an alcohol selected from the group consisting of ethanol, isopropanol, or a combination thereof. In some embodiments, the electron donor is selected from the group consisting of NADH, NADPH, or a combination thereof.

In particular aspects, the one or more second engineered metabolic pathways to produce an electron donor is a xylose fermentation pathway. In certain embodiments, the engineered xylose fermentation pathway comprises upregulation of the native and/or heterologous enzymes xylose reductase (XR) and xylitol dehydrogenase (XDH). In some embodiments, the XR reaction has a preference for NADPH or is NADPH-specific, and/or the XDH reaction has a preference for NADH or is NADH-specific. In certain embodiments, the native and/or heterologous XDH enzyme is from *Scheffersomyces stipitis.* In further embodiments, the XDH enzyme is encoded by a *xyl2* polynucleotide. In some embodiments, the native and/or heterologous XR enzyme is from *Scheffersomyces stipitis, Neurospora crassa,* or *Candida boidinii*. In certain embodiments, the XR enzyme is encoded by a *xyl1* polynucleotide or an aldolase reductase.

In some embodiments, the first and second engineered metabolic pathways in the recombinant microorganism result in ATP production. In further embodiments, the first and second engineered metabolic pathways in the recombinant microorganism result in net ATP production. In certain embodiments, the one or more first engineered metabolic pathways comprises activating or upregulating one or more heterologous enzymes selected from the group consisting of acetyl-CoA acetyltransferase (thiolase), acetoacetyl-CoA transferase, acetoacetate decarboxylase, a secondary alcohol dehydrogenase, or combinations thereof. In some embodiments, the one or more first engineered metabolic pathways comprises activating or upregulating a heterologous ADP-producing acetyl-CoA synthase enzyme. In some embodiments, the one or more first engineered metabolic pathways comprises activating or upregulating the acetate kinase/phosphotransacetylase (AK/PTA) couple. In particular aspects, the first and second engineered metabolic pathways result in ATP production.

In certain embodiments, the one or more second engineered metabolic pathways to produce an electron donor is the oxidative branch of the pentose phosphate pathway (PPP). In some embodiments, the engineered PPP comprises activation or upregulation of the native enzyme glucose-6-P dehydrogenase. In certain embodiments, the native glucose-6-P dehydrogenase enzyme is from *Saccharomyces cerevisiae.* In further embodiments the glucose-6-P dehydrogenase is encoded by a *zwf1* polynucleotide.

In some embodiments, the recombinant microorganism that converts acetate to an alcohol further comprises altering the expression of transcription factors that regulate expression of enzymes of the PPP pathway. In certain embodiments, the transcription factor is Stb5p. In further embodiments, the Stb5p is from *Saccharomyces cerevisiae.*

In certain embodiments, the one or more second engineered metabolic pathways of the recombinant microorganism that converts acetate to an alcohol to produce an electron donor is a pathway that competes with the oxidative branch of the PPP. In some embodiments, the engineered pathway that competes with the oxidative branch of the PPP comprises downregulation of the native enzyme glucose-6-P isomerase. In further embodiments, the native glucose-6-P isomerase enzyme is from *Saccharomyces cerevisiae.* In some embodiments, the glucose-6-P isomerase is encoded by a *pgi1* polynucleotide.

In certain embodiments, the one or more second engineered metabolic pathways of the recombinant microorganism that converts acetate to an alcohol to produce an electron donor comprises the ribulose-monophosphate pathway (RuMP). In some embodiments, the engineered RuMP pathway converts fructose-6-P to ribulose-5-P and formaldehyde. In further embodiments, the engineered RuMP pathway comprises upregulating a heterologous enzyme selected from the group consisting of 6-phospho-3-hexuloisomerase, 3-hexulose-6-phosphate synthase, and the combination thereof.

In certain embodiments, the one or more second engineered metabolic pathways of the recombinant microorganism that converts acetate to an alcohol to produce an electron donor comprises upregulating native enzymes that degrade formaldehyde or formate. In some embodiments, the formaldehyde degrading enzymes convert formaldehyde to formate. In further embodiments, the formaldehyde degrading enzymes are formaldehyde dehydrogenase and S-formylglutathione hydrolase. In some embodiments, the formate degrading enzyme converts formate to $CO_2$. In further

embodiments, the formate degrading enzyme is formate dehydrogenase. In some embodiments, the formaldehyde is oxidized to form $CO_2$.

In some embodiments, the formate dehydrogenase is from a yeast microorganism. In some emodiments, the yeast microorganism is *S. cerevisiae* or *Candida boidinii.* In further embodiments, the formate dehydrogenase from *S. cerevisiae* is FDH1. In some embodiments, the formate dehydrogenase from *Candida boidinii* is FDH3. In some embodiments, the microorganism consumes or uses more acetate than a microorganism not comprising the enzyme that degrades formate. In further embodiments, the recombinant microorganism has an acetate uptake (g/L) under anaerobic conditions from: (a) at least about 1.0 fold more acetate uptake than that taken up by a recombinant microorganism not comprising said enzyme that degrades formate; (b) at least about 1.1 fold more acetate uptake than that taken up by a recombinant microorganism not comprising said enzyme that degrades formate; (c) at least about 1.2 fold more acetate uptake than that taken up by a recombinant microorganism not comprising said enzyme that degrades formate; (d) at least about 1.3 fold more acetate uptake than that taken up by a recombinant microorganism not comprising said enzyme that degrades formate; (e) at least about 1.4 fold more acetate uptake than that taken up by a recombinant microorganism not comprising said enzyme that degrades formate;(f) at least about 1.5 fold more acetate uptake than that taken up by a recombinant microorganism not comprising said enzyme that degrades formate; (g) at least about 2.0 fold more acetate uptake than that taken up by a recombinant microorganism not comprising said enzyme that degrades formate; (h) at least about 2.5 fold more acetate uptake than that taken up by a recombinant microorganism not comprising said enzyme that degrades formate; (i) at least about 3.0 fold more acetate uptake than that taken up by a recombinant microorganism not comprising said enzyme that degrades formate; (j) at least about 4.0 fold more acetate uptake than that taken up by a recombinant microorganism not comprising said enzyme that degrades formate; (k) at least about 5.0 fold more acetate uptake than that taken up by a recombinant microorganism not comprising said enzyme that degrades formate; or (1) at least about 10 fold more acetate uptake than that taken up by a recombinant microorganism not comprising said enzyme that degrades formate. In some embodiments, the recombinant microorganism has an acetate uptake under anaerobic conditions at least about 0.32 g/L, at least about 0.37 g/L, at least about 0.46 g/L, or at least about 0.48 g/L.

In certain emodiments, the recombinant microorganism comprises a) one or more native and/or heterologous enzymes that function in one or more first engineered metabolic pathways to convert acetate to an alcohol, wherein said one or more native and/or heterologous enzymes is activated, upregulated or downregulated; and b) one or more native and/or heterologous *zwf1* polynucleotides; wherein one or more native and/or heterologous enzymes is an NADPH-specific alcohol dehydrogenase. In other emodiments, the NADPH-specific alcohol dehydrogenase is from a microorganism selected from the group consisting of *T. pseudethanolicus, C. beijerinckii, Entamoeba histolytica, Cucumis melo,* and *S. cerevisiae.* In further embodiments, the NADPH-specific alcohol dehydrogenase is *T. pseudethanolicus* adhB. In other embodiments, the NADPH-specific alcohol dehydrogenase is *C. beijerinckii* 2° Adh. In other embodiments, the NADPH-specific alcohol dehydrogenase is *S. cerevisiae* ARI1. In some embodiments, the NADPH-specific alcohol dehydrogenase is *Entamoeba histolytica* ADH1. In other embodiments, the NADPH-specific alcohol dehydrogenase is *Cucumis melo* ADH1.

In certain embodiments, the one or more native enzymes that function in one or more first engineered metabolic pathways to convert acetate to an alcohol is an NADH-specific alcohol dehydrogenase. In other embodiments, the alcohol dehydrogenase is downregulated. In further embodiments, the downregulated alcohol dehydrogenase is an NADH-ADH selected from ADH1, ADH2, ADH3, ADH4, ADH5, or SFA1 from *Saccharomyces.* In some embodiments, the recombinant microorganism consumes or uses more acetate than a microorganism not comprising said NADPH-specific alcohol dehydrogenase.

In other embodiments, the recombinant microorganism has an acetate uptake (g/L) under anaerobic conditions: (a) at least about 1.1 fold more acetate uptake than that taken up by a recombinant microorganism not comprising said NADPH-specific alcohol dehydrogenase; (b) at least about 1.2 fold more acetate uptake than that taken up by a recombinant microorganism not comprising said NADPH-specific alcohol dehydrogenase; (c) at least about 1.3 fold more acetate uptake than that taken up by a recombinant microorganism not comprising said NADPH-specific alcohol dehydrogenase; (d) at least about 1.4 fold more acetate uptake than that taken up by a recombinant microorganism not comprising said NADPH-specific alcohol dehydrogenase; (e) at least about 1.5 fold more acetate uptake than that taken up by a recombinant microorganism not comprising said NADPH-specific alcohol dehydrogenase; (f) at least about 1.6 fold more acetate uptake than that taken up by a recombinant microorganism not comprising said NADPH-specific alcohol dehydrogenase; (g) at least about 1.9 fold more acetate uptake than that taken up by a recombinant microorganism not comprising said NADPH-specific alcohol dehydrogenase; (h) at least about 2.0 fold more acetate uptake than that taken up by a recombinant microorganism not comprising said NADPH-specific alcohol dehydrogenase; (i) at least about 2.1 fold more acetate uptake than that taken up by a recombinant microorganism not comprising said NADPH-specific alcohol dehydrogenase; (j) at least about 2.3 fold more acetate uptake than that taken up by a recombinant microorganism not comprising said NADPH-specific alcohol dehydrogenase; (k) at least about 2.4 fold more acetate uptake than that taken up by a recombinant microorganism not comprising said NADPH-specific alcohol dehydrogenase; (1) at least about 2.5 fold more acetate uptake than that taken up by a recombinant microorganism not comprising said NADPH-specific alcohol dehydrogenase; (m) at least about 2.7 fold more acetate uptake than that taken up by a recombinant microorganism not comprising said NADPH-specific alcohol dehydrogenase; (n) at least about 2.8 fold more acetate uptake than that taken up by a recombinant microorganism not comprising said NADPH-specific alcohol dehydrogenase; (o) at least about 2.9 fold more acetate uptake than that taken up by a recombinant microorganism not comprising said NADPH-specific alcohol dehydrogenase; or (p) at least about 3.1 fold more acetate uptake than that taken up by a recombinant microorganism not comprising said NADPH-specific alcohol dehydrogenase.

In further emodiments, the recombinant microorganism has an acetate uptake under anaerobic conditions at least about 0.35 g/L, at least about 0.36 g/L, at least about 0.38 g/L, at least about 0.40 g/L, at least about 0.44 g/L, at least about 0.45 g/L, at least about 0.47 g/L, at least about 0.48 g/L, at least about 0.51 g/L, at least about 0.53 g/L, at least about 0.59 g/L, at least about 0.61 g/L, at least about 0.63 g/L, at least about 0.65 g/L, at least about 0.66 g/L, at least about 0.70 g/L, at least about 0.79 g/L, at least about 0.8 g/L, at least about 0.83 g/l, at least about 0.84 g/L, at least about 0.87 g/L, at least about 0.9 g/L, at least about 0.91 g/L, at least about 0.96 g/L, at least about 0.99 g/L, at least about 1.00 g/L, at least about 1.01 g/L at least about 1.02 g/L, at least about 1.18 g/L, at least about 1.20 g/L, at least about 1.23 g/L, at least about 3.2 g/L, or at least about 3.3 g/L. In other embodiments, the recombinant microorganism has an acetate uptake under anaerobic conditions from about 0.35 g/L to about 3.3 g/L.

In certain embodiments, the recombinant microorganism further comprises one or more native and/or heterologous acetyl-CoA synthetases, and wherein said one or more native and/or heterologous acetyl-CoA synthetases is activated or upregulated. In other embodiments, the acetyl-CoA synthetase is encoded by a polynucleotide selected from the group consisting of an ACS1 polynucleotide and an ACS2 polynucleotide. In further embodiments, the ACS1 polynucleotide or the ACS2 polynucleotide is from a yeast microorganism. In other embodiments, the ACS1 polynucleotide is from *Saccharomyces cerevisiae* or *Saccharomyces kluyveri.* In further emodiments, the ACS2 polynucleotide is from *Saccharomyces cerevisiae* or *Saccharomyces kluyveri.*

In certain embodiments, the one or more native and/or heterologous enzymes of the recombinant microorganism that converts acetate to an alcohol is from *Mycobacterium gastri.*

In certain embodiments, the one or more second engineered metabolic pathways of the recombinant microorganism that converts acetate to an alcohol to produce an electron donor comprises the dihydroxyacetone (DHA) pathway. In some embodiments, the engineered DHA pathway interconverts dihydroxyacetone and glyceraldehyde-3-P into xylose-5-P and formaldehyde. In further embodiments, the engineered DHA pathway comprises upregulating the heterologous enzyme formaldehyde transketolase (EC 2.2.1.3).

In certain embodiments, the one or more second engineered metabolic pathways of the recombinant microorganism that converts acetate to an alcohol to produce an electron donor comprises upregulating native and/or heterologous enzymes that produce dihydroxyacetone. In some embodiments, the native and/or heterologous enzymes that produce dihydroxyacetone are selected from the group consisting of glycerol dehydrogenase, dihydroxyacetone phosphatase, and a combination thereof. In further embodiments, the native and/or heterologous glycerol dehydrogenase is from a microorganism selected from the group consisting of *Hansenula polymorpha, E. coli, Pichia angusta,* and *Saccharomyces cerevisiae.* In some embodiments, the glycerol dehydrogenase is encoded by a polynucleotide selected from the group consisting of *gdh, gldA, and gcyl.*

In certain embodiments, the one or more second engineered metabolic pathways of the recombinant microorganism that converts acetate to an alcohol to produce an electron donor comprises downregulating a native dihydroxyacetone kinase enzyme. In some embodiments, the dihydroxyacetone kinase is encoded by a polynucleotide selected from the group consisting of *dak1, dak2,* and a combination thereof.

In certain embodiments, the recombinant microorganism that converts acetate to an alcohol further comprises overexpressing a glycerol/proton-symporter. In some embodiments, the glycerol/proton-symporter is encoded by a *stl1* polynucleotide.

In certain embodiments, the recombinant microorganism that converts acetate to an alcohol further comprises overexpression of a native and/or heterologous transhydrogenase enzyme. In some embodiments, the transhydrogenase catalyzes the interconversion of NADPH and NAD to NADP and NADH. In further embodiments, the transhydrogenase is from a microorganism selected from the group consisting of *Escherichia coli* and *Azotobacter vinelandii.*

In certain embodiments, the recombinant microorganism that converts acetate to an alcohol further comprises overexpression of a native and/or heterologous glutamate dehydrogenase enzyme. In some embodiments, the glutamate dehydrogenase is encoded by a *gdh2* polynucleotide.

In certain embodiments of the invention, in the recombinant microorganism that converts acetate to an alcohol, one of the engineered metabolic pathways comprises the conversion of acetate to acetyl-CoA and conversion of acetyl-CoA to ethanol.

In certain embodiments, the one or more downregulated native enzymes of the microorganism that converts acetate to an alcohol is encoded by a *gpd1* polynucleotide, a *gpd2* polynucleotide, or both a *gpd1* polynucleotide and a *gpd2* polynucleotide.

In certain embodiments, the microorganism that converts acetate to an alcohol produces ethanol.

In certain embodiments, the microorganism that converts acetate to an alcohol is selected from the group consisting of *Saccharomyces cerevisiae, Kluyveromyces lactis, Kluyveromyces marxianus, Pichia pastoris, Yarrowia lipolytica, Hansenula polymorpha, Phaffia rhodozyma, Candida utilisutilis, Arxula adeninivorans, Pichia stipitis, Debaryomyces hansenii, Debaryomyces polymorphus, Schizosaccharomyces pombe, Candida albicans,* and *Schwanniomycesoccidentalis.* In some embodiments, the microorganism is *Saccharomyces cerevisiae.*

In certain embodiments, in the microorganism that converts acetate to an alcohol, acetate is converted to acetyl-CoA by an acetyl-CoA transferase (ACS). In some embodiments, the acetate is converted to acetyl-P by an acetate kinase and the acetyl-P is converted to acetyl-CoA by a phosphotransacetylase. In some embodiments, the acetyl-CoA transferase (ACS) is encoded by an ACS1 polynucleotide. In further embodiments, the acetate kinase and the phosphotransacetylase are from one or more of an *Escherichia,* a *Thermoanaerobacter,* a *Clostridia,* or a *Bacillus* species. In some embodiments, acetyl-CoA is converted to acetaldehyde by an acetaldehyde dehydrogenase and the acetaldehyde is converted to ethanol by an alcohol dehydrogenase. In some embodiments, the acetaldehyde dehydrogenase is from *C. phytofermentans.* In further embodiments, the acetaldehyde dehydrogenase is an NADPH-specific acetaldehyde dehydrogenase. In some embodiments, the NADPH-specific acetaldehyde dehydrogenase is from *T. pseudethanolicus.* In further embodiments, the NADPH-specific acetaldehyde dehydrogenase is *T. pseudethanolicus* adhB. In some embodiments, the alcohol dehydrogenase is an NADPH-specific alcohol dehydrogenase. In further embodiments, the NADPH-specific alcohol dehydrogenase is from a microorganism selected from the group consisting of *T. pseudethanolicus, C. beijerinckii, Entamoeba histolytica, Cucumis melo,* and *S. cerevisiae.* In some embodiments, the NADPH-specific alcohol dehydrogenase is *T. pseudethanolicus* adhB. In some embodiments, the NADPH-specific alcohol dehydrogenase is *C. beijerinckii* 2° Adh. In certain embodiments, the NADPH-specific alcohol dehydrogenase is *S. cerevisiae* ARI1. In some embodiments, the NADPH-specific alcohol dehydrogenase is *Entamoeba histolytica* ADH1. In certain embodiments, the NADPH-specific alcohol dehydrogenase is *Cucumis melo* ADH1.

In certain embodiments, the microorganism consumes or uses more acetate than a microorganism not comprising said NADPH-specific alcohol dehydrogenase. In some embodiments, the recombinant microorganism has an acetate uptake (g/L) under anaerobic conditions: (a) at least about 1.1 fold more acetate uptake than that taken up by a recombinant microorganism not comprising said NADPH-specific alcohol dehydrogenase; (b) at least about 1.2 fold more acetate uptake than that taken up by a recombinant microorganism not comprising said NADPH-specific alcohol dehydrogenase; (c) at least about 1.3 fold more acetate uptake than that taken up by a recombinant microorganism not comprising said NADPH-specific alcohol dehydrogenase; (d) at least about 1.4 fold more acetate uptake than that taken up by a recombinant microorganism not comprising said NADPH-specific alcohol dehydrogenase; (e) at least about 1.5 fold more acetate uptake than that taken up by a recombinant microorganism not comprising said NADPH-specific alcohol dehydrogenase; (f) at least about 1.6 fold more acetate uptake than that taken up by a recombinant microorganism not comprising said NADPH-specific alcohol dehydrogenase; (g) at least about 1.9 fold more acetate uptake than that taken up by a recombinant microorganism not comprising said NADPH-specific alcohol dehydrogenase; (h) at least about 2.0 fold more acetate uptake than that taken up by a recombinant microorganism not comprising said NADPH-specific alcohol dehydrogenase; (i) at least about 2.1 fold more acetate uptake than that taken up by a recombinant microorganism not comprising said NADPH-specific alcohol dehydrogenase; (j) at least about 2.3 fold more acetate uptake than that taken up by a recombinant microorganism not comprising said NADPH-specific alcohol dehydrogenase; (k) at least about 2.4 fold more acetate uptake than that taken up by a recombinant microorganism not comprising said NADPH-specific alcohol dehydrogenase; (l) at least about 2.5 fold more acetate uptake than that taken up by a recombinant microorganism not comprising said NADPH-specific alcohol dehydrogenase; (m) at least about 2.7 fold more acetate uptake than that taken up by a recombinant microorganism not comprising said NADPH-specific alcohol dehydrogenase; (n) at least about 2.8 fold more acetate uptake than that taken up by a recombinant microorganism not comprising said NADPH-specific alcohol dehydrogenase; (o) at least about 2.9 fold more acetate uptake than that taken up by a recombinant microorganism not comprising said NADPH-specific alcohol dehydrogenase; or (p) at least about 3.1 fold more acetate uptake than that taken up by a recombinant microorganism not comprising said NADPH-specific alcohol dehydrogenase.

In further embodiments, the recombinant microorganism has an acetate uptake under anaerobic conditions at least about 0.35 g/L, at least about 0.36 g/L, at least about 0.38 g/L, at least about 0.40 g/L, at least about 0.44 g/L, at least about 0.45 g/L, at least about 0.47 g/L, at least about 0.48 g/L, at least about 0.51 g/L, at least about 0.53 g/L, at least about 0.59 g/L, at least about 0.61 g/L, at least about 0.63 g/L, at least about 0.65 g/L, at least about 0.66 g/L, at least about 0.70 g/L, at least about 0.79 g/L, at least about 0.8 g/L, at least about 0.83 g/l, at least about 0.84 g/L, at least about 0.87 g/L, at least about 0.9 g/L, at least about 0.91 g/L, at least about 0.96 g/L, at least about 0.99 g/L, at least about 1.00 g/L, at least about 1.01 g/L at least about 1.02 g/L, at least about 1.18 g/L, at least about 1.20 g/L, at least about 1.23 g/L, at least about 3.2 g/L, or at least about 3.3 g/L. In other embodiments, the recombinant microorganism has an acetate uptake under anaerobic conditions from about 0.35 g/L to about 3.3 g/L.

In certain embodiments, in the recombinant microorganism that converts acetate to an alcohol, acetyl-CoA is converted to ethanol by a bifunctional acetaldehyde/alcohol dehydrogenase. In some embodiments, the bifunctional acetaldehyde/alcohol dehydrogenase is from *E. coli, C. acetobutylicum, T. saccharolyticum, C. thermocellum,* or *C. phytofermentans.*

Another aspect of the invention relates to a recombinant microorganism comprising one or more native and/or heterologous enzymes that function in one or more engineered metabolic pathways to convert acetate to acetone, wherein the one or more native and/or heterologous enzymes is activated, upregulated or downregulated; and one or more native and/or heterologous enzymes that function in one or more second engineered metabolic pathways to produce an electron donor used in the conversion of acetate to acetone, wherein the one or more native and/or heterologous enzymes is activated, upregulated or downregulated. In some embodiments, the acetate is produced as a by-product of biomass processing. In certain embodiments, one of the engineered metabolic pathways comprises the conversion of acetate to acetyl-CoA; conversion of acetyl-CoA to acetoacetyl-CoA; conversion of acetoacetyl-CoA to acetoacetate; and conversion of acetoacetate to acetone.

In certain embodiments, the recombinant microorganism that converts acetate to acetone produces acetone. In some embodiments, the recombinant microorganism is *Escherichia coli.* In certain embodiments, the recombinant microorganism is a thermophilic or mesophilic bacterium. In further embodiments, the recombinant microorganism is a species of the genera *Thermoanaerobacterium, Thermoanaerobacter, Clostridium, Geobacillus, Saccharococcus, Paenibacillus, Bacillus, Caldicellulosiruptor, Anaerocellum,* or *Anoxybacillus.* In some embodiments, the recombinant microorganism is a bacterium selected from the group consisting of *Thermoanaerobacteriumthermosulfurigenes, Thermoanaerobacteriumaotearoense, Thermoanaerobacteriumpolysaccharolyticum, Thermoanaerobacteriumzeae, Thermoanaerobacteriumxylanolyticum, Thermoanaerobacterium saccharolyticum, Thermoanaerobiumbrockii, Thermoanaerobacteriumthermosaccharolyticum, Thermoanaerobacter thermohydrosulfuricus, Thermoanaerobacterethanolicus, Thermoanaerobacterbrocki, Clostridium thermocellum, Clostridium cellulolyticum, Clostridium phytofermentans, Clostridium straminosolvens, Geobacillus thermoglucosidasius, Geobacillus stearothermophilus, Saccharococcus caldoxylosilyticus, Saccharoccus thermophilus, Paenibacillus campinasensis, Bacillus flavothermus, Anoxybacillus kamchatkensis, Anoxybacillus gonensis, Caldicellulosiruptor acetigenus, Caldicellulosiruptor saccharolyticus, Caldicellulosiruptor kristjanssonii, Caldicellulosiruptor owensensis, Caldicellulosiruptor lactoaceticus,* and *Anaerocellumthermophilum.*

In certain embodiments, the recombinant microorganism that converts acetate to acetone is selected from the group consisting of *Clostridium thermocellum* and *Thermoanaerobacterium saccharolyticum.* In some embodiments, the recombinant microorganism is selected from the group consisting of *Saccharomyces cerevisiae, Kluyveromyces lactis, Kluyveromyces marxianus, Pichia pastoris, Yarrowia lipolytica, Hansenula polymorpha, Phaffia rhodozyma, Candida utilis, Arxula adeninivorans, Pichia stipitis, Debaryomyces hansenii, Debaryomyces polymorphus, Schizosaccharomyces pombe, Candida albicans,* and *Schwanniomycesoccidentalis.* In further embodiments, the recombinant microorganism is *Saccharomyces cerevisiae.*

In certain embodiments, in the recombinant microorganism that converts acetate to acetone, the acetate is converted to acetyl-CoA by an acetyl-CoA synthetase. In some embodiments, the acetate is converted to acetyl-P by an acetate kinase and the acetyl-P is converted to acetyl-CoA by a phosphotransacetylase. In further embodiments, the acetyl-CoA is converted to acetoacetyl-CoA by a thiolase. In some embodiments, the acetoacetyl-CoA is converted to acetoacetate by a CoA transferase. In certain embodiments, the acetoacetate is converted to acetone by an acetoacetate decarboxylase. In some embodiments, the acetyl-CoA synthetase is encoded by a polynucleotide selected from the group consisting of a yeast ACS1 polynucleotide and a yeast ACS2 polynucleotide. In further embodiments, the yeast ACS1 polynucleotide is from *Saccharomyces cerevisiae* or *Saccharomyces kluyveri.* In certain embodiments, the yeast ACS2 polynucleotide is from *Saccharomyces cerevisiae* or *Saccharomyces kluyveri.* In some embodiments, the acetate kinase and phosphotransacetylase are from *T. saccharolyticum.* In some embodiments, the thiolase, CoA transferase, and acetoacetate decarboxylase are from *C. acetobutylicum.* In further embodiments, the thiolase is from *C. acetobutylicum* or *T. thermosaccharolyticum.* In some embodiments, the CoA transferase is from a bacterial source. In further embodiments, the bacterial source is selected from the group consisting of *Thermoanaerobacter tengcongensis, Thermoanaerbacterium thermosaccharolyticum, Thermosipho africanus,* and *Paenibacillus macerans.* In some embodiments, the acetoacetate decarboxylase is from a bacterial source. In further embodiments, the bacterial source is selected from the group consisting of *C. acetobutylicum, Paenibacillus macerans, Acidothermus cellulolyticus, Bacillus amyloliquefaciens,* and *Rubrobacter xylanophilus.*

In certain embodiments, in the recombinant microorganism that converts acetate to acetone, one of said engineered metabolic pathways comprises the conversion of acetate to acetyl-CoA; conversion of acetyl-CoA to acetoacetyl-CoA; conversion of acetoacetyl-CoA to acetoacetate; conversion of acetoacetate to acetone; and conversion of acetone to isopropanol. In further embodiments, the recombinant microorganism is selected from the group consisting of *Saccharomyces cerevisiae, Kluyveromyces lactis, Kluyveromyces marxianus, Pichia pastoris, Yarrowia lipolytica, Hansenula polymorpha, Phaffia rhodozyma, Candida utilis, Arxula adeninivorans, Pichia stipitis, Debaryomyces hansenii, Debaryomyces polymorphus, Schizosaccharomyces pombe, Candida albicans,* and *Schwanniomycesoccidentalis.* In some embodiments, the recombinant microorganism is *Saccharomyces cerevisiae.*

In certain embodiments, in the recombinant microorganism, acetate is converted to acetyl-CoA by an acetyl-CoA synthetase. In some embodiments, the acetyl-CoA is converted to acetoacetyl-CoA by a thiolase. In some embodiments, the acetoacetyl-CoA is converted to acetoacetate by a CoA transferase. In certain embodiments, the acetoacetate is converted to acetone by an acetoacetate decarboxylase. In some embodiments, the acetone is converted to isopropanol by an alcohol dehydrogenase. In further embodiments, the acetyl-CoA synthetase is encoded by a polynucleotide selected from the group consisting of a yeast ACS1 polynucleotide and a yeast ACS2 polynucleotide. In some embodiments, the CoA transferase is from a bacterial source. In certain embodiments, the acetoacetate decarboxylase is from a bacterial source.

In certain embodiments, the invention relates to a recombinant microorganism comprising one or more native and/or heterologous enzymes that function in one or more engineered metabolic pathways to convert acetate to an alcohol, wherein one of said native and/or heterologous enzymes is an NADPH-specific alcohol dehydrogenase. In some embodiments, the NADPH-specific alcohol dehydrogenase is from a microorganism selected from the group consisting of *T. pseudethanolicus, C. beijerinckii, Entamoeba histolytica, Cucumis melo,* and *S. cerevisiae.* In some embodiments, the NADPH-specific alcohol dehydrogenase is encoded by any one of SEQ ID NOs:30, 32, 33, 35, or 36 or a fragment, variant, or derivative thereof that retains the function of an alcohol dehydrogenase.

In certain embodiments, the invention relates to a recombinant microorganism comprising one or more native and/or heterologous enzymes that function in one or more engineered metabolic pathways to convert acetate to an alcohol, wherein a first native and/or heterologous enzyme is an NADPH-specific alcohol dehydrogenase and wherein a second native and/or heterologous enzyme is an acetyl-CoA synthetase. In some embodiments, the NADPH-specific alcohol dehydrogenase is from *Entamoeba histolytica.* In some embodiments, the NADPH-specific alcohol dehydrogenase is encoded by SEQ ID NO:35 or a fragment, variant, or derivative thereof that retains the function of an alcohol dehydrogenase. In some embodiments, the acetyl-CoA synthetase is from a yeast microorganism or from a bacterial microorganism. In some embodiments, the acetyl-CoA synthetase is from *Saccharomyces cerevisiae, Saccharomyces kluyveri, Zygosaccharomyces bailii,* or *Acetobacter aceti.* In other embodiments, the acetyl-CoA synthetase is encoded by any one of SEQ ID NOs:37-40, 57, 58 or a fragment, variant, or derivative thereof that retains the function of an acetyl-CoA synthetase.

In certain embodiments, the invention relates to a recombinant microorganism comprising one or more native and/or heterologous enzymes that function in one or more engineered metabolic pathways to convert acetate to an alcohol, wherein a first native and/or heterologous enzyme is an NADPH-specific alcohol dehydrogenase and wherein a second native and/or heterologous enzyme is an NADH-specific alcohol dehydrogenase. In some embodiments, the NADPH-specific alcohol dehydrogenase is from *Entamoeba histolytica.* In some embodiments, the NADPH-specific alcohol dehydrogenase is encoded by SEQ ID NO:35 or a fragment, variant, or derivative thereof that retains the function of an alcohol dehydrogenase. In some embodiments, the NADH-specific alcohol dehydrogenase is downregulated. In some embodiments, the downregulated NADH-specific alcohol dehydrogenase is selected from ADH1, ADH2, ADH3, ADH4, ADH5, or SFA1 from *Saccharomyces.*

In certain embodiments, the invention relates to a recombinant microorganism comprising a) one or more native and/or heterologous enzymes that function in one or more first engineered metabolic pathways to convert acetate to an alcohol, wherein said one or more native and/or heterologous enzymes is activated, upregulated or downregulated; and b) one or more native and/or heterologous enzymes that function in one or more second engineered metabolic pathways to produce an electron donor used in the conversion of acetate to an alcohol, wherein one of said native and/or heterologous enzymes is a formate dehydrogenase. In some embodiments, the formate dehydrogenase is from a yeast microorganism. In some embodiments, the yeast microorganism is *S. cerevisiae* or *Candida boidinii.* In other embodiments, the formate dehydrogenase from *S. cerevisiae* is FDH1 or from *Candida boidinii* is FDH3. In some embodiments, the formate dehydrogenase from is encoded by SEQ ID NO:46, 47, or a fragment, variant, or derivative thereof that retains the function of a formate dehydrogenase.

Another aspect of the invention relates to a method for increasing acetate uptake from a biomass comprising contacting said biomass with a recombinant microorganism of the invention. In further emdodiments, the method further comprises increasing the amount of sugars of the biomass. In other embodiments, the the sugars are increased by the addition of an exogenous sugar source to the biomass. In further embodiments, the sugars are increased by the addition of one or more enzymes to the biomass or the recombinant microorganisms of the invention that use or break-down cellulose, hemicellulose and/or other biomass components. In other embodiments, the sugars are increased by the addition of a CBP microorganism that uses or breaks-down cellulose, hemicellulose and/or other biomass components.

Another aspect of the invention relates to a process for converting biomass to ethanol, acetone, or isopropanol comprising contacting biomass with a recombinant microorganism of the invention. In some embodiments, the biomass comprises lignocellulosic biomass. In further embodiments, the lignocellulosic biomass is selected from the group consisting of grass, switch grass, cord grass, rye grass, reed canary grass, mixed prairie grass, miscanthus, sugar-processing residues, sugarcane bagasse, sugarcane straw, agricultural wastes, rice straw, rice hulls, barley straw, corn cobs, cereal straw, wheat straw, canola straw, oat straw, oat hulls, corn fiber, stover, soybean stover, corn stover, forestry wastes, recycled wood pulp fiber, paper sludge, sawdust, hardwood, softwood, agave, and combinations thereof.

In certain embodiments, the process reduces or removes acetate from the consolidated bioprocessing (CBP) media. In some embodiments, the reduction or removal of acetate occurs during fermentation.

The invention further relates to an engineered metabolic pathway for reducing or removing acetate from consolidated bioprocessing (CBP) media.

According to an aspect of the invention there is:

a recombinant microorganism comprising:

a) one or more native and/or heterologous enzymes that function in a first engineered metabolic pathway to convert acetate to an alcohol, wherein said one or more native and/or heterologous enzymes is activated, upregulated or downregulated and is recombinantly introduced into said microorganism; and

b) one or more native and/or heterologous enzymes that function in a second engineered metabolic pathway to produce NADH, NADPH, or a combination thereof for use in the conversion of acetate to an alcohol, wherein said one or more native and/or heterologous enzymes is activated, upregulated or downregulated and is recombinantly introduced into said microorganism.

The acetate may be produced as a by-product of biomass processing. The alcohol is preferably selected from the group consisting of ethanol, isopropanol, or a combination thereof. The second engineered metabolic pathway to produce an electron donor may be a xylose fermentation pathway and comprises upregulation of the native and/or heterologous enzymes xylose reductase (XR) and xylitol dehydrogenase (XDH). The native and/or heterologous XDH enzyme is from *Scheffersomyces stipites* and optionally is encoded by a *xyl2* polynucleotide. The native and/or heterologous XR enzyme is preferably from *Scheffersomyces stipitis, Neurospora crassa,* or *Candida boidinii* and optionally is encoded by a *xyl1* polynucleotide or an aldolase reductase. The first and second engineered metabolic pathways preferably result in ATP production. The first engineered metabolic pathways preferably comprise activating or upregulating one or more heterologous enzymes selected from the group consisting of acetyl-CoA acetyltransferase (thiolase), acetoacetyl-CoA transferase, acetoacetate decarboxylase, a secondary alcohol dehydrogenase, and combinations thereof. The first engineered metabolic pathways may comprise activating or upregulating a heterologous ADP-producing acetyl-CoA synthase enzyme. The first engineered metabolic pathway may comprise activating or upregulating the acetate kinase/phosphotransacetylase (AK/PTA) couple. Preferably said first and second engineered metabolic pathways result in ATP production. Preferably said second engineered metabolic pathway comprises activation or upregulation of the native enzyme glucose-6-P dehydrogenase of the oxidative branch of the pentose phosphate pathway (PPP). Preferably said native glucose-6-P dehydrogenase enzyme is from *Saccharomyces cerevisiae,* and optionally is encoded by a *zwf1* polynucleotide. Preferably further comprises altering the expression of transcription factors that regulate expression of enzymes of the pentose phosphate pathway (PPP). Preferably the transcription factor is Stb5p, and optionally is from *Saccharomyces cerevisiae.* Preferably said second engineered metabolic pathway comprises downregulation of the native enzyme glucose-6-P isomerase that competes with the oxidative branch of the PPP. Preferably said native glucose-6-P isomerase enzyme is from *Saccharomyces cerevisiae,* and optionally is encoded by a *pgi1* polynucleotide. Preferably said second engineered metabolic pathway to produce an electron donor comprises the ribulose-monophosphate pathway (RuMP) that converts fructose-6-P to ribulose-5-P and formaldehyde. Preferably said RuMP pathway comprises upregulating a heterologous enzyme selected from the group consisting of 6-phospho-3-hexuloisomerase, 3-hexulose-6-phosphate synthase, and the combination thereof. Preferably further comprises upregulating native enzymes that degrade formaldehyde or formate. The formaldehyde degrading enzymes are formaldehyde dehydrogenase and S-formylglutathione hydrolase. The formate degrading enzyme is preferably formate dehydrogenase. The second engineered metabolic pathway to produce an electron donor preferably comprises the dihydroxyacetone (DHA) pathway that interconverts dihydroxyacetone and glyceraldehyde-3-P into xylose-5-P and formaldehyde. The DHA pathway preferably comprises upregulating the heterologous enzyme formaldehyde transketolase (EC 2.2.1.3). The recombinant microorganism preferably further comprises upregulating native and/or heterologous enzymes that produce dihydroxyacetone, and optionally are enzymes selected from the group consisting of glycerol dehydrogenase, dihydroxyacetone phosphatase, and a combination thereof. Preferably said native and/or heterologous glycerol dehydrogenase is from a microorganism selected from the group consisting of *Hansenula polymorpha, E. coli, Pichia angusta,* and *Saccharomyces cerevisiae,* and optionally are encoded by a polynucleotide selected from the group consisting of *gdh, gldA,* and *gcy1.* The formaldehyde is preferably oxidized to form $CO_2$. The one or more second engineered metabolic pathways to produce an electron donor preferably comprises downregulating a native dihydroxyacetone kinase enzyme encoded by a polynucleotide selected from the group consisting of *dak1, dak2,* and a combination thereof. The microorganism preferably further comprises overexpression of a glycerol/proton-symporter, and optionally is encoded by a *stl1* polynucleotide. The microorganism properly further comprises overexpression of a native and/or heterologous transhydrogenase enzyme that catalyzes the interconversion of NADPH and NAD to NADP and NADH, and optionally is from a microorganism selected from the group consisting of *Escherichia coli* and *Azotobacter vinelandii.*

The microorganism preferably further comprises overexpression of a native and/or heterologous glutamate dehydrogenase enzyme, and optionally is encoded by a *gdh2* polynucleotide. One of said engineered metabolic pathways preferably comprises the following steps: (a) conversion of acetate to acetyl-CoA and (b) conversion of acetyl-CoA to ethanol. The one or more downregulated native enzymes is preferably encoded by a *gpd1* polynucleotide, a *gpd2* polynucleotide, or both a *gpd1* polynucleotide and a *gpd2* polynucleotide. The acetyl-CoA is preferably converted to acetaldehyde by an acetaldehyde dehydrogenase; and wherein said acetaldehyde is converted to ethanol by an alcohol dehydrogenase. The acetaldehyde dehydrogenase is preferably an NADPH-specific acetaldehyde dehydrogenase, and optionally is *T. pseudethanolicus* adhB. The alcohol dehydrogenase is preferably an NADPH-specific alcohol dehydrogenase, and optionally is from a microorganism selected from the group consisting of *T. pseudethanolicus, C. beijerinckii, Entamoeba histolytica, Cucumis melo,* and *S. cerevisiae.* The acetyl-CoA is preferably converted to ethanol by a bifunctional acetaldehyde/alcohol dehydrogenase, and optionally is from *E. coli, C. acetobutylicum, T. saccharolyticum, C. thermocellum,* or *C. phytofermentans.*

According to another aspect of the invention there is:
a recombinant microorganism comprising

a) one or more native and/or heterologous enzymes that function in one or more first engineered metabolic pathways to convert acetate to acetone, wherein said one or more native and/or heterologous enzymes is activated, upregulated or downregulated and is recombinantly introduced into said microorganism; and
b) one or more native and/or heterologous enzymes that function in one or more second engineered metabolic pathways to produce an electron donor used in the conversion of acetate to isopropanol, wherein said one or more native and/or heterologous enzymes is activated, upregulated or downregulated and is recombinantly introduced into said microorganism;
wherein one of said first or second engineered metabolic pathways comprises the following steps: (a) conversion of acetate to acetyl-CoA; (b) conversion of acetyl-CoA to acetoacetyl-CoA; (c) conversion of acetoacetyl-CoA to acetoacetate; (d) conversion of acetoacetate to acetone; and (e) conversion of acetone to isopropanol.

According to another aspect of the invention there is:
a process for converting biomass to ethanol, acetone, or isopropanol comprising contacting biomass with a recombinant microorganism as described according to any of the preceeding statements.

The biomass preferably comprises lignocellulosic biomass, and optionally is selected from the group consisting of grass, switch grass, cord grass, rye grass, reed canary grass, mixed prairie grass, miscanthus, sugar-processing residues, sugarcane bagasse, sugarcane straw, agricultural wastes, rice straw, rice hulls, barley straw, corn cobs, cereal straw, wheat straw, canola straw, oat straw, oat hulls, corn fiber, stover, soybean stover, corn stover, forestry wastes, recycled wood pulp fiber, paper sludge, sawdust, hardwood, softwood, agave, and combinations thereof. The process reduces or removes acetate from the consolidated bioprocessing (CBP) media.

According to another aspect of the invention there is:
an engineered metabolic pathway for reducing or removing acetate from consolidated bioprocessing (CBP) media as described in any of the preceeding statements.

According to another aspect of the invention there is:
a method for increasing acetate uptake from a biomass comprising contacting said biomass with a recombinant microorganism as described in any of the preceeding statements.

The method may comprise increasing the amount of sugars of the biomass, and optionally wherein said sugars are increased by the addition of an exogenous sugar source to the biomass or are increased by the addition of one or more enzymes that use or break-down cellulose, hemicellulose and/or other biomass components.

The sugars are preferably increased by the addition of a CBP microorganism that uses or breaks-down cellulose, hemicellulose and/or other biomass components.

BRIEF DESCRIPTION OF THE DRAWINGS/FIGURES

Figure 1 shows a schematic for a pathway for converting acetate to ethanol using the endogenous acetyl-CoA synthetase (ACS).
Figure 2 shows a schematic for a pathway for converting acetate to ethanol using an ADP-ACS or the acetate kinase/phospho-transacetylase (AK/PTA) couple.
Figure 3 shows a schematic for a pathway for converting acetate to isopropanol using ACS, acetyl-CoA acetyltransferase (ACoAAT), acetoacetyl-CoA transferase (ACoAT), acetoacetate decarboxylase (ADC), and secondary alcohol dehydrogenase (SADH).

Figure 4 shows a schematic for a pathway for converting xylose to ethanol using either xylose isomerase, for which the conversion is redox neutral, or an NADP+-dependent xylose reductase and NADH-dependent xylitol dehydrogenase, in which case an NADPH shortage and NADH surplus is created. This NADPH shortage can be relieved by directing part of the carbon flux through the oxidative pentose phosphate pathway, which generates 2 NADPH for every $CO_2$ formed.

Figure 5 shows a schematic for a ribulose-monophosphate (RuMP) pathway for converting fructose 6-P to ribulose 5-phosphate and $CO_2$ to generate 2 NADH.

Figure 6 shows a schematic for a dihydroxyacetone (DHA) pathway for converting glycerol or dihydroxyacetone phosphate to DHA and its subsequent conversion to $CO_2$ to generate 2 NADH.

Figure 7 shows a schematic for integration of *B. adolescentis* AdhE in the GPD1 locus.

Figure 8 depicts a vector used for integration of *B. adolescentis* AdhE in the GPD1 locus.

Figure 9 shows a schematic for integration of *B. adolescentis* AdhE in the GPD2 locus.

Figure 10 depicts a vector used for integration of *B. adolescentis* AdhE in the GPD2 locus.

Figure 11 shows a schematic for integration of GDH2 in the FCY1 locus.

Figure 12 depicts a vector used for integration of GDH2 in the FCY1 locus.

Figure 13 shows a schematic for integration of endogenous pentose phosphate genes TAL1, XKS1, TKL1, RPE1, and RKI1 in the GRE3 locus.

Figure 14 depicts a vector used for integration of endogenous pentose phosphate genes TAL1, XKS1, TKL1, RPE1, and RKI1 in the GRE3 locus.

Figure 15 shows a schematic for integration of *Scheffersomyces stipites* XYL1 and XYL2 genes and *Piromyces* sp. E2 adhE gene in the GPD1 locus.

Figure 16 depicts a vector used for integration of *Scheffersomyces stipites* XYL1 and XYL2 genes and *Piromyces* sp. E2 adhE gene in the GPD1 locus.

Figure 17 shows a schematic for integration of STB5 and GDH2 in the FCY1 locus.

Figure 18 depicts a vector used for integration of STB5 and GDH2 in the FCY1 locus.

Figure 19 shows a schematic for integration of *Mycobacterium gastri* rmpA, *O. polymorpha* formaldehyde dehydrogenase, *O. polymorpha* formate dehydrogenase, and *Mycobacterium gastri* rmpB in the FCY1 locus.

Figure 20 depicts a vector used for integration of *Mycobacterium gastri* rmpA, *O. polymorpha* formaldehyde dehydrogenase, *O. polymorpha* formate dehydrogenase, and *Mycobacterium gastri* rmpB in the FCY1 locus.

Figure 21 shows schematics for deletion of the DAK1 and DAK2 genes.

Figure 22 shows a schematic for deletion of the DAK1 gene.

Figure 23 shows a schematic for deletion of the DAK2 gene.

Figure 24 shows a schematic for integration of *O. polymorpha* glycerol dehydrogenase, *O. polymorpha* formaldehyde dehydrogenase, *O. polymorpha* formate dehydrogenase, transketolase (TKL1), and *Piromyces* sp. E2 adhE in the FCY1 locus.

Figure 25 depicts a vector used for integration of *O. polymorpha* glycerol dehydrogenase, *O. polymorpha* formaldehyde dehydrogenase, *O. polymorpha* formate dehydrogenase, transketolase (TKL1), and *Piromyces* sp. E2 adhE in the FCY1 locus.

Figure 26 shows a schematic for replacing both chromosomal copies of GRE3 with an expression cassette containing genes from the pentose phosphate pathway.

Figure 27 depicts a vector for replacing both chromosomal copies of GRE3 with an expression cassette containing genes from the pentose phosphate pathway.

Figure 28 shows a schematic for integration of *T. pseudethanolicus* adhB with the Enol promoter in the FCY1 locus.

Figure 29 shows a schematic for integration of *T. pseudethanolicus* adhB with the TPIlp promoter in the FCY1 locus.

Figure 30 shows a schematic for integration of *C. beijerinckii* 2° Adh (Cbe adhB) with the Eno1p promoter in the FCY1 locus.

Figure 31 shows a schematic for integration of *C. beijerinckii* 2° Adh with the TPIlp promoter in the FCY1 locus.

Figure 32 shows a schematic for a construct used to express *C. beijerinckii* 2° Adh. Zeo depicts the Zeo cassette.

Figure 33 shows a schematic for a construct used to express ARI1 using the Enol promoter. Zeo depicts the Zeo cassette.

Figure 34 shows a schematic for a construct used to express ARI1 using the TPIlp promoter. Zeo depicts the Zeo cassette.

Figure 35 shows a schematic for a construct used to express *Entamoeba histolytica* ADH1 from the Enol promoter. Zeo depicts the Zeo cassette.

Figure 36 shows a schematic for a construct used to express *Entamoeba histolytica* ADH1 from the TPIlp promoter. Zeo depicts the Zeo cassette.

Figure 37 shows a schematic for a construct used to express *Cucumis melo* ADH1 from the Enol promoter. Zeo depicts the Zeo cassette.

Figure 38 shows a schematic for a construct used to express *Cucumis melo* ADH1 from the TPIlp promoter. Zeo depicts the Zeo cassette.

Figure 39 shows a schematic of a construct to delete ADH1.

Figure 40 shows a schematic of a construct to delete ADH1.

Figure 41 shows acetate consumption for *C. beijerinckii* 2° Adh and *Entamoeba histolytica* ADH expressed in an ADH1 wild-type, single copy deletion, or double copy deletion yeast mutants.

Figure 42 shows a schematic of an ADH1 deletion.

Figure 43 shows a schematic for a construct (MA741) used to express two copies of *Entamoeba histolytica* ADH1 (EhADH1) from the TPIlp promoter for integration at YLR296W.

Figure 44 shows a schematic for a construct (MA743) used to express two copies of *Entamoeba histolytica* ADH1 (EhADH1) from the TPIlp promoter and a copy of ZWF1 (glucose-6-P dehydrogenase) from the Enol promoter for integration at YLR296W.

Figure 45 shows a schematic for a construct (MA742) used to express two copies of *Entamoeba histolytica* ADH1 (EhADH1) from the TPIlp promoter and a copy of STB5 from the Enol promoter for integration at YLR296W.

Figure 46 shows a schematic for ethanol production and NAD(P)H balance without ADH engineering.

Figure 47 shows a schematic for ethanol production and NAD(P)H balance with ADH engineering.

Figure 48 shows a schematic for a construct (MA421) used to express a copy of *S. cerevisiae* FDH1 from the ADH1 promoter.

Figure 49 shows a schematic for a construct (MA422) used to express two copies of C. boidinii FDH3 from the TPI1 and PFK1 promoters.

Figure 50 shows a schematic for a construct used to express two copies of *Entamoeba histolytica* ADH1 (EhADH1) from the TPIlp promoter, *S. cerevisiae* STB5 from the Enol promoter, and *S. cerevisiae* ACS2 from the PYK1 promoter.

Figure 51 shows a schematic for a construct used to express two copies of *Entamoeba histolytica* ADH1 (EhADH1) from the TPIlp promoter, *S. cerevisiae* ZWF1 from the Enol promoter, and *S. cerevisiae* ACS2 from the PYK1 promoter.

Figure 52 shows a schematic for a construct used to express two copies of *Entamoeba histolytica* ADH1 (EhADH1) from the TPIlp promoter and *S. cerevisiae* ACS2 from the PYK1 promoter.

Figure 53 shows a schematic for a construct used to express the NADPH-ADH from *E. histolytica.*

Figure 54 shows a schematic for assembly MA1181 used to replace the endogenous FCY1 ORF with a two-copy expression cassette of *E. histolytica* ADH1.

Figure 55 shows a schematic for assembly MA905 used to introduce two copies of *E. coli* udhA into the apt2 locus.

Figure 56 shows a schematic for assembly MA483 used to introduce two copies of *E. coli* udhA into the YLR296W locus.

Figure 57A shows ethanol production from pressure bottle fermentations on pre-treated agricultural waste by control strains and strains expressing *E. coli* udhA.

Figure 57B shows acetate consumption from pressure bottle fermentations on pre-treated agricultural waste by control strains and strains expressing *E. coli* udhA.

Figure 57C shows glycerol production from pressure bottle fermentations on pre-treated agricultural waste by control strains and strains expressing *E. coli* udhA.

Figure 58A shows ethanol production from pressure bottle fermentations on pre-treated corn stover by control strains and strains expressing *E. coli* udhA.

Figure 58B shows acetate consumption from pressure bottle fermentations on pre-treated corn stover by control strains and strains expressing *E. coli* udhA.

Figure 58C shows glycerol production from pressure bottle fermentations on pre-treated corn stover by control strains and strains expressing *E. coli* udhA.

Figure 59 shows a schematic for a contstruct that can used to express *Azotobacter vinelandii* sthA.

## DETAILED DESCRIPTION OF THE INVENTION

Aspects of the present invention relate to the engineering of a microorganism to detoxify biomass-derived acetate via metabolic conversion to ethanol, acetone, or isopropanol by improving the availability of redox cofactors NADH or NADPH. To overcome the inhibitory effects of acetate, the acetate can be converted to a less inhibitory compound that is a product of bacterial or yeast fermentation, as described herein. Less inhibitory compounds such as ethanol, acetone, or isopropanol, can be readily recovered from the fermentation media. In addition, the present invention relates to the engineering of a microorganism to provide additional electron donors, thereby producing additional electrons, which facilitate more efficient conversion of acetate to the less inhibitory compounds. Additional advantages of the present invention over existing means for reducing acetate include:

- Reduced cost compared to chemical or physical acetate removal systems;
- Reduced loss of sugar yield (washing) compared to chemical or physical acetate removal systems;
- Reduced demand for base addition during fermentation;
- Reduced overall fermentation cost;
- Improved pH control;
- Reduced costs, including capital, operating, and environmental, for wastewater treatment and water recycling; and
- Improved metabolic conversion of acetate by optimization of pathways that produce or balance electron donors.

**Definitions**

Unless defined otherwise, all technical and scientific terms used herein have the same meaning as commonly understood by one of ordinary skill in the art to which this invention belongs. Also, unless otherwise required by context, singular terms shall include pluralities and plural terms shall include the singular. All publications, patents and other references mentioned herein are incorporated by reference in their entireties for all purposes.

The term "heterologous" when used in reference to a polynucleotide, a gene, a polypeptide, or an enzyme refers to a polynucleotide, gene, polypeptide, or an enzyme not normally found in the host organism. "Heterologous" also includes a native coding region, or portion thereof, that is reintroduced into the source organism in a form that is different from the corresponding native gene, *e.g.,* not in its natural location in the organism's genome. The heterologous polynucleotide or gene may be introduced into the host organism by, *e.g.,* gene transfer. A heterologous gene may include a native coding region that is a portion of a chimeric gene including non-native regulatory regions that is reintroduced into the native host. Foreign genes can comprise native genes inserted into a non-native organism, or chimeric genes.

The term "heterologous polynucleotide" is intended to include a polynucleotide that encodes one or more polypeptides or portions or fragments of polypeptides. A heterologous polynucleotide may be derived from any source, *e.g.*, eukaryotes, prokaryotes, viruses, or synthetic polynucleotide fragments.

The terms "promoter" or "surrogate promoter" is intended to include a polynucleotide that can transcriptionally control a gene-of-interest that it does not transcriptionally control in nature. In certain embodiments, the transcriptional control of a surrogate promoter results in an increase in expression of the gene-of-interest. In certain embodiments, a surrogate promoter is placed 5' to the gene-of-interest. A surrogate promoter may be used to replace the natural promoter, or may be used in addition to the natural promoter. A surrogate promoter may be endogenous with regard to the host cell in which it is used, or it may be a heterologous polynucleotide sequence introduced into the host cell, *e.g.,* exogenous with regard to the host cell in which it is used.

The terms "gene(s)" or "polynucleotide" or "polynucleotide sequence(s)" are intended to include nucleic acid molecules, *e.g.*, polynucleotides which include an open reading frame encoding a polypeptide, and can further include non-coding regulatory sequences, and introns. In addition, the terms are intended to include one or more genes that map to a functional locus. In addition, the terms are intended to include a specific gene for a selected purpose. The gene may be endogenous to the host cell or may be recombinantly introduced into the host cell, *e.g.,* as a plasmid maintained episomally or a plasmid (or fragment thereof) that is stably integrated into the genome. In addition to the plasmid form, a gene may, for example, be in the form of linear DNA. In certain embodiments, the gene or polynucleotide is involved in at least one step in the bioconversion of acetate to a non-charged solvent, including but not limited to, acetone, isopropanol, or ethanol. Accordingly, the term is intended to include any gene encoding a polypeptide, such as the enzymes acetate kinase (ACK), phosphotransacetylase (PTA), lactate dehydrogenase (LDH), pyruvate formate lyase (PFL), aldehyde dehydrogenase (ADH) and/or alcohol dehydrogenase (ADH), acetyl-CoA transferase (ACS), acetaldehyde dehydrogenase, acetaldehyde/alcohol dehydrogenase (*e.g.,* a bifunctional acetaldehyde/alcohol dehydrogenase), glycerol-3-phosphate dehydrogenase (GPD), acetyl-CoA synthetase, thiolase, CoA transferase, acetoacetate decarboxylase, alcohol acetyltransferase enzymes in the D-xylose pathway, such as xylose isomerase and xylulokinase, enzymes in the L-arabinose pathway, such as L-arabinose isomerase and L-ribulose-5-phosphate 4-epimerase. The term gene is also intended to cover all copies of a particular gene, *e.g.,* all of the DNA sequences in a cell encoding a particular gene product.

The term "transcriptional control" is intended to include the ability to modulate gene expression at the level of transcription. In certain embodiments, transcription, and thus gene expression, is modulated by replacing or adding a surrogate promoter near the 5' end of the coding region of a gene-of-interest, thereby resulting in altered gene expression. In certain embodiments, the transcriptional control of one or more genes is engineered to result in the optimal expression of such genes, *e.g.,* in a desired ratio. The term also includes inducible transcriptional control as recognized in the art.

The term "expression" is intended to include the expression of a gene at least at the level of mRNA production.

The term "expression product" is intended to include the resultant product, *e.g.,* a polypeptide, of an expressed gene.

The term "increased expression" is intended to include an alteration in gene expression at least at the level of increased mRNA production and, preferably, at the level of polypeptide expression. The term "increased production" is intended to include an increase in the amount of a polypeptide expressed, in the level of the enzymatic activity of the polypeptide, or a combination thereof, as compared to the native production of, or the enzymatic activity, of the polypeptide.

The terms "activity," "activities," "enzymatic activity," and "enzymatic activities" are used interchangeably and are intended to include any functional activity normally attributed to a selected polypeptide when produced under favorable conditions. Typically, the activity of a selected polypeptide encompasses the total enzymatic activity associated with the produced polypeptide. The polypeptide produced by a host cell and having enzymatic activity may be located in the intracellular space of the cell, cell-associated, secreted into the extracellular milieu, or a combination thereof. Techniques for determining total activity as compared to secreted activity are described herein and are known in the art.

The term "xylanolytic activity" is intended to include the ability to hydrolyze glycosidic linkages in oligopentoses and polypentoses.

The term "cellulolytic activity" is intended to include the ability to hydrolyze glycosidic linkages in oligohexoses and polyhexoses. Cellulolytic activity may also include the ability to depolymerize or debranch cellulose and hemicellulose.

As used herein, the term "lactate dehydrogenase" or "LDH" is intended to include the enzymes capable of converting pyruvate into lactate. It is understood that LDH can also catalyze the oxidation of hydroxybutyrate. LDH includes those enzymes that correspond to Enzyme Commission Number 1.1.1.27.

As used herein the term "alcohol dehydrogenase" or "ADH" is intended to include the enzymes capable of converting acetaldehyde into an alcohol, such as ethanol. ADH also includes the enzymes capable of converting acetone to isopropanol. ADH includes those enzymes that correspond to Enzyme Commission Number 1.1.1.1.

As used herein, the term "phosphotransacetylase" or "PTA" is intended to include the enzymes capable of converting acetyl-phosphate into acetyl-CoA. PTA includes those enzymes that correspond to Enzyme Commission Number 2.3.1.8.

As used herein, the term "acetate kinase" or "ACK" is intended to include the enzymes capable of converting acetate into acetyl-phosphate. ACK includes those enzymes that correspond to Enzyme Commission Number 2.7.2.1.

As used herein, the term "pyruvate formate lyase" or "PFL" is intended to include the enzymes capable of converting pyruvate into acetyl-CoA and formate. PFL includes those enzymes that correspond to Enzyme Commission Number 2.3.1.54.

As used herein, the term "acetaldehyde dehydrogenase" or "ACDH" is intended to include the enzymes capable of converting acetyl-CoA to acetaldehyde. ACDH includes those enzymes that correspond to Enzyme Commission Number 1.2.1.3.

As used herein, the term "acetaldehyde/alcohol dehydrogenase" is intended to include the enzymes capable of converting acetyl-CoA to ethanol. Acetaldehyde/alcohol dehydrogenase includes those enzymes that correspond to Enzyme Commission Numbers 1.2.1.10 and 1.1.1.1.

As used herein, the term "glycerol-3-phosphate dehydrogenase" or "GPD" is intended to include the enzymes capable of converting dihydroxyacetone phosphate to glycerol-3-phosphate. GPD includes those enzymes that correspond to Enzyme Commission Number 1.1.1.8.

As used herein, the term "acetyl-CoA synthetase" or "ACS" is intended to include the enzymes capable of converting acetate to acetyl-CoA. Acetyl-CoA synthetase includes those enzymes that correspond to Enzyme Commission Number 6.2.1.1.

As used herein, the term "thiolase" is intended to include the enzymes capable of converting acetyl-CoA to acetoacetyl-CoA. Thiolase includes those enzymes that correspond to Enzyme Commission Number 2.3.1.9.

As used herein, the term "CoA transferase" is intended to include the enzymes capable of converting acetate and acetoacetyl-CoA to acetoacetate and acetyl-CoA. CoA transferase includes those enzymes that correspond to Enzyme Commission Number 2.8.3.8.

As used herein, the term "acetoacetate decarboxylase" is intended to include the enzymes capable of converting acetoacetate to acetone and carbon dioxide. Acetoacetate decarboxylase includes those enzymes that correspond to Enzyme Commission Number 4.1.1.4.

As used herein, the term "alcohol acetyltransferase" is intended to include the enzymes capable of converting acetyl-CoA and ethanol to ethyl acetate. Alcohol acetyltransferase includes those enzymes that correspond to Enzyme Commission Number 2.3.1.84.

The term "pyruvate decarboxylase activity" is intended to include the ability of a polypeptide to enzymatically convert pyruvate into acetaldehyde and carbon dioxide (*e.g.,* "pyruvate decarboxylase" or "PDC"). Typically, the activity of a selected polypeptide encompasses the total enzymatic activity associated with the produced polypeptide, comprising, *e.g.,* the superior substrate affinity of the enzyme, thermostability, stability at different pHs, or a combination of these attributes. PDC includes those enzymes that correspond to Enzyme Commission Number 4.1.1.1.

A "xylose metabolizing enzyme" can be any enzyme involved in xylose digestion, metabolism and/or hydrolysis, including a xylose isomerase, xylulokinase, xylose reductase, xylose dehydrogenase, xylitol dehydrogenase, xylonate dehydratase, a transketolase, and a transaldolase protein.

A "xylulokinase" (XK) as used herein, is meant for refer to an enzyme that catalyzes the chemical reaction: ATP + D-xylulose ⇄ ADP + D-xylulose 5-phosphate. Thus, the two substrates of this enzyme are ATP and D-xylulose, whereas its two products are ADP and D-xylulose 5-phosphate. This enzyme belongs to the family of transferases, specifically those transferring phosphorus-containing groups (phosphotransferases) with an alcohol group as acceptor. The systematic name of this enzyme class is ATP:D-xylulose 5-phosphotransferase. Other names in common use include xylulokinase (phosphorylating), and D-xylulokinase. This enzyme participates in pentose and glucuronate interconversions. XK includes those enzymes that correspond to Enzyme Commission Number 2.7.1.17.

A "xylose isomerase" (XI) as used herein, is meant to refer to an enzyme that catalyzes the chemical reaction: D-xylose ⇄ D-xylulose. This enzyme belongs to the family of isomerases, specifically those intramolecular oxidoreductases interconverting aldoses and ketoses. The systematic name of this enzyme class is D-xylose aldose-ketose-isomerase. Other names in common use include D-xylose isomerase, D-xylose ketoisomerase, and D-xylose ketol-isomerase. This enzyme participates in pentose and glucuronate interconversions and fructose and mannose metabolism. The enzyme is used industrially to convert glucose to fructose in the manufacture of high-fructose corn syrup. It is sometimes referred to as "glucose isomerase". XI includes those enzymes that correspond to Enzyme Commission Number 5.3.1.5.

As used herein, the term "glucose-6-phosphate isomerase" is intended to include the enzymes capable of converting glucose-6-phosphate into fructose-6-phosphate. Glucose-6-phosphate isomerases include those enzymes that correspond to Enzyme Commission Number 5.3.1.9.

As used herein, the term "transhydrogenase" is intended to include the enzymes capable of converting NADPH and $NAD^+$ to $NADP^+$ and NADH. Transhydrogenases include those enzymes that correspond to Enzyme Commission Number 1.6.1.1.

As used herein, the term "xylose reductase" is intended to include the enzymes capable of converting xylose and $NADP^+$ to NADPH and xylitol. Xylose reductases include those enzymes that correspond to Enzyme Commission Number 1.1.1.307.

As used herein, the term "xylitol dehydrogenase" is intended to include the enzymes capable of converting xylitol and $NAD^+$ to NADH and xylulose. Xylitol dehydrogenases include those enzymes that correspond to Enzyme Commission Numbers 1.1.1.9, 1.1.1.10, and 1.1.1. B19.

As used herein, the term "glucose-6-phosphate dehydrogenase" or "glucose-6-P dehydrogenase" is intended to include the enzymes capable of converting glucose-6-phosphate and $NADP^+$ to NADPH and 6-phosphoglucono-δ-lactone. Glucose-6-phosphate dehydrogenases include those enzymes that correspond to Enzyme Commission Number 1.1.1.49.

As used herein, the term "6-phospho-3-hexuloisomerase" or "PHI" is intended to include the enzymes capable of converting fructose-6-P to D-arabino-3-hexulose-6-P. 6-phospho-3-hexuloisomerases include those enzymes that correspond to Enzyme Commission Number 5.3.1.27.

As used herein, the term "3-hexulose-6-phosphate synthase" or "HPS" is intended to include the enzymes capable of converting D-arabino-3-hexulose-6-P to ribulose-5-phosphate and formaldehyde. 3-hexulose-6-phosphate synthases include those enzymes that correspond to Enzyme Commission Number 4.1.2.43.

As used herein, the term "formaldehyde dehydrogenase" is intended to include the enzymes capable of converting formaldehyde and $NAD^+$ to NADH and formate. Formaldehyde dehydrogenases include those enzymes that correspond to Enzyme Commission Number 1.2.1.46.

As used herein, the term "S-formylglutathione hydrolase" is intended to include the enzymes capable of converting s-formylglutathione to glutathione and formate. S-formylglutathione hydrolases include those enzymes that correspond to Enzyme Commission Number 3.1.2.12.

As used herein, the term "formate dehydrogenase" is intended to include the enzymes capable of converting formate and $NAD^+$ to NADH and $CO_2$. Formate dehydrogenases include those enzymes that correspond to Enzyme Commission Number 1.2.1.2.

As used herein, the term "formaldehyde transketolase" is intended to include the enzymes capable of converting dihydroxyacetone and glyceraldehyde-3-P to xylulose-5-P and formaldehyde. Formaldehyde transketolases include those enzymes that correspond to Enzyme Commission Number 2.2.1.3.

As used herein, the term "dihydroxyacetone phosphatase" is intended to include the enzymes capable of converting dihydroxyacetone-phosphate to dihydroxyacetone. Dihydroxyacetone phosphatases include those enzymes that correspond to Enzyme Commission Number 3.1.3.1. *See also* Filburn, C.R., "Acid Phosphatase Isozymes of Xenoupus laevis Tadpole Tails: I. Spearation and Partial Characterization," Archives of Biochem. And Biophysics 159:683-93 (1973).

As used herein, the term "dihydroxyacetone kinase" is intended to include the enzymes capable of converting dihydroxyacetone to dihydroxyacetone phosphate. Dihydroxyacetone kinases include those enzymes that correspond to Enzyme Commission Number 2.7.1.29.

As used herein, the term "glutamate dehydrogenase" is intended to include the enzymes capable of converting L-glutamate and NAD(P)$^+$ to 2-oxoglutarate and NAD(P)H. Glutamate dehydrogenases include those enzymes that correspond to Enzyme Commission Numbers 1.4.1.2, 1.4.1.3, and 1.4.1.4.

The term "ethanologenic" is intended to include the ability of a microorganism to produce ethanol from a carbohydrate as a fermentation product. The term is intended to include, but is not limited to, naturally occurring ethanologenic organisms, ethanologenic organisms with naturally occurring or induced mutations, and ethanologenic organisms which have been genetically modified.

The terms "fermenting" and "fermentation" are intended to include the enzymatic process (*e.g.,* cellular or acellular, *e.g.,* a lysate or purified polypeptide mixture) by which ethanol is produced from a carbohydrate, in particular, as a product of fermentation.

The term "secreted" is intended to include the movement of polypeptides to the periplasmic space or extracellular milieu. The term "increased secretion" is intended to include situations in which a given polypeptide is secreted at an increased level (*i.e.,* in excess of the naturally-occurring amount of secretion). In certain embodiments, the term "increased secretion" refers to an increase in secretion of a given polypeptide that is at least about 10% or at least about 100%, 200%, 300%, 400%, 500%, 600%, 700%, 800%, 900%, 1000%, or more, as compared to the naturally-occurring level of secretion.

The term "secretory polypeptide" is intended to include any polypeptide(s), alone or in combination with other polypeptides, that facilitate the transport of another polypeptide from the intracellular space of a cell to the extracellular milieu. In certain embodiments, the secretory polypeptide(s) encompass all the necessary secretory polypeptides sufficient to impart secretory activity to a Gram-negative or Gram-positive host cell or to a yeast host cell. Typically, secretory proteins are encoded in a single region or locus that may be isolated from one host cell and transferred to another host cell using genetic engineering. In certain embodiments, the secretory polypeptide(s) are derived from any bacterial cell having secretory activity or any yeast cell having secretory activity. In certain embodiments, the secretory polypeptide(s) are derived from a host cell having Type II secretory activity. In certain embodiments, the host cell is a thermophilic bacterial cell. In certain embodiments, the host cell is a yeast cell.

The term "derived from" is intended to include the isolation (in whole or in part) of a polynucleotide segment from an indicated source or the purification of a polypeptide from an indicated source. The term is intended to include, for example, direct cloning, PCR amplification, or artificial synthesis from or based on a sequence associated with the indicated polynucleotide source.

By "thermophilic" is meant an organism that thrives at a temperature of about 45°C or higher.

By "mesophilic" is meant an organism that thrives at a temperature of about 20-45°C.

The term "organic acid" is art-recognized. "Organic acid," as used herein, also includes certain organic solvents such as ethanol. The term "lactic acid" refers to the organic acid 2-hydroxypropionic acid in either the free acid or salt form. The salt form of lactic acid is referred to as "lactate" regardless of the neutralizing agent, *i.e.,* calcium carbonate or ammonium hydroxide. The term "acetic acid" refers to the organic acid methanecarboxylic acid, also known as ethanoic acid, in either free acid or salt form. The salt form of acetic acid is referred to as "acetate."

Certain embodiments of the present invention provide for the "insertion," (*e.g.,* the addition, integration, incorporation, or introduction) of certain genes or particular polynucleotide sequences within thermophilic or mesophilic microorganisms, which insertion of genes or particular polynucleotide sequences may be understood to encompass "genetic modification(s)" or "transformation(s)" such that the resulting strains of said thermophilic or mesophilic microorganisms may be understood to be "genetically modified" or "transformed." In certain embodiments, strains may be of bacterial, fungal, or yeast origin.

Certain embodiments of the present invention provide for the "inactivation" or "deletion" of certain genes or particular polynucleotide sequences within thermophilic or mesophilic microorganisms, which "inactivation" or "deletion" of genes or particular polynucleotide sequences may be understood to encompass "genetic modification(s)" or "transformation(s)" such that the resulting strains of said thermophilic or mesophilic microorganisms may be understood to be "genetically modified" or "transformed." In certain embodiments, strains may be of bacterial, fungal, or yeast origin.

The term "CBP organism" is intended to include microorganisms of the invention, *e.g.*, microorganisms that have properties suitable for CBP.

In one aspect of the invention, the genes or particular polynucleotide sequences are inserted to activate the activity for which they encode, such as the expression of an enzyme. In certain embodiments, genes encoding enzymes in the metabolic production of ethanol, *e.g.*, enzymes that metabolize pentose and/or hexose sugars, may be added to a mesophilic or thermophilic organism. In certain embodiments of the invention, the enzyme may confer the ability to metabolize a pentose sugar and be involved, for example, in the D-xylose pathway and/or L-arabinose pathway. In certain embodiments of the invention, genes encoding enzymes in the conversion of acetate to a non-charged solvent, including but not limited to, acetone, isopropanol, or ethanol, may be added to a mesophilic or thermophilic organism.

In one aspect of the invention, the genes or particular polynucleotide sequences are partially, substantially, or completely deleted, silenced, inactivated, or down-regulated in order to inactivate the activity for which they encode, such as the expression of an enzyme. Deletions provide maximum stability because there is no opportunity for a reverse mutation to restore function. Alternatively, genes can be partially, substantially, or completely deleted, silenced, inactivated, or down-regulated by insertion of nucleic acid sequences that disrupt the function and/or expression of the gene (*e.g.,* PI transduction or other methods known in the art). The terms "eliminate," "elimination," and "knockout" are used interchangeably with the terms "deletion," "partial deletion," "substantial deletion," or "complete deletion." In certain embodiments, strains of thermophilic or mesophilic microorganisms of interest may be engineered by site directed homologous recombination to knockout the production of organic acids. In still other embodiments, RNAi or antisense DNA (asDNA) may be used to partially, substantially, or completely silence, inactivate, or down-regulate a particular gene of interest.

In certain embodiments, the genes targeted for deletion or inactivation as described herein may be endogenous to the native strain of the microorganism, and may thus be understood to be referred to as "native gene(s)" or "endogenous gene(s)." An organism is in "a native state" if it has not been genetically engineered or otherwise manipulated by the hand of man in a manner that intentionally alters the genetic and/or phenotypic constitution of the organism. For example, wild-type organisms may be considered to be in a native state. In other embodiments, the gene(s) targeted for deletion or inactivation may be non-native to the organism.

Similarly, the enzymes of the invention as described herein can be endogenous to the native strain of the microorganism, and can thus be understood to be referred to as "native" or "endogenous."

The term "upregulated" means increased in activity, *e.g*., increase in enzymatic activity of the enzyme as compared to activity in a native host organism.

The term "downregulated" means decreased in activity, *e.g*., decrease in enzymatic activity of the enzyme as compared to activity in a native host organism.

The term "activated" means expressed or metabolically functional.

The term "adapted for growing" means selection of an organism for growth under conditions in which the organism does not otherwise grow or in which the organism grows slowly or minimally. Thus, an organism that is said to be adapted for growing under the selected condition, grows better than an organism that has not been adapted for growing under the selected conditions. Growth can be measured by any methods known in the art, including, but not limited to, measurement of optical density or specific growth rate.

The term "biomass inhibitors" means the inhibitors present in biomass that inhibit processing of the biomass by organisms, including but not limited to, CBP organisms. Biomass inhibitors include, but are not limited to, acids, including without limitation, acetic, lactic, 2-furoic, 3,4-dihydroxybenzoic, 3,5-dihydroxybenzoic, vanillic, homovanillic, syringic, gallic, and ferulic acids; aldehydes, including without limitation, 5-hydroxymethylfurfural, furfural, 3,4-hydroxybenzaldehyde, vanillin, and syringaldehyde. Biomass inhibitors include products removed from pretreated cellulosic material or produced as a result of treating or processing cellulosic material, including but not limited to, inhibitors removed from pretreated mixed hardwood or any other pretreated biomass.

## Biomass

Biomass can include any type of biomass known in the art or described herein. The terms "lignocellulosic material," "lignocellulosic substrate," and "cellulosic biomass" mean any type of biomass comprising cellulose, hemicellulose, lignin, or combinations thereof, such as but not limited to woody biomass, forage grasses, herbaceous energy crops, non-woody-plant biomass, agricultural wastes and/or agricultural residues, forestry residues and/or forestry wastes, paper-production sludge and/or waste paper sludge, waste-water-treatment sludge, municipal solid waste, corn fiber from wet and dry mill corn ethanol plants, and sugar-processing residues. The terms "hemicellulosics," "hemicellulosic portions," and "hemicellulosic fractions" mean the non-lignin, non-cellulose elements of lignocellulosic material, such as but not limited to hemicellulose (*i.e.,* comprising xyloglucan, xylan, glucuronoxylan, arabinoxylan, mannan, glucomannan, and galactoglucomannan, among others), pectins (*e.g.,* homogalacturonans, rhamnogalacturonan I and II, and xylogalacturonan), and proteoglycans (*e.g.,* arabinogalactan-protein, extensin, and proline-rich proteins).

In a non-limiting example, the lignocellulosic material can include, but is not limited to, woody biomass, such as recycled wood pulp fiber, sawdust, hardwood, softwood, and combinations thereof; grasses, such as switch grass, cord grass, rye grass, reed canary grass, miscanthus, or a combination thereof; sugar-processing residues, such as but not limited to sugar cane bagasse; agricultural wastes, such as but not limited to rice straw, rice hulls, barley straw, corn cobs, cereal straw, wheat straw, canola straw, oat straw, oat hulls, and corn fiber; stover, such as but not limited to soybean stover, corn stover; succulents, such as but not limited to, Agave; and forestry wastes, such as but not limited to, recycled wood pulp fiber, sawdust, hardwood (*e.g.,* poplar, oak, maple, birch, willow), softwood, or any combination thereof. Lignocellulosic material may comprise one species of fiber; alternatively, lignocellulosic material may comprise a mixture of fibers that originate from different lignocellulosic materials. Other lignocellulosic materials are agricultural wastes, such as cereal straws, including wheat straw, barley straw, canola straw and oat straw; corn fiber; stovers, such as corn stover and soybean stover; grasses, such as switch grass, reed canary grass, cord grass, and miscanthus; or combinations thereof.

Paper sludge is also a viable feedstock for lactate or acetate production. Paper sludge is solid residue arising from pulping and paper-making, and is typically removed from process wastewater in a primary clarifier. At a disposal cost of $30/wet ton, the cost of sludge disposal equates to $5/ton of paper that is produced for sale. The cost of disposing of wet sludge is a significant incentive to convert the material for other uses, such as conversion to ethanol. Processes provided by the present invention are widely applicable. Moreover, the saccharification and/or fermentation products may be used to produce ethanol or higher value added chemicals, such as organic acids, aromatics, esters, acetone and polymer intermediates.

**Acetate**

Acetate is produced from acetyl-CoA in two reaction steps catalyzed by phosphotransacetlyase (PTA) and acetate kinase (ACK). The reactions mediated by these enzymes are shown below:

PTA reaction: acetyl-CoA + phosphate = CoA + acetyl phosphate (EC 2.3.1.8)
ACK reaction: ADP + acetyl phosphate = ATP + acetate (EC 2.7.2.1)

Both *C. thermocellum* and *C. cellulolyticum* make acetate under standard fermentation conditions and have well annotated genes encoding PTA and ACK (see Table 7 of Published U.S. Appl. No. 2012/0094343 A1, which is incorporated by reference herein in its entirety).

**Consolidated Bioprocessing**

Consolidated bioprocessing (CBP) is a processing strategy for cellulosic biomass that involves consolidating into a single process step four biologically-mediated events: enzyme production, hydrolysis, hexose fermentation, and pentose fermentation. Implementing this strategy requires development of microorganisms that both utilize cellulose, hemicellulosics, and other biomass components while also producing a product of interest at sufficiently high yield and concentrations. The feasibility of CBP is supported by kinetic and bioenergetic analysis. *See* van Walsum and Lynd (1998) *Biotech. Bioeng.* 58:316.

**Xylose metabolism**

Xylose is a five-carbon monosaccharide that can be metabolized into useful products by a variety of organisms. There are two main pathways of xylose metabolism, each unique in the characteristic enzymes they utilize. One pathway is called the "Xylose Reductase-Xylitol Dehydrogenase" or XR-XDH pathway. Xylose reductase (XR) and xylitol dehydrogenase (XDH) are the two main enzymes used in this method of xylose degradation. XR, encoded by the XYL1 gene, is responsible for the reduction of xylose to xylitol and is aided by cofactors NADH or NADPH. Xylitol is then oxidized to xylulose by XDH, which is expressed through the XYL2 gene, and accomplished exclusively with the cofactor NAD+. Because of the varying cofactors needed in this pathway and the degree to which they are available for usage (*e.g.,* XR consumes NADPH and XDH produces NADH), an imbalance can result in an overproduction of xylitol byproduct and an inefficient production of desirable ethanol. Varying expression of the XR and XDH enzyme levels have been tested in the laboratory in the attempt to optimize the efficiency of the xylose metabolism pathway.

The other pathway for xylose metabolism is called the "Xylose Isomerase" (XI) pathway. Enzyme XI is responsible for direct conversion of xylose into xylulose, and does not proceed via a xylitol intermediate. Both pathways create xylulose, although the enzymes utilized are different. After production of xylulose both the XR-XDH and XI pathways proceed through enzyme xylulokinase (XK), encoded on gene XKS1, to further modify xylulose into xylulose-5-P where it then enters the pentose phosphate pathway for further catabolism. XI includes those enzymes that correspond to Enzyme Commission Number 5.3.1.5. Suitable xylose isomerases of the present invention include xylose isomerases derived from *Piromyces sp.,* and *B. thetaiotamicron,* although any xylose isomerase that functions when expressed in host cells of the invention can be used.

Studies on flux through the pentose phosphate pathway during xylose metabolism have revealed that limiting the speed of this step may be beneficial to the efficiency of fermentation to ethanol. Modifications to this flux that may improve ethanol production include a) lowering phosphoglucose isomerase activity, b) deleting the GND1 gene, and c) deleting the ZWF1 gene. Jeppsson, M., et al., "The level of glucose-6-phosphate dehydrogenase activity strongly influences xylose fermentation and inhibitor sensitivity in recombinant Saccharomyces cerevisiae strains," Yeast 20:1263-1272 (2003). Since the pentose phosphate pathway produces additional NADPH during metabolism, limiting this step will help to correct the already evident imbalance between NAD(P)H and NAD+ cofactors and reduce xylitol byproduct. An alternative approach is to improve the kinetics of the oxidative branch of the PPP over those of competing pathways. This could be achieved by various approaches, *e.g.*, by directly increasing the expression of the rate-limiting enzyme(s) of the oxidative branch of the PPP pathway, such as glucose-6-P dehydrogenase (encoded endogenously by *ZWF1*), changing the expression of regulating transcription factors like Stb5p (Cadière, A., et al., "The Saccharomyces cerevisiae zinc factor protein Stb5p is required as a basal regulator of the pentose phosphate pathway," FEMS Yeast Research 10:819-827 (2010)), or directly down-regulating the expression of genes involved in competing pathways like glucose-6-P isomerase (encoded by PGI1). Producing more $CO_2$ in the oxidative branch of the PPP would increase the availability of NADPH and increase the NADPH/NADP ratio. This would stimulate the flux of acetate-consuming pathways that (at least partially) consume NADPH, as would for example be the case for ethanol-to-isopropanol conversion that relies on a NADPH-consuming secondary alcohol dehydrogenase to convert acetone to isopropanol, or an acetate-to-ethanol pathway that uses a NADPH-consuming acetaldehyde dehydrogenase and/or alcohol dehydrogenase. Another experiment comparing the two xylose metabolizing pathways revealed that the XI pathway was best able to metabolize xylose to produce the greatest ethanol yield, while the XR-XDH pathway reached a much faster rate of ethanol production (Karhumaa et al., Microb Cell Fact. 2007 Feb 5; 6:5). *See also* U.S. Published Appl. No. 2008/0261287 A1, incorporated herein by reference in its entirety.

In one embodiment, the invention comprises combining the XR/XDH pathway for ethanolic xylose fermentation with acetate-to-ethanol conversion through the ACDH pathway. In the proposed pathway, the NADPH consumed in the XR/XDH pathway is regenerated through the pentose phosphate pathway (PPP), while the NADH produced in the XR/XDH pathway is consumed through the acetate-to-ethanol conversion. In contrast to NADH oxidation via glycerol formation, acetate consumption via ACDH results in an overall positive ATP yield. The overall pathway would allow for anaerobic growth on xylose and acetate, providing a selective pressure for improved xylose and acetate consumption and reduced glycerol and xylitol production. It would uncouple acetate uptake from biomass formation, instead providing a fixed stoichiometry between xylose and acetate uptake. This solution to the redox imbalance of the XR/XDH conversion might make the kinetically faster XR/XDH pathway a viable candidate for industrial ethanol production, while the acetate consumption can improve the ethanol yield on xylose by up to 20%. Acetate consumption would furthermore reduce the toxicity of the cellulosic feedstock hydrolysate.

**Ribulose-Monophosphate Pathway**

In another embodiment, the invention comprises introducing the heterologous ribulose-monophosphate (RuMP) pathway found in various bacteria and archaea, which also produces $CO_2$ while conferring electrons to redox carriers. The RuMP pathway relies on the expression of two heterologous genes: 6-phospho-3-hexuloisomerase(PHI) and 3-hexulose-6-phosphate synthase (HPS). PHI converts fructose-6-P to D-arabino-3-hexulose-6-P, and HPS converts the latter to ribulose-5-P and formaldehyde. While this conversion is redox neutral, the produced formaldehyde can then be converted to $CO_2$ by the action of the endogenous enzymes formaldehyde dehydrogenase and S-formylglutathione hydrolase (which produce formate and NADH) and formate dehydrogenase (which convert the formate to $CO_2$, producing a second NADH).

The RuMP pathway has been characterized as a reversible pathway, and many of the characterized enzymes have been found in thermophiles. Candidate genes can be derived from the mesophilic *Mycobacterium gastri, Bacillus subtilis, Methylococcus capsulatus,* and *Thermococcus kodakaraensis. See* Mitsui, R., et al., "A Novel Operon Encoding Formaldehyde Fixation: the Ribulose Monophosphate Pathway in the Gram-Positive Facultative Methylotrophic Bacterium Mycobacterium gastri MB19, "Journal of Bacteriology 182:944 -948 (2000); Yasueda, H., et al., "Bacillus subtilis yckG and yckF Encode Two Key Enzymes of the Ribulose Monophosphate Pathway Used by Methylotrophs, and yckH is Required for Their Expression," J. of Bacteriol. 181:7154-60 (1999); Ferenci, T., et al., "Purification and properties of 3-hexulose phosphate synthase and phospho-3-hexuloisomerase from Methylococcus capsulatus," Biochem J. 144:477-86 (1974); Orita, I., et al., "The Ribulose Monophosphate Pathway Substitutes for the Missing Pentose Phosphate Pathway in the Archaeon Thermococcus kodakaraensis," J. Bacteriol. 188:4698-4704 (2006).

**Dihydroxyacetone Pathway**

In another embodiment, the invention comprises using the dihydroxyacetone pathway (DHA), which also produces $CO_2$ while conferring electrons to redox carriers. In one embodiment, the invention comprises a DHA pathway that is endogenous to *S. cerevisiae* and comprises the genes glycerol dehydrogenase and formaldehyde transketolase and results in formaldehyde oxidation to $CO_2$. In another embodiment, the invention comprises a DHA pathway that comprises heterologous enzymes such as *gdh* from *Ogataea polymorpha. See* Nguyen, H.T.T. & Nevoigt, E., "Engineering of Saccharomyces cerevisiae for the production of dihydroxyacetone (DHA) from sugars: A proof of concept," Metabolic Engineering 11:335-46 (2009). The DHA pathway is conceptually similar to the RuMP pathway as both rely on the formation of formaldehyde and the subsequent oxidation of the formaldehyde to $CO_2$, producing NADH. With the DHA pathway, formaldehyde is produced by the action of formaldehyde transketolase (EC 2.2.1.3), which interconverts dihydroxyacetone and glyceraldehyde-3-P into xylulose-5-P and formaldehyde. *See* Figure 6. The required dihydroxyacetone can be produced by either glycerol dehydrogenase or dihydroxyacetone phosphatase:

glycerol + NAD(P) $\rightarrow$ dihydroxyacetone + NAD(P)H (glycerol dehydrogenase) or

dihydroxyacetone-P $\rightarrow$ dihydroxyacetone (dihydroxyacetone phosphatase)

dihydroxyacetone + glyceraldehyde-3-P $\rightarrow$ xylulose-5-P + formaldehyde (formaldehyde transketolase)

formaldehyde $\rightarrow$ $CO_2$ + 2 NADH (formaldehyde dehydrogenase, S-formylglutathione hydrolase, and formate dehydrogenase)

DHA degradation via formaldehyde transketolase has been described for *S. cerevisiae,* and baker's yeast has an endogenous glycerol dehydrogenase, encoded by GCY1. *See* Molin, M., and A. Blomberg, "Dihydroxyacetone detoxification in Saccharomyces cerevisiae involves formaldehyde dissimilation," Mol. Microbiol. 60:925-938 (2006) and Yu, K. O., et al., "Engineering of glycerol utilization pathway for ethanol production by Saccharomyces cerevisiae," Bioresource Technol. 101:4157-4161 (2010). Glycerol dehydrogenases from several organisms, including *Hansenula polymorpha* (*gdh*), *E. coli* (*gldA*) and *Pichia angusta* (*gdh*), have also been functionally expressed in *S. cerevisiae. See* Jung, J.-Y., et al., "Production of 1,2-propanediol from glycerol in Saccharomyces cerevisiae," J. Microbiol. Biotechnol. 21:846-853 (2011) and Nguyen, H. T. T., and Nevoigt, E., "Engineering of Saccharomyces cerevisiae for the production of dihydroxyacetone (DHA) from sugars: A proof of concept, "Metabolic Engineering 11:335-346 (2009). Dihydroxyacetone-P-specific phosphatase-activity has been found in the bacterium *Zymomonas mobilis. See* Horbach, S., et al., "Enzymes involved in the formation of glycerol 3-phosphate and the by-products dihydroxyacetone and glycerol in Zymomonas mobilis," FEMS Microbiology Letters 120:37-44 (1994).

**Transhydrogenase**

In another embodiment, the invention comprises the introduction of a transhydrogenase for the production of electron donors to be used in the conversion of acetate to ethanol or isopropanol.

As the (cytosolic) NADPH/NADP ratio in *S. cerevisiae* is typically assumed to be higher than the NADH/NAD ratio, introduction of a transhydrogenase should create a flux towards NADH formation as transhydrogenases catalyze the following reaction: NADPH + $NAD^+ \rightleftarrows NADP^+$ + NADH. Transhydrogenases from *Escherichia coli* and *Azotobacter vinelandii* have been successfully expressed in *S. cerevisiae,* and observed changes in the metabolic profiles (increased glycerol, acetate and 2-oxoglutarate production, decreased xylitol production) indeed pointed to a net conversion of NADPH into NADH. *See* Anderlund, M., et al., "Expression of the Escherichia coli pntA and pntB Genes, Encoding Nicotinamide Nucleotide Transhydrogenase, in Saccharomyces cerevisiae and Its Effect on Product Formation during Anaerobic Glucose Fermentation," Appl. Envirol. Microbiol. 65:2333-340 (1999); Heux, S., et al., "Glucose utilization of strains lacking PGI1 and expressing a transhydrogenase suggests differences in the pentose phosphate capacity among Saccharomyces cerevisiae strains," FEMS Yeast Research 8:217-224 (2008); Jeppsson, M., *et al.,* (2003); Jeun, Y.-S., et al., "Expression of Azotobacter vinelandii soluble transhydrogenase perturbs xylose reductase-mediated conversion of xylose to xylitol by recombinant Saccharomyces cerevisiae," Journal of Molecular Catalysis B: Enzymatic 26:251-256 (2003); and Nissen, T. L., et al., "Expression of a cytoplasmic transhydrogenase in Saccharomyces cerevisiae results in formation of 2-oxoglutarate due to depletion of the NADPH pool," Yeast 18:19-32 (2001).

With this approach, additional NADH becomes available for acetate-to-ethanol conversion, and the consumed NADPH could be replenished by increasing the flux through the pentose phosphate pathway.

**Glutamate Dehydrogenase**

In another embodiment, the invention comprises the introduction of a NADPH/NADH-cycling reaction. One such cycle consists of the combination of cytosolic NAD-specific and NADP-specific glutamate dehydrogenases (GDH), which catalyze the reversible reaction:

L-glutamate + $H_2O$ + NAD(P)+ $\rightleftarrows$ 2-oxoglutarate + $NH_3$ + NAD(P)H + $H^+$

Overexpressing the native NAD-GDH encoded by *GDH2* (SEQ ID NO:1) has been shown to rescue growth in a phosphoglucose isomerase *pgi1 S. cerevisiae* deletion mutant, but only as long as glucose-6-phosphate dehydrogenase and the NADP-GDH encoded by *GDH1* were left intact. *See* Boles, E., et al., "The role of the NAD-dependent glutamate dehydrogenase in restoring growth on glucose of a Saccharomyces cerevisiae phosphoglucose isomerase mutant," European Journal of Biochemistry 217:469-477 (1993). This strongly suggests that the increased NADPH production, the result of redirection of glucose into the pentose phosphate pathway, which normally proves fatal, could be balanced by conversion of NADPH to NADH by this GDH-cycle, with the produced NADH being reoxidized via respiration.

As with transhydrogenase, when the cytosolic NADPH/NADP ratio is higher than the NADH/NAD ratio, introducing a GDH-cycling reaction would generate additional NADH at the expense of NADPH. The latter can then again be replenished by an increased flux through the pentose phosphate pathway. In one embodiment, the invention comprises a copy of *GDH2* under the control of a strong constitutive promoter (*e.g.,* pTPI1) that is integrated in the genomic DNA of *S. cerevisiae* which also expresses a NADH-specific acetaldehyde dehydrogenase. *See* Figures 11 and 12.

The DNA and amino acid sequences for *S. cerevisiae GDH2* are provided as SEQ ID NOs:1 and 2, respectively. The sequence for the strong constitutive promoter pTPI1 is provided as SEQ ID NO:3.

**Glycerol Reduction**

Anaerobic growth conditions require the production of endogenous electron acceptors, such as the coenzyme nicotinamide adenine dinucleotide ($NAD^+$). In cellular redox reactions, the $NAD^+$/NADH couple plays a vital role as a reservoir and carrier of reducing equivalents. Ansell, R., et al., EMBO J. 16:2179-87 (1997). Cellular glycerol production, which generates an $NAD^+$, serves as a redox valve to remove excess reducing power during anaerobic fermentation in yeast. Glycerol production is, however, an energetically wasteful process that expends ATP and results in the loss of a reduced three-carbon compound. Ansell, R., et al., EMBO J. 16:2179-87 (1997). To generate glycerol from a starting glucose molecule, glycerol 3-phosphate dehydrogenase (GPD) reduces dihydroxyacetone phosphate to glycerol 3-phosphate and glycerol 3-phosphatase (GPP) dephosphorylates glycerol 3-phosphate to glycerol. Despite being energetically wasteful, glycerol production is a necessary metabolic process for anaerobic growth as deleting GPD activity completely inhibits growth under anaerobic conditions. *See* Ansell, R., et al., EMBO J. 16:2179-87 (1997).

GPD is encoded by two isogenes, *gpd1* and *gpd2.* GPD1 encodes the major isoform in anaerobically growing cells, while GPD2 is required for glycerol production in the absence of oxygen, which stimulates its expression. Pahlman, A-K., et al., J. Biol. Chem. 276:3555-63(2001). The first step in the conversion of dihydroxyacetone phosphate to glycerol by GPD is rate controlling. Guo, Z.P., et al., Metab. Eng. 13:49-59 (2011). GPP is also encoded by two isogenes, *gpp1* and *gpp2.* The deletion of GPP genes arrests growth when shifted to anaerobic conditions, demonstrating that GPP is important for cellular tolerance to osmotic and anaerobic stress. *See* Pahlman, A-K., et al., J. Biol. Chem. 276:3555-63(2001).

Because glycerol is a major by-product of anaerobic production of ethanol, many efforts have been made to delete cellular production of glycerol. However, because of the reducing equivalents produced by glycerol synthesis, deletion of the glycerol synthesis pathway cannot be done without compensating for this valuable metabolic function. Attempts to delete glycerol production and engineer alternate electron acceptors have been made. Lidén, G., et al., Appl. Env. Microbiol. 62:3894-96 (1996); Medina, V.G., et al., Appl. Env. Microbiol. 76:190-195 (2010). Lidén and Medina both deleted the *gpd1* and *gpd2* genes and attempted to bypass glycerol formation using additional carbon sources. Lidén engineered a xylose reductase from *Pichia stipitis* into an *S. cerevisiae gpd1/2* deletion strain. The xylose reductase activity facilitated the anaerobic growth of the glycerol-deleted strain in the presence of xylose. *See* Lidén, G., et al., Appl. Env. Microbiol. 62:3894-96 (1996). Medina engineered an acetylaldehyde dehydrogenase, *mhpF,* from *E. coli* into an *S. cerevisiae gpd1/2* deletion strain to convert acetyl-CoA to acetaldehyde. The acetylaldehyde dehydrogenase activity facilitated the anaerobic growth of the glycerol-deletion strain in the presence of acetic acid but not in the presence of glucose as the sole source of carbon. Medina, V.G., et al., Appl. Env. Microbiol. 76:190-195 (2010); *see also* EP 2277989. Medina noted several issues with the mhpF-containing strain that needed to be addressed before implementing industrially, including significantly reduced growth and product formation rates than yeast comprising GPD1 and GPD2.

Thus, in some embodiments of the invention, the recombinant host cells comprise a deletion or alteration of one or more glycerol producing enzymes. Additional deletions or alterations to modulate glycerol production include, but are not limited to, engineering a pyruvate formate lyase in a recombinant host cell, and are described in U.S. Appl. No. 61/472,085, incorporated by reference herein in its entirety.

**Microorganisms**

The present invention includes multiple strategies for the development of microorganisms with the combination of substrate-utilization and product-formation properties required for CBP. The "native cellulolytic strategy" involves engineering naturally occurring cellulolytic microorganisms to improve product-related properties, such as yield and titer. The "recombinant cellulolytic strategy" involves engineering natively non-cellulolytic organisms that exhibit high product yields and titers to express a heterologous cellulase system that enables cellulose utilization or hemicellulose utilization or both.

Many bacteria have the ability to ferment simple hexose sugars into a mixture of acidic and pH-neutral products via the process of glycolysis. The glycolytic pathway is abundant and comprises a series of enzymatic steps whereby a six carbon glucose molecule is broken down, via multiple intermediates, into two molecules of the three-carbon compound pyruvate. This process results in the net generation of ATP (biological energy supply) and the reduced cofactor NADH.

Pyruvate is an important intermediary compound of metabolism. For example, under aerobic conditions pyruvate may be oxidized to acetyl coenzyme A (acetyl-CoA), which then enters the tricarboxylic acid cycle (TCA), which in turn generates synthetic precursors, $CO_2$, and reduced cofactors. The cofactors are then oxidized by donating hydrogen equivalents, via a series of enzymatic steps, to oxygen resulting in the formation of water and ATP. This process of energy formation is known as oxidative phosphorylation.

Under anaerobic conditions (no available oxygen), fermentation occurs in which the degradation products of organic compounds serve as hydrogen donors and acceptors. Excess NADH from glycolysis is oxidized in reactions involving the reduction of organic substrates to products, such as lactate and ethanol. In addition, ATP is regenerated from the production of organic acids, such as acetate, in a process known as substrate level phosphorylation. Therefore, the fermentation products of glycolysis and pyruvate metabolism include a variety of organic acids, alcohols and $CO_2$.

Most facultative anaerobes metabolize pyruvate aerobically via pyruvate dehydrogenase (PDH) and the tricarboxylic acid cycle (TCA). Under anaerobic conditions, the main energy pathway for the metabolism of pyruvate is via pyruvate-formate-lyase (PFL) pathway to give formate and acetyl-CoA. Acetyl-CoA is then converted to acetate, via phosphotransacetylase (PTA) and acetate kinase (ACK) with the co-production of ATP, or reduced to ethanol via acetaldehyde dehydrogenase (ACDH) and alcohol dehydrogenase (ADH). In order to maintain a balance of reducing equivalents, excess NADH produced from glycolysis is re-oxidized to NAD+ by lactate dehydrogenase (LDH) during the reduction of pyruvate to lactate. NADH can also be re-oxidized by ACDH and ADH during the reduction of acetyl-CoA to ethanol, but this is a minor reaction in cells with a functional LDH.

**Host Cells**

Host cells useful in the present invention include any prokaryotic or eukaryotic cells; for example, microorganisms selected from bacterial, algal, and yeast cells. Among host cells thus suitable for the present invention are microorganisms, for example, of the genera *Aeromonas, Aspergillus, Bacillus, Escherichia, Kluyveromyces, Pichia, Rhodococcus, Saccharomyces* and *Streptomyces.*

In some embodiments, the host cells are microorganisms. In one embodiment the microorganism is a yeast. According to the present invention the yeast host cell can be, for example, from the genera *Saccharomyces, Kluyveromyces, Candida, Pichia, Schizosaccharomyces, Hansenula, Kloeckera, Schwanniomyces,* and *Yarrowia.* Yeast species as host cells may include, for example, *S. cerevisiae, S. bulderi, S. barnetti, S. exiguus, S. uvarum, S. diastaticus, K. lactis, K. marxianus,* or *K. fragilis.* In some embodiments, the yeast is selected from the group consisting of *Saccharomyces cerevisiae, Schizzosaccharomyces pombe, Candida albicans, Pichia pastoris, Pichia stipitis, Yarrowia lipolytica, Hansenula polymorpha, Phaffia rhodozyma, Candida utilis, Arxula adeninivorans, Debaryomyces hansenii, Debaryomyces polymorphus, Schizosaccharomyces pombe* and *Schwanniomyces occidentalis.* In one particular embodiment, the yeast is *Saccharomyces cerevisiae.* In another embodiment, the yeast is a thermotolerant *Saccharomyces cerevisiae.* The selection of an appropriate host is deemed to be within the scope of those skilled in the art from the teachings herein.

In some embodiments, the host cell is an oleaginous cell. The oleaginous host cell can be an oleaginous yeast cell. For example, the oleaginous yeast host cell can be from the genera *Blakeslea, Candida, Cryptococcus, Cunninghamella, Lipomyces, Mortierella, Mucor, Phycomyces, Pythium, Rhodosporidum, Rhodotorula, Trichosporon* or *Yarrowia.* According to the present invention, the oleaginous host cell can be an oleaginous microalgae host cell. For example, the oleaginous microalgae host cell can be from the genera *Thraustochytrium* or *Schizochytrium.* Biodiesel could then be produced from the triglyceride produced by the oleaginous organisms using conventional lipid transesterification processes. In some particular embodiments, the oleaginous host cells can be induced to secrete synthesized lipids. Embodiments using oleaginous host cells are advantageous because they can produce biodiesel from lignocellulosic feedstocks which, relative to oilseed substrates, are cheaper, can be grown more densely, show lower life cycle carbon dioxide emissions, and can be cultivated on marginal lands.

In some embodiments, the host cell is a thermotolerant host cell. Thermotolerant host cells can be particularly useful in simultaneous saccharification and fermentation processes by allowing externally produced cellulases and ethanol-producing host cells to perform optimally in similar temperature ranges.

Thermotolerant host cells can include, for example, *Issatchenkia orientalis, Pichia mississippiensis, Pichia mexicana, Pichia farinosa, Clavispora opuntiae, Clavispora lusitaniae, Candida mexicana, Hansenula polymorpha* and *Kluyveromyces* host cells. In some embodiments, the thermotolerant cell is an *S. cerevisiae* strain, or other yeast strain, that has been adapted to grow in high temperatures, for example, by selection for growth at high temperatures in a cytostat.

In some particular embodiments, the host cell is a *Kluyveromyces* host cell. For example, the *Kluyveromyces* host cell can be a *K. lactis, K. marxianus, K. blattae, K. phaffii, K. yarrowii, K. aestuarii, K. dobzhanskii, K. wickerhamii,*

*K. thermotolerans,* or *K. waltii* host cell. In one embodiment, the host cell is a *K. lactis,* or *K. marxianus* host cell. In another embodiment, the host cell is a *K. marxianus* host cell.

In some embodiments, the thermotolerant host cell can grow at temperatures above about 30° C, about 31° C, about 32° C, about 33° C, about 34° C, about 35° C, about 36° C, about 37° C, about 38° C, about 39° C, about 40° C, about 41° C or about 42° C. In some embodiments of the present invention the thermotolerant host cell can produce ethanol from cellulose at temperatures above about 30° C, about 31° C, about 32° C, about 33° C, about 34° C, about 35° C, about 36° C, about 37° C, about 38° C, about 39° C, about 40° C, about 41° C, about 42° C, or about 43 °C, or about 44 °C, or about 45 °C, or about 50° C.

In some embodiments of the present invention, the thermotolerant host cell can grow at temperatures from about 30° C to 60° C, about 30° C to 55° C, about 30° C to 50° C, about 40° C to 60° C, about 40° C to 55° C or about 40° C to 50° C. In some embodiments of the present invention, the thermotolerant host cell can produce ethanol from cellulose at temperatures from about 30° C to 60° C, about 30° C to 55° C, about 30° C to 50° C, about 40° C to 60° C, about 40° C to 55° C or about 40° C to 50° C.

In some embodiments, the host cell has the ability to metabolize xylose. Detailed information regarding the development of the xylose-utilizing technology can be found in the following publications: Kuyper M. et al,. FEMS Yeast Res. 4: 655-64 (2004), Kuyper M. et al,. FEMS Yeast Res. 5:399-409 (2005), and Kuyper M. et al,. FEMS Yeast Res. 5:925-34 (2005), which are herein incorporated by reference in their entirety. For example, xylose-utilization can be accomplished in *S. cerevisiae* by heterologously expressing the xylose isomerase gene, *XylA, e.g.,* from the anaerobic fungus *Piromyces sp. E2,* overexpressing five *S. cerevisiae* enzymes involved in the conversion of xylulose to glycolytic intermediates (xylulokinase, ribulose 5-phosphate isomerase, ribulose 5-phosphate epimerase, transketolase and transaldolase) and deleting the GRE3 gene encoding aldose reductase to minimize xylitol production.

The host cells can contain antibiotic markers or can contain no antibiotic markers.

In certain embodiments, the host cell is a microorganism that is a species of the genera *Thermoanaerobacterium, Thermoanaerobacter, Clostridium, Geobacillus, Saccharococcus, Paenibacillus, Bacillus, Caldicellulosiruptor, Anaerocellum, or Anoxybacillus.* In certain embodiments, the host cell is a bacterium selected from the group consisting of: *Thermoanaerobacterium thermosulfurigenes, Thermoanaerobacterium aotearoense, Thermoanaerobacterium polysaccharolyticum, Thermoanaerobacterium zeae, Thermoanaerobacterium xylanolyticum, Thermoanaerobacterium saccharolyticum, Thermoanaerobium brockii, Thermoanaerobacterium thermosaccharolyticum, Thermoanaerobacter thermohydrosulfuricus, Thermoanaerobacter ethanolicus, Thermoanaerobacter brocki, Clostridium thermocellum, Clostridium cellulolyticum, Clostridium phytofermentans, Clostridium straminosolvens, Geobacillus thermoglucosidasius, Geobacillus stearothermophilus, Saccharococcus caldoxylosilyticus, Saccharoccus thermophilus, Paenibacillus campinasensis, Bacillus flavothermus, Anoxybacillus kamchatkensis, Anoxybacillus gonensis, Caldicellulosiruptor acetigenus, Caldicellulosiruptor saccharolyticus, Caldicellulosiruptor kristjanssonii, Caldicellulosiruptor owensensis, Caldicellulosiruptor lactoaceticus,* and *Anaerocellumthermophilum.* In certain embodiments, the host cell is *Clostridium thermocellum, Clostridium cellulolyticum,* or *Thermoanaerobacterium saccharolyticum.*

## Codon Optimized Polynucleotides

The polynucleotides encoding heterologous enzymes described herein can be codon-optimized. As used herein the term "codon-optimized coding region" means a nucleic acid coding region that has been adapted for expression in the cells of a given organism by replacing at least one, or more than one, or a significant number, of codons with one or more codons that are more frequently used in the genes of that organism.

In general, highly expressed genes in an organism are biased towards codons that are recognized by the most abundant transfer RNA (tRNA) species in that organism. One measure of this bias is the "codon adaptation index" or "CAI," which measures the extent to which the codons used to encode each amino acid in a particular gene are those which occur most frequently in a reference set of highly expressed genes from an organism.

The CAI of codon optimized sequences of the present invention corresponds to between about 0.8 and 1.0, between about 0.8 and 0.9, or about 1.0. A codon optimized sequence may be further modified for expression in a particular organism, depending on that organism's biological constraints. For example, large runs of "As" or "Ts" (*e.g.,* runs greater than 3, 4, 5, 6, 7, 8, 9, or 10 consecutive bases) can be removed from the sequences if these are known to effect transcription negatively. Furthermore, specific restriction enzyme sites may be removed for molecular cloning purposes. Examples of such restriction enzyme sites include PacI, AscI, BamHI, BglII, EcoRI and XhoI. Additionally, the DNA sequence can be checked for direct repeats, inverted repeats and mirror repeats with lengths of ten bases or longer, which can be modified manually by replacing codons with "second best" codons, *i.e.,* codons that occur at the second highest frequency within the particular organism for which the sequence is being optimized.

Deviations in the nucleotide sequence that comprise the codons encoding the amino acids of any polypeptide chain allow for variations in the sequence coding for the gene. Since each codon consists of three nucleotides, and the nucleotides comprising DNA are restricted to four specific bases, there are 64 possible combinations of nucleotides, 61 of which encode amino acids (the remaining three codons encode signals ending translation). The "genetic code" which shows which codons encode which amino acids is reproduced herein as Table 1. As a result, many amino acids are designated by more than one codon. For example, the amino acids alanine and proline are coded for by four triplets, serine and arginine by six, whereas tryptophan and methionine are coded by just one triplet. This degeneracy allows for DNA base composition to vary over a wide range without altering the amino acid sequence of the proteins encoded by the DNA.

TABLE 1: The Standard Genetic Code

| | **T** | **C** | **A** | **G** |
|---|---|---|---|---|
| **T** | TTT Phe (F)<br>TTC"<br>TTA Leu (L)<br>TTG" | TCT Ser (S)<br>TCC"<br>TCA"<br>TCG" | TAT Tyr (Y)<br>TAC"<br>TAA **Ter**<br>TAG **Ter** | rGT Cys (C)<br>TGC<br>TGA **Ter**<br>TGG Trp (W) |
| **C** | CTT Leu (L)<br>CTC"<br>CTA"<br>CTG" | CCT Pro (P)<br>CCC"<br>CCA"<br>CCG" | CAT His (H)<br>CAC"<br>CAA Gln (Q)<br>CAG" | CGT Arg (R)<br>CGC"<br>CGA"<br>CGG" |
| **A** | ATT Ile (I)<br>ATC"<br>ATA"<br>**ATG** Met (M) | ACT Thr (T)<br>ACC"<br>ACA"<br>ACG" | AAT Asn (N)<br>AAC"<br>AAA Lys (K)<br>AAG" | AGT Ser (S)<br>AGC "<br>AGA Arg (R)<br>AGG " |
| **G** | GTT Val (V)<br>GTC"<br>GTA"<br>GTG" | GCT Ala (A)<br>GCC"<br>GCA"<br>GCG" | GAT Asp (D)<br>GAC"<br>GAA Glu (E)<br>GAG" | GGT Gly (G) "<br>GGC "<br>GGA "<br>GGG |

Many organisms display a bias for use of particular codons to code for insertion of a particular amino acid in a growing peptide chain. Codon preference or codon bias, differences in codon usage between organisms, is afforded by degeneracy of the genetic code, and is well documented among many organisms. Codon bias often correlates with the efficiency of translation of messenger RNA (mRNA), which is in turn believed to be dependent on, *inter alia,* the properties of the codons being translated and the availability of particular tRNA molecules. The predominance of selected tRNAs in a cell is generally a reflection of the codons used most frequently in peptide synthesis. Accordingly, genes can be tailored for optimal gene expression in a given organism based on codon optimization.

Given the large number of gene sequences available for a wide variety of animal, plant and microbial species, it is possible to calculate the relative frequencies of codon usage. Codon usage tables are readily available, for example, at kazusa.or.jp/codon/ (visited August 10, 2012), and these tables can be adapted in a number of ways. *See* Nakamura, Y., et al. "Codon usage tabulated from the international DNA sequence databases: status for the year 2000," Nucl. Acids Res. 28:292 (2000). Codon usage tables for yeast, calculated from GenBank Release 128.0 [15 February 2002], are reproduced below as Table 2. This table uses mRNA nomenclature, and so instead of thymine (T) which is found in DNA, the tables use uracil (U) which is found in RNA. The table has been adapted so that frequencies are calculated for each amino acid, rather than for all 64 codons.

TABLE 2: Codon Usage Table for *Saccharomyces cerevisiae* Genes

| Amino Acid | Codon | Number | Frequency per hundred |
|---|---|---|---|
| Phe | UUU | 170666 | 26.1 |
| Phe | UUC | 120510 | 18.4 |
| | | | |
| Leu | UUA | 170884 | 26.2 |
| Leu | UUG | 177573 | 27.2 |
| Leu | CUU | 80076 | 12.3 |

(continued)

| Amino Acid | Codon | Number | Frequency per hundred |
|---|---|---|---|
| Leu | CUC | 35545 | 5.4 |
| Leu | CUA | 87619 | 13.4 |
| Leu | CUG | 68494 | 10.5 |
| | | | |
| Ile | AUU | 196893 | 30.1 |
| Ile | AUC | 112176 | 17.2 |
| Ile | AUA | 116254 | 17.8 |
| | | | |
| Met | AUG | 136805 | 20.9 |
| | | | |
| Val | GUU | 144243 | 22.1 |
| Val | GUC | 76947 | 11.8 |
| Val | GUA | 76927 | 11.8 |
| Val | GUG | 70337 | 10.8 |
| | | | |
| Ser | UCU | 153557 | 23.5 |
| Ser | UCC | 92923 | 14.2 |
| Ser | UCA | 122028 | 18.7 |
| Ser | UCG | 55951 | 8.6 |
| Ser | AGU | 92466 | 14.2 |
| Ser | AGC | 63726 | 9.8 |
| | | | |
| Pro | CCU | 88263 | 13.5 |
| Pro | CCC | 44309 | 6.8 |
| Pro | CCA | 119641 | 18.3 |
| Pro | CCG | 34597 | 5.3 |
| | | | |
| Thr | ACU | 132522 | 20.3 |
| Thr | ACC | 83207 | 12.7 |
| Thr | ACA | 116084 | 17.8 |
| Thr | ACG | 52045 | 8.0 |
| | | | |
| Ala | GCU | 138358 | 21.2 |
| Ala | GCC | 82357 | 12.6 |
| Ala | GCA | 105910 | 16.2 |
| Ala | GCG | 40358 | 6.2 |
| | | | |
| Tyr | UAU | 122728 | 18.8 |

(continued)

| Amino Acid | Codon | Number | Frequency per hundred |
|---|---|---|---|
| Tyr | UAC | 96596 | 14.8 |
| | | | |
| His | CAU | 89007 | 13.6 |
| His | CAC | 50785 | 7.8 |
| | | | |
| Gln | CAA | 178251 | 27.3 |
| Gln | CAG | 79121 | 12.1 |
| | | | |
| Asn | AAU | 233124 | 35.7 |
| Asn | AAC | 162199 | 24.8 |
| | | | |
| Lys | AAA | 273618 | 41.9 |
| Lys | AAG | 201361 | 30.8 |
| | | | |
| Asp | GAU | 245641 | 37.6 |
| Asp | GAC | 132048 | 20.2 |
| | | | |
| Glu | GAA | 297944 | 45.6 |
| Glu | GAG | 125717 | 19.2 |
| | | | |
| Cys | UGU | 52903 | 8.1 |
| Cys | UGC | 31095 | 4.8 |
| | | | |
| Trp | UGG | 67789 | 10.4 |
| | | | |
| Arg | CGU | 41791 | 6.4 |
| Arg | CGC | 16993 | 2.6 |
| Arg | CGA | 19562 | 3.0 |
| Arg | CGG | 11351 | 1.7 |
| Arg | AGA | 139081 | 21.3 |
| Arg | AGG | 60289 | 9.2 |
| | | | |
| Gly | GGU | 156109 | 23.9 |
| Gly | GGC | 63903 | 9.8 |
| Gly | GGA | 71216 | 10.9 |
| Gly | GGG | 39359 | 6.0 |
| | | | |
| Stop | UAA | 6913 | 1.1 |

(continued)

| Amino Acid | Codon | Number | Frequency per hundred |
|---|---|---|---|
| Stop | UAG | 3312 | 0.5 |
| Stop | UGA | 4447 | 0.7 |

By utilizing this or similar tables, one of ordinary skill in the art can apply the frequencies to any given polypeptide sequence, and produce a nucleic acid fragment of a codon-optimized coding region which encodes the polypeptide, but which uses codons optimal for a given species. Codon-optimized coding regions can be designed by various different methods.

In one method, a codon usage table is used to find the single most frequent codon used for any given amino acid, and that codon is used each time that particular amino acid appears in the polypeptide sequence. For example, referring to Table 2 above, for leucine, the most frequent codon is UUG, which is used 27.2% of the time. Thus all the leucine residues in a given amino acid sequence would be assigned the codon UUG.

In another method, the actual frequencies of the codons are distributed randomly throughout the coding sequence. Thus, using this method for optimization, if a hypothetical polypeptide sequence had 100 leucine residues, referring to Table 2 for frequency of usage in the *S. cerevisiae,* about 5, or 5% of the leucine codons would be CUC, about 11, or 11% of the leucine codons would be CUG, about 12, or 12% of the leucine codons would be CUU, about 13, or 13% of the leucine codons would be CUA, about 26, or 26% of the leucine codons would be UUA, and about 27, or 27% of the leucine codons would be UUG.

These frequencies would be distributed randomly throughout the leucine codons in the coding region encoding the hypothetical polypeptide. As will be understood by those of ordinary skill in the art, the distribution of codons in the sequence can vary significantly using this method; however, the sequence always encodes the same polypeptide.

When using the methods above, the term "about" is used precisely to account for fractional percentages of codon frequencies for a given amino acid. As used herein, "about" is defined as one amino acid more or one amino acid less than the value given. The whole number value of amino acids is rounded up if the fractional frequency of usage is 0.50 or greater, and is rounded down if the fractional frequency of use is 0.49 or less. Using again the example of the frequency of usage of leucine in human genes for a hypothetical polypeptide having 62 leucine residues, the fractional frequency of codon usage would be calculated by multiplying 62 by the frequencies for the various codons. Thus, 7.28 percent of 62 equals 4.51 UUA codons, or "about 5," *i.e.,* 4, 5, or 6 UUA codons, 12.66 percent of 62 equals 7.85 UUG codons or "about 8," *i.e.,* 7, 8, or 9 UUG codons, 12.87 percent of 62 equals 7.98 CUU codons, or "about 8," *i.e.,* 7, 8, or 9 CUU codons, 19.56 percent of 62 equals 12.13 CUC codons or "about 12," *i.e.,* 11, 12, or 13 CUC codons, 7.00 percent of 62 equals 4.34 CUA codons or "about 4," *i.e.,* 3, 4, or 5 CUA codons, and 40.62 percent of 62 equals 25.19 CUG codons, or "about 25," *i.e.,* 24, 25, or 26 CUG codons.

Randomly assigning codons at an optimized frequency to encode a given polypeptide sequence, can be done manually by calculating codon frequencies for each amino acid, and then assigning the codons to the polypeptide sequence randomly. Additionally, various algorithms and computer software programs are readily available to those of ordinary skill in the art. For example, the "EditSeq" function in the Lasergene Package, available from DNAstar, Inc., Madison, WI, the backtranslation function in the VectorNTI Suite, available from InforMax, Inc., Bethesda, MD, and the "backtranslate" function in the GCG--Wisconsin Package, available from Accelrys, Inc., San Diego, CA. In addition, various resources are publicly available to codon-optimize coding region sequences, *e.g.,* the "backtranslation" function at entelechon.com/bioinformatics/backtranslation.php?lang=eng (visited August 10, 2012) and the "backtranseq" function available at emboss.bioinformatics.nl/cgi-bin/emboss/backtranseq (visited December 18, 2009). Constructing a rudimentary algorithm to assign codons based on a given frequency can also easily be accomplished with basic mathematical functions by one of ordinary skill in the art.

A number of options are available for synthesizing codon optimized coding regions designed by any of the methods described above, using standard and routine molecular biological manipulations well known to those of ordinary skill in the art. In one approach, a series of complementary oligonucleotide pairs of 80-90 nucleotides each in length and spanning the length of the desired sequence is synthesized by standard methods. These oligonucleotide pairs are synthesized such that upon annealing, they form double stranded fragments of 80-90 base pairs, containing cohesive ends, *e.g.,* each oligonucleotide in the pair is synthesized to extend 3, 4, 5, 6, 7, 8, 9, 10, or more bases beyond the region that is complementary to the other oligonucleotide in the pair. The single-stranded ends of each pair of oligonucleotides are designed to anneal with the single-stranded end of another pair of oligonucleotides. The oligonucleotide pairs are allowed to anneal, and approximately five to six of these double-stranded fragments are then allowed to anneal together via the cohesive single stranded ends, and then they ligated together and cloned into a standard bacterial cloning vector, for example, a TOPO® vector available from Invitrogen Corporation, Carlsbad, CA. The construct is then sequenced by standard methods. Several of these constructs consisting of 5 to 6 fragments of 80 to 90 base pair fragments ligated together, *i.e.,* fragments of about 500 base pairs, are prepared, such that the entire desired sequence is represented in a series of plasmid constructs. The inserts of these plasmids are then cut with appropriate restriction enzymes and ligated together to form the final construct. The final construct is then cloned into a standard bacterial cloning vector, and sequenced. Additional methods would be immediately apparent to the skilled artisan. In addition, gene synthesis is readily available commercially.

In additional embodiments, a full-length polypeptide sequence is codon-optimized for a given species resulting in a codon-optimized coding region encoding the entire polypeptide, and then nucleic acid fragments of the codon-optimized coding region, which encode fragments, variants, and derivatives of the polypeptide, are made from the original codon-optimized coding region. As would be well understood by those of ordinary skill in the art, if codons have been randomly assigned to the full-length coding region based on their frequency of use in a given species, nucleic acid fragments encoding fragments, variants, and derivatives would not necessarily be fully codon optimized for the given species. However, such sequences are still much closer to the codon usage of the desired species than the native codon usage. The advantage of this approach is that synthesizing codon-optimized nucleic acid fragments encoding each fragment, variant, and derivative of a given polypeptide, although routine, would be time consuming and would result in significant expense.

**Vectors and Methods of Using Vectors in Host Cells**

In another aspect, the present invention relates to vectors which include polynucleotides of the present invention, host cells which are genetically engineered with vectors of the invention and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the genes of the present invention. The culture conditions, such as temperature, pH and the like, are those previously used with the host cell selected for expression, and will be apparent to the ordinarily skilled artisan.

The polynucleotides of the present invention can be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, *e.g.*, derivatives of SV40; bacterial plasmids; and yeast plasmids. However, any other vector may be used as long as it is replicable and viable in the host.

The appropriate DNA sequence can be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively associated with an appropriate expression control sequence(s) (promoter) to direct mRNA synthesis. Any suitable promoter to drive gene expression in the host cells of the invention may be used.

In addition, the expression vectors may contain one or more selectable marker genes to provide a phenotypic trait for selection of transformed host cells such as *URA3, HIS3, LEU2, TRP1, LYS2* or *ADE2,* dihydrofolate reductase, neomycin (G418) resistance or zeocin resistance for eukaryotic cell culture, or tetracycline or ampicillin resistance in prokaryotic cell culture, *e.g., Clostridium thermocellum.*

The expression vector may also contain a ribosome binding site for translation initiation and/or a transcription terminator. The vector may also include appropriate sequences for amplifying expression, or may include additional regulatory regions.

The vector containing the appropriate DNA sequence as herein, as well as an appropriate promoter or control sequence, may be employed to transform an appropriate host to permit the host to express the protein.

Thus, in certain aspects, the present invention relates to host cells containing the above-described constructs. The host cell can be a host cell as described elsewhere in the application. The host cell can be, for example, a lower eukaryotic cell, such as a yeast cell, *e.g., Saccharomyces cerevisiae* or *Kluyveromyces,* or the host cell can be a prokaryotic cell, such as a bacterial cell, *e.g., Clostridium thermocellum.*

The selection of an appropriate host is deemed to be within the scope of those skilled in the art from the teachings herein. In one embodiment, the vector is integrated into the genome of the host cell. In another embodiment, the vector is present in the host cell as an extrachromosomal plasmid.

## Transposons

To select for foreign DNA that has entered a host it is preferable that the DNA be stably maintained in the organism of interest. With regard to plasmids, there are two processes by which this can occur. One is through the use of replicative plasmids. These plasmids have origins of replication that are recognized by the host and allow the plasmids to replicate as stable, autonomous, extrachromosomal elements that are partitioned during cell division into daughter cells. The second process occurs through the integration of a plasmid onto the chromosome. This predominately happens by homologous recombination and results in the insertion of the entire plasmid, or parts of the plasmid, into the host chromosome. Thus, the plasmid and selectable marker(s) are replicated as an integral piece of the chromosome and segregated into daughter cells. Therefore, to ascertain if plasmid DNA is entering a cell during a transformation event through the use of selectable markers requires the use of a replicative plasmid or the ability to recombine the plasmid onto the chromosome. These qualifiers cannot always be met, especially when handling organisms that do not have a suite of genetic tools.

One way to avoid issues regarding plasmid-associated markers is through the use of transposons. A transposon is a mobile DNA element, defined by mosaic DNA sequences that are recognized by enzymatic machinery referred to as a transposase. The function of the transposase is to randomly insert the transposon DNA into host or target DNA. A selectable marker can be cloned onto a transposon by standard genetic engineering. The resulting DNA fragment can be coupled to the transposase machinery in an *in vitro* reaction and the complex can be introduced into target cells by electroporation. Stable insertion of the marker onto the chromosome requires only the function of the transposase machinery and alleviates the need for homologous recombination or replicative plasmids.

The random nature associated with the integration of transposons has the added advantage of acting as a form of mutagenesis. Libraries can be created that comprise amalgamations of transposon mutants. These libraries can be used in screens or selections to produce mutants with desired phenotypes. For instance, a transposon library of a CBP organism could be screened for the ability to produce more ethanol, or less lactic acid and/or more acetate.

## Native cellulolytic strategy

Naturally occurring cellulolytic microorganisms are starting points for CBP organism development via the native strategy. Anaerobes and facultative anaerobes are of particular interest. The primary objective is to improve the engineering of the detoxification of biomass derived acetate to a non-charged solvent, including but not limited to, acetone, isopropanol, or ethanol. Metabolic engineering of mixed-acid fermentations in relation to, for example, ethanol production, has been successful in the case of mesophilic, non-cellulolytic, enteric bacteria. Recent developments in suitable gene-transfer techniques allow for this type of work to be undertaken with cellulolytic bacteria.

## Recombinant cellulolytic strategy

Non-cellulolytic microorganisms with desired product-formation properties are starting points for CBP organism development by the recombinant cellulolytic strategy. The primary objective of such developments is to engineer a heterologous cellulase system that enables growth and fermentation on pretreated lignocellulose. The heterologous production of cellulases has been pursued primarily with bacterial hosts producing ethanol at high yield (engineered strains of *E. coli, Klebsiella oxytoca,* and *Zymomonas mobilis*) and the yeast *Saccharomyces cerevisiae.* Cellulase expression in strains of *K. oxytoca* resulted in increased hydrolysis yields - but not growth without added cellulase - for microcrystalline cellulose, and anaerobic growth on amorphous cellulose. Although dozens of saccharolytic enzymes have been functionally expressed in *S. cerevisiae,* anaerobic growth on cellulose as the result of such expression has not been definitively demonstrated.

Aspects of the present invention relate to the use of thermophilic or mesophilic microorganisms as hosts for modification via the native cellulolytic strategy. Their potential in process applications in biotechnology stems from their ability to grow at relatively high temperatures with attendant high metabolic rates, production of physically and chemically stable enzymes, and elevated yields of end products. Major groups of thermophilic bacteria include eubacteria and archaebacteria. Thermophilic eubacteria include: phototropic bacteria, such as cyanobacteria, purple bacteria, and green bacteria; Gram-positive bacteria, such as *Bacillus, Clostridium,* Lactic acid bacteria, and Actinomyces; and other eubacteria, such as *Thiobacillus,* Spirochete, *Desulfotomaculum,* Gram-negative aerobes, Gram-negative anaerobes, and *Thermotoga.* Within archaebacteria are considered Methanogens, extreme thermophiles (an art-recognized term), and *Thermoplasma.* In certain embodiments, the present invention relates to Gram-negative organotrophic thermophiles of the genera *Thermus,* Gram-positive eubacteria, such as genera *Clostridium,* and also which comprise both rods and cocci, genera in group of eubacteria, such as *Thermosipho* and *Thermotoga,* genera of Archaebacteria, such as *Thermococcus, Thermoproteus* (rod-shaped), *Thermofilum* (rod-shaped), *Pyrodictium, Acidianus, Sulfolobus, Pyrobaculum, Pyrococcus, Thermodiscus, Staphylothermus, Desulfurococcus, Archaeoglobus,* and *Methanopyrus.* Some examples of thermophilic or mesophilic (including bacteria, procaryotic microorganism, and fungi), which may be suitable for the present invention include, but are not limited to: *Clostridium thermosulfurogenes, Clostridium cellulolyticum, Clostridium thermocellum, Clostridium thermohydrosulfuricum, Clostridium thermoaceticum, Clostridium thermosaccharolyticum, Clostridium tartarivorum, Clostridium thermocellulaseum, Clostridium phytofermentans, Clostridium straminosolvens, Thermoanaerobacterium thermosaccarolyticum, Thermoanaerobacterium saccharolyticum, Thermobacteroides acetoethylicus, Thermoanaerobium brockii, Methanobacterium thermoautotrophicum, Anaerocellum thermophilium, Pyrodictium occultum, Thermoproteus neutrophilus, Thermofilum librum, Thermothrix thioparus, Desulfovibrio thermophilus, Thermoplasma acidophilum, Hydrogenomonas thermophilus, Thermomicrobium roseum, Thermus flavas, Thermus ruber, Pyrococcus furiosus, Thermus aquaticus, Thermus thermophilus, Chloroflexus aurantiacus, Thermococcus litoralis, Pyrodictium abyssi, Bacillus stearothermophilus, Cyanidium caldarium, Mastigocladus laminosus, Chlamydothrix calidissima, Chlamydothrix penicillata, Thiothrix carnea, Phormidium tenuissimum, Phormidium geysericola, Phormidium subterraneum, Phormidium bijahensi, Oscillatoria filiformis, Synechococcus lividus, Chloroflexus aurantiacus, Pyrodictium brockii, Thiobacillus thiooxidans, Sulfolobus acidocaldarius, Thiobacillus thermophilica, Bacillus stearothermophilus, Cercosulcifer hamathensis, Vahlkampfia reichi, Cyclidium citrullus, Dactylaria gallopava, Synechococcus lividus, Synechococcus elongatus, Synechococcus minervae, Synechocystis aquatilus, Aphanocapsa thermalis, Oscillatoria terebriformis, Oscillatoria amphibia, Oscillatoria germinata, Oscillatoria okenii, Phormidium laminosum, Phormidium parparasiens, Symploca thermalis, Bacillus acidocaldarias, Bacillus coagulans, Bacillus thermocatenalatus, Bacillus licheniformis, Bacillus pamilas, Bacillus macerans, Bacillus circulans, Bacillus laterosporus, Bacillus brevis, Bacillus subtilis, Bacillus sphaericus, Desulfotomaculum nigrificans, Streptococcus thermophilus, Lactobacillus thermophilus, Lactobacillus bulgaricus, Bifidobacterium thermophilum, Streptomyces fragmentosporus, Streptomyces thermonitrificans, Streptomyces thermovulgaris, Pseudonocardia thermophila, Thermoactinomyces vulgaris, Thermoactinomyces sacchari, Thermoactinomyces candidas, Thermomonospora curvata, Thermomonospora viridis, Thermomonospora citrina, Microbispora thermodiastatica, Microbispora aerata, Microbispora bispora, Actinobifida dichotomica, Actinobifida chromogena, Micropolyspora caesia, Micropolyspora faeni, Micropolyspora cectivugida, Micropolyspora cabrobrunea, Micropolyspora thermovirida, Micropolyspora viridinigra, Methanobacterium thermoautothropicum, Caldicellulosiruptor acetigenus, Caldicellulosiruptor saccharolyticus, Caldicellulosiruptor kristjanssonii, Caldicellulosiruptor owensensis, Caldicellulosiruptor lactoaceticus,* variants thereof, and/or progeny thereof.

In particular embodiments, the present invention relates to thermophilic bacteria selected from the group consisting of *Clostridium cellulolyticum, Clostridium thermocellum,* and *Thermoanaerobacterium saccharolyticum.*

In certain embodiments, the present invention relates to thermophilic bacteria selected from the group consisting of *Fervidobacterium gondwanense, Clostridium thermolacticum, Moorella sp.,* and *Rhodothermus marinus.*

In certain embodiments, the present invention relates to thermophilic bacteria of the genera *Thermoanaerobacterium* or *Thermoanaerobacter,* including, but not limited to, species selected from the group consisting of: *Thermoanaerobacteriumthermosulfurigenes, Thermoanaerobacteriumaotearoense, Thermoanaerobacteriumpolysaccharolyticum, Thermoanaerobacteriumzeae, Thermoanaerobacteriumxylanolyticum, Thermoanaerobacterium saccharolyticum, Thermoanaerobiumbrockii, Thermoanaerobacteriumthermosaccharolyticum, Thermoanaerobacter thermohydrosulfuricus, Thermoanaerobacterethanolicus, Thermoanaerobacterbrockii,* variants thereof, and progeny thereof.

In certain embodiments, the present invention relates to microorganisms of the genera *Geobacillus, Saccharococcus, Paenibacillus, Bacillus,* and *Anoxybacillus,* including, but not limited to, species selected from the group consisting of: *Geobacillus thermoglucosidasius, Geobacillus stearothermophilus, Saccharococcus caldoxylosilyticus, Saccharoccus thermophilus, Paenibacillus campinasensis, Bacillus flavothermus, Anoxybacillus kamchatkensis, Anoxybacillus gonensis,* variants thereof, and progeny thereof.

In certain embodiments, the present invention relates to mesophilic bacteria selected from the group consisting of *Saccharophagus degradans; Flavobacterium johnsoniae; Fibrobacter succinogenes; Clostridium hungatei*; *Clostridium phytofermentans*; *Clostridium cellulolyticum*; *Clostridium aldrichii*; *Clostridium termitididis; Acetivibrio cellulolyticus; Acetivibrio ethanolgignens; Acetivibrio multivorans; Bacteroides cellulosolvens;* and *Alkalibacter saccharofomentans,* variants thereof and progeny thereof.

**Organism development via the native cellulolytic strategy**

One approach to organism development for CBP begins with organisms that naturally utilize cellulose, hemicellulose and/or other biomass components, which are then genetically engineering to enhance product yield and tolerance. For example, *Clostridium thermocellum* is a thermophilic bacterium that has among the highest rates of cellulose utilization reported. Other organisms of interest are xylose-utilizing thermophiles such as *Thermoanaerobacterium saccharolyticum* and *Thermoanaerobacterium thermosaccharolyticum.* Organic acid production may be responsible for the low concentrations of produced ethanol generally associated with these organisms. Thus, one objective is to eliminate production of acetic and lactic acid in these organisms via metabolic engineering. Substantial efforts have been devoted to developing gene transfer systems for the above-described target organisms and multiple *C. thermocellum* isolates from nature have been characterized. *See* McLaughlin et al. (2002) Environ. Sci. Technol. 36:2122. Metabolic engineering of thermophilic, saccharolytic bacteria is an active area of interest, and knockout of lactate dehydrogenase in *T. saccharolyticum* has recently been reported. *See* Desai et al. (2004) Appl. Microbiol. Biotechnol. 65:600. Knockout of acetate kinase and phosphotransacetylase in this organism is also possible.

**Organism development via the recombinant cellulolytic strategy**

An alternative approach to organism development for CBP involves conferring the ability to grow on lignocellulosic materials to microorganisms that naturally have high product yield and tolerance via expression of a heterologous cellulasic system and perhaps other features. For example, *Saccharomyces cerevisiae* has been engineered to express over two dozen different saccharolytic enzymes. *See* Lynd et al. (2002) Microbiol. Mol. Biol. Rev. 66:506.

Whereas cellulosic hydrolysis has been approached in the literature primarily in the context of an enzymatically-oriented intellectual paradigm, the CBP processing strategy requires that cellulosic hydrolysis be viewed in terms of a microbial paradigm. This microbial paradigm naturally leads to an emphasis on different fundamental issues, organisms, cellulasic systems, and applied milestones compared to those of the enzymatic paradigm. In this context, *C. thermocellum* has been a model organism because of its high growth rate on cellulose together with its potential utility for CBP.

In certain embodiments, organisms useful in the present invention may be applicable to the process known as simultaneous saccharification and fermentation (SSF), which is intended to include the use of said microorganisms and/or one or more recombinant hosts (or extracts thereof, including purified or unpurified extracts) for the contemporaneous degradation or depolymerization of a complex sugar *(i.e.,* cellulosic biomass) and bioconversion of that sugar residue into ethanol by fermentation.

**Ethanol Production**

According to the present invention, a recombinant microorganism can be used to produce ethanol from biomass, which is referred to herein as lignocellulosic material, lignocellulosic substrate, or cellulosic biomass. Methods of producing ethanol can be accomplished, for example, by contacting the biomass with a recombinant microorganism as described herein, and as described in commonly owned International Appl. No. PCT/US2009/002902, International Appl. No. PCT/US2009/003972, International Appl. No. PCT/US2009/003970, Published International Appl. No. WO 2010/060056, International Appl. No. PCT/US2009/069443, International Appl. No. PCT/US2009/064128, International Appl. No. PCT/IB2009/005881, International Appl. No. PCT/US2011/039192, U.S. Appl. No. 61/116,981, U.S. Published Appl. No. 2012/0129229 A1, U.S. Appl. No. 61/351,165, U.S. Appl. No. 13/701,652, and U.S. Appl. No. 61/420,142, the contents of each are incorporated by reference herein in their entirety.

In addition, to produce ethanol, the recombinant microorganisms as described herein can be combined, either as recombinant host cells or as engineered metabolic pathways in recombinant host cells, with the recombinant microorganisms described in commonly owned International Appl. No. PCT/US2009/002902, International Appl. No. PCT/US2009/003972, International Appl. No. PCT/US2009/003970, International Patent Application Publication No.. WO 2010/060056, International Appl. No. PCT/US2009/069443, International Appl. No. PCT/US2009/064128, International Appl. No. PCT/IB2009/005881, International Appl. No. PCT/US2011/039192, U.S. Appl. No. 61/351,165, U.S. Appl. No. 13/701,652, and U.S. Appl. No. 61/420,142, the contents of each are incorporated by reference herein in their entirety. The recombinant microorganism as described herein can also be engineered with the enzymes and/or metabolic pathways described in commonly owned International Appl. No. PCT/US2009/002902, International Appl. No. PCT/US2009/003972, International Appl. No. PCT/US2009/003970, International Patent Application Publication No. WO 2010/060056, International Appl. No. PCT/US2009/069443, International Appl. No. PCT/US2009/064128, International Appl. No. PCT/IB2009/005881, International Appl. No. PCT/US2011/039192, U.S. Appl. No. 61/351,165, U.S. Appl. No. 13/701,652, and U.S. Appl. No. 61/420,142, the contents of each are incorporated by reference herein in their entirety.

Numerous cellulosic substrates can be used in accordance with the present invention. Substrates for cellulose activity assays can be divided into two categories, soluble and insoluble, based on their solubility in water. Soluble substrates include cellodextrins or derivatives, carboxymethyl cellulose (CMC), or hydroxyethyl cellulose (HEC). Insoluble substrates include crystalline cellulose, microcrystalline cellulose (Avicel), amorphous cellulose, such as phosphoric acid swollen cellulose (PASC), dyed or fluorescent cellulose, and pretreated lignocellulosic biomass. These substrates are generally highly ordered cellulosic material and thus only sparingly soluble.

It will be appreciated that suitable lignocellulosic material may be any feedstock that contains soluble and/or insoluble cellulose, where the insoluble cellulose may be in a crystalline or non-crystalline form. In various embodiments, the lignocellulosic biomass comprises, for example, wood, corn, corn stover, sawdust, bark, leaves, agricultural and forestry residues, grasses such as switchgrass, ruminant digestion products, municipal wastes, paper mill effluent, newspaper, cardboard or combinations thereof.

In some embodiments, the invention is directed to a method for hydrolyzing a cellulosic substrate, for example a cellulosic substrate as described above, by contacting the cellulosic substrate with a recombinant microorganism of the invention. In some embodiments, the invention is directed to a method for hydrolyzing a cellulosic substrate, for example a cellulosic substrate as described above, by contacting the cellulosic substrate with a co-culture comprising yeast cells expressing heterologous cellulases.

In some embodiments, the invention is directed to a method for fermenting cellulose. Such methods can be accomplished, for example, by culturing a host cell or co-culture in a medium that contains insoluble cellulose to allow saccharification and fermentation of the cellulose.

The production of ethanol can, according to the present invention, be performed at temperatures of at least about 30° C, about 31° C, about 32° C, about 33° C, about 34° C, about 35° C, about 36° C, about 37° C, about 38° C, about 39° C, about 40° C, about 41° C, about 42° C, about 43 °C, about 44 °C, about 45 °C, about 46 °C, about 47 °C, about 48 °C, about 49 °C, or about 50° C. In some embodiments of the present invention the thermotolerant host cell can produce ethanol from cellulose at temperatures above about 30° C, about 31° C, about 32° C, about 33° C, about 34° C, about 35° C, about 36° C, about 37° C, about 38° C, about 39° C, about 40° C, about 41° C, about 42° C, or about 43 °C, or about 44 °C, or about 45 °C, or about 50° C. In some embodiments of the present invention, the thermotolerant host cell can produce ethanol from cellulose at temperatures from about 30° C to 60° C, about 30° C to 55° C, about 30° C to 50° C, about 40° C to 60° C, about 40° C to 55° C or about 40° C to 50° C.

In some embodiments, methods of producing ethanol can comprise contacting a cellulosic substrate with a recombinant microorganism or co-culture of the invention and additionally contacting the cellulosic substrate with externally produced cellulase enzymes. Exemplary externally produced cellulase enzymes are commercially available and are known to those of skill in the art.

In some embodiments, the methods comprise producing ethanol at a particular rate. For example, in some embodiments, ethanol is produced at a rate of at least about 0.1 mg per hour per liter, at least about 0.25 mg per hour per liter, at least about 0.5 mg per hour per liter, at least about 0.75 mg per hour per liter, at least about 1.0 mg per hour per liter, at least about 2.0 mg per hour per liter, at least about 5.0 mg per hour per liter, at least about 10 mg per hour per liter, at least about 15 mg per hour per liter, at least about 20.0 mg per hour per liter, at least about 25 mg per hour per liter, at least about 30 mg per hour per liter, at least about 50 mg per hour per liter, at least about 100 mg per hour per liter, at least about 200 mg per hour per liter, at least about 300 mg per hour per liter, at least about 400 mg per hour per liter, or at least about 500 mg per hour per liter.

In some embodiments, the host cells of the present invention can produce ethanol at a rate of at least about 0.1 mg per hour per liter, at least about 0.25 mg per hour per liter, at least about 0.5 mg per hour per liter, at least about 0.75 mg per hour per liter, at least about 1.0 mg per hour per liter, at least about 2.0 mg per hour per liter, at least about 5.0 mg per hour per liter, at least about 10 mg per hour per liter, at least about 15 mg per hour per liter, at least about 20.0 mg per hour per liter, at least about 25 mg per hour per liter, at least about 30 mg per hour per liter, at least about 50 mg per hour per liter, at least about 100 mg per hour per liter, at least about 200 mg per hour per liter, at least about 300 mg per hour per liter, at least about 400 mg per hour per liter, or at least about 500 mg per hour per liter more than a control strain (lacking heterologous cellulases) and grown under the same conditions. In some embodiments, the ethanol can be produced in the absence of any externally added cellulases.

Ethanol production can be measured using any method known in the art. For example, the quantity of ethanol in fermentation samples can be assessed using HPLC analysis. Many ethanol assay kits are commercially available that use, for example, alcohol oxidase enzyme based assays. Methods of determining ethanol production are within the scope of those skilled in the art from the teachings herein. The U.S. Department of Energy (DOE) provides a method for calculating theoretical ethanol yield. Accordingly, if the weight percentages are known of C6 sugars (*i.e.,* glucan, galactan, mannan), the theoretical yield of ethanol in gallons per dry ton of total C6 polymers can be determined by applying a conversion factor as follows:

(1.11 pounds of C6 sugar/pound of polymeric sugar) x (0.51 pounds of ethanol/pound of sugar) x (2000 pounds of ethanol/ton of C6 polymeric sugar) x (1 gallon of ethanol/6.55 pounds of ethanol) x (1/100%), *wherein the factor* (1 gallon of ethanol/6.55 pounds of ethanol) *is taken as the specific gravity of ethanol at 20°C.*

And if the weight percentages are known of C5 sugars (*i.e.,* xylan, arabinan), the theoretical yield of ethanol in gallons per dry ton of total C5 polymers can be determined by applying a conversion factor as follows:

(1.136 pounds of C5 sugar/pound of C5 polymeric sugar) x (0.51 pounds of ethanol/pound of sugar) x (2000 pounds of ethanol/ton of C5 polymeric sugar) x (1 gallon of ethanol/6.55 pounds of ethanol) x (1/100%), *wherein the factor* (1 gallon of ethanol/6.55 pounds of ethanol) *is taken as the specific gravity of ethanol at 20°C.*

It follows that by adding the theoretical yield of ethanol in gallons per dry ton of the total C6 polymers to the theoretical yield of ethanol in gallons per dry ton of the total C5 polymers gives the total theoretical yield of ethanol in gallons per dry ton of feedstock.

Applying this analysis, the DOE provides the following examples of theoretical yield of ethanol in gallons per dry ton of feedstock: corn grain, 124.4; corn stover, 113.0; rice straw, 109.9; cotton gin trash, 56.8; forest thinnings, 81.5;

hardwood sawdust, 100.8; bagasse, 111.5; and mixed paper, 116.2. It is important to note that these are theoretical yields. The DOE warns that depending on the nature of the feedstock and the process employed, actual yield could be anywhere from 60% to 90% of theoretical, and further states that "achieving high yield may be costly, however, so lower yield processes may often be more cost effective." (*Ibid.*)

**TDK Counterselection**

In the field of genetic engineering, cells containing an engineering event are often identified through use of positive selections. This is done by creating genetic linkage between the positive selection encoded by a dominant marker such as an antibiotic resistance gene, the desired genetic modification, and the target loci. Once the modifications are identified, it is often desirable to remove the dominant marker so that it can be reused during subsequent genetic engineering events.

However, if a dominant marker does not also have a counter selection, a gene expressing a protein that confers a counter-selection, must be genetically linked to the dominant marker, the desired genetic modification, and the target loci. To avoid such limitations, the methods of the invention include linking and/or designing a transformation associated with recombination between the thymidine kinase gene (TDK) from the Herpes Simplex Virus Type 1 (GenBank Accession No. AAA45811; SEQ ID NO:4) and one or more antibiotic resistance genes. *See, e.g.,* Argyros, D. A., et al., "High Ethanol Titers from Cellulose by Using Metabolically Engineered Thermophilic, Anaerobic Microbes," Appl. Environ. Microbiol. 77(23):8288-94 (2011). Examples of such antibiotic resistant genes, include but are not limited to aminoglycoside phosphotransferase (Kan; resistant to G418), nourseothricin acetyltransferase (Nat; resistant to nourseothricin), hygromycin B phosphotransferase (hph; resistant to hygromycin B), or a product of the Sh ble gene 1 (ble; resistant to Zeocin). Using such counter-selection methods with linked positive/negative selectable markers, transformants comprising the desired genetic modification have been obtained as described further in the examples below.

EXAMPLES

The invention now being generally described, it will be more readily understood by reference to the following examples, which are included merely for purposes of illustration of certain aspects and embodiments of the present invention, and are not intended to limit the invention.

The following examples describe *S. cerevisiae* genotypes for improved acetate-to-ethanol conversion by improving the availability of redox cofactors NADH or NADPH. Homologous recombination within the yeast cell can be used for genomic integrations and the construction of plasmids. With this approach, DNA fragments (containing promoters, terminators and open reading frames) are synthesized by PCR, with overlapping regions to adjoining fragments and/or the integration site. After cotransformation of the yeast with the synthesized fragments, the yeast are screened for those containing complete assemblies. Anybody skilled in the art can design the necessary primers and perform the required transformations, and only the final DNA sequences are included in the examples below. In many cases the genomic integration site is first pre-marked with one of two antibiotic markers (to target both alleles in diploid strains) and a marker for counter-selection (such as the Herpes simplex HSV-1 thymidine kinase *tdk* gene, which introduces a sensitivity to fluoro-deoxyuracil, to facilitate the isolation of correct transformants. *See* Argyros, D. A., *et al.,* (2011).

Promoter and terminator pairs in the following examples are exemplary. Possible promoters include, but are not limited to: ADH1, TPI1, ENOI, PFK1, ADH5, XKS1. Possible terminators include, but are not limited to: FBA1, PDC1, ENOI, HXT2, ALD6, SOL3.

Example 1

The present prophetic example describes engineering of a recombinant microorganism to increase flux through the oxidative pentose phosphate pathway (PPP) by creating a redox imbalance in xylose consumption using xylose reductase (XR) and xylitol dehydrogenase (XDH) that is coupled with the conversion of acetate to ethanol or isopropanol.

Current methods rely on xylose isomerase to enable *S. cerevisiae* to consume xylose. An alternative pathway that uses XR and XDH has been studied in the scientific literature, but achieving efficient ethanol production using this method has been difficult because of the pathway's redox imbalance. *See* Watanabe, S. et al., "Ethanol production from xylose by recombinant Saccharomyces cerevisiae expressing protein engineered NADP+-dependent xylitol dehydrogenase," J. Biotechnol. 130:316-19 (2007). XRs typically have a higher affinity for the cofactor NADPH, whereas most XDHs are NAD-specific. *See* Watanabe, S. *et al.,* (2007).

Recently an acetate-to-ethanol pathway has been described in U.S. Patent Appl. No. 13/696,207, which is incorporated by reference herein in its entirety. *See also* Medina, V. G., et al., "Elimination of Glycerol Production in Anaerobic Cultures of a Saccharomyces cerevisiae Strain Engineered To Use Acetic Acid as an Electron Acceptor," Appl. Environ. Microbiol. 76:190-195 (2010). This pathway, which relies on the introduction of a heterologous acetaldehyde dehydrogenase (ACDH), consumes two NADH molecules per every molecule of acetate converted. *See* Figure 1. As described herein, this NADH-consuming pathway can be used to balance the surplus NADH generated by XDH during xylose fermentation. The NADPH required by XR can be produced by redirecting part of the fructose-6-P produced by the PPP into the oxidative path of the PPP, which produces 2 NADPH per $CO_2$. Xylose fermentation via NADPH-specific XR and NAD-specific XDH together with acetate-to-ethanol conversion via ACDH generates a net amount of ATP (equation 1), whereas no ATP is generated when the surplus NADH is reoxidized via NADH-specific glycerol formation.

$$2\ \text{xylose} + \text{acetate} \rightarrow 4\ \text{ethanol} + 4\ \text{CO2} + \text{ATP}\ \text{(equation 1)}$$

The pathway of the present invention stoichiometrically couples acetate consumption to xylose fermentation in a 1:2 molar ratio. The overall reaction results in the formation of sufficient ATP to allow for growth of the microorganisms. In the absence of other ATP-yielding reactions, it would also be possible to use natural selection to select for mutant microorganisms with faster anaerobic ethanolic fermentation on xylose/acetate mixtures and increased tolerance to industrial feedstocks.

A similar strategy is employed for an acetate-to-isopropanol pathway based on the expression of the heterologous enzymes acetyl-CoA acetyltransferase (thiolase), acetoacetyl-CoA transferase, acetoacetate decarboxylase and a secondary alcohol dehydrogenase. *See* Figure 3. However, to produce a positive ATP yield, additional engineering is done, e.g., by replacing or supplementing the endogenous AMP-producing acetyl-CoA synthetase (ACS) (also referred to as acetyl-CoA ligase) by an ADP-producing variant, or using the acetate kinase/phosphotransacetylase (AK/PTA) couple. The endogenous AMP-producing ACS consumes one ATP per acetate and produces AMP. The use of an ADP-producing ACS, or the enzymes acetate kinase and phosphotransacetylase, consumes one ATP molecule per acetate molecule, however ADP is produced instead of AMP. The energy released by the conversion of ATP to AMP is about twice that of the conversion of ATP to ADP, thus using an ATP-to-ADP conversion is more energy efficient (to stress this difference, ATP requirements in Figure 2 have been normalized to ATP-to-ADP, so the ATP-to-AMP conversion of AMP-ACS counts as 2 ATP to 2 ADP). *See* Figure 2. By replacing an AMP-forming acetyl-CoA synthetase with an ADP-forming variant or by AK/PTA, the resulting pathway increases the yield of ATP by four molecules (equation 2).

$$4\quad \text{acetate} + 2\ \text{xylose} + \text{ATP} \rightarrow 2\ \text{isopropanol} + 3\ \text{ethanol} + 6\ CO_2\ \text{(equation 2)}$$

Testing this strategy involves engineering a yeast such as *S. cerevisiae* to use XR and XDH for xylose consumption and to convert acetate-to-ethanol by introducing an ACDH, and demonstrating anaerobic ethanol production with the combined consumption of xylose and acetate.

NADPH-specific XR and NADH-specific XDH are overexpressed in a strain overexpressing an NADH-dependent ACDH. To improve xylose consumption *XKS1* may also be overexpressed. In one embodiment of the invention, one or more genes of the pentose phosphate pathway (either endogenous or heterologous genes) are also overexpressed, which can improve xylose metabolism. For example, the endogenous pentose phosphate genes transaldolase (*TAL1*), xylulokinase (*XKS1*), transketolase (*TKL1*), ribulose-phosphate 3-epimerase (*RPE1*) and ribulose 5-phosphate isomerase (*RKI1*) are overexpressed in the *gre3* locus. *See* Figures 13 and 14.

Glycerol production can also be reduced to enable growth, *e.g.,* by deleting *gpd1. See, e.g.,* U.S. Patent Appl. No. 13/696,207, which is incorporated by reference herein in its entirety. For example, the *Scheffersomyces stipitis XYL1* and *XYL2* genes and *Piromyces adhE* are overexpressed in the *gpd1* locus. *See* Figures 15 and 16. *XYL1* can be replaced by either the *Candida boidinii AR* or the *Neurospora crassa XR* gene.

This strain is grown under anaerobic conditions in media containing xylose as well as acetate. Because of the need to balance the use of redox cofactors and generate ATP, it is expected that the surplus NADH formed during the fermentation of xylose to ethanol is to a large extent used for the conversion of acetate to ethanol via the NADH-dependent ACDH.

Examples of XR sequences include: *Scheffersomyces stipitis* XYL1 (SEQ ID NO:5), *Candida boidinii* Aldolase Reductase (SEQ ID NO:6), and *Neurospora crassa* Xylose Reductase (codon-optimized for *S. cerevisiae* by DNA 2.0) (SEQ ID NO:7).

Examples of XDH sequences include: *Scheffersomyces stipitis* XYL2 (SEQ ID NO:8).

The nucleotide sequence for *Piromyces adhE* is provided as SEQ ID NO:9.

Examples of ACS sequences include: *Entamoeba histolytica* ACS Q9NAT4 (ADP-forming) (SEQ ID NO: 10), *Giardia intestinalis* ACS (ADP-forming) (SEQ ID NO:11), *Pyrococcus furiosus* ACS Q9Y8L1 (ADP-forming) (SEQ ID NO:12), *Pyrococcus furiosus* ACS Q9Y8L0 (ADP-forming) (SEQ ID NO:13), *Pyrococcus furiosus* ACS E7FI45 (ADP-forming) (SEQ ID NO:14), and *Pyrococcus furiosus* ACS E7FHP1 (ADP-forming) (SEQ ID NO:15).

The amino acid sequence for *S. cerevisiae TAL1* is provided in SEQ ID NO: 16. The amino acid sequence for *S. cerevisiae XKS1* is provided in SEQ ID NO:17. The amino acid sequence for *S. cerevisiae TKL1* is provided in SEQ ID NO:18. The amino acid sequence for *S. cerevisiae RPE1* is provided in SEQ ID NO:19. The amino acid sequence for *S. cerevisiae RKI1* is provided in SEQ ID NO:20.

The upstream sequence used to delete *S. cerevisiae GRE3* is provided in SEQ ID NO:21. The downstream sequence used to delete *S. cerevisiae GRE3* is provided in SEQ ID NO:22.

2μ multi-copy vectors have been constructed expressing the *XYL2* XDH from *Scheffersomyces stipitis* (formerly *Pichia stipitis*) and one of the following three XRs: *XYL1* from *S. stipitis* (which has comparable affinity for NADH and NADPH), the more NADPH-specific XR from *Neurospora crassa* (codon-optimized), or aldolase reductase from *Candida boidinii. See* Figures 15 and 16.

Transformation of strain M2566, in which *GRE3* has been replaced by a cassette of PPP genes (including *XKS1* under the *HXT3* promoter), with the plasmid carrying *S. stipitis* XR and XDH and selection on aerobic YNX agar plates resulted in a low number of colonies. The M2566 strain was derived from strain M2390 (described in U.S. Patent Appl. No. 13/696,207 and U.S. Patent Appl. No. 13/701,652, both of which are incorporated by reference herein in their entirety). In M2566, both chromosomal copies of GRE3 (M2390 is a diploid strain) have been replaced with an expression cassette with genes from the pentose phosphate pathway, for example *XKS* and *TKL1.* Overexpressing these pentose phosphate pathway genes in *S. cerevisiae* generally improves xylose fermentation when either xylose isomerase or xylose reductase/xylitol dehydrogenase are expressed. A schematic and vector map of the cassette used to create the M2566 strain are depicted in Figures 26 and 27, respectively. To create this strain, YNX agar media containing 6.7 g/l yeast nitrogen base with amino acids (Sigma Y1250), 20 g/l bacta agar, and 20 g/l xylose was used. The YNX agar media was supplemented with nourseothricin to allow selection based on the presence of the plasmid and the agar plates were incubated at 35° C for several days.

Further steps will encompass integrating XR, XDH and ACDH into the genome of M2566 using the techniques described above, for increased stability of expression, and selecting for growth under anaerobic conditions on xylose/acetate mixtures such as the synthetic medium described in Verduyn et al. "Effect of benzoic acid on metabolic fluxes in yeasts: a continuous-culture study on the regulation of respiration and alcoholic fermentation," Yeast 8(7):501-17 (1992), supplemented with 420 mg/l Tween-80 and 10 mg/l ergosterol, to allow for anaerobic growth, and with xylose and acetate in an approximately 2:1 molar ratio. For example, endogenous *GPD1* (encoding a glycerol-3-phosphate dehydrogenase) can be replaced with the XR/XDH/ACDH expression cassette (see Fig. 16) as glycerol formation competes with the acetate-to-ethanol conversion for NADH, and deleting *GPD1* has previously been shown to reduce glycerol production in U.S. Patent Appl. No. 13/696,207, which is incorporated by reference herein.

Example 2

The present example describes engineering of a recombinant microorganism to increase flux through the oxidative pentose phosphate pathway (PPP) by overexpressing pathway genes or reducing the expression of competing pathways that is coupled with the conversion of acetate to ethanol or isopropanol.

The strategy of Example 1 relies on two redox imbalanced pathways that counterbalance each other. An alternative approach is to improve the kinetics of the oxidative branch of the PPP over those of competing pathways. This is achieved by various approaches, including directly increasing the expression of the rate-limiting enzyme(s) of the oxidative branch of the PPP pathway, such as glucose-6-P dehydrogenase (encoded endogenously by *ZWF1,* SEQ ID NO:23), changing the expression of regulating transcription factors like Stb5p (also referred to as Stb5) (Cadière, A., et al., "The Saccharomyces cerevisiae zinc factor protein Stb5p is required as a basal regulator of the pentose phosphate pathway," FEMS Yeast Research 10:819-827 (2010)), which controls the flux distribution between glycolysis and the oxidative pentose phosphate pathway by modulating activities of enzymes involved in both pathways, or directly down-regulating the expression of genes involved in competing pathways (e.g., glycolysis), such as glucose-6-P isomerase (encoded by PGI1 in *S. cerevisiae*). A similar effect might be achieved by increasing the expression of the other genes of the oxidative pentose phosphate pathway, including the 6-phosphogluconolactonases *SOL3* and *SOL4,* and the 6-phosphogluconate dehydrogenases *GND1* and GND2.

The sequence for *Saccharomyces cerevisiae stb5* is provided in SEQ ID NO:24.

STB5 is overexpressed in a strain overexpressing either an NADPH-dependent acetaldehyde dehydrogenase, or an NADH-dependent acetaldehyde dehydrogenase, *e.g., B. adolescentis adhE,* in combination with genes that could affect the conversion of NADPH into NADH, such as *gdh2* (SEQ ID NO:1) or a transhydrogenase (*see* Example 5). *See* Figures 17 and 18. In the latter case, competition with glycerol formation (another NADH-consuming reaction) can be prevented by deleting *gpd1* and *gpd2. See* Figures 7-10.

The strain is grown under anaerobic conditions in media containing glucose as well as acetate. Overexpressing STB5 is expected to force more glucose through the oxidative pentose phosphate pathway, generating more NADPH, which will improve the conversion of acetate to ethanol via, *e.g.,* an NADPH-dependent acetaldehyde dehydrogenase.

The amino acid sequence for *B. adolescentis adhE* is provided in SEQ ID NO:25. The upstream sequence used for deleting the *Gpd1* gene is provided in SEQ ID NO:26. The downstream sequence used for deleting the *Gpd1* gene is provided in SEQ ID NO:27. The sequence of the *Gpd2* promoter region used for deleting the *Gpd2* gene is provided in SEQ ID NO:28. The downstream sequence used for deleting the *Gpd2* gene is provided in SEQ ID NO:29.

Producing more $CO_2$ in the oxidative branch of the PPP increases the availability of NADPH and increases the NADPH/NADP ratio. This stimulates the flux of acetate-consuming pathways, for example ethanol-to-isopropanol conversion that relies on a NADPH-consuming secondary alcohol dehydrogenase to convert acetone to isopropanol, or an acetate-to-ethanol pathway that uses a NADPH-consuming acetaldehyde dehydrogenase (ACDH) and/or alcohol dehydrogenase (ADH), that (at least partially) consume NADPH. Thus, while the supply of NADH is fairly limited, yeast have more flexibility to create NADPH via the oxidative pentose phosphate pathway where there is a demand for NADPH consumption. *See* Celton, M., et al., "A constraint-based model analysis of the metabolic consequences of increased NADPH oxidation in Saccharomyces cerevisiae," Metabolic Eng. 14(4):366-79 (2012).

For example, wild-type yeast do not possess endogenous ACDH activity and exogenously introduced ACDH enzymes are thought to only participate in the acetate-to-ethanol pathway. The adhB from *T. pseudethanolicus* is a gene that may have NADPH-specific ACDH activity and can be used in the above process. *See* Burdette D. and Zeikus, J. G., "Purification of acetaldehyde dehydrogenase and alcohol dehydrogenases from Thermoanaerobacter ethanolicus 39E and characterization of the secondary-alcohol dehydrogenase (2° Adh) as a bifunctional alcohol dehydrogenase-acetyl-CoA reductive thioesterase," Biochem J. 302:163-70 (1994). The nucleotide sequence for *T. pseudethanolicus* adhB is provided in SEQ ID NO:30.

Preliminary screening of *T. pseudethanolicus* adhB in the M2390 strain, to create the M4596 and M4598 strains, did not result in an increase in acetate uptake compared to control strain M2390 (described in U.S. Patent Appl. No. 13/696,207 and U.S. Patent Appl. No. 13/701,652, both of which are incorporated by reference herein in their entirety). *T. pseudethanolicus* adhB was introduced in M2390 in the FCY1 locus (both chromosomal copies), using two different promoter/terminator pairs, as demonstrated by the schematics and vector maps depicted in Figures 28 and 29. The strains were grown anaerobically in YPD (40 g/l glucose, 4 g/l acetate, pH 5.5) media. Final acetate concentrations for M2390 and the M4596 and M4598 strains were very similar, suggesting that introduction of the *T. pseudethanolicus* adhB gene did not increase conversion of acetate to ethanol. Because the latter two strains showed improved conversion of acetone to IPA compared to M2390, this confirmed that the *T. pseudethanolicus* adhB gene was expressed. That the enzyme appears to be more active with acetone suggests that the intracellular metabolite levels and protein characteristics significantly favor conversion of acetone to IPA over conversion of acetyl-CoA to acetaldehyde and/or acetaldehyde to ethanol. *See* Burdette D. and Zeikus, J.G. However, additional NADPH-specific ACDH enzymes can be used and tested for increased acetate uptake.

Modifying ADH activity in yeast is different from modifying ACDH activity, which is not present endogenously. NADH-specific ADHs are present in very high levels in yeast (around 10 U/mg protein; *see* van den Brink, J., et al., "Dynamics of Glycolytic Regulation during Adaptation of Saccharomyces cerevisiae to Fermentative Metabolism," Appl. Environ. Microbiol. 74(18):5710-23 (2008)), and play an important role in standard ethanolic fermentation. As a result, high expression levels of NADPH-specific ADHs can be used, and may be needed, to compete with the activity of NADH-specific ADHs. As an alternative approach, the activity of NADH-specific ADHs can be reduced by deletion, modification, or downregulation of some of the endogenous enzymes with this activity. For example, ADH1 is an attractive target because it has been reported to be responsible for about 90% of all ADH activity. Other example ADHs depend on the host organism (including but not limited to ADH2-5 and SFA1 from *Saccharomyces; see* Ida, Y., et al., "Stable disruption of ethanol production by deletion of the genes encoding alcohol dehydrogenase isozymes in Saccharomyces cerevisiae," J. Biosci. Bioeng. 113(2):192-95 (2012)), and can be identified through various genomic resources as available from the National Center for Biotechnology Information (ncbi.nlm.nih.gov) and the Saccharomyces Genome Database (yeastgenome.org). Full deletion of endogenous NADH-specific ADHs, however, would likely cripple the yeast. *See* Cordier, H., et al., "A metabolic and genomic study of engineered Saccharomyces cerevisiae strains for high glycerol production," Metab. Engineer. 9(4):364-78. There is an advantage, however, to expressing NADPH-specific ADHs in the presence of native NADH-specific ADHs, because the total flux through ADH (sugar-to-ethanol + acetate-to-ethanol) is much larger than the acetate-to-ethanol flux. As a result, even if the NADPH-specific ADH flux is only 5% of the original NADH-specific ADH flux, that amount of NADPH-ADH flux would still allow for 0.8 g extra acetate uptake per 100 g sugar (any NADPH used in the sugar-to-ethanol conversion saves an equal amount of NADH that can be used in the acetate-to-ethanol route).

Most of the NADPH-specific ADHs described in the literature (EC 1.1.1.2; *see, e.g.,* brenda-enzymes.org/php/result_flat.php4?ecno=1.1.1.2) are thought to be localized to the mitochondria or are from thermophiles, and most are thought to function best at high pH. While some may not function in the slightly acidic yeast cytosol, there are several candidate enzymes. First, there are the secondary alcohol dehydrogenases (2° Adh) from *T. pseudethanolicus* (adhB) and C. *beijerinckii.* The *T. pseudethanolicus* adhB is the same as that described above. The amino acid sequence for the *C. beijerinckii* 2° Adh is provided in SEQ ID NO:31.

Figure 32 depicts a schematic for the construct used to express *C. beijerinckii* 2° Adh (Cbe adhB). The constructs used to create strains M4597 and M4599, which contain *C. beijerinckii* 2° Adh expressed from the *FCY1* locus, are depicted in Figures 30 and 31. It may be desirable to use a codon-optimized version of the *C. beijerinckii* 2° Adh. The nucleotide sequence for a codon-optimized *C. beijerinckii* 2° Adh is provided in SEQ ID NO:32.

While *T. pseudethanolicus* adhB and *C. beijerinckii* 2° Adh likely prefer acetone as a substrate, they can be tested for the desired NADPH specificity and function with acetaldehyde as a substrate. *See* Burdette D. and Zeikus, J. G. The secondary alcohol dehydrogenases from *T. pseudethanolicus* and *C. beijerinckii* in *S. cerevisiae,* were expressed and both improved the conversion of acetone to isopropanol. The strains were grown anaerobically in YPD media (40 g/l glucose, 10 g/l acetone, pH 5). After 5 days, 1.9 g/l IPA was detected in the M2390 (control) culture. With *T. pseudethanolicus adhB,* the IPA titers were 8.1 g/l (ENOl promoter, ENOl terminator) and 3.1 g/l (TPI1 promoter, FBA1 terminator). With the *C. beijerinckii* 2° Adh, the IPA titers were 4.1 g/l (ENOl promoter, ENOl terminator) and 5.1 g/l (TPI1 promoter, FBA1 terminator).

A third gene that may possess the desired NADPH-ADH activity is the *S. cerevisiae* gene ARI1. *See* GenBank Accession No. FJ851468. The nucleotide and amino acid sequences for ARI1 are provided in SEQ ID NOs:33 and 34, respectively.

ARI1 has been shown to reduce a broad range of aldehydes. *See* Liu, Z. L., and Moon, J., "A novel NADPH-dependent aldehyde reductase gene from Saccharomyces cerevisiae NRRL Y-12632 involved in the detoxification of aldehyde inhibitors derived from lignocellulosic biomass conversion," Gene 446(1):1-10 (2009). Overexpression of ARI1 improves tolerance to furfural and hydroxymethylfurfural and ARI1 has been demonstrated to act on acetaldehyde as a substrate. *See* Liu, Z. L., and Moon, J., (2009). Constructs used to create overexpression of ARI1 are depicted in Figures 33 and 34.

Additional genes that may have NADPH-specific ADH activity include *Entamoeba histolytica ADH1* and *Cucumis melo ADH1. See* Kumar, A., et al., "Cloning and expression of an NADP(+)-dependent alcohol dehydrogenase gene of Entamoeba histolytica," PNAS 89(21:10188-92 (1992) and Manriquez, D., et al., "Two highly divergent alcohol dehydrogenases of melon exhibit fruit ripening-specific expression and distinct biochemical characteristics," Plant Molecular Biology 61(4):675-85 (2006). Constructs used to create strains expressing *Entamoeba histolytica ADH1* or *Cucumis melo ADH1* are depicted in Figures 35-38.

The nucleotide sequence for *Entamoeba histolytica ADH1* is provided in SEQ ID NO:35. The nucleotide sequence for *Cucumis melo ADH1* is provided in SEQ ID NO:36.

The activity of the above genes can be determined by using a gpd1/2 double knockout strain with an NADH-specific ACDH integrated into a host genome, *e.g.*, M2594. The M2594 strain is derived from M2390 (described above) in which all chromosomal copies of *GPD1* and *GPD2* (M2390 is a diploid strain) have been replaced with an expression cassette with two copies of *Bifidobacterium adolescentis adhE* (the first AdhE reuses the original GPD promotor, while the second in reverse orientation is introduced with a new promotor, and both AdhE have a new terminator). *See* Figures 7-10.

The candidate gene(s) can be expressed in high copy number and transformants screened for improved acetate uptake. This can be accomplished by integrating the gene candidates into chromosomal rDNA loci; a transformation method that allows integration of multiple copies of a gene cassette into the genome, given the multiple rDNA sequences in the genome that are highly homologous. The integration cassettes can include an antibiotic marker and xylosidase gene that can be used for selection of transformants. In addition, derivative strains of M2594 in which either one or both copies of the endogenous ADH1 have been deleted can be employed. Contructs that can be used for the deletion of ADH1 are depicted in Figures 39 and 40. Given that ADH1 is responsible for most of the yeast's NADH-specific alcohol dehydrogenase activity, reducing the expression of ADH1 may allow for the new genes to more readily compete with the high native levels of NADH-specific alcohol dehydrogenases. The screening of these strains can be performed with YPD or a Sigmacell medium, with HPLC to measure acetate levels.

Overexpression of an acetyl-CoA synthetase, for example, a gene encoding ACS1 or ACS2, in the above strains with NADPH-specific ADH activity may lead to improved acetate-to-ethanol conversion. Examples of genes encoding ACS1 and ACS2 include those from yeast and other microorganisms, including but not limited to, *Saccharomyces cerevisiae, Saccharomyces kluyveri, Zygosaccharomyces bailii,* and *Acetobacter aceti* ACS1 and/or ACS2. *See, e.g.,* Rodrigues, F., et al., "The Fate of Acetic Acid during Glucose Co-Metabolism by the Spoilage Yeast Zygosaccharomyces bailii," PLOS One 7(12):e52402 (2012); Sousa, M.J., et al., "Mechanisms underlying the transport and intracellular metabolism of acetic acid in the presence of glucose in the yeast Zygosaccharomyces bailii," Microbiology 144(3):665-70 (1998); Rodrigues, F., et al., "Isolation of an acetyl-CoA synthetase gene (ZbACS2) from Zygosaccharomyces bailii," Yeast 21(4):325-31 (2004); Vilela-Moura, A., et al., "Reduction of volatile acidity of wines by selected yeast strains," Appl. Microbiol. Biotechnol. 80(5):881-90 (2008); and O'Sullivan, J. and Ettlinger, L., "Characterization of the acetyl-CoA synthetase of Acetobacter aceti," Biochimica et Biophysica Acta (BBA) - Lipid and Lipid Metabolism, 450(3):410-17 (1976). These genes, *e.g.,* encoding the *S. cerevisiae* ACS2, are integrated in an expression vector to analyze its effect on acetate uptake and ethanol production. *See* Figures 50-52. ACS2 can be engineered with the *E. histolytica* ADH1 (SEQ ID NO:35) and/or the *S. cerevisiae* ZWF1 or STB5 (SEQ ID NOs:23 or 24, respectively) for effect on acetate uptake and ethanol.

The nucleotide sequence for *Saccharomyces cerevisiae acs1* is provided in SEQ ID NO:37. The nucleotide sequence for *Saccharomyces kluyveri acs1* is provided in SEQ ID NO:38. The nucleotide sequence for *Saccharomyces cerevisiae acs2* is provided in SEQ ID NO:39. The nucleotide sequence for *Saccharomyces kluyveri acs2* is provided in SEQ ID NO:40. The nucleotide sequence for *Zygosaccharomyces bailii* ACS is provided in SEQ ID NO:57. The nucleotide sequence for *Acetobacter aceti* ACS is provided in SEQ ID NO:58.

**Identifying Active NADPH-ADHs**

As described above, due to high NADH-ADH activity in wild-type *S. cerevisiae,* and to achieve sufficiently high expression of NADPH-ADH, the NADH-ADH gene candidates were integrated in the rDNA sites, which allows for high-copy genomic integration. The integration cassettes included antibiotic markers and a xylosidase gene, as discussed above, and transformants were selected for Zeocin resistance. For each transformation, approximately two dozen transformants were screened for xylosidase activity, and the transformants with the highest activity were tested for acetate uptake. The background strain was M4868, based on M2594 (described above), in which endognenous ADH1 is marked with two antibiotic markers. Each candidate NADPH-ADH was tested with either a TPI1 promoter and FBA1 terminator, or an ENOl promoter and ENOl terminator. *See* Figures 32-38.

To test for acetate uptake, the transformants were grown overnight in an aerobic tube with 5 ml YPD media (40 g/l glucose, 10 g/l acetone, pH 5). The following day, cells were collected by centrifugation, washed with demineralized water, and resuspended in 2 ml demineralized water. 100 ul of the cell suspension was used to inoculate 150 ml medium bottles containing 20 ml of YPD media with 40 g/l glucose and 4 g/l acetate (added as potassium acetate), set to pH 5.5 with HCl. Bottles were capped and flushed with a gas mixture of 5% $CO_2$ and 95% $N_2$ to remove oxygen, and incubated at 35° C in a shaker at 150 RPM for 48 hours. At 48 hours the bottles were sampled to determine glucose, acetate and ethanol concentrations, and pH using HPLC.

The results are shown below in Table 3. Each row represents a single bottle from a single transformant. All tested NADPH-ADHs, with the possible exception of the *C. melo* ADH1, improved acetate uptake. The highest acetate uptake was obtained with strain M4868 expressing ADH1 from *E. histolytica* using TPIlp and FBA1t.

Table 3. Acetate uptake for various NADPH-ADHs.

| | | Concentration (g/l) | | Consumption (g/l) | Consumption relative to M2594 (fold difference) |
|---|---|---|---|---|---|
| Background | ADH (in rDNA) | Acetate | Ethanol | Acetate | Acetate |
| M4868 | T. pseudethanolicus adhB (pENO1/ENO1t) | 2.96 | 19.75 | 0.51 | 1.5 |
| M4868 | T. pseudethanolicus adhB (pENO1/ENO1t) | 3.00 | 19.89 | 0.47 | 1.4 |
| M4868 | C. beijerinckii adhB (pTPI1/FBA1t) | 2.77 | 20.11 | 0.70 | 2.1 |
| M4868 | C. beijerinckii adhB (pTPI1/FBA1t) | 2.56 | 20.16 | 0.91 | 2.7 |
| M4868 | C. beijerinckii adhB (pTPI1/FBA1t) | 2.82 | 20.03 | 0.65 | 2.0 |
| M4868 | C. beijerinckii adhB (pTPI1/FBA1t) | 2.81 | 20.13 | 0.66 | 2.0 |
| M4868 | S. cerevisiae ARI1 (pENO1/ENO1t) | 3.07 | 20.00 | 0.40 | 1.2 |
| M4868 | S. cerevisiae ARI1 (pENO1/ENO1t) | 3.03 | 20.03 | 0.44 | 1.3 |
| M4868 | S. cerevisiae ARI1 (pTPI1/FBA1t) | 3.00 | 19.90 | 0.47 | 1.4 |

(continued)

| | | Concentration (g/l) | | Consumption (g/l) | Consumption relative to M2594 (fold difference) |
|---|---|---|---|---|---|
| Background | ADH (in rDNA) | Acetate | Ethanol | Acetate | Acetate |
| M4868 | S. cerevisiae ARI1 (pTPI1/FBA1t) | 2.94 | 19.97 | 0.53 | 1.6 |
| M4868 | C. melo ADH1 (pENO1/ENO1t) | 3.09 | 19.90 | 0.38 | 1.1 |
| M4868 | C. melo ADH1 (pENO1/ENO1t) | 3.12 | 19.94 | 0.35 | 1.1 |
| M4868 | C. melo ADH1 (pTPI1/FBA1t) | 3.15 | 19.83 | 0.32 | 1.0 |
| M4868 | C. melo ADH1 (pTPI1/FBA1t) | 3.11 | 19.83 | 0.36 | 1.1 |
| M4868 | E. histolytica (pENO1/ENO1t) | 2.63 | 19.97 | 0.84 | 2.5 |
| M4868 | E. histolytica (pENO1/ENO1t) | 2.64 | 19.98 | 0.83 | 2.5 |
| M4868 | E. histolytica (pTPI1/FBA1t) | 2.51 | 20.09 | 0.96 | 2.9 |
| M4868 | E. histolytica (pTPI1/FBA1t) | 2.45 | 20.23 | 1.02 | 3.1 |
| M2594 | - | 3.14 | 20.01 | 0.33 | 1.0 |
| Medium | | 3.47 | | | |
| | | | | | |
| Genotypes:<br>M2594: gpd1::adhE gpd2::adhE<br>M4868: gpd1::adhE gpd2::adhE | | | | | |

**Deletion of ADH1**

Using the NADPH-ADH results, mutants with one or both copies of the endogenous ADH1 deleted were tested. The screening process of above was repeated with two additional backgrounds: M2594 (with two functional copies of ADH1) and M4867 (with a single copy ADH1 deletion), with NADPH-ADHs, from *E. histolytica* and C. *beijerinckii.* These additional transformants demonstrated that expressing NADPH-ADH has little effect on acetate uptake in M2594, but increased acetate consumption in a single knockout of ADH1 (M4867) and in strain M4868 compared to M2594. The results are shown below in Table 4. The data for several isolates for each background/NADPH-ADH/promoter/terminator combination are shown in Figure 41.

Table 4. Acetate uptake for ADH1 deletion mutants.

| | Concentration (g/l) | | Consumption (g/l) | Consumption relative to M2594 (fold difference) |
|---|---|---|---|---|
| Modification | Acetate | Ethanol | Acetate | Acetate |
| C. beijerinckii adhB (pTPI1/FBA1t) | 3.11 | 19.49 | 0.59 | 1.4 |
| E. histolytica (pENO1/ENO1t) | 3.23 | 19.34 | 0.48 | 1.1 |
| E. histolytica (pENO1/ENO1t) | 3.26 | 19.52 | 0.45 | 1.0 |

(continued)

| | Concentration (g/l) | | Consumption (g/l) | Consumption relative to M2594 (fold difference) |
|---|---|---|---|---|
| Modification | Acetate | Ethanol | Acetate | Acetate |
| E. histolytica (pTPI1/FBA1t) | 3.23 | 19.33 | 0.47 | 1.1 |
| E. histolytica (pTPI1/FBA1t) | 3.26 | 19.44 | 0.45 | 1.0 |
| C. beijerinckii adhB (pTPI1/FBA1t) | 2.91 | 19.69 | 0.79 | 1.9 |
| C. beijerinckii adhB (pTPI1/FBA1t) | 2.83 | 19.59 | 0.87 | 2.0 |
| E. histolytica (pENO1/ENO1t) | 3.08 | 19.50 | 0.63 | 1.5 |
| E. histolytica (pENO1/ENO1t) | 3.10 | 19.59 | 0.61 | 1.4 |
| E. histolytica (pENO1/ENO1t) | 3.01 | 19.59 | 0.70 | 1.6 |
| E. histolytica (pENO1/ENO1t) | 2.50 | 19.81 | 1.20 | 2.8 |
| E. histolytica (pTPI1/FBA1t) | 2.52 | 19.76 | 1.18 | 2.8 |
| E. histolytica (pTPI1/FBA1t) | 2.69 | 19.86 | 1.01 | 2.4 |
| wild-type | 3.56 | 18.76 | 0.14 | 0.3 |
| gpd1::adhE gpd2::adhE | 3.28 | 19.45 | 0.42 | 1.0 |
| gpd1::adhE gpd2::adhE adh1/ADH1 | 3.29 | 19.70 | 0.42 | 1.0 |
| gpd1::adhE gpd2::adhE adh1/adh1 | 3.27 | 19.54 | 0.44 | 1.0 |
| M4868 + C. beijerinckii adhB (pTPI1/FBA1t) | 2.71 | 19.77 | 1.00 | 2.3 |
| M4868 + E. histolytica (pENO1/ENO1t) | 2.71 | 20.00 | 0.99 | 2.3 |
| M4868 + E. histolytica (pTPI1/FBA1t) | 2.48 | 19.62 | 1.23 | 2.9 |
| Medium | 3.70 | | | |

Additional strains that express the NADPH-ADH from *E. histolytica* without any changes to the endogenous NADH-ADH1 were created using the strategy depicted in Figure 53. Strain M6571 is a restocked version of M2594 and is genotypically identical to M2594.

Strains M6950 and M6951 have the *E. histolytica* ADH1 expressed at the site of the endogenous FCY1 gene, using two promoter/terminator combinations in an opposed orientation. Strains M6950 and 6951 were constructed by integrating the assembly MA1181 into M2594, using methods described above, and replacing the original FCY1 ORF with a two-copy expression cassette *of E. histolytica* ADH1. *See* Figure 54. Transformants were selected for 5FC resistance using FCY1 as a counterselectable marker. Experimental results for the various strains with 40 or 110 g/L glucose in bottles is provided in Tables 5 and 6. The 40 g/L glucose bottles were sparged with $N_2/CO_2$ prior to incubation, whereas the 110 g/L bottles were not.

Table 5. Acetate uptake for *E. histolytica* ADH1 expressing strains grown in 40 g/L glucose.

| YPD (40 g/l) Sampled after 48 hours | | | | | | |
|---|---|---|---|---|---|---|
| | | HPLC Concentrations (g/l) | | | | |
| Bottle no. | Strain | Glucose | Glycerol | Acetate | Ethanol | Acetate consumption (g/l) |
| 1 | M2390 | | 1.0 | 4.8 | 17.2 | 0.0 |

(continued)

| YPD (40 g/l) Sampled after 48 hours | | | | | | |
|---|---|---|---|---|---|---|
| | | HPLC Concentrations (g/l) | | | | |
| Bottle no. | Strain | Glucose | Glycerol | Acetate | Ethanol | Acetate consumption (g/l) |
| 2 | M2594 | | 0.1 | 4.5 | 17.7 | -0.2 |
| 4 | M6950 | | 0.1 | 4.2 | 17.8 | -0.6 |
| 5 | M6951 | 0.1 | 0.1 | 4.2 | 17.7 | -0.6 |
| 10 | M5553 | | 0.1 | 4.6 | 17.6 | -0.2 |
| 11 | M5582 | | 0.1 | 4.1 | 17.9 | -0.7 |
| 12 | M5586 | | 0.2 | 3.7 | 18.0 | -1.0 |
| | Media | 35.9 | 0.1 | 4.7 | | |

Table 6. Acetate uptake for *E. histolytica* ADH1 expressing strains grown in 100 g/L glucose.

| YPD (110 g/l), not flushed; Sampled after 72 hours | | | | | | |
|---|---|---|---|---|---|---|
| | | Concentrations (g/l) | | | | |
| Bottle no. | Strain | Glucose | Glycerol | Acetate | Ethanol | Acetate consumption (g/l) |
| 13 | M2390 | | 2.5 | 4.6 | 51.0 | -0.3 |
| 14 | M2594 | | 0.2 | 3.9 | 52.8 | -1.0 |
| 16 | M6950 | | 0.2 | 2.5 | 53.5 | -2.4 |
| 17 | M6951 | | 0.2 | 2.4 | 53.7 | -2.5 |
| 22 | M5553 | | 0.1 | 4.1 | 53.2 | -0.8 |
| 23 | M5582 | | 0.3 | 2.1 | 53.9 | -2.8 |
| 24 | M5586 | 11.8 | 1.3 | 1.9 | 46.0 | -3.0 |
| | M6571 | | 0.1 | 4.1 | 52.8 | -0.8 |
| | Media | 110.1 | 0.1 | 4.9 | | |

As demonstrated in Table 6, acetate consumption in strains M6950 and M6951 is comparable to that of strain M5582, in which both copies of endogenous ADH1 are deleted and *E. histolytica* ADH1 is expressed (*see* Tables 7-9 below). Thus, while deleting one or both copies of endogenous ADH1 in microorganisms expressing exogenous NADPH-specific ADHs might be beneficial in the context of acetate consumption, it is not required to obtain a significant improvement in acetate uptake.

**Improving NADPH availability**

To determine if acetate uptake can be further increased above the NADPH-ADH results described above for the ADH1 double knockout strains, STB5 or ZWF1 were overexpressed. Strains were reconstructed, targeting the NADPH-ADH to the site of YLR296W, to eliminate uncertainty regarding the copy number of the rDNA integration cassettes (*see* Figures 43-45). To facilitate the strain construction, the ADH1 ORFs were cleanly deleted (not leaving any antibiotic markers; Figure 42), resulting in strain M5553. Transformants expressed 4 copies of the E. *histolytica* ADH1 and two copies of ZWF1 or STB5.

Screening of several transformants indicated that STB5 overexpression slightly reduced acetate uptake, whereas ZWF1 overexpression increased acetate uptake, compared to overexpression of *E. histolytica* ADH1 alone. The results are shown below in Tables 7 and 8.

Table 7. Acetate uptake for strains overexpressing *E. histolytica* ADH1 and either STB5 or ZWF1.

| | | Concentration (g/l) | | Consumption (g/l) |
|---|---|---|---|---|
| Strain | Modification | Acetate | Ethanol | Acetate |
| M2390 | wild-type | 4.52 | 18.09 | 0.1 |
| M2594 | gpd1::adhE gpd2::adhE | 4.31 | 18.88 | 0.3 |
| M4868 | M2594 adh1 marked by antibiotic markers | 4.30 | 18.85 | 0.3 |
| M5279 | M4868 + E. histolytica ADH1 (pENO1/ENO1t) (rDNA) | 3.91 | 18.83 | 0.7 |
| M5280 | M4868 + E. histolytica ADH1 (pTPI1/FBA1t) (rDNA) | 3.70 | 19.15 | 0.9 |
| M5553 | M2594 adh1/adh1 | 4.31 | 18.93 | 0.3 |
| M5582 | M5553 + E. histolytica ADH1 (4x) | 3.72 | 19.22 | 0.9 |
| M5583 | M5553 + E. histolytica ADH1 (4x) | 3.67 | 19.16 | 0.9 |
| M5584 | M5553 + E. histolytica ADH1 (4x) + STB5 (2x) | 3.88 | 19.20 | 0.7 |
| M5585 | M5553 + E. histolytica ADH1 (4x) + STB5 (2x) | 3.85 | 19.21 | 0.8 |
| M5586 | M5553 + E. histolytica ADH1 (4x) + ZWF1 (2x) | 3.44 | 19.12 | 1.2 |

Table 8. Acetate uptake for strains overexpressing *E. histolytica* ADH1 and either STB5 or ZWF1.

| | | Concentration (g/l) | | Consumption (g/l) |
|---|---|---|---|---|
| Strain | Modification | Acetate | Ethanol | Acetate |
| M2390 | wild-type | 3.73 | 17.70 | 0.0 |
| M2594 | gpd1::adhE gpd2::adhE | 3.33 | 18.55 | 0.4 |
| M5280 | M4868 + E. histolytica ADH1 (pTPI1/FBA1t) (rDNA) | 2.76 | 18.75 | 1.0 |
| M5582 | M5553 + E. histolytica ADH1 (4x) | 2.80 | 18.73 | 1.0 |
| M5583 | M5553 + E. histolytica ADH1 (4x) | 2.80 | 18.73 | 1.0 |
| M5584 | M5553 + E. histolytica ADH1 (4x) + STB5 (2x) | 2.93 | 18.75 | 0.8 |
| M5585 | M5553 + E. histolytica ADH1 (4x) + STB5 (2x) | 2.89 | 18.70 | 0.9 |
| M5586 | M5553 + E. histolytica ADH1 (4x) + ZWF1 (2x) | 2.48 | 18.88 | 1.3 |

**Higher sugar concentrations**

To determine if acetate uptake can be increased above the NADPH-ADH results described above in the presence of an increased sugar concentration, strains were screened in YPD with 120 g/l glucose and 5.5 g/l acetate, pH 5.5. The bottles in these high-sugar concentration experiments were not flushed with a nitrogen/carbon dioxide mixture because flushing the bottles does not always result in finishing the fermentation, which can leave residual sugar behind. Acetate consumption increased up to 3.3 g/l under an increased sugar concentration. *See* Table 9.

Table 9. Acetate uptake at an increased sugar concentration.

| | | Concentration (g/l) | | Consumption (g/l) |
|---|---|---|---|---|
| Strain | Modification | Acetate | Ethanol | Acetate |
| M2390 | wild-type | 5.3 | 52.4 | 0.0 |
| M2390 | wild-type | 5.4 | 52.1 | -0.1 |
| M2594 | gpd1::adhE gpd2::adhE | 4.3 | 54.6 | 1.0 |
| M2594 | gpd1::adhE gpd2::adhE | 4.5 | 54.9 | 0.8 |
| M5553 | M2594 adhl | 4.3 | 54.7 | 1.0 |
| M5553 | M2594 adhl | 4.5 | 54.7 | 0.8 |
| M5582 | M5553 + EhADH1 (4x) | 2.0 | 55.5 | 3.3 |
| M5582 | M5553 + EhADH1 (4x) | 2.1 | 55.6 | 3.2 |
| | Medium | 5.3 | | |

**Strain Construction**

Construction of M2390 and M2594 are described above. Strain M4867 was constructed by deleting a single copy of ADH1 using the cassette depicted in Figure 2. M4868 was constructed by deleting both copies of ADH1 using the cassettes depicted in Figures 39 and 40. Strain M5553 is similarly based on M2594, but has clean deletions of two copies of ADH1 (*i.e.,* the promoter and terminator were left intact, but the open reading frame (ORF) was removed). *See* Figure 2. The *S. cerevisiae* ADH1 nucleotide sequence for reference strain S288C is provided in SEQ ID NO:41.

Strains M5582, M5584 and M5586 are based on M5553, and overexpress ADH1 from E. *histolytica* as well as endogenous STB5 (M5584 only) or ZWF1 (M5586 only). *See* Figures 43-45. The sequence of these genes is provided above. Each of these integrations replaces the ORF of YLR296W. Integration cassettes containing either hygromycin or zeocin resistance markers allowed targeting of both YLR296W sites in the diploid strain. *See* Figures 43-45.

**Summary**

As demonstrated above, deleting endogenous NADH-ADH and introducing heterologous NADPH-ADH improved conversion of acetate to ethanol. Without wishing to be bound by any theory, the improvement may be due to the introduction of a redox imbalance in sugar fermentation, leading to a net conversion of NADPH to NADH. A smaller but additional beneficial effect is that the acetate-to-ethanol pathway itself, for which a heterologous NADH-dependent acetaldehyde dehydrogenase is expressed, also relies on alcohol dehydrogenase. With NADPH-ADH, the conversion of acetate to ethanol consumes less NADH and more NADPH. Because the yeast strains were tested anaerobically, and because these strains are glycerol-3-phosphate dehydrogenase negative, the only way the cells can reoxidize NADH is by taking up acetate and converting it to ethanol. In addition, further improvements in acetate uptake were obtained by overexpressing ZWF1, whereas overexpressing STB5 had less of an effect.

Figures 46 and 47 show how the use of redox cofactors is affected by expressing NADPH-ADH. In the extreme case where yeast balance the use of NADH and NADPH *(i.e.,* as much NADH is consumed as is produced; same for NADPH), and where yeast directs all of the ATP it generates from sugar fermentation to the conversion of acetate to ethanol, 29 g/l acetate can be consumed per 100 g/l glucose (or xylose). In this case, two-thirds of the ADH activity is NADPH-dependent, and one-third is NADH-dependent. The above strains might be unable to grow when completely lacking in NADH-ADH activity, because this would produce more NADH than can be consumed with the limited amount of ATP available from sugar metabolism. The strains containing deletions in both copies of *ADH1* (which results in partial replacement of cytosolic NADH-ADHs with NADPH-ADH) grew, however, so modifying the cofactor preference for ADH demonstrated cell viability and increased acetate consumption and ethanol production with an NADPH-preferring ADH.

Example 3

The present prophetic example describes engineering of a recombinant microorganism to use the ribulose-monophosphate pathway (RuMP) for production of electron donors to be used in the conversion of acetate to ethanol or isopropanol.

Instead of relying on the endogenous oxidative branch of the PPP as described in Example 2, the heterologous RuMP pathway found in various bacteria and archaea, including *Bacillus subtilis, Methylococcus capsulatus,* and *Thermococcus kodakaraensis,* which also produces $CO_2$ while conferring electrons to redox carriers, can be introduced. *See* Yurimoto, H., et al., "Genomic organization and biochemistry of the ribulose monophosphate pathway and its application in biotechnology," Appl. Microbiol. Biotechnol. 84:407-416 (2009).

This pathway relies on the expression of two heterologous genes, 6-phospho-3-hexuloisomerase (PHI) and 3-hexulose-6-phosphate synthase (HPS). Examples of PHI and HPS enzymes include *Mycobacterium gastri rmpB* and *Mycobacterium gastri rmpA,* respectively. PHI converts fructose-6-P to D-arabino-3-hexulose-6-P, and HPS converts the latter to ribulose-5-P and formaldehyde. *See* Figure 5. While this conversion is redox neutral, the produced formaldehyde can then be converted to $CO_2$ by the action of the endogenous enzymes formaldehyde dehydrogenase (*SFA1*) and S-formylglutathione hydrolase *(YJL068C),* which produce formate and NADH, and formate dehydrogenase (*FDH1*), which converts the formate to $CO_2$, producing a second NADH. These enzymes can be overexpressed or upregulated.

A beneficial effect of FDH1 overexpression on formate consumption has been demonstrated. *See* Geertman, J-M. A., et al., "Engineering NADH metabolism in Saccharomyces cerevisiae: formate as an electron donor for glycerol production by anaerobic, glucose-limited chemostat cultures," FEMS Yeast Research 6(8):1193-1203 (2006). It is also possible to overexpress heterologous genes, like the formaldehyde and formate dehydrogenases from *O. polymorpha,* which improve formaldehyde consumption in *S. cerevisiae. See* Baerends, R. J. S., et al., "Engineering and Analysis of a Saccharomyces cerevisiae Strain That Uses Formaldehyde as an Auxiliary Substrate," Appl. Environ. Microbiol. 74(1):3182-88 (2008). Overexpression of an NADH-dependent acetaldehyde dehydrogenase may also be employed to enable conversion of acetate to ethanol. Competition with glycerol formation (another NADH-consuming reaction) can be prevented by deleting *gpd1* and *gpd2.*

This strain is grown under anaerobic conditions in media containing C6 and/or C5 sugars, as well as acetate. *See* Figures 19 and 20. The RuMP pathway, combined with formaldehyde degradation to $CO_2$, can generate NADH, which will improve the conversion of acetate to ethanol via an NADH-dependent acetaldehyde dehydrogenase.

The sequence for *Mycobacterium gastri rmpB* (PHI) is provided in SEQ ID NO:42. The sequence for *Mycobacterium gastri rmpA* (HPS) is provided in SEQ ID NO:43. The sequence for *Saccharomyces cerevisiae SFA1* is provided in SEQ ID NO:44. The sequence for *Saccharomyces cerevisiae YJL068C* is provided in SEQ ID NO:45. The sequence for *Saccharomyces cerevisiae FDH1* is provided in SEQ ID NO:46. The sequence for *Candida boidinii FDH3* is provided in SEQ ID NO:47.

To bring this strategy into practice, first the formaldehyde or formate degrading enzymes can be overexpressed or upregulated in a yeast such as *S. cerevisiae,* and then assayed to verify that the increased NADH production allows for increased acetate consumption in cultures supplemented with formaldehyde and/or formate. This assay involves the addition of formaldehyde or formate to the medium and determining whether these compounds are taken up by the yeast and if it produces more ethanol, using techniques described herein and in WO 2012/138942 (PCT/US2012/032443), incorporated by reference herein in its entirety. Once this has been demonstrated, functional expression of PHI and HPS that confer this benefit without the need for formaldehyde/formate supplementation can be screened. Functional expression of PHI and HPS in the pathway can be screened by measuring for improved acetate uptake and ethanol titers as described herein and in U.S. Patent Appl. No. 13/696,207, incorporated by reference herein in its entirety. Figure 20 depicts a construct used to create a microorganism containing this engineered RuMP pathway.

**FDH Expression**

Acetate consumption and availability of NADH was measured by expression of a formate dehydrogenase from *S. cerevisiae* (FDH1; SEQ ID NO: 46) or from *Candida boidinii* (FDH3; SEQ ID NO: 47). Two cassettes, one with a single copy of the *S. cerevisiae* FDH1 (ADH1 promoter and PDC1 terminator) (Figure 48), and one with two copies of the *Candida boidinii* FDH3 (TPI1 promoter, FBA1 terminator, and PFK1 promoter, HXT2 terminator) (Figure 49), were expressed in M2594. Two verified transformants per cassette were tested in anaerobic bottles on YPD (40 g/l glucose, 3 g/l acetate, and 2 g/l formate, pH 4.8 (set with HCl)), which were sparged with 5% $CO_2$ / 95 % $N_2$ after inoculation to remove oxygen, and incubated for 48 hours at 35 °C and 150 RPM.

Acetate and formate consumption were measured for the FDH transformants, as well as for the M2390 and M2594 background strains, according to the assay described above. The results are shown in Table 10. Both the *S. cerevisiae* FDH1 and the C. *boidinii* FDH3 transformants demonstrated improved acetate consumption compared to the M2390 strain. The C. *boidinii* FDH3 transformants showed the highest acetate consumption, which may be in part due to expression of two copies of the gene or promoter/terminator selection. Thus, expression of a formate degrading enzyme such as FDH increases acetate consumption and ethanol production.

Table 10. Acetate uptake for strains overexpressing *S. cerevisiae* FDH1 or C. *boidinii* FDH3.

| | | Concentration (g/l) | | | Consumption (g/l) | |
|---|---|---|---|---|---|---|
| Background | Modification | Acetate | Ethanol | Formate | Acetate | Formate |
| M2390 | Wild-type | 2.57 | 18.9 | 1.62 | 0.10 | 0.17 |
| M2594 | gpd1::adhE gpd2::adhE | 2.35 | 19.7 | 1.61 | 0.32 | 0.18 |
| M4109 | gpd1::adhE gpd2::adhE fcy1::FDH1 | 2.35 | 19.8 | 1.57 | 0.32 | 0.21 |
| M4110 | gpd1::adhE gpd2::adhE fcy1::FDH1 | 2.30 | 19.5 | 1.57 | 0.37 | 0.22 |
| M4111 | gpd1::adhE gpd2::adhE fcy1::C.boidinii FDH3 | 2.21 | 20.0 | 1.35 | 0.46 | 0.44 |
| M4112 | gpd1::adhE gpd2::adhE fcy1::C.boidinii FDH3 | 2.19 | 19.7 | 1.32 | 0.48 | 0.47 |
| | Medium | 2.67 | | 1.79 | | |

Example 4

The present prophetic example describes engineering of a recombinant microorganism to use the dihydroxyacetone pathway (DHA) for production of electron donors to be used in the conversion of acetate to ethanol or isopropanol.

The DHA pathway is conceptually similar to the RuMP pathway of Example 3, as both rely on the formation of formaldehyde and the subsequent oxidation of the formaldehyde to $CO_2$, producing NADH. With the DHA pathway, formaldehyde is produced by the action of formaldehyde transketolase (EC 2.2.1.3), which interconverts dihydroxyacetone and glyceraldehyde-3-P into xylulose-5-P and formaldehyde. *See* Figure 6. The required dihydroxyacetone can be produced by either glycerol dehydrogenase or dihydroxyacetone phosphatase:

glycerol + NAD(P) $\rightarrow$ dihydroxyacetone + NAD(P)H (catalyzed by glycerol dehydrogenase)

or

dihydroxyacetone-P $\rightarrow$ dihydroxyacetone (catalyzed by dihydroxyacetone phosphatase)

dihydroxyacetone + glyceraldehyde-3-P $\rightarrow$ xylulose-5-P + formaldehyde (catalyzed by formaldehyde transketolase)

formaldehyde $\rightarrow$ $CO_2$ + 2 NADH (catalyzed by formaldehyde dehydrogenase, S-formylglutathione hydrolase, and formate dehydrogenase)

DHA degradation via formaldehyde transketolase has been described for *S. cerevisiae,* and baker's yeast has an endogenous glycerol dehydrogenase, encoded by GCY1. *See* Molin, M., and A. Blomberg, "Dihydroxyacetone detoxification in Saccharomyces cerevisiae involves formaldehyde dissimilation," Mol. Microbiol. 60:925-938 (2006) and Yu, K. O., et al., "Engineering of glycerol utilization pathway for ethanol production by Saccharomyces cerevisiae," Bioresource Technol. 101:4157-4161 (2010). Glycerol dehydrogenases from several organisms, including *Hansenula polymorpha* (*gdh*), *E. coli* (*gldA*) and *Pichia angusta* (*gdh*), have also been functionally expressed in *S. cerevisiae. See* Jung, J-Y., et al., "Production of 1,2-propanediol from glycerol in Saccharomyces cerevisiae," J. Microbiol. Biotechnol. 21:846-853 (2011) and Nguyen, H. T. T., and Nevoigt, E., "Engineering of Saccharomyces cerevisiae for the production of dihydroxyacetone (DHA) from sugars: A proof of concept," Metabolic Engineering 11:335-346 (2009). Dihydroxyacetone-P-specific phosphatase-activity has been found in the bacterium *Zymomonas mobilis. See* Horbach, S., et al., "Enzymes involved in the formation of glycerol 3-phosphate and the by-products dihydroxyacetone and glycerol in Zymomonas mobilis," FEMS Microbiology Letters 120:37-44 (1994).

To prevent conversion of dihydroxyacetone to dihydroxyacetone phosphate, expression of the *DAK1*/*DAK2* genes, which encode dihydroxyacetone kinases, can be downregulated For example, the *DAK1*/*DAK2* genes can be deleted. *See* Figures 20-22. Dihydroxyacetone kinases convert DHA to DHAP. In this pathway, NADH is generated via the conversion of glycerol, produced from DHAP, to $CO_2$ and xylulose-5-P. Rephosphorylating DHA would result in a futile cycle. If a glycerol dehydrogenase is used and the medium contains glycerol (either introduced by the feedstock or released by the cells), the *STL1*-encoded glycerol/proton-symporter can be overexpressed or upregulated to take up glycerol from the medium. A source of DHA is required for this pathway to function. Extracellular glycerol is an attractive source, although it might not be present in all media, and it may not be economical to add it. In the case where glycerol is present, expressing a transporter is likely to improve the capacity of the cell to take up glycerol, especially at lower glycerol concentrations. *See* International Patent Application Publication No WO2011/149353, which is incorporated by reference herein in its entirety.

The desired strain comprises overexpression of glycerol dehydrogenase and transketolase to convert glycerol to xylulose-5-P and formaldehyde, and overexpression of formaldehyde dehydrogenase and formate dehydrogenase to convert formaldehyde to $CO_2$. In addition, deletion of both dihydroxyacetone kinases (DAK1 and DAK2) is desired to prevent (re)phosphorylation of dihydroxyacetone. Further, the strain overexpresses an NADH-dependent acetaldehyde dehydrogenase, *e.g., Piromyces sp. E2 adhE,* to enable conversion of acetate to ethanol. *See* Figures 24 and 25.

This strain can be grown under anaerobic conditions in media containing C6 and/or C5 sugars, as well as acetate. The dihydroxyacetone (DHA) pathway, combined with formaldehyde degradation to $CO_2$, can generate NADH and improve the conversion of acetate to ethanol via an NADH-dependent acetaldehyde dehydrogenase.

The sequence for *O. polymorpha* Glycerol dehydrogenase is provided in SEQ ID NO:48. The sequence for *S. cerevisiae* Transketolase *TKL1* is provided in SEQ ID NO:18. The sequence for *O. polymorpha* Formaldehyde dehydrogenase *FLD1* is provided in SEQ ID NO:49. The sequence for *O. polymorpha* Formate dehydrogenase is provided in SEQ ID NO:50. The sequence for the *S. cerevisiae* dihydroxyacetone kinase *DAK1* is provided in SEQ ID NO:51. The sequence for the *S. cerevisiae* dihydroxyacetone kinase *DAK2* is provided in SEQ ID NO:52. The nucleotide sequence upstream of the *DAK1* gene used to create a *DAK1* clean deletion is provided in SEQ ID NO:53. The nucleotide sequence downstream of the *DAK1* gene used to create a *DAK1* clean deletion is provided in SEQ ID NO:54. The nucleotide sequence upstream of the *DAK2* gene used to create a *DAK2* clean deletion is provided in SEQ ID NO:55. The nucleotide sequence downstream of the *DAK2* gene used to create a *DAK2* clean deletion is provided in SEQ ID NO:56.

Example 5

The present example describes engineering of a recombinant microorganism to use a transhydrogenase for the production of electron donors to be used in the conversion of acetate to ethanol or isopropanol.

Transhydrogenases catalyze the interconversion of:

$$\text{NADPH} + \text{NAD} \rightleftarrows \text{NADP} + \text{NADH (equation 3)}$$

As the (cytosolic) NADPH/NADP ratio in *S. cerevisiae* is typically assumed to be higher than the NADH/NAD ratio, introduction of a transhydrogenase should create a flux towards NADH formation. Transhydrogenases from *Escherichia coli* (udhA) and *Azotobacter vinelandii* (sthA) have been successfully expressed in *S. cerevisiae,* and observed changes in the metabolic profiles (increased glycerol, acetate and 2-oxoglutarate production, decreased xylitol production) indeed pointed to a net conversion of NADPH into NADH. *See* Anderlund, M., et al., "Expression of the Escherichia coli pntA and pntB Genes, Encoding Nicotinamide Nucleotide Transhydrogenase, in Saccharomyces cerevisiae and Its Effect on Product Formation during Anaerobic Glucose Fermentation," Appl. Envirol Microbiol. 65:2333-2340 (1999); Heux, S., et al., "Glucose utilization of strains lacking PGI1 and expressing a transhydrogenase suggests differences in the pentose phosphate capacity among Saccharomyces cerevisiae strains," FEMS Yeast Research 8:217-224 (2008); Jeppsson, M., et al., "The level of glucose-6-phosphate dehydrogenase activity strongly influences xylose fermentation and inhibitor sensitivity in recombinant Saccharomyces cerevisiae strains," Yeast 20:1263-1272 (2003); Jeun, Y.-S., et al., "Expression of Azotobacter vinelandii soluble transhydrogenase perturbs xylose reductase-mediated conversion of xylose to xylitol by recombinant Saccharomyces cerevisiae," Journal of Molecular Catalysis B: Enzymatic 26:251-256 (2003); and Nissen, T.L., et al.," Expression of a cytoplasmic transhydrogenase in Saccharomyces cerevisiae results in formation of 2-oxoglutarate due to depletion of the NADPH pool," Yeast 18:19-32 (2001).

With this approach, additional NADH becomes available for acetate-to-ethanol conversion, and the consumed NADPH could be replenished by increasing the flux through the pentose phosphate pathway. The nucleotide sequence for *E. coli* udhA is provided as SEQ ID NO:59, and the amino acid sequence for *E. coli* udhA is provided as SEQ ID NO:60. The nucleotide sequence for codon-optimized *Azotobacter vinelandii* sthA is provided as SEQ ID NO:61, and the amino acid sequence for codon-optimized *Azotobacter vinelandii* sthA is provided as SEQ ID NO:62. A contstruct that can used to express *Azotobacter vinelandii* sthA is depicted in Figure 59.

The following example describes the engineering of a recombinant microorganism to increase acetate conversion to ethanol by overexpressing the transhydrogenase, *E. coli* udhA, in xylose utilizing strains. *E. coli* udhA was overexpressed in the engineered xylose utilizing strains M3799 and M4044. M4044 is a glycerol-reduction strain derived from M3799 and contains a gpd2 gene deletion with the integration of two copies of *B. adolescentis* adhE. Strains M4044 and M3799 are described in commonly owned International Appl. No. PCT/US2013/000090, which is hereby incorporated by reference in its entirety. Strains M3799 and M4044 were pre-marked with dominant (kanMX and Nat) and negative (fcy1) selection markers at the apt2 and YLR296W sites, respectively. Two copies of the udhA were introduced into the pre-marked strains using the 5FC counterselection previously described. *See* Figures 55 and 56. The udhA+ strains M7215 and M7216 were generated by insertion of MA905 (Figure 55) into the pre-marked M3799 strain. The udhA+ strains M4610 and M4611 were generated by insertion of MA483 (Figure 56) into the pre-marked glycerol-reduction background strain M4044.

To determine if the udhA transhydrogenase was capable of influencing the acetate-to-ethanol conversion in a glycerol reduction strain expressing the *B. adolescentis* adhE (Δgpd2::adhE-adhE), strain M4610 (Δgpd2::adhE ΔYLR296W::udhA) was compared to the parental strain M4044 (Δgpd2::adhE) in fermentation on a pre-treated agricultural waste (Figures 57A-C). Fermentations were performed at 33° C and 35° C and were buffered with $CaCO_3$. Cells were inoculated at 0.5 g/L. M4610 (Δgpd2::adhE ΔYLR296W::udhA) fermentation had -0.5 g/L less acetic acid compared to the parental strain M4044 (Δgpd2::adhE), at both 33° C and 35° C, indicating that the udhA strain M4610 was consuming more acetic acid than the parental strain M4044 (Figure 57B). In addition, the udhA+ strain M4610 had a faster fermentation rate compared to M4044. At 25.5 hours of fermentation the udhA+ strain M4610 had 10% higher ethanol titer than the parental strain M4044. At the end of this fermentation (48.5 hours) the background strain had reached similar ethanol titers as the udhA strain (Figure 57A). The glycerol production was also affected by the introduction of udhA. A non-glycerol reduction strain background run in this same fermentation was making ~2 g/L of glycerol at 33° C and -1.6 g/L at 35° C (data not shown). The glycerol reduction strain M4044 made 30% of the total glycerol made by the non-glycerol reduction strains (0.47 g/L). The udhA+ strain M4610 produced 2-fold more glycerol (~1 g/L) compared to M4044 (Figure 57C). Without wishing to be bound by any one theory, this data suggests that udhA drives acetate consumption, leads to increased rate of ethanol production, and an overall increase in glycerol production. This is consistent with the role of udhA in converting NADPH to NADH because NADH is required for glycerol production (these strains still have gpdl) and acetate-to-ethanol conversion.

The glycerol reduction udhA strains as well as the udhA+ strains in the non-glycerol-reduction M3799 background were tested for their fermentation performance on pre-treated corn stover, another commercially relevant substrate. The data from these experiments are depicted in Figures 58A-C. Fermentations were performed at 35° C for 70 hours in pressure bottles and were buffered with $CaCO_3$. Cells were inoculated at 0.5 g/L, and ethanol, acetic acid and glycerol levels were determined. The rate of ethanol production was increased for both the M3799 udhA+ strains, M7215 and M7216, as well as the udhA+ glycerol-reduction (Δgpd2::adhE ΔYLR296W::udhA) strains M4610 and M4611. At 22 hours the udhA+ strains M7215 and M7216 produced 4.5-6% more ethanol compared to the parental strain M3799 while the udhA+ glycerol reduction strains M4610 and M4611 had produced 56-60% more ethanol than the parental strain M4044 (Figure 58A). M4044, did not show any acetic acid consumption on this material, but addition of udhA led to consumption of 0.8-0.85 g/L of acetate for strains M4610 and M4611 (Figure 58B). While M7215 and M7216 did not show any acetate consumption as expected, they did show a slight increase (-0.4 g/L) in glycerol production compared to their parental strain M3799 (Figure 58C). The increase in glycerol production for the M3799 udhA strains and the increase in acetate consumption by the M4044 udhA strains on this material further suggest that udhA is functioning in these strains to convert NADPH to NADH.

These results suggest that the udhA is functioning in these strains to convert NADPH to NADH in both non-glycerol-reduction strains and in acetate-to-ethanol strains. The beneficial effect of a higher rate of ethanol production is likely attributable to an increased NADH availability for acetate-to-ethanol conversion (reducing the toxicity of acetate) and glycerol production (improving cell robustness). In addition, without being bound by an theory, consumption of NADPH by the transhydrogenase may benefit activity of xylose isomerase by reducing xylitol formation by any NADPH-dependent xylose reductases (because xylitol is a potent inhibitor of xylose isomerase).

Example 6

Conceptually similar to the introduction of a transhydrogenase is the creation of a NADPH/NADH-cycling reaction. The reaction catalyzed by the overexpression of NADP- and NAD-dependent glutamate dehydrogenases is close to equilibrium, resulting in some conversion back and forth between NADPH and NADH. As the cytosolic NADPH/NADP ratio is expected to be higher than the NADH/NAD ratio, the reverse glutamate-forming reaction will preferentially use NADPH, and the forward glutamate-consuming reaction will preferentially use NAD, resulting in a net conversion of NADPH and NAD to NADP and NADH, the same reaction catalyzed by transhydrogenase. One such cycle consists of the combination of cytosolic NAD-specific and NADP-specific glutamate dehydrogenases (GDH), which catalyze the reversible reaction:

$$\text{L-glutamate} + H_2O + NAD(P)^+ \rightleftarrows \text{2-oxoglutarate} + NH_3 + NAD(P)H + H^+$$

Overexpressing the native NAD-GDH encoded by *GDH2* (SEQ ID NO: 1) has been shown to rescue growth in a phosphoglucose isomerase *pgil S. cerevisiae* deletion mutant, but only as long as glucose-6-phosphate dehydrogenase and the NADP-GDH encoded by *GDH1* were left intact. *See* Boles, E., et al., "The role of the NAD-dependent glutamate dehydrogenase in restoring growth on glucose of a Saccharomyces cerevisiae phosphoglucose isomerase mutant," European Journal of Biochemistry 217:469-477 (1993). This strongly suggests that the increased NADPH production, the result of redirection of glucose into the pentose phosphate pathway, which normally proves fatal, could be balanced by conversion of NADPH to NADH by this GDH-cycle, with the produced NADH being reoxidized via respiration.

As with transhydrogenase, when the cytosolic NADPH/NADP ratio is higher than the NADH/NAD ratio, introducing a GDH-cycling reaction would generate additional NADH at the expense of NADPH. The latter can then again be replenished by an increased flux through the pentose phosphate pathway.

GDH2 is overexpressed in a strain overexpressing an NADH-dependent acetaldehyde dehydrogenase. Competition with glycerol formation (another NADH-consuming reaction) is prevented by deleting *gpd1* and *gpd2.* In one embodiment of the invention, *adhE* from *Bifidobacterium adolescentis* is integrated into the *gpd1* and *gpd2* loci, resulting in deletion of *gpd1* and *gpd2. See* Figures 7-10.

This strain is grown under anaerobic conditions in media containing C6 and/or C5 sugars, as well as acetate. The strain may generate more NADH under these conditions than a strain which does not overexpress GDH2 (due to a net transfer of electrons from NADPH to NADH), allowing for improved conversion of acetate to ethanol via the NADH-dependent acetaldehyde dehydrogenase.

Following are particular embodiments of the disclosed invention.

E1. A recombinant microorganism comprising: a) one or more native and/or heterologous enzymes that function in one or more first engineered metabolic pathways to convert acetate to an alcohol, wherein said one or more native and/or heterologous enzymes is activated, upregulated or downregulated; and b) one or more native and/or heterologous enzymes that function in one or more second engineered metabolic pathways to produce an electron donor used in the conversion of acetate to an alcohol, wherein said one or more native and/or heterologous enzymes is activated, upregulated or downregulated. E2. The recombinant microorganism of E1, wherein said acetate is produced as a by-product of biomass processing. E3. The recombinant microorganism of E1 or E2, wherein said alcohol is selected from the group consisting of ethanol, isopropanol, or a combination thereof. E4. The recombinant microorganism of any of E1-E3, wherein said electron donor is selected from the group consisting of NADH, NADPH, or a combination thereof. E5. The recombinant microorganism of any one of E1-E4, wherein said one or more second engineered metabolic pathways to produce an electron donor is a xylose fermentation pathway. E6. The recombinant microorganism of E5, wherein said engineered xylose fermentation pathway comprises upregulation of the native and/or heterologous enzymes xylose reductase (XR) and xylitol dehydrogenase (XDH). E7. The recombinant microorganism of E6, wherein said native and/or heterologous XDH enzyme is from *Scheffersomyces stipitis.* E8. The recombinant microorganism of E7, wherein said XDH enzyme is encoded by a *xyl2* polynucleotide. E9. The recombinant microorganism of E6, wherein said native and/or heterologous XR enzyme is from *Scheffersomyces stipitis, Neurospora crassa,* or *Candida boidinii.*

E10. The recombinant microorganism of E9, wherein said XR enzyme is encoded by a *xyl1* polynucleotide or an aldolase reductase. E11. The recombinant microorganism of any one of E1-E10, wherein said first and second engineered metabolic pathways result in ATP production. E12. The recombinant microorganism of any one of E1-E10, wherein said one or more first engineered metabolic pathways comprises activating or upregulating one or more heterologous enzymes selected from the group consisting of acetyl-CoA acetyltransferase (thiolase), acetoacetyl-CoA transferase, acetoacetate decarboxylase, a secondary alcohol dehydrogenase, and combinations thereof. E13. The recombinant microorganism of any one of E1-E10, wherein one or more first engineered metabolic pathways comprises activating or upregulating a heterologous ADP-producing acetyl-CoA synthase enzyme. E14. The recombinant microorganism of any one of E1-E10, wherein one or more first engineered metabolic pathways comprises activating or upregulating the acetate kinase/phosphotransacetylase (AK/PTA) couple. E15. The recombinant microorganism of any one of E13 and E14, wherein said first and second engineered metabolic pathways result in ATP production. E16. The recombinant microorganism of any one of E1-E4, wherein said one or more second engineered metabolic pathways to produce an electron donor is the oxidative branch of the pentose phosphate pathway (PPP). E17. The recombinant microorganism of E16, wherein said engineered PPP comprises activation or upregulation of the native enzyme glucose-6-P dehydrogenase. E18. The recombinant microorganism of E17, wherein said native glucose-6-P dehydrogenase enzyme is from *Saccharomyces cerevisiae.* E19. The recombinant microorganism of E18, wherein said glucose-6-P dehydrogenase is encoded by a *zwf1* polynucleotide. E20. The recombinant microorganism of E1-E4, further comprising altering the expression of transcription factors that regulate expression of enzymes of the PPP pathway. E21. The recombinant microorganism of E20, wherein the transcription factor is Stb5p. E22. The recombinant microorganisms of E21, wherein the Stb5p is from *Saccharomyces cerevisiae.* E23. The recombinant microorganism of any one of E1-E4, wherein said one or more second engineered metabolic pathways to produce an electron donor is a pathway that competes with the oxidative branch of the PPP. E24. The recombinant microorganism of E23, wherein said engineered pathway that competes with the oxidative branch of the PPP comprises downregulation of the native enzyme glucose-6-P isomerase. E25. The recombinant microorganism of E24, wherein said native glucose-6-P isomerase enzyme is from *Saccharomyces cerevisiae.* E26. The recombinant microorganism of E25, wherein said glucose-6-P isomerase is encoded by a *pgi1* polynucleotide. E27. The recombinant microorganism of any one of E1-E4, wherein said one or more second engineered metabolic pathways to produce an electron donor comprises the ribulose-monophosphate pathway (RuMP). E28. The recombinant microorganism of E27, wherein said engineered RuMP pathway converts fructose-6-P to ribulose-5-P and formaldehyde. E29. The recombinant microorganism of E28, wherein said engineered RuMP pathway comprises upregulating a heterologous enzyme selected from the group consisting of 6-phospho-3-hexuloisomerase, 3-hexulose-6-phosphate synthase, and the combination thereof.

E30. The recombinant microorganism of any one of E27-E29, wherein said one or more second engineered metabolic pathways to produce an electron donor comprises upregulating native enzymes that degrade formaldehyde or formate. E31. The recombinant microorganism of E30, wherein the formaldehyde degrading enzymes convert formaldehyde to formate. E32. The recombinant microorganism of E31, wherein the formaldehyde degrading enzymes are formaldehyde dehydrogenase and S-formylglutathione hydrolase. E33. The recombinant microorganism of any of E30-E32, wherein the formate degrading enzyme converts formate to $CO_2$. E34. The recombinant microorganism of E33, wherein the formate degrading enzyme is formate dehydrogenase. E35. The recombinant microorganism of any one of E27-E34, wherein said one or more native and/or heterologous enzymes is from *Mycobacterium gastri.*

E36. The recombinant microorganism of any one of E1-E4, wherein said one or more second engineered metabolic pathways to produce an electron donor comprises the dihydroxyacetone (DHA) pathway. E37. The recombinant microorganism of E36, wherein said engineered DHA pathway interconverts dihydroxyacetone and glyceraldehyde-3-P into xylose-5-P and formaldehyde. E38. The recombinant microorganism of E37, wherein said engineered DHA pathway comprises upregulating the heterologous enzyme formaldehyde transketolase (EC 2.2.1.3). E39. The recombinant microorganism of any one of E36-E38, wherein said one or more second engineered metabolic pathways to produce an electron donor comprises upregulating native and/or heterologous enzymes that produce dihydroxyacetone.

E40. The recombinant microorganism of E39, wherein said native and/or heterologous enzymes that produce dihydroxyacetone are selected from the group consisting of glycerol dehydrogenase, dihydroxyacetone phosphatase, and a combination thereof. E41. The recombinant microorganism of E40, wherein said native and/or heterologous glycerol dehydrogenase is from a microorganism selected from the group consisting of *Hansenula polymorpha, E. coli, Pichia angusta,* and *Saccharomyces cerevisiae.* E42. The recombinant microorganism of E41, wherein said glycerol dehydrogenase is encoded by a polynucleotide selected from the group consisting of *gdh, gldA,* and *gcy1.*

E43. The recombinant microorganism of any one of E37-E42, wherein said formaldehyde is oxidized to form $CO_2$.

E44. The recombinant microorganism of any one of E39-E43, wherein said one or more second engineered metabolic pathways to produce an electron donor comprises downregulating a native dihydroxyacetone kinase enzyme. E45. The recombinant microorganism of E44, wherein the dihydroxyacetone kinase is encoded by a polynucleotide selected from the group consisting of *dak1, dak2,* and a combination thereof. E46. The recombinant microorganism of any one of E39-E45, wherein said microorganism further comprises overexpression of a glycerol/proton-symporter.

E47. The recombinant microorganism of E46, wherein said glycerol/proton-symporter is encoded by a *stl1* polynucleotide. E48. The recombinant microorganism of any one of E1-E47, wherein said microorganism further comprises overexpression of a native and/or heterologous transhydrogenase enzyme. E49. The recombinant microorganism of E48, wherein said transhydrogenase catalyzes the interconversion of NADPH and NAD to NADP and NADH.

E50. The recombinant microorganism of any one of E48 and E49, wherein said transhydrogenase is from a microorganism selected from the group consisting of *Escherichia coli* and *Azotobacter vinelandii.* E51. The recombinant microorganism of any one of E1-E47, wherein said microorganism further comprises overexpression of a native and/or heterologous glutamate dehydrogenase enzyme. E52. The recombinant microorganism of E51, wherein said glutamate dehydrogenase is encoded by a *gdh2* polynucleotide. E53. The recombinant microorganism of any one of E1-E52, wherein one of said engineered metabolic pathways comprises the following steps: (a) conversion of acetate to acetyl-CoA and (b) conversion of acetyl-CoA to ethanol. E54. The recombinant microorganism of any one of E1-E52, wherein said one or more downregulated native enzymes is encoded by a *gpd1* polynucleotide, a *gpd2* polynucleotide, or both a *gpd1* polynucleotide and a *gpd2* polynucleotide. E55. The recombinant microorganism of any one of E1-E54, wherein said microorganism produces ethanol. E56. The recombinant microorganism of any one of E1-E55, wherein said microorganism is selected from the group consisting of *Saccharomyces cerevisiae, Kluyveromyces lactis, Kluyveromyces marxianus, Pichia pastoris, Yarrowia lipolytica, Hansenula polymorpha, Phaffia rhodozyma, Candida utilis, Arxula adeninivorans, Pichia stipitis, Debaryomyces hansenii, Debaryomyces polymorphus, Schizosaccharomyces pombe, Candida albicans,* and *Schwanniomycesoccidentalis.* E57. The recombinant microorganism of E56, wherein said microorganism is *Saccharomyces cerevisiae.* E58. The recombinant microorganism of any one of E1-E57, wherein said acetate is converted to acetyl-CoA by an acetyl-CoA transferase (ACS). E59. The recombinant microorganism of any one of E1-E57, wherein said acetate is converted to acetyl-P by an acetate kinase; and wherein said acetyl-P is converted to acetyl-CoA by a phosphotransacetylase.

E60. The recombinant microorganism of E59, wherein said acetate kinase and said phosphotransacetylase are from one or more of an *Escherichia,* a *Thermoanaerobacter,* a *Clostridia,* or a *Bacillus* species. E61. The recombinant microorganism of any one of E1-E60, wherein said acetyl-CoA is converted to acetaldehyde by an acetaldehyde dehydrogenase; and wherein said acetaldehyde is converted to ethanol by an alcohol dehydrogenase. E62. The recombinant microorganism of E61, wherein said acetaldehyde dehydrogenase is an NADPH-specific acetaldehyde dehydrogenase. E63. The recombinant microorganism of E62, wherein said NADPH-specific acetaldehyde dehydrogenase is from *T. pseudethanolicus.* E64. The recombinant microorganism of E63, wherein said NADPH-specific acetaldehyde dehydrogenase is *T. pseudethanolicus* adhB. E65. The recombinant microorganism of E61, wherein said alcohol dehydrogenase is an NADPH-specific alcohol dehydrogenase. E66. The recombinant microorganism of E65, wherein said NADPH-specific alcohol dehydrogenase is from a microorganism selected from the group consisting of *T. pseudethanolicus, C. beijerinckii, Entamoeba histolytica, Cucumis melo,* and *S. cerevisiae.* E67. The recombinant microorganism of E66, wherein said NADPH-specific alcohol dehydrogenase is *T. pseudethanolicus* adhB. E68. The recombinant microorganism of E66, wherein said NADPH-specific alcohol dehydrogenase is C. *beijerinckii* 2° Adh. E69. The recombinant microorganism of E66, wherein said NADPH-specific alcohol dehydrogenase is *S. cerevisiae* ARI1.

E70. The recombinant microorganism of E66, wherein said NADPH-specific alcohol dehydrogenase is *Entamoeba histolytica* ADH1. E71. The recombinant microorganism of E66, wherein said NADPH-specific alcohol dehydrogenase is *Cucumis melo* ADH1. E72. The recombinant microorganism of any one of E1-E71, wherein said acetyl-CoA is converted to ethanol by a bifunctional acetaldehyde/alcohol dehydrogenase. E73. The recombinant microorganism of any one of E58 or E61-E72, wherein said acetyl-CoA transferase (ACS) is encoded by an ACS1 polynucleotide. E74. The recombinant microorganism of E61, wherein said acetaldehyde dehydrogenase is from *C. phytofermentans.* E75. The recombinant microorganism of E72, wherein said bifunctional acetaldehyde/alcohol dehydrogenase is from *E. coli, C. acetobutylicum, T. saccharolyticum, C. thermocellum,* or *C. phytofermentans.* E76. A recombinant microorganism comprising a) one or more native and/or heterologous enzymes that function in one or more engineered metabolic pathways to convert acetate to acetone, wherein said one or more native and/or heterologous enzymes is activated, upregulated or downregulated; and b) one or more native and/or heterologous enzymes that function in one or more second engineered metabolic pathways to produce an electron donor used in the conversion of acetate to isopropanol, wherein said one or more native and/or heterologous enzymes is activated, upregulated or downregulated. E77. The recombinant organism of E76, wherein said acetate is produced as a by-product of biomass processing. E78 The recombinant microorganism of E76 or E77, wherein one of said engineered metabolic pathways comprises the following steps: (a) conversion of acetate to acetyl-CoA; (b) conversion of acetyl-CoA to acetoacetyl-CoA; (c) conversion of acetoacetyl-CoA to acetoacetate; (d) conversion of acetoacetate to acetone; and (e) conversion of acetone to isopropanol. E79. The recombinant microorganism of any one of E76-E78, wherein said microorganism produces isopropanol.

E80. The recombinant microorganism of any one of E76-E79, wherein said microorganism is *Escherichia coli.* E81. The recombinant microorganism of any one of E76-E79, wherein said microorganism is a thermophilic or mesophilic bacterium. E82. The recombinant microorganism of E81, wherein said thermophilic or mesophilic bacterium is a species of the genera Thermoanaerobacterium, Thermoanaerobacter, Clostridium, Geobacillus, Saccharococcus, Paenibacillus, Bacillus, Caldicellulosiruptor, Anaerocellum, or Anoxybacillus. E83. The recombinant microorganism of E82, wherein said microorganism is a bacterium selected from the group consisting of: *Thermoanaerobacteriumthermosulfurigenes, Thermoanaerobacteriumaotearoense, Thermoanaerobacteriumpolysaccharolyticum, Thermoanaerobacteriumzeae, Thermoanaerobacteriumxylanolyticum, Thermoanaerobacterium saccharolyticum, Thermoanaerobiumbrockii, Thermoanaerobacteriumthermosaccharolyticum, Thermoanaerobacter thermohydrosulfuricus, Thermoanaerobacterethanolicus, Thermoanaerobacterbrocki, Clostridium thermocellum, Clostridium cellulolyticum, Clostridium phytofermentans, Clostridium straminosolvens, Geobacillus thermoglucosidasius, Geobacillus stearothermophilus, Saccharococcus caldoxylosilyticus, Saccharoccus thermophilus, Paenibacillus campinasensis, Bacillus flavothermus, Anoxybacillus kamchatkensis, Anoxybacillus gonensis, Caldicellulosiruptor acetigenus, Caldicellulosiruptor saccharolyticus, Caldicellulosiruptor kristjanssonii, Caldicellulosiruptor owensensis, Caldicellulosiruptor lactoaceticus,* and *Anaerocellumthermophilum.* E84. The recombinant microorganism of E83, wherein said microorganism is selected from the group consisting of *Clostridium thermocellum* and *Thermoanaerobacterium saccharolyticum.* E85. The recombinant microorganism of any one of E76-E79, wherein said microorganism is selected from the group consisting of *Saccharomyces cerevisiae, Kluyveromyces lactis, Kluyveromyces marxianus, Pichiapastoris, Yarrowia lipolytica, Hansenula polymorpha, Phaffia rhodozyma, Candida utilis, Arxula adeninivorans, Pichia stipitis, Debaryomyces hansenii, Debaryomyces polymorphus, Schizosaccharomyces pombe, Candida albicans,* and *Schwanniomycesoccidentalis.* E86. The recombinant microorganism of E85, wherein said microorganism is *Saccharomyces cerevisiae.* E87. The recombinant microorganism of any one of E76-E86, wherein said acetate is converted to acetyl-CoA by an acetyl-CoA synthetase. E88. The recombinant microorganism of any one of E76-E86, wherein said acetate is converted to acetyl-P by an acetate kinase; and wherein said acetyl-P is converted to acetyl-CoA by a phosphotransacetylase. E89. The recombinant microorganism of any one of E76-E88, wherein said acetyl-CoA is converted to acetoacetyl-CoA by a thiolase.

E90. The recombinant microorganism of any one of E76-E89, wherein said acetoacetyl-CoA is converted to acetoacetate by a CoA transferase. E91. The recombinant microorganism of any one of E76-E90, wherein said acetoacetate is converted to acetone by an acetoacetate decarboxylase. E92. The recombinant microorganism of E87, wherein said acetyl-CoA synthetase is encoded by a polynucleotide selected from the group consisting of a yeast ACS1 polynucleotide and a yeast ACS2 polynucleotide. E93. The recombinant microorganism of E92, wherein said yeast ACS1 polynucleotide is from *Saccharomyces cerevisiae* or *Saccharomyces kluyveri.* E94. The recombinant microorganism of E92, wherein said yeast ACS2 polynucleotide is from *Saccharomyces cerevisiae* or *Saccharomyces kluyveri.* E95. The recombinant microorganism of E88, wherein said acetate kinase and said phosphotransacetylase are from *T. saccharolyticum.* E96. The recombinant microorganism of any one of E89-E91, wherein said thiolase, said CoA transferase, and said acetoacetate decarboxylase are from C. *acetobutylicum.* E97. The recombinant microorganism of E89, wherein said thiolase is from *C. acetobutylicum* or *T. thermosaccharolyticum.* E98. The recombinant microorganism of E90, wherein said CoA transferase is from a bacterial source. E99. The recombinant microorganism of E98, wherein said bacterial source is selected from the group consisting of *Thermoanaerobacter tengcongensis, Thermoanaerbacterium thermosaccharolyticum, Thermosipho africanus,* and *Paenibacillus macerans.*

E100. The recombinant microorganism of E91, wherein said acetoacetate decarboxylase is from a bacterial source. E101. The recombinant microorganism of E100, wherein said bacterial source is selected from the group consisting of *C. acetobutylicum, Paenibacillus macerans, Acidothermus cellulolyticus, Bacillus amyloliquefaciens,* and *Rubrobacter xylanophilus.* E102. The recombinant microorganism of any one of E1-E54 and E76-E101, wherein one of said engineered metabolic pathways comprises the following steps: (a) conversion of acetate to acetyl-CoA; (b) conversion of acetyl-CoA to acetoacetyl-CoA; (c) conversion of acetoacetyl-CoA to acetoacetate; (d) conversion of acetoacetate to acetone; and (e) conversion of acetone to isopropanol. E103. The recombinant microorganism of E102, wherein said microorganism is selected from the group consisting of Saccharomyces cerevisiae, Kluyveromyces lactis, Kluyveromyces marxianus, Pichia pastoris, Yarrowia lipolytica, Hansenula polymorpha, Phaffia rhodozyma, Candida utilis, Arxula adeninivorans, Pichia stipitis, Debaryomyces hansenii, Debaryomyces polymorphus, Schizosaccharomyces pombe, Candida albicans, and Schwanniomycesoccidentalis. E104. The recombinant microorganism of E103, wherein said microorganism is *Saccharomyces cerevisiae.* E105. The recombinant microorganism of any one of E102-E104, wherein said acetate is converted to acetyl-CoA by an acetyl-CoA synthetase. E106. The recombinant microorganism of any one of E102-E105, wherein said acetyl-CoA is converted to acetoacetyl-CoA by a thiolase. E107. The recombinant microorganism of any one of E102-E106, wherein said acetoacetyl-CoA is converted to acetoacetate by a CoA transferase. E108. The recombinant microorganism of any one of E102-E107, wherein said acetoacetate is converted to acetone by an acetoacetate decarboxylase. E109. The recombinant microorganism of any one of E102-E108, wherein said acetone is converted to isopropanol by an alcohol dehydrogenase.

E110. The recombinant microorganism of E105, wherein said acetyl-CoA synthetase is encoded by a polynucleotide selected from the group consisting of a yeast ACS1 polynucleotide and a yeast ACS2 polynucleotide. E111. The recombinant microorganism of E107, wherein said CoA transferase is from a bacterial source. E112. The recombinant microorganism of E108, wherein said acetoacetate decarboxylase is from a bacterial source. E113. A process for converting biomass to ethanol, acetone, or isopropanol comprising contacting biomass with a recombinant microorganism according to any one of E1-E112. E114. The process of E113, wherein said biomass comprises lignocellulosic biomass. E115. The process of E114, wherein said lignocellulosic biomass is selected from the group consisting of grass, switch grass, cord grass, rye grass, reed canary grass, mixed prairie grass, miscanthus, sugar-processing residues, sugarcane bagasse, sugarcane straw, agricultural wastes, rice straw, rice hulls, barley straw, corn cobs, cereal straw, wheat straw, canola straw, oat straw, oat hulls, corn fiber, stover, soybean stover, corn stover, forestry wastes, recycled wood pulp fiber, paper sludge, sawdust, hardwood, softwood, agave, and combinations thereof. E116. The process of E115, wherein said process reduces or removes acetate from the consolidated bioprocessing (CBP) media. E117. The process of any one of E114-E116, wherein said reduction or removal of acetate occurs during fermentation. E118. An engineered metabolic pathway for reducing or removing acetate from consolidated bioprocessing (CBP) media according to any one of E1-E112. E119. The recombinant microorganism of any one of E27-E29, wherein said one or more second engineered metabolic pathways to produce an electron donor comprises upregulating an enzyme that degrades formate.

E120. The recombinant microorganism of E119, wherein the formate degrading enzyme converts formate to $CO_2$. E121. The recombinant microorganism of E120, wherein the formate degrading enzyme is formate dehydrogenase. E122. The recombinant microorganism of E121, wherein the formate dehydrogenase is from a yeast microorganism.

E123. The recombinant microorganism of E122, wherein the yeast microorganism is *S. cerevisiae* or *Candida boidinii.* E124. The recombinant microorganism of E123, wherein the formate dehydrogenase from *S. cerevisiae* is FDH1. E125. The recombinant microorganism of E123, wherein the formate dehydrogenase from *Candida boidinii* is FDH3. E126. The recombinant microorganism of any one of E119-E125, wherein said microorganism consumes or uses more acetate than a microorganism not comprising said enzyme that degrades formate. E127. The recombinant microorganism of E126, wherein said recombinant microorganism has an acetate uptake (g/L) under anaerobic conditions selected from: (a) at least about 1.0 fold more acetate uptake than that taken up by a recombinant microorganism not comprising said enzyme that degrades formate; (b) at least about 1.1 fold more acetate uptake than that taken up by a recombinant microorganism not comprising said enzyme that degrades formate; (c) at least about 1.2 fold more acetate uptake than that taken up by a recombinant microorganism not comprising said enzyme that degrades formate; (d) at least about 1.3 fold more acetate uptake than that taken up by a recombinant microorganism not comprising said enzyme that degrades formate; (e) at least about 1.4 fold more acetate uptake than that taken up by a recombinant microorganism not comprising said enzyme that degrades formate; (f) at least about 1.5 fold more acetate uptake than that taken up by a recombinant microorganism not comprising said enzyme that degrades formate; (g) at least about 2.0 fold more acetate uptake than that taken up by a recombinant microorganism not comprising said enzyme that degrades formate; (h) at least about 2.5 fold more acetate uptake than that taken up by a recombinant microorganism not comprising said enzyme that degrades formate; (i) at least about 3.0 fold more acetate uptake than that taken up by a recombinant microorganism not comprising said enzyme that degrades formate; (j) at least about 4.0 fold more acetate uptake than that taken up by a recombinant microorganism not comprising said enzyme that degrades formate; (k) at least about 5.0 fold more acetate uptake than that taken up by a recombinant microorganism not comprising said enzyme that degrades formate; or (1) at least about 10 fold more acetate uptake than that taken up by a recombinant microorganism not comprising said enzyme that degrades formate. E128. The recombinant microorganism of E126, wherein said recombinant microorganism has an acetate uptake under anaerobic conditions selected from at least about 0.32 g/L, at least about 0.37 g/L, at least about 0.46 g/L, or at least about 0.48 g/L. E129. The recombinant microorganism of any one of E65-E71, wherein said microorganism consumes or uses more acetate than a microorganism not comprising said NADPH-specific alcohol dehydrogenase.

E130. The recombinant microorganism of E129, wherein said recombinant microorganism has an acetate uptake (g/L) under anaerobic conditions selected from: (a) at least about 1.1 fold more acetate uptake than that taken up by a recombinant microorganism not comprising said NADPH-specific alcohol dehydrogenase; (b) at least about 1.2 fold more acetate uptake than that taken up by a recombinant microorganism not comprising said NADPH-specific alcohol dehydrogenase; (c) at least about 1.3 fold more acetate uptake than that taken up by a recombinant microorganism not comprising said NADPH-specific alcohol dehydrogenase; (d) at least about 1.4 fold more acetate uptake than that taken up by a recombinant microorganism not comprising said NADPH-specific alcohol dehydrogenase; (e) at least about 1.5 fold more acetate uptake than that taken up by a recombinant microorganism not comprising said NADPH-specific alcohol dehydrogenase; (f) at least about 1.6 fold more acetate uptake than that taken up by a recombinant microorganism not comprising said NADPH-specific alcohol dehydrogenase; (g) at least about 1.9 fold more acetate uptake than that taken up by a recombinant microorganism not comprising said NADPH-specific alcohol dehydrogenase; (h) at least about 2.0 fold more acetate uptake than that taken up by a recombinant microorganism not comprising said NADPH-specific alcohol dehydrogenase; (i) at least about 2.1 fold more acetate uptake than that taken up by a recombinant microorganism not comprising said NADPH-specific alcohol dehydrogenase; (j) at least about 2.3 fold more acetate uptake than that taken up by a recombinant microorganism not comprising said NADPH-specific alcohol dehydrogenase; (k) at least about 2.4 fold more acetate uptake than that taken up by a recombinant microorganism not comprising said NADPH-specific alcohol dehydrogenase; (1) at least about 2.5 fold more acetate uptake than that taken up by a recombinant microorganism not comprising said NADPH-specific alcohol dehydrogenase; (m) at least about 2.7 fold more acetate uptake than that taken up by a recombinant microorganism not comprising said NADPH-specific alcohol dehydrogenase; (n) at least about 2.8 fold more acetate uptake than that taken up by a recombinant microorganism not comprising said NADPH-specific alcohol dehydrogenase; (o) at least about 2.9 fold more acetate uptake than that taken up by a recombinant microorganism not comprising said NADPH-specific alcohol dehydrogenase; or (p) at least about 3.1 fold more acetate uptake than that taken up by a recombinant microorganism not comprising said NADPH-specific alcohol dehydrogenase. E131. The recombinant microorganism of E129, wherein said recombinant microorganism has an acetate uptake under anaerobic conditions selected from at least about 0.35 g/L, at least about 0.36 g/L, at least about 0.38 g/L, at least about 0.40 g/L, at least about 0.44 g/L, at least about 0.45 g/L, at least about 0.47 g/L, at least about 0.48 g/L, at least about 0.51 g/L, at least about 0.53 g/L, at least about 0.59 g/L, at least about 0.61 g/L, at least about 0.63 g/L, at least about 0.65 g/L, at least about 0.66 g/L, at least about 0.70 g/L, at least about 0.79 g/L, at least about 0.8 g/L, at least about 0.83 g/l, at least about 0.84 g/L, at least about 0.87 g/L, at least about 0.9 g/L, at least about 0.91 g/L, at least about 0.96 g/L, at least about 0.99 g/L, at least about 1.00 g/L, at least about 1.01 g/L at least about 1.02 g/L, at least about 1.18 g/L, at least about 1.20 g/L, at least about 1.23 g/L, at least about 3.2 g/L, or at least about 3.3 g/L. E132. The recombinant microorganism of E129, wherein said recombinant microorganism has an acetate uptake under anaerobic conditions from about 0.35 g/L to about 3.3 g/L. E133. A recombinant microorganism comprising: a) one or more native and/or heterologous enzymes that function in one or more first engineered metabolic pathways to convert acetate to an alcohol, wherein said one or more native and/or heterologous enzymes is activated, upregulated or downregulated; and b) one or more native and/or heterologous *zwf1* polynucleotides; wherein one or more native and/or heterologous enzymes is an NADPH-specific alcohol dehydrogenase. E134. The recombinant microorganism of E133, wherein said NADPH-specific alcohol dehydrogenase is from a microorganism selected from the group consisting of *T. pseudethanolicus, C. beijerinckii, Entamoeba histolytica, Cucumis melo,* and *S. cerevisiae.* E135. The recombinant microorganism of E133, wherein said NADPH-specific alcohol dehydrogenase is *T. pseudethanolicus* adhB. E136. The recombinant microorganism of E133, wherein said NADPH-specific alcohol dehydrogenase is **C.** *beijerinckii* 2° Adh. E137. The recombinant microorganism of E133, wherein said NADPH-specific alcohol dehydrogenase is *S. cerevisiae* ARI1. E138. The recombinant microorganism of E133, wherein said NADPH-specific alcohol dehydrogenase is *Entamoeba histolytica* ADH1. E139. The recombinant microorganism of E133, wherein said NADPH-specific alcohol dehydrogenase is *Cucumis melo* ADH1.

E140. The recombinant microorganism of any one of E133-E139, wherein said one or more native enzymes that function in one or more first engineered metabolic pathways to convert acetate to an alcohol is an NADH-specific alcohol dehydrogenase. E141. The recombinant microorganism of any one of E133-E140, wherein said NADH-specific alcohol dehydrogenase is downregulated. E142. The recombinant microorganism of any one of E133-E141, wherein said NADH-specific alcohol dehydrogenase is selected from ADH1, ADH2, ADH3, ADH4, ADH5, or SFA1 from *Saccharomyces.* E143. The recombinant microorganism of any one of E133-E142, wherein said microorganism consumes or uses more acetate than a microorganism not comprising said NADPH-specific alcohol dehydrogenase. E144. The recombinant microorganism of E143, wherein said recombinant microorganism has an acetate uptake (g/L) under anaerobic conditions selected from: (a) at least about 1.1 fold more acetate uptake than that taken up by a recombinant microorganism not comprising said NADPH-specific alcohol dehydrogenase; (b) at least about 1.2 fold more acetate uptake than that taken up by a recombinant microorganism not comprising said NADPH-specific alcohol dehydrogenase; (c) at least about 1.3 fold more acetate uptake than that taken up by a recombinant microorganism not comprising said NADPH-specific alcohol dehydrogenase; (d) at least about 1.4 fold more acetate uptake than that taken up by a recombinant microorganism not comprising said NADPH-specific alcohol dehydrogenase; (e) at least about 1.5 fold more acetate uptake than that taken up by a recombinant microorganism not comprising said NADPH-specific alcohol dehydrogenase; (f) at least about 1.6 fold more acetate uptake than that taken up by a recombinant microorganism not comprising said NADPH-specific alcohol dehydrogenase; (g) at least about 1.9 fold more acetate uptake than that taken up by a recombinant microorganism not comprising said NADPH-specific alcohol dehydrogenase; (h) at least about 2.0 fold more acetate uptake than that taken up by a recombinant microorganism not comprising said NADPH-specific alcohol dehydrogenase; (i) at least about 2.1 fold more acetate uptake than that taken up by a recombinant microorganism not comprising said NADPH-specific alcohol dehydrogenase; (j) at least about 2.3 fold more acetate uptake than that taken up by a recombinant microorganism not comprising said NADPH-specific alcohol dehydrogenase; (k) at least about 2.4 fold more acetate uptake than that taken up by a recombinant microorganism not comprising said NADPH-specific alcohol dehydrogenase; (1) at least about 2.5 fold more acetate uptake than that taken up by a recombinant microorganism not comprising said NADPH-specific alcohol dehydrogenase; (m) at least about 2.7 fold more acetate uptake than that taken up by a recombinant microorganism not comprising said NADPH-specific alcohol dehydrogenase; (n) at least about 2.8 fold more acetate uptake than that taken up by a recombinant microorganism not comprising said NADPH-specific alcohol dehydrogenase; (o) at least about 2.9 fold more acetate uptake than that taken up by a recombinant microorganism not comprising said NADPH-specific alcohol dehydrogenase; or (p) at least about 3.1 fold more acetate uptake than that taken up by a recombinant microorganism not comprising said NADPH-specific alcohol dehydrogenase. E145. The recombinant microorganism of any one of E133-E143, wherein said recombinant microorganism has an acetate uptake under anaerobic conditions selected from at least about 0.35 g/L, at least about 0.36 g/L, at least about 0.38 g/L, at least about 0.40 g/L, at least about 0.44 g/L, at least about 0.45 g/L, at least about 0.47 g/L, at least about 0.48 g/L, at least about 0.51 g/L, at least about 0.53 g/L, at least about 0.59 g/L, at least about 0.61 g/L, at least about 0.63 g/L, at least about 0.65 g/L, at least about 0.66 g/L, at least about 0.70 g/L, at least about 0.79 g/L, at least about 0.8 g/L, at least about 0.83 g/l, at least about 0.84 g/L, at least about 0.87 g/L, at least about 0.9 g/L, at least about 0.91 g/L, at least about 0.96 g/L, at least about 0.99 g/L, at least about 1.00 g/L, at least about 1.01 g/L at least about 1.02 g/L, at least about 1.18 g/L, at least about 1.20 g/L, at least about 1.23 g/L, at least about 3.2 g/L, or at least about 3.3 g/L. E146. The recombinant microorganism of any one of E133-E143, wherein said recombinant microorganism has an acetate uptake under anaerobic conditions from about 0.35 g/L to about 3.3 g/L. E147. The recombinant microorganism of any one of E133-E146, wherein the recombinant microorganism further comprises one or more native and/or heterologous acetyl-CoA synthetases, and wherein said one or more native and/or heterologous acetyl-CoA synthetases is activated or upregulated. E148. The recombinant microorganism of E147, wherein said acetyl-CoA synthetase is encoded by a polynucleotide selected from the group consisting of an ACS1 polynucleotide and an ACS2 polynucleotide. E149. The recombinant microorganism of E148, wherein said ACS1 polynucleotide or said ACS2 polynucleotide is from a yeast microorganism.

E150. The recombinant microorganism of E149, wherein said ACS1 polynucleotide is from *Saccharomyces cerevisiae* or *Saccharomyces kluyveri.* E151. The recombinant microorganism of E149, wherein said ACS2 polynucleotide is from *Saccharomyces cerevisiae* or *Saccharomyces kluyveri.* E152. A method for increasing acetate uptake from a biomass comprising contacting said biomass with a recombinant microorganism according to any one of E65 to E71 or E119 to E151. E153. The method of E152 further comprising increasing the amount of sugars of the biomass. E154. The method of E153, wherein said sugars are increased by the addition of an exogenous sugar source to the biomass. E155. The method of E153 or E154, wherein said sugars are increased by the addition of one or more enzymes that use or break-down cellulose, hemicellulose and/or other biomass components. E156. The method of any one of E153-E155, wherein said sugars are increased by the addition of a CBP microorganism that uses or breaks-down cellulose, hemicellulose and/or other biomass components. E157. The recombinant microorganism of E5, wherein said xylose reductase (XR) has a preference for NADPH or is NADPH-specific. E158. The recombinant microorganism of E5, wherein said xylitol dehydrogenase (XDH) has a preference for NADH or is NADH-specific. E159. A recombinant microorganism comprising: one or more native and/or heterologous enzymes that function in one or more engineered metabolic pathways to convert acetate to an alcohol, wherein one of said native and/or heterologous enzymes is an NADPH-specific alcohol dehydrogenase.

E160. The recombinant microorganism of E159, wherein said NADPH-specific alcohol dehydrogenase is from a microorganism selected from the group consisting of *T. pseudethanolicus, C. beijerinckii, Entamoeba histolytica, Cucumis melo,* and *S. cerevisiae.* E161. The recombinant microorganism of E159, wherein said NADPH-specific alcohol dehydrogenase is encoded by any one of SEQ ID NOs:30, 32, 33, 35, or 36 or a fragment, variant, or derivative thereof that retains the function of an alcohol dehydrogenase. E162. A recombinant microorganism comprising: one or more native and/or heterologous enzymees that function in one or more engineered metabolic pathways to convert acetate to an alcohol, wherein a first native and/or heterologous enzyme is an NADPH-specific alcohol dehydrogenase and wherein a second native and/or heterologous enzyme is an acetyl-CoA synthetase. E163. The recombinant microorganism of E162, wherein said NADPH-specific alcohol dehydrogenase is from *Entamoeba histolytica.* E164. The recombinant microorganism of E162, wherein said NADPH-specific alcohol dehydrogenase is encoded by SEQ ID NO:35 or a fragment, variant, or derivative thereof that retains the function of an alcohol dehydrogenase. E165. The recombinant microorganism of any one of E162-E164, wherein said acetyl-CoA synthetase is from a yeast microorganism or from a bacterial microorganism. E166. The recombinant microorganism of any one of E162-E164, wherein said acetyl-CoA synthetase is from *Saccharomyces cerevisiae, Saccharomyces kluyveri, Zygosaccharomyces bailii,* or *Acetobacter aceti.* E167. The recombinant microorganism of any one of E162-E164, wherein said acetyl-CoA synthetase is encoded by any one of SEQ ID NOs:37-40, 57, 58 or a fragment, variant, or derivative thereof that retains the function of an acetyl-CoA synthetase. E168. A recombinant microorganism comprising: one or more native and/or heterologous enzymes that function in one or more engineered metabolic pathways to convert acetate to an alcohol, wherein a first native and/or heterologous enzyme is an NADPH-specific alcohol dehydrogenase and wherein a second native and/or heterologous enzyme is an NADH-specific alcohol dehydrogenase. E169. The recombinant microorganism of E168, wherein said NADPH-specific alcohol dehydrogenase is from *Entamoeba histolytica.*

E170. The recombinant microorganism of E168, wherein said NADPH-specific alcohol dehydrogenase is encoded by SEQ ID NO:35 or a fragment, variant, or derivative thereof that retains the function of an alcohol dehydrogenase. E171. The recombinant microorganism of E168, wherein said NADH-specific alcohol dehydrogenase is downregulated. E172. The recombinant microorganism of E171, wherein said downregulated NADH-specific alcohol dehydrogenase is selected from ADH1, ADH2, ADH3, ADH4, ADH5, or SFA1 from *Saccharomyces.* E173. A recombinant microorganism comprising: a one or more native and/or heterologous enzymes that function in one or more first engineered metabolic pathways to convert acetate to an alcohol, wherein said one or more native and/or heterologous enzymes is activated, upregulated or downregulated; and b) one or more native and/or heterologous enzymes that function in one or more second engineered metabolic pathways to produce an electron donor used in the conversion of acetate to an alcohol, wherein one of said native and/or heterologous enzymes is a formate dehydrogenase. E174. The recombinant microorganism of E173, wherein the formate dehydrogenase is from a yeast microorganism. E175. The recombinant microorganism of E174, wherein the yeast microorganism is *S. cerevisiae* or *Candida boidinii.* E176. The recombinant microorganism of E175, wherein the formate dehydrogenase from *S. cerevisiae* is FDH1. E177. The recombinant microorganism of E175, wherein the formate dehydrogenase from *Candida boidinii* is FDH3. E178. The recombinant microorganism of E173, wherein the formate dehydrogenase from is encoded by SEQ ID NO:46, 47, or a fragment, variant, or derivative thereof that retains the function of a formate dehydrogenase. E179. The recombinant microorganism of any one of E48-E50, wherein said microorganism consumes or uses more acetate than a microorganism not comprising overexpression of said native and/or heterologous transhydrogenase enzyme.

E180. The recombinant microorganism of any one of E48-E50, wherein said microorganism produces more ethanol than a microorganism not comprising overexpression of said native and/or heterologous transhydrogenase enzyme.

E181. The recombinant microorganism of any one of E48-E50, wherein said microorganism produces more glycerol than a microorganism not comprising overexpression of said native and/or heterologous transhydrogenase enzyme.

E182. The recombinant microorganism of E179, wherein the microorganism has an acetate uptake (g/L) selected from at least about 0.35 g/L, at least about 0.36 g/L, at least about 0.38 g/L, at least about 0.40 g/L, at least about 0.44 g/L, at least about 0.45 g/L, at least about 0.47 g/L, at least about 0.48 g/L, at least about 0.51 g/L, at least about 0.53 g/L, at least about 0.59 g/L, at least about 0.61 g/L, at least about 0.63 g/L, at least about 0.65 g/L, at least about 0.66 g/L, at least about 0.70 g/L, at least about 0.79 g/L, at least about 0.8 g/L, at least about 0.83 g/l, at least about 0.84 g/L, or at least about 085 g/L. E183. The recombinant microorganism of E180, wherein the microorganism produces ethanol at a level selected from: (a) at least about 2% more ethanol produced by a recombinant microorganism not comprising said transhydrogenase; (b) at least about 3% more ethanol produced by a recombinant microorganism not comprising said transhydrogenase; (c) at least about 4% more ethanol produced by a recombinant microorganism not comprising said transhydrogenase; (d) at least about 4.5% more ethanol produced by a recombinant microorganism not comprising said transhydrogenase; (e) at least about 5% more ethanol produced by a recombinant microorganism not comprising said transhydrogenase; (f) at least about 6% more ethanol produced by a recombinant microorganism not comprising said transhydrogenase; (g) at least about 10% more ethanol produced by a recombinant microorganism not comprising said transhydrogenase; (h) at least about 15% more ethanol produced by a recombinant microorganism not comprising said transhydrogenase; (i) at least about 20% more ethanol produced by a recombinant microorganism not comprising said transhydrogenase; (j) at least about 25% more ethanol produced by a recombinant microorganism not comprising said transhydrogenase; (k) at least about 30% more ethanol produced by a recombinant microorganism not comprising said transhydrogenase; (1) at least about 35% more ethanol produced by a recombinant microorganism not comprising said transhydrogenase; (m) at least about 40% more ethanol produced by a recombinant microorganism not comprising said transhydrogenase; (n) at least about 45% more ethanol produced by a recombinant microorganism not comprising said transhydrogenase; (o) at least about 50% more ethanol produced by a recombinant microorganism not comprising said transhydrogenase; (p) at least about 55% more ethanol produced by a recombinant microorganism not comprising said transhydrogenase; (q) at least about 56% more ethanol produced by a recombinant microorganism not comprising said transhydrogenase; and (r) at least about 60% more ethanol produced by a recombinant microorganism not comprising said transhydrogenase. E184. The recombinant microorganism of E181, wherein the microorganism produces glycerol (g/L) selected from at least about 0.10 g/L, at least about 0.15 g/L, at least about 0.20 g/L, at least about 0.25 g/L, at least about 0.30 g/L, at least about 0.35 g/L, at least about 0.36 g/L, at least about 0.38 g/L, or at least about 0.40 g/L. E185. The recombinant microorganism of E181, wherein the microorganism produces glycerol (g/L) selected from: (a) at least about 1.1 fold more glycerol than that produced by a recombinant microorganism not comprising said transhydrogenase; (b) at least about 1.2 fold more glycerol than that produced by a recombinant microorganism not comprising said transhydrogenase; (c) at least about 1.3 fold more glycerol than that produced by a recombinant microorganism not comprising said transhydrogenase; (d) at least about 1.4 fold more glycerol than that produced by a recombinant microorganism not comprising said transhydrogenase; (e) at least about 1.5 fold more glycerol than that produced by a recombinant microorganism not comprising said transhydrogenase; (f) at least about 1.6 fold more glycerol than that produced by a recombinant microorganism not comprising said transhydrogenase; (g) at least about 1.9 fold more glycerol than that produced by a recombinant microorganism not comprising said transhydrogenase; and (h) at least about 2.0 fold more glycerol than that produced by a recombinant microorganism not comprising said transhydrogenase. E186. A method for increasing acetate uptake from a biomass comprising contacting said biomass with a recombinant microorganism according to any one of E179-E185, wherein said biomass is pre-treated agricultural waste or pre-treated corn stover. E187. A method for increasing ethanol production from a biomass comprising contacting said biomass with a recombinant microorganism according to any one of E179-E185, wherein said biomass is pre-treated agricultural waste or pre-treated corn stover. E188. A method for increasing glycerol production from a biomass comprising contacting said biomass with a recombinant microorganism according to any one of E179-E185, wherein said biomass is pre-treated agricultural waste or pre-treated corn stover.

Incorporation by Reference

All of the references cited herein are hereby incorporated by reference in their entirety.

Equivalents

Those skilled in the art will recognize, or be able to ascertain using no more than routine experimentation, many equivalents to the specific embodiments of the invention described herein. Such equivalents are intended to be encompassed by the following claims.

## Claims

**1.** A recombinant microorganism comprising:

a) one or more native and/or heterologous enzymes that function in a first engineered metabolic pathway to convert acetate to an alcohol, wherein said one or more native and/or heterologous enzymes is activated, upregulated or downregulated and is recombinantly introduced into said microorganism,
b) one or more native and/or heterologous enzymes that function in a second engineered metabolic pathway to produce NADH, NADPH, or a combination thereof for use in the conversion of acetate to an alcohol, wherein said one or more native and/or heterologous enzymes is activated, upregulated or downregulated and is recombinantly introduced into said microorganism, wherein said second engineered metabolic pathway comprises activation or upregulation of the native enzyme glucose-6-P dehydrogenase of the oxidation branch of the pentose phosphate pathway (PPP);
wherein one of said engineered metabolic pathways comprises the following steps: (1) conversion of acetate to acetyl-CoA and (2) conversion of acetyl-CoA to ethanol,
wherein said acetyl-CoA is converted to acetaldehyde by an acetaldehyde dehydrogenase;
wherein said acetaldehyde is converted to ethanol by an alcohol dehydrogenase;
wherein said acetaldehyde dehydrogenase is an NADPH- specific acetaldehyde dehydrogenase; and
wherein said alcohol dehydrogenase is an NADPH-specific alcohol dehydrogenase or a combination thereof;
wherein said alcohol is ethanol.

**2.** The recombinant microorganism of claim 1 or claim 2, wherein said first and second engineered metabolic pathways result in ATP production.

**3.** The recombinant microorganism of any one of claims 1 to 3, wherein said first engineered metabolic pathways comprises activating or upregulating one or more heterologous enzymes selected from the group consisting of acetyl-CoA acetyltransferase (thiolase), acetoacetyl-CoA transferase, acetoacetate decarboxylase, a secondary alcohol dehydrogenase, and combinations thereof;
preferably wherein said first engineered metabolic pathways comprises activating or upregulating a heterologous ADP-producing acetyl-CoA synthase enzyme;
or wherein said first engineered metabolic pathways comprises activating or upregulating the acetate kinase/phosphotransacetylase (AK/PTA) couple;
more preferably wherein said first and second engineered metabolic pathways result in ATP production.

**4.** The recombinant microorganism of claim 1, wherein
said native glucose-6-P dehydrogenase enzyme is from *Saccharomyces cerevisiae,* and optionally is encoded by a *zwf1* polynucleotide.

**5.** The recombinant microorganism of claim 1, further comprising altering the expression of transcription factors that regulate expression of enzymes of the pentose phosphate pathway (PPP);
preferably wherein the transcription factor is Stb5p, and optionally is from *Saccharomyces cerevisiae.*

**6.** The recombinant microorganism of claim 1, wherein said second engineered metabolic pathway comprises downregulation of the native enzyme glucose-6-P isomerase that competes with the oxidative branch of the PPP;
preferably wherein said native glucose-6-P isomerase enzyme is from *Saccharomyces cerevisiae,* and optionally is encoded by a *pgi1* polynucleotide.

**7.** The recombinant microorganism of any one of claims 1 to 6, wherein said microorganism further comprises overexpression of a native and/or heterologous transhydrogenase enzyme that catalyzes the interconversion of NADPH and NAD to NADP and NADH, and optionally is from a microorganism selected from the group consisting of *Escherichia coli* and *Azotobacter vinelandii.*

**8.** The recombinant microorganism of any one of claims 1 to 7, wherein said microorganism further comprises overexpression of a native and/or heterologous glutamate dehydrogenase enzyme, and optionally is encoded by a *gdh2* polynucleotide

**9.** The recombinant microorganism of any one of claims 1 to 8, wherein
said NADPH-specific acetaldehyde dehydrogenase is *T. pseudoethanolicus* adhB.

**10.** The recombinant microorganism of any one of claims 1 to 6, wherein said NADPH-specific alcohol dehydrogenase is from a microorganism selected from the group consisting of *T. pseudethanolicus, C. beijerinckii, Entamoeba histolytica, Cucumis melo,* and *S. cerevisiae.*

**11.** The recombinant microorganism of any one of claims 1 to 10, wherein said acetyl-CoA is converted to ethanol by a bifunctional acetaldehyde/alcohol dehydrogenase, and optionally is from *E. coli, C. acetobutylicum, T. saccharolyticum, C. thermocellum,* or *C. phytofermentans* .

**12.** The recombinant microorganism of any one of claims 1 to 11, wherein said one or more downregulated native enzymes is encoded by a *gpd1* polynucleotide, a *gpd2* polynucleotide, or both a *gpd1* polynucleotide and a *gpd2* polynucleotide.

**13.** A process for converting biomass to ethanol, acetone, or isopropanol comprising contacting biomass with a recombinant microorganism according to any one of claims 1-12;
preferably wherein said biomass comprises lignocellulosic biomass, and optionally is selected from the group consisting of grass, switch grass, cord grass, rye grass, reed canary grass, mixed prairie grass, miscanthus, sugar-processing residues, sugarcane bagasse, sugarcane straw, agricultural wastes, rice straw, rice hulls, barley straw, corn cobs, cereal straw, wheat straw, canola straw, oat straw, oat hulls, corn fiber, stover, soybean stover, corn stover, forestry wastes, recycled wood pulp fiber, paper sludge, sawdust, hardwood, softwood, agave, and combinations thereof;
more preferably wherein said process reduces or removes acetate from the consolidated bioprocessing (CBP) media.

**14.** An engineered metabolic pathway for reducing or removing acetate from consolidated bioprocessing (CBP) media according to any one of claims 1-12.

**15.** A method for increasing acetate uptake from a biomass comprising contacting said biomass with a recombinant microorganism according to claim 9;
optionally further comprising increasing the amount of sugars of the biomass, and optionally wherein said sugars are increased by the addition of an exogenous sugar source to the biomass or are increased by the addition of one or more enzymes that use or breakdown cellulose, hemicellulose and/or other biomass components;
preferably wherein said sugars are increased by the addition of a CBP microorganism that uses or breaks-down cellulose, hemicellulose and/or other biomass components.

Figure 1

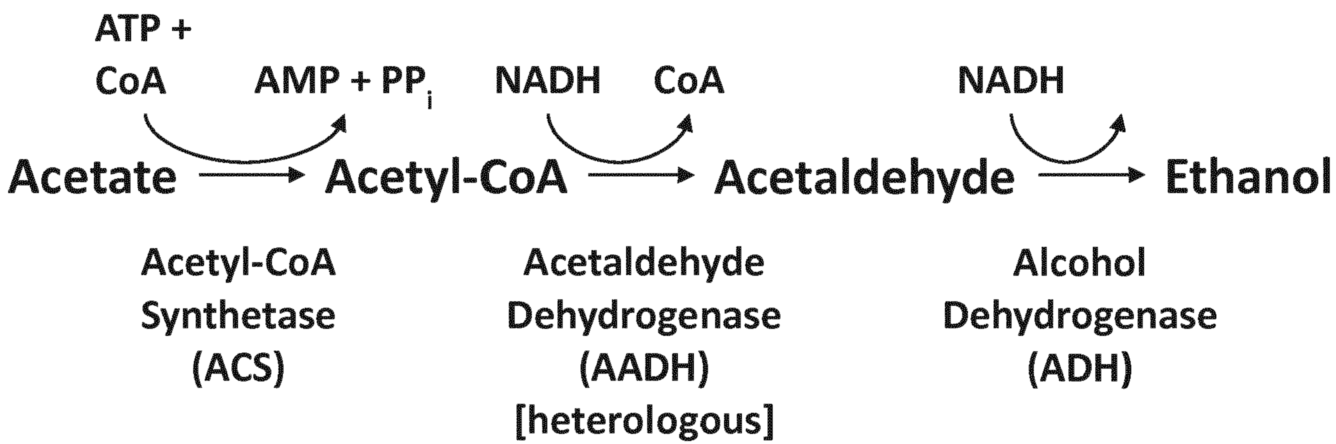


**Net reaction**

acetate + 2 ATP + 2 NADH → ethanol + 2 ADP + 2 $P_i$

Figure 2

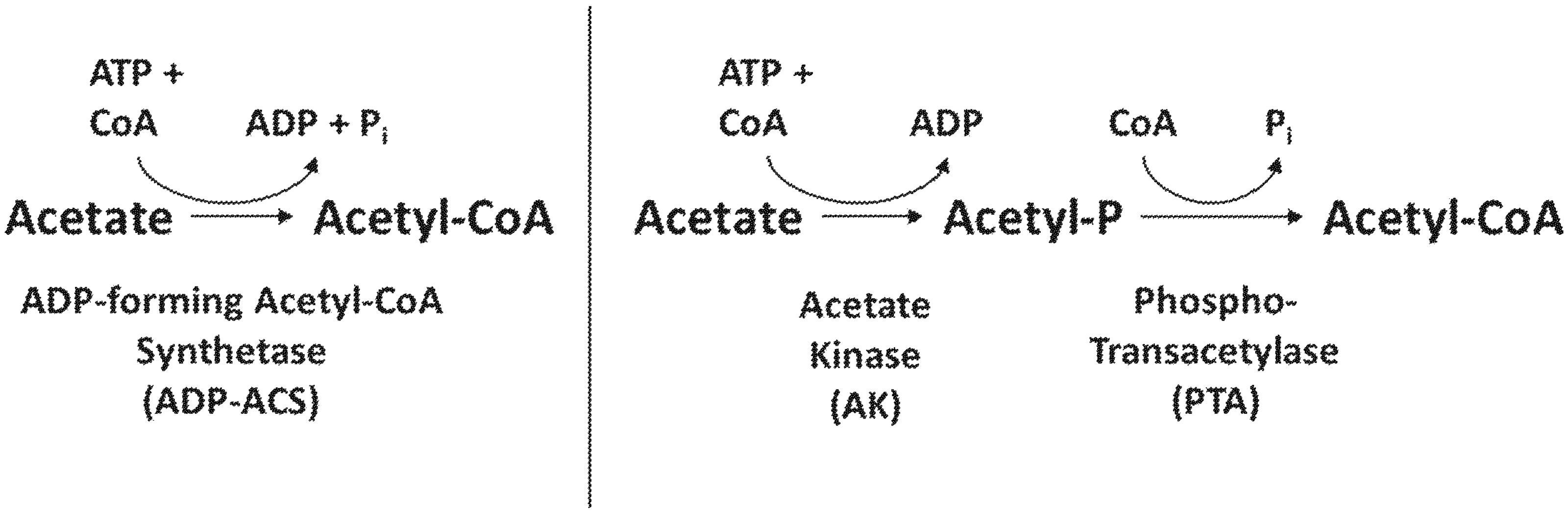


**Net reaction**

| | |
|---|---|
| ACS | acetate + 2 ATP + 2 NADH → ethanol + 2 ADP + 2 $P_i$ |
| ADP-ACS or AK + PTA | acetate + ATP + 2 NADH → ethanol + ADP + $P_i$ |

Figure 3

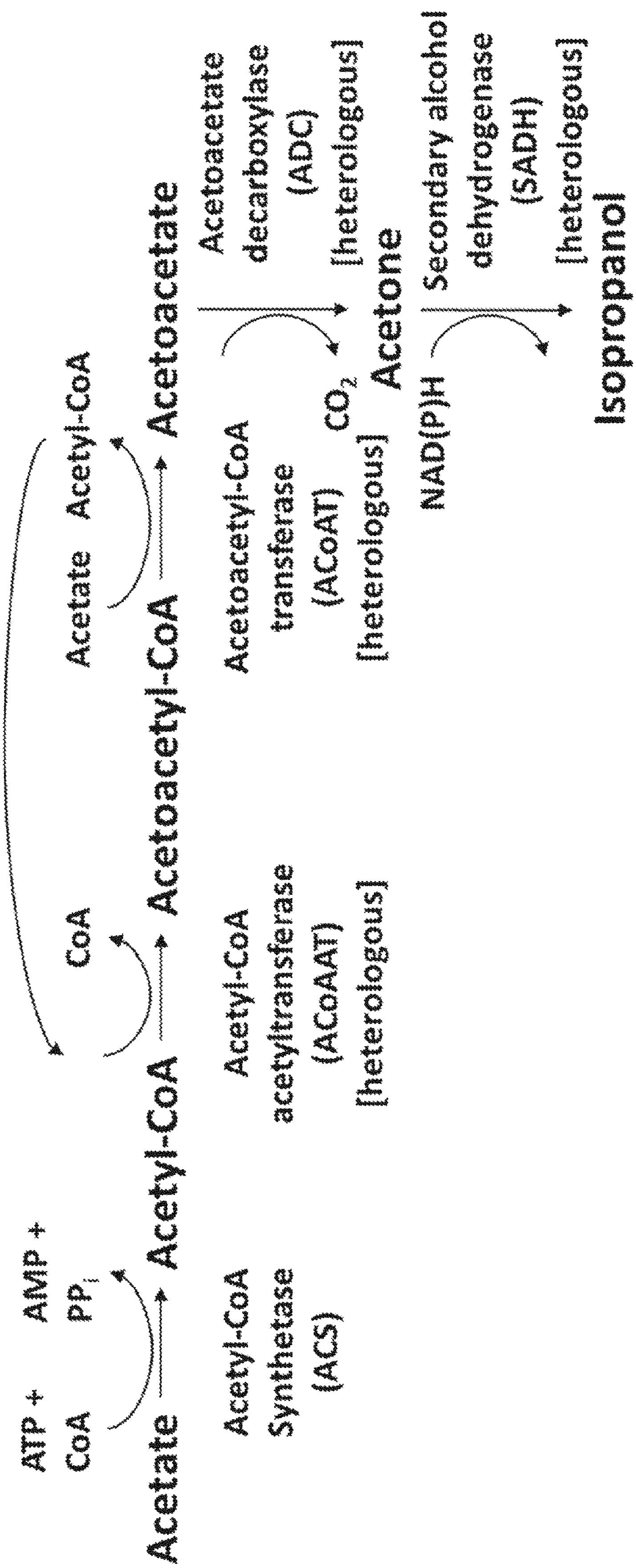


**Net reaction**

2 acetate + 2 ATP + NAD(P)H → isopropanol + $CO_2$ + 2 ADP + 2 $P_i$

Figure 4

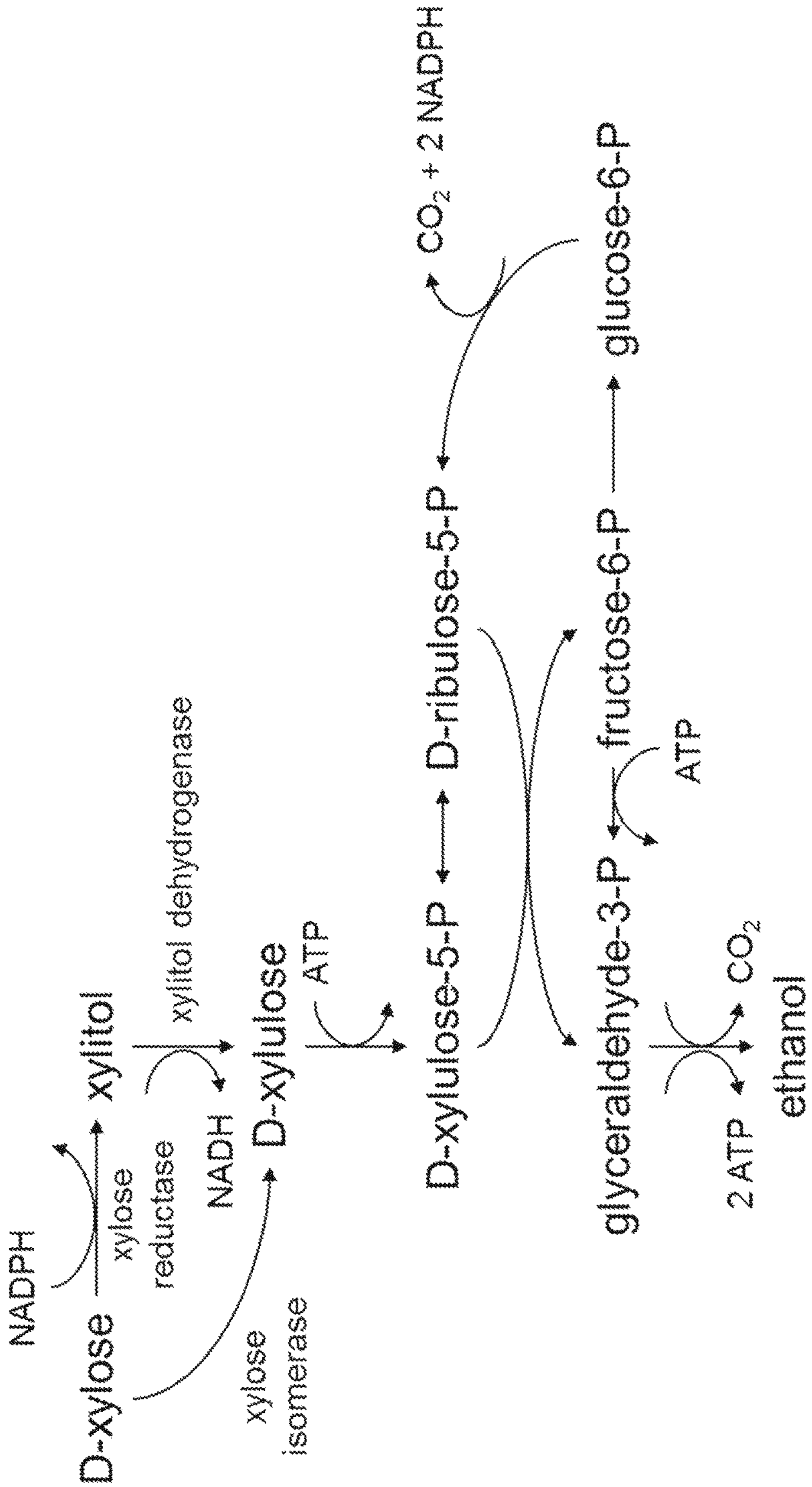
D-xylose
NADPH
xylose reductase
xylitol
xylitol dehydrogenase
NADH
D-xylulose
xylose isomerase
ATP
D-xylulose-5-P
D-ribulose-5-P
$CO_2$ + 2 NADPH
glucose-6-P
fructose-6-P
ATP
glyceraldehyde-3-P
2 ATP
$CO_2$
ethanol

Figure 5

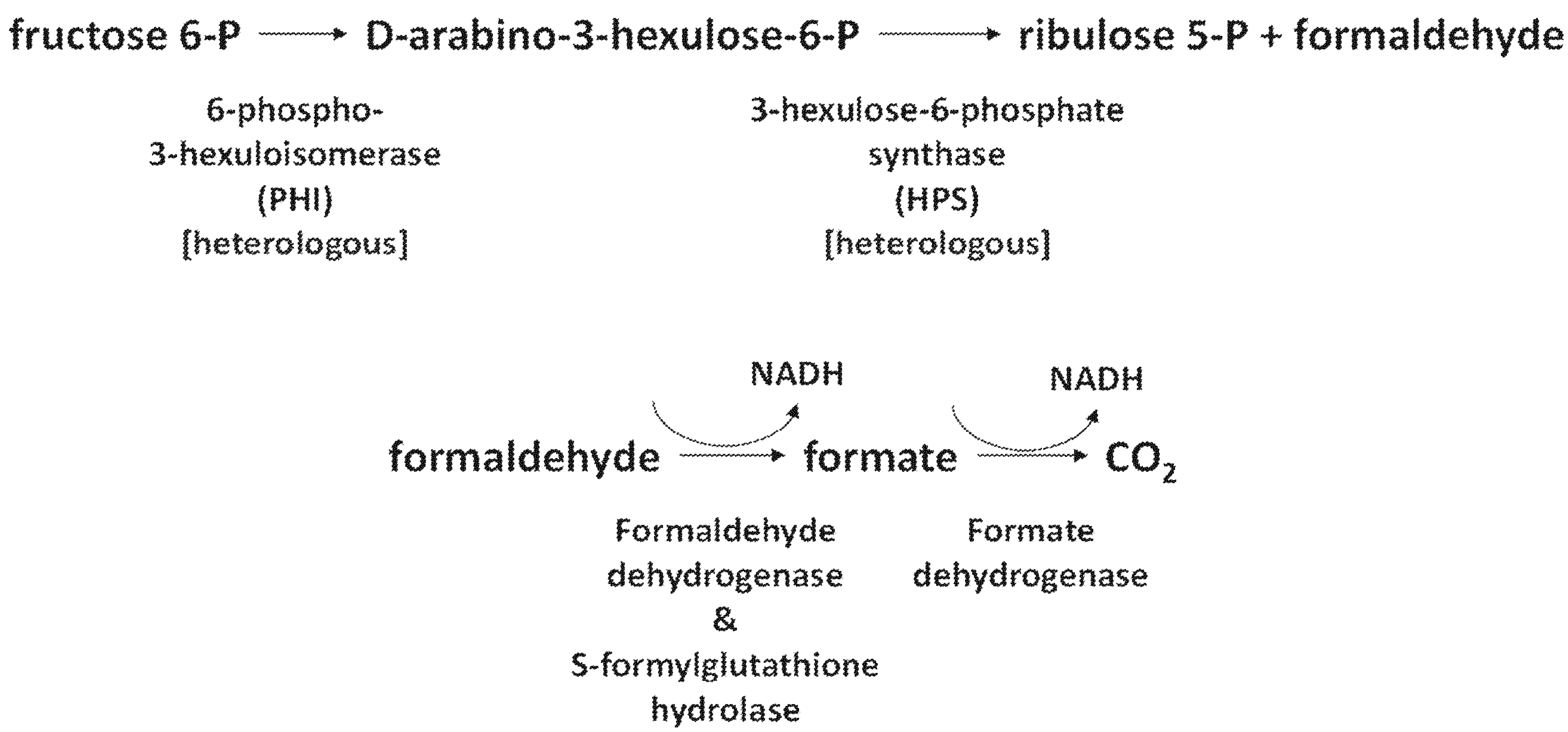
fructose 6-P
D-arabino-3-hexulose-6-P
ribulose 5-P + formaldehyde
6-phospho-
3-hexuloisomerase
(PHI)
[heterologous]
3-hexulose-6-phosphate
synthase
(HPS)
[heterologous]
NADH
NADH
formaldehyde
formate
CO2
Formaldehyde
dehydrogenase
&
S-formylglutathione
hydrolase
Formate
dehydrogenase

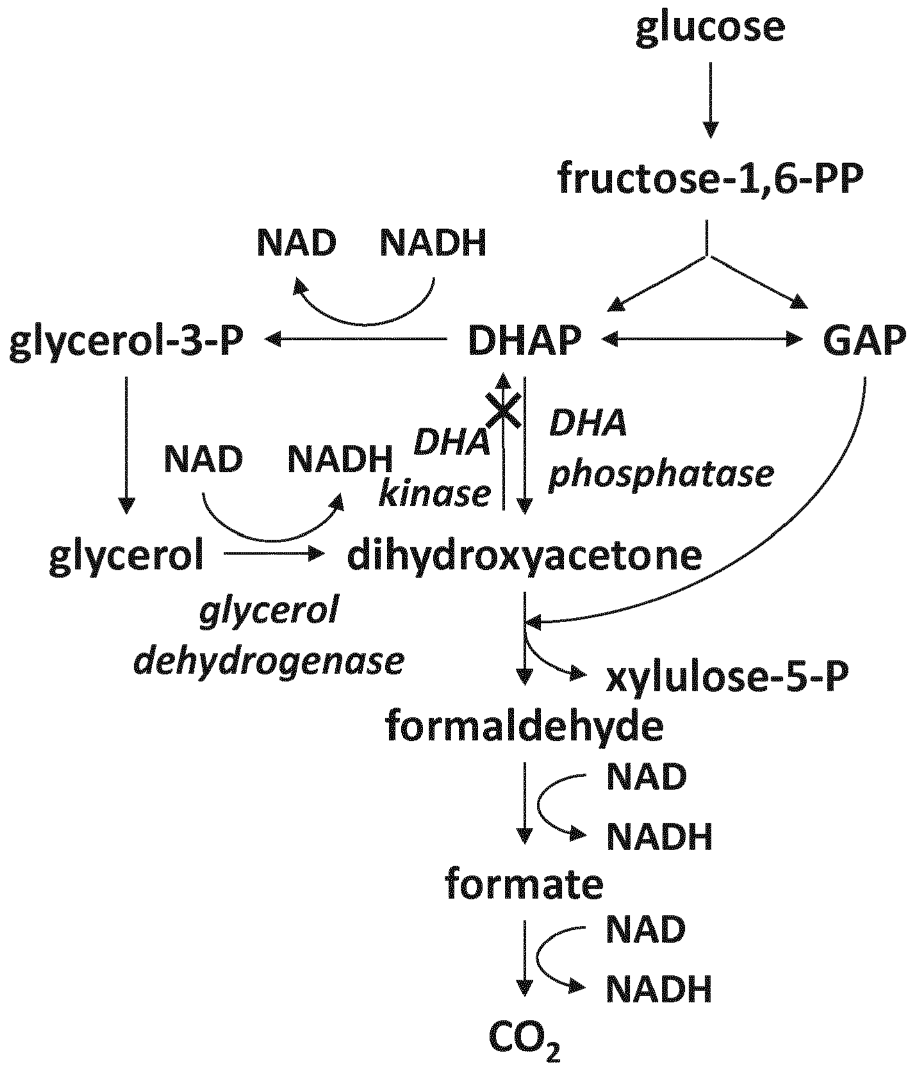
glucose
fructose-1,6-PP
NAD
NADH
glycerol-3-P
DHAP
GAP
DHA kinase
DHA phosphatase
NAD
NADH
glycerol
dihydroxyacetone
glycerol dehydrogenase
xylulose-5-P
formaldehyde
NAD
NADH
formate
NAD
NADH
$CO_2$

Figure 7

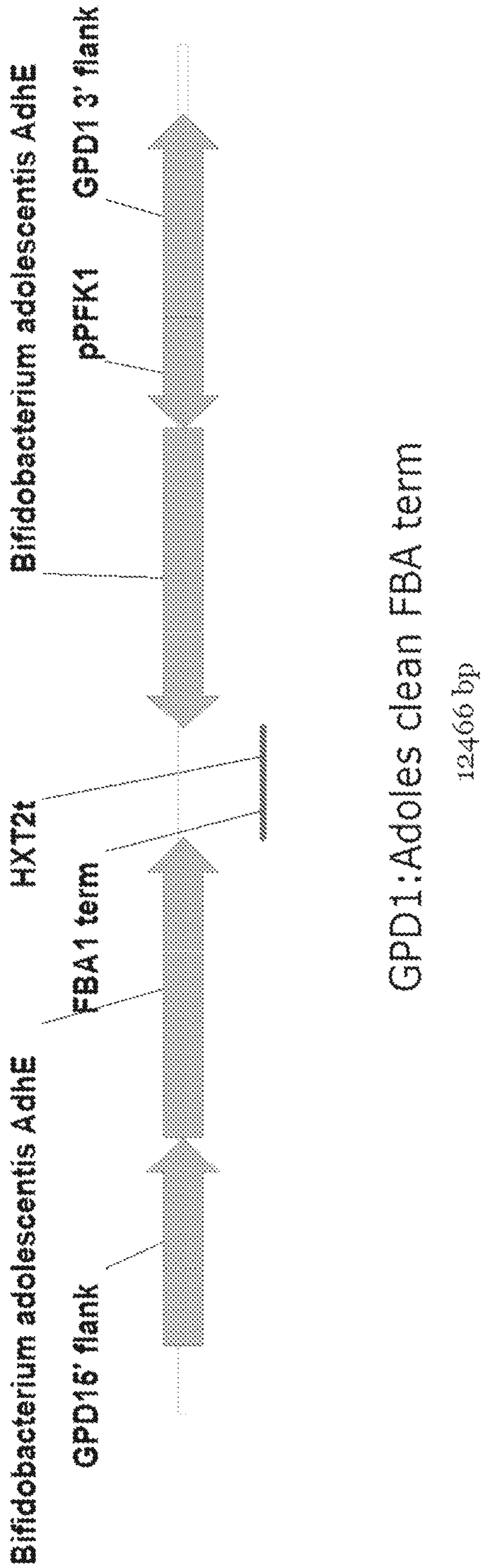
GPD15' flank
Bifidobacterium adolescentis AdhE
FBA1 term
HXT2t
Bifidobacterium adolescentis AdhE
pPFK1
GPD1 3' flank
GPD1:Adoles clean FBA term
12466 bp

Figure 8

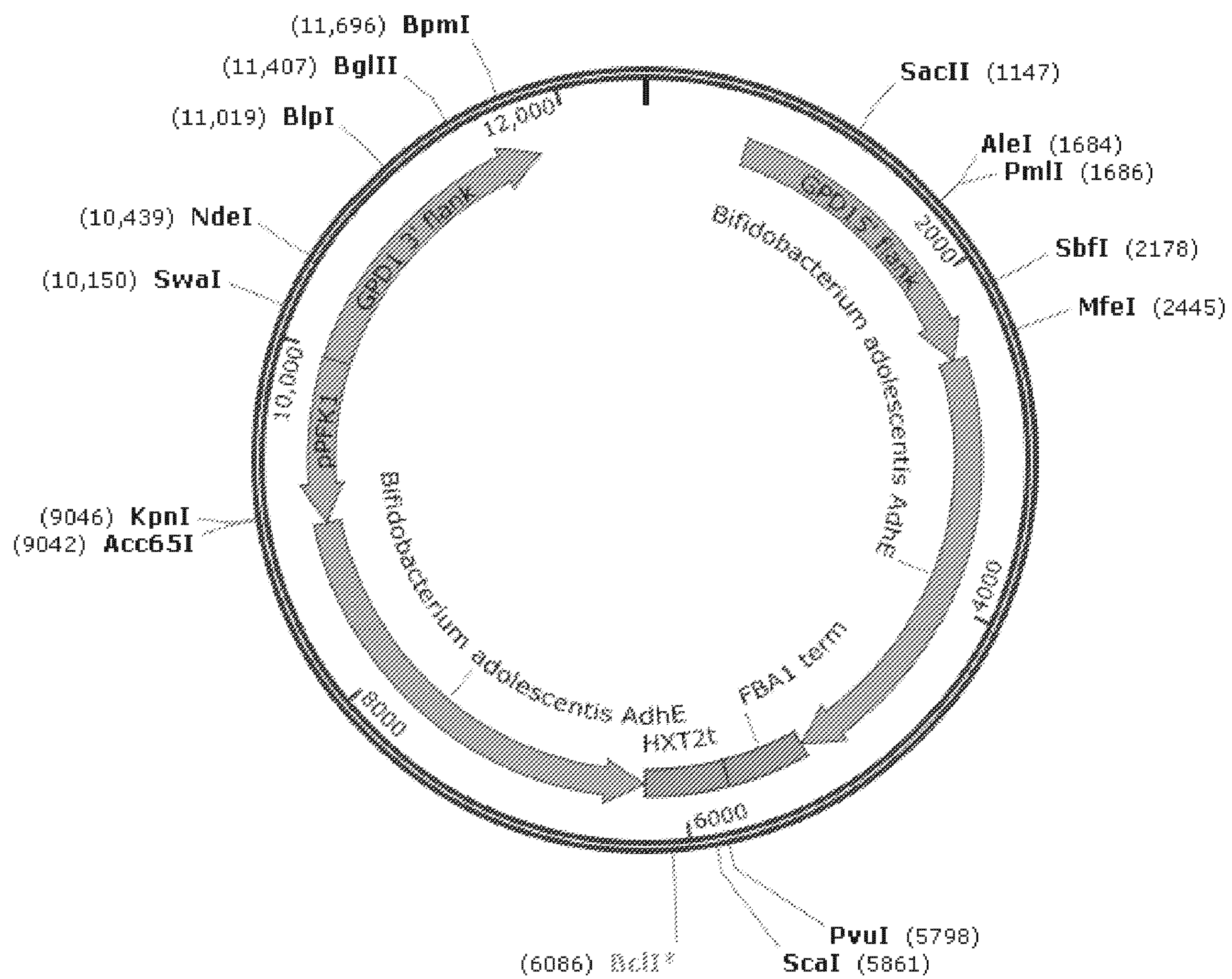


GPD1 replaced by 2x B adolescentis AdhE
12,466 bp

Figure 9

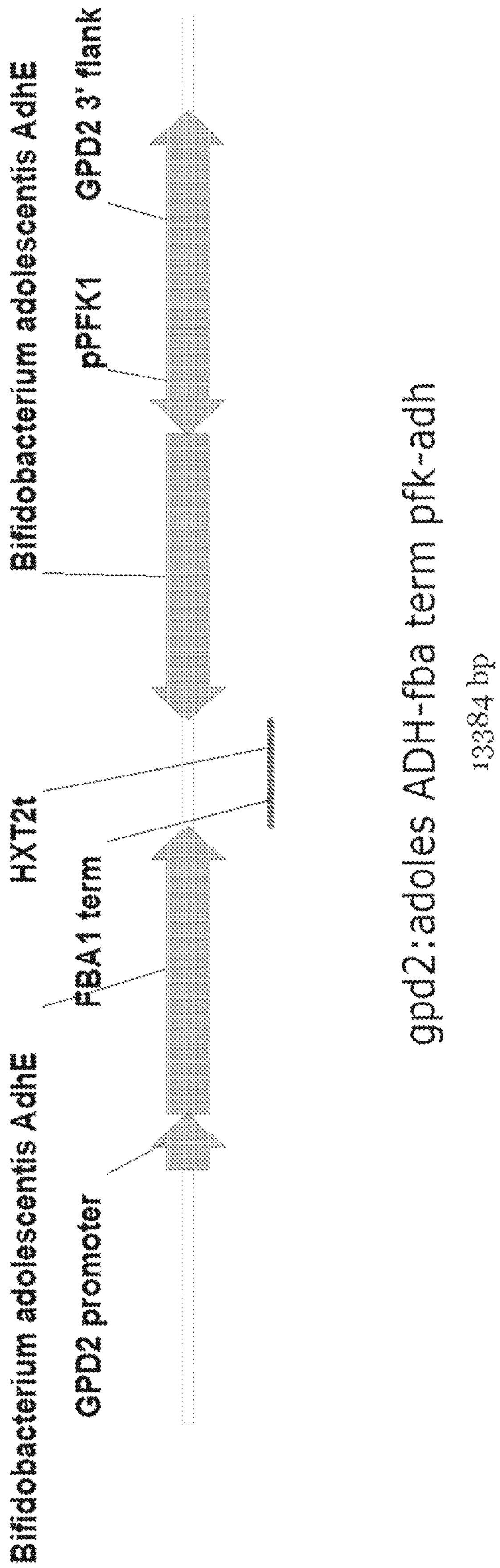
Bifidobacterium adolescentis AdhE
GPD2 promoter
FBA1 term
HXT2t
Bifidobacterium adolescentis AdhE
pPFK1
GPD2 3' flank
gpd2:adoles ADH-fba term pfk-adh
13384 bp

Figure 10

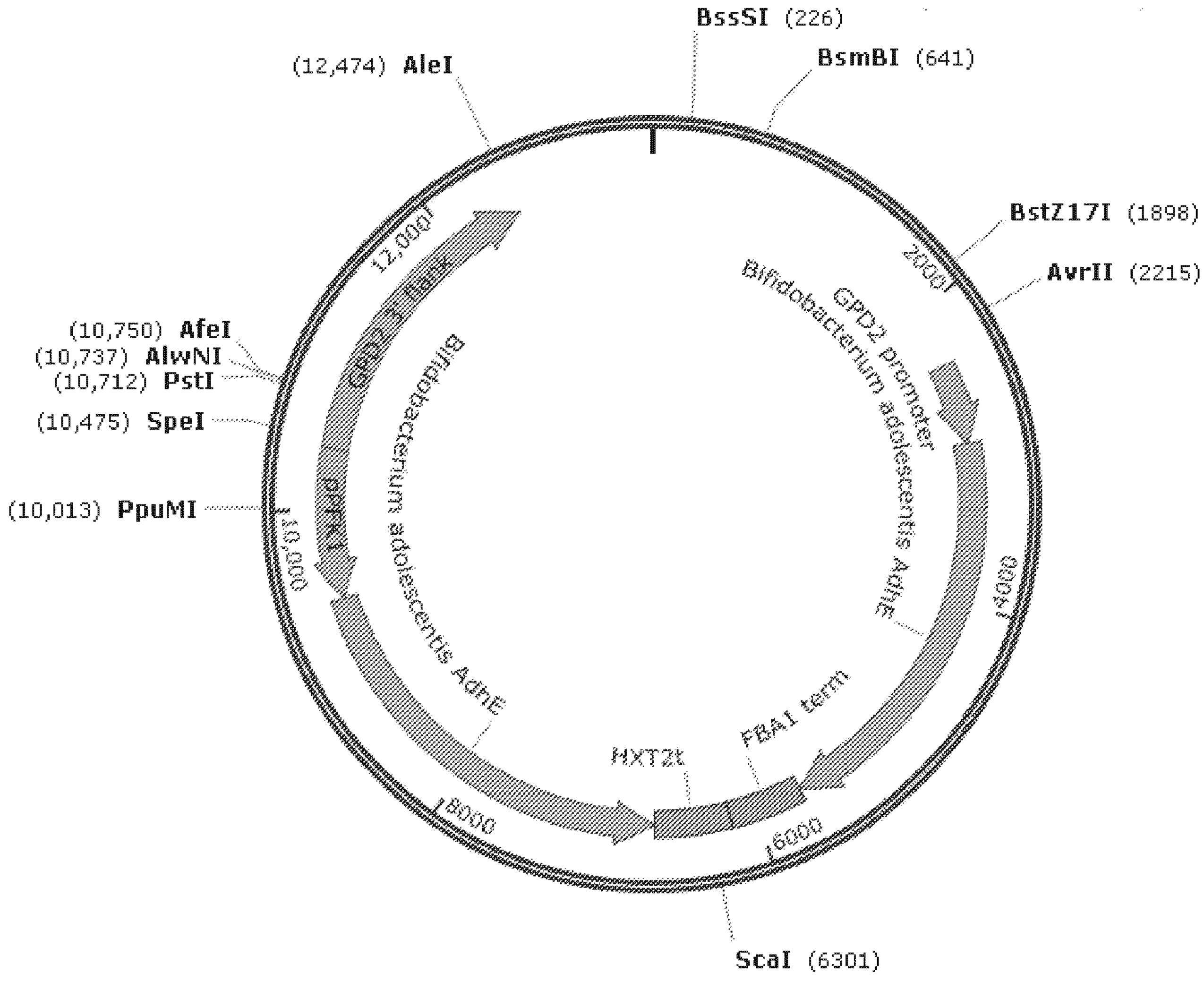


GPD2 replaced by 2x B adolescentis AdhE
13,384 bp

Figure 11

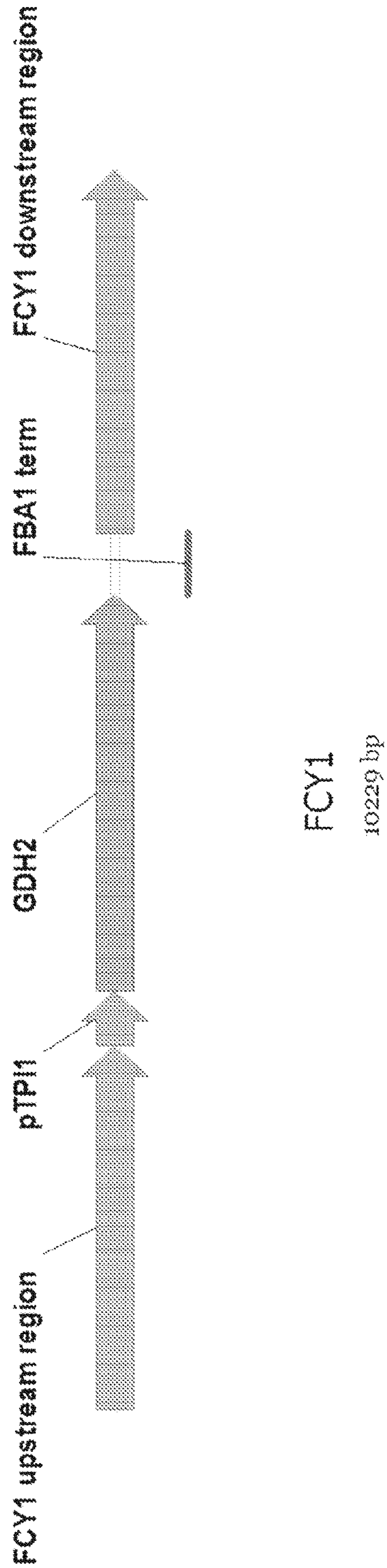
FCY1 upstream region
pTPI1
GDH2
FBA1 term
FCY1 downstream region
FCY1
10229 bp

Figure 12

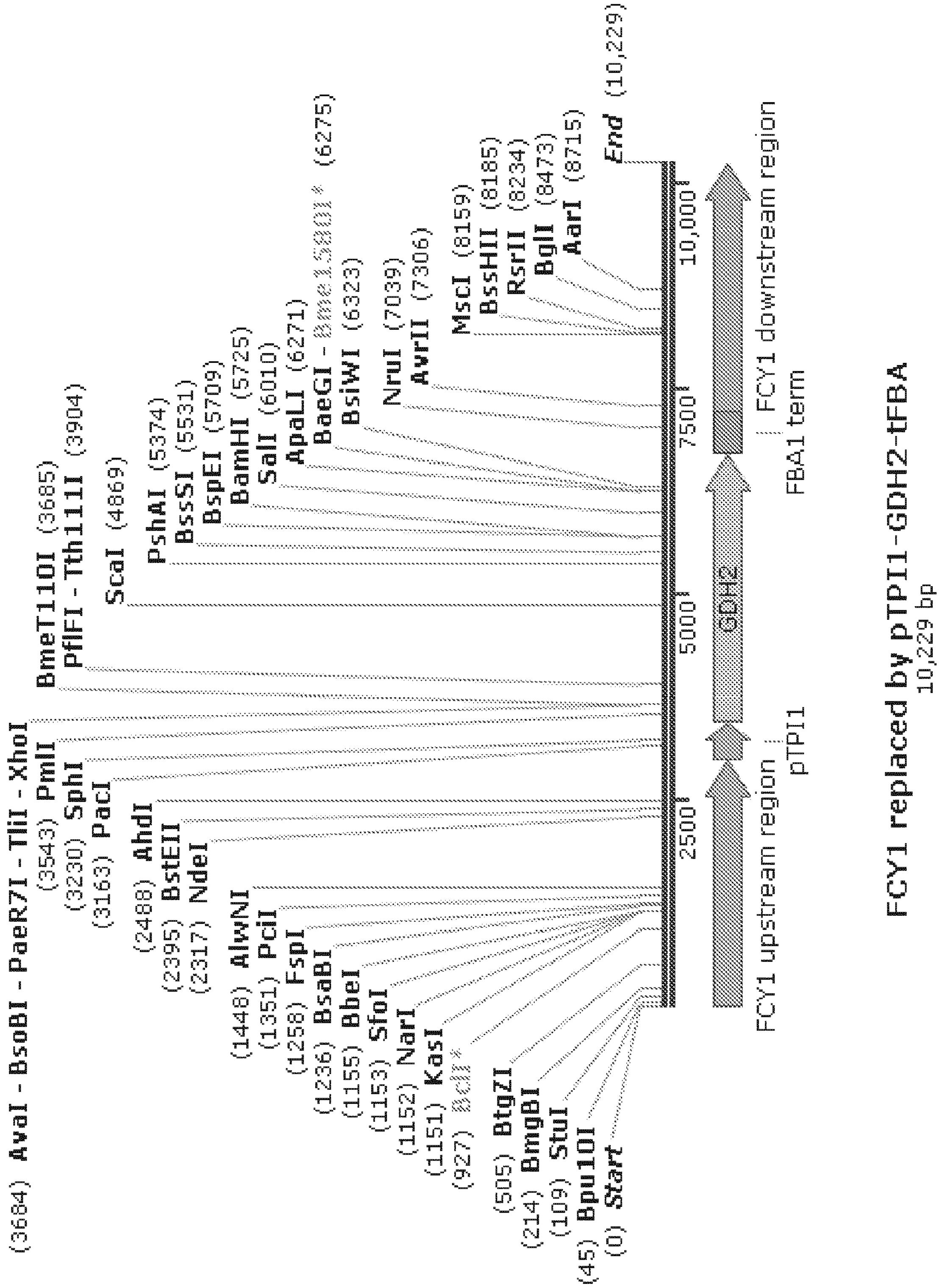

(3684) AvaI - BsoBI - PaeR7I - TliI - XhoI
(3543) PmlI
(3230) SphI
(3163) PacI
(2488) AhdI
(2395) BstEII
(2317) NdeI
(1448) AlwNI
(1351) PciI
(1258) FspI
(1236) BsaBI
(1155) BbeI
(1153) SfoI
(1152) NarI
(1151) KasI
(927) BclI*
(505) BtgZI
(214) BmgBI
(109) StuI
(45) Bpu10I
(0) Start
BmeT110I (3685)
PflFI - Tth111I (3904)
ScaI (4869)
PshAI (5374)
BssSI (5531)
BspEI (5709)
BamHI (5725)
SalI (6010)
ApaLI (6271)
BaeGI - Bme1580I* (6275)
BsiWI (6323)
NruI (7039)
AvrII (7306)
MscI (8159)
BssHII (8185)
RsrII (8234)
BglI (8473)
AarI (8715)
End (10,229)
2500
5000
7500
10,000
FCY1 upstream region
pTPI1
GDH2
FBA1 term
FCY1 downstream region
FCY1 replaced by pTPI1-GDH2-tFBA
10,229 bp

Figure 13

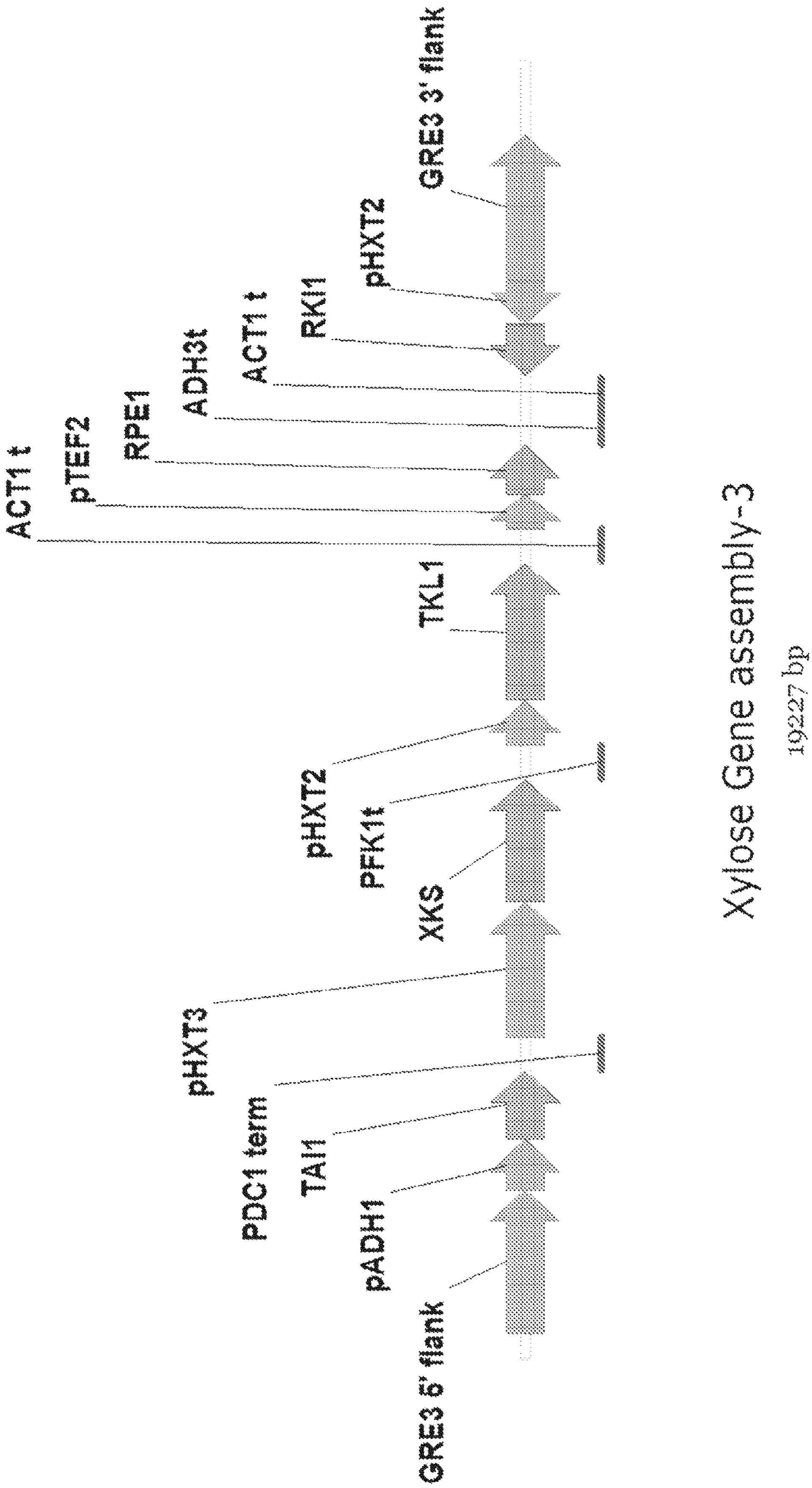
GRE3 5' flank
pADH1
TAI1
PDC1 term
pHXT3
XKS
PFK1t
pHXT2
TKL1
ACT1 t
pTEF2
RPE1
ADH3t
ACT1 t
RKI1
pHXT2
GRE3 3' flank
Xylose Gene assembly-3
19227 bp

Figure 14

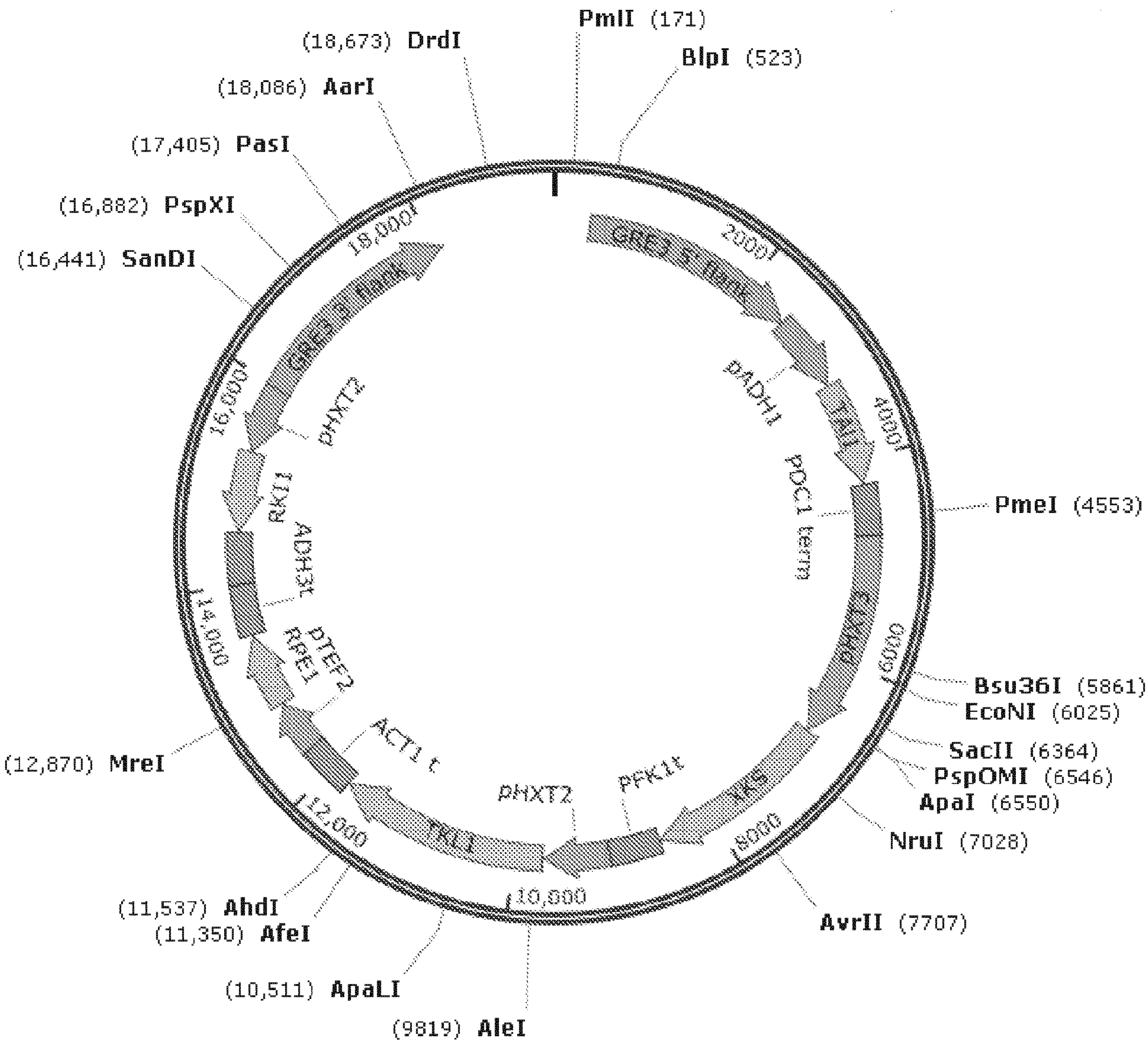


GRE3 replaced by TAL1 XKS1 TKL1 RPE1 and RKI1
19,227 bp

Figure 15

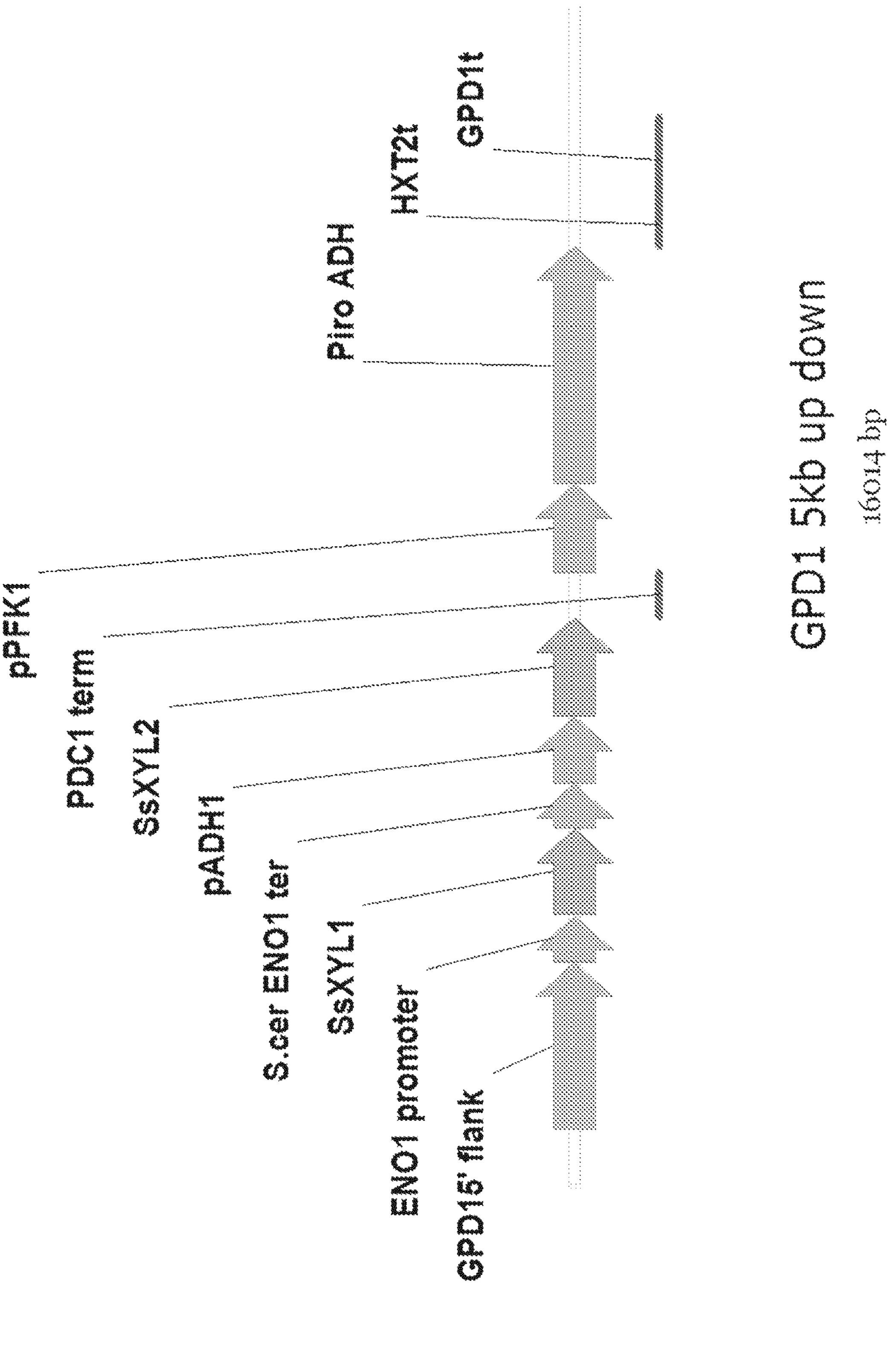
GPD15' flank
ENO1 promoter
SsXYL1
S.cer ENO1 ter
pADH1
SsXYL2
PDC1 term
pPFK1
Piro ADH
HXT2t
GPD1t
GPD1 5kb up down
16014 bp

Figure 16

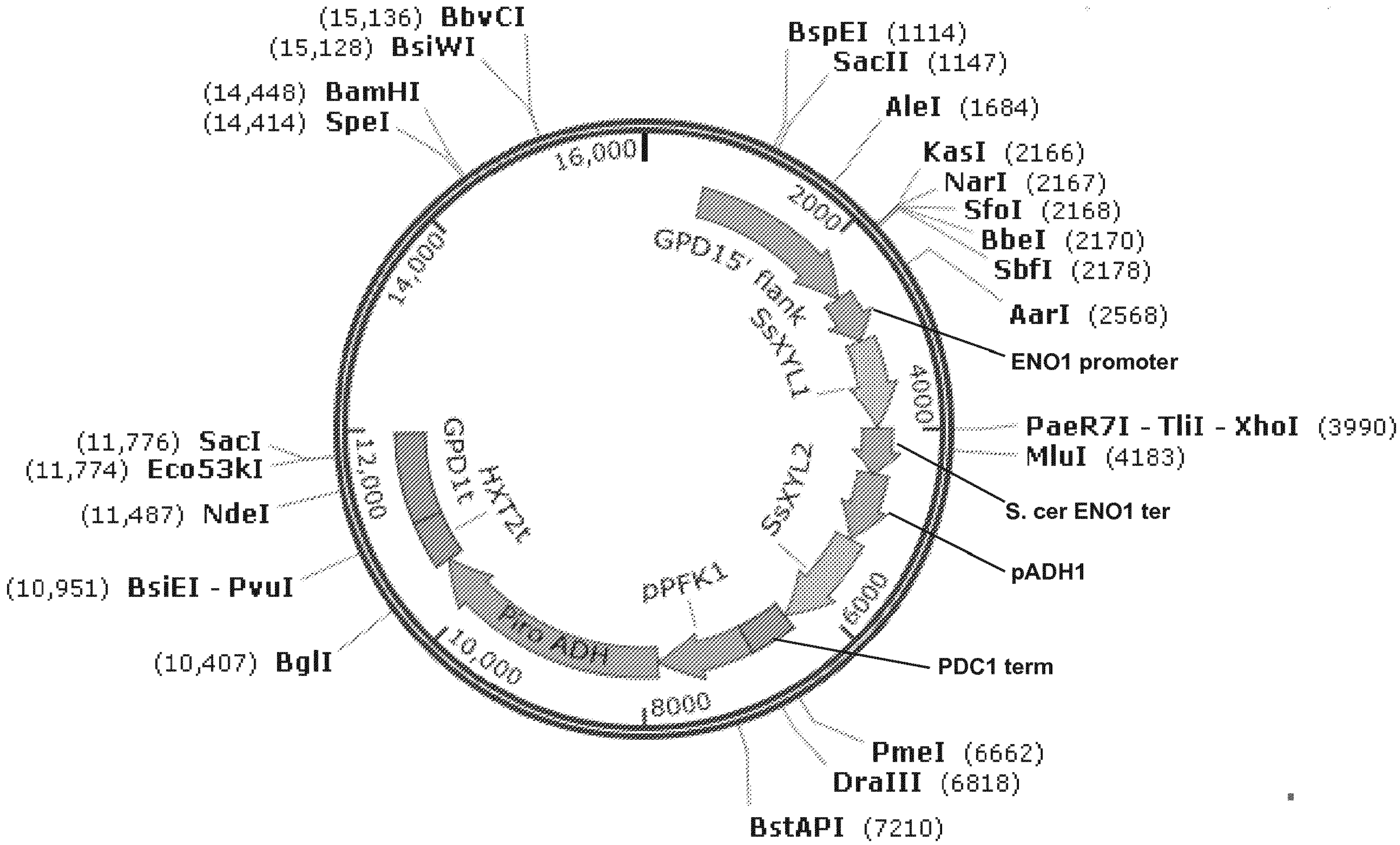


GPD1 replaced by SsXYL1 SsXYL2 and AADH
16,014 bp

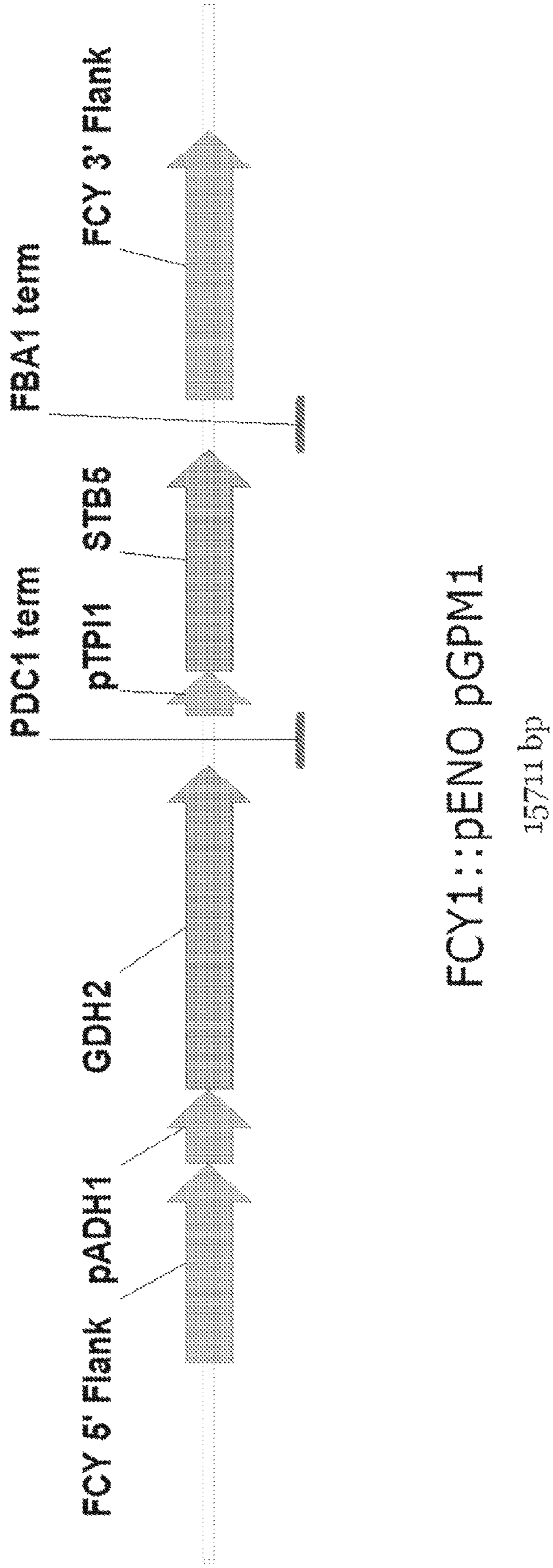
FCY 5' Flank
pADH1
GDH2
PDC1 term
pTPI1
STB5
FBA1 term
FCY 3' Flank
FCY1::pENO pGPM1
15711 bp

Figure 17

Figure 18

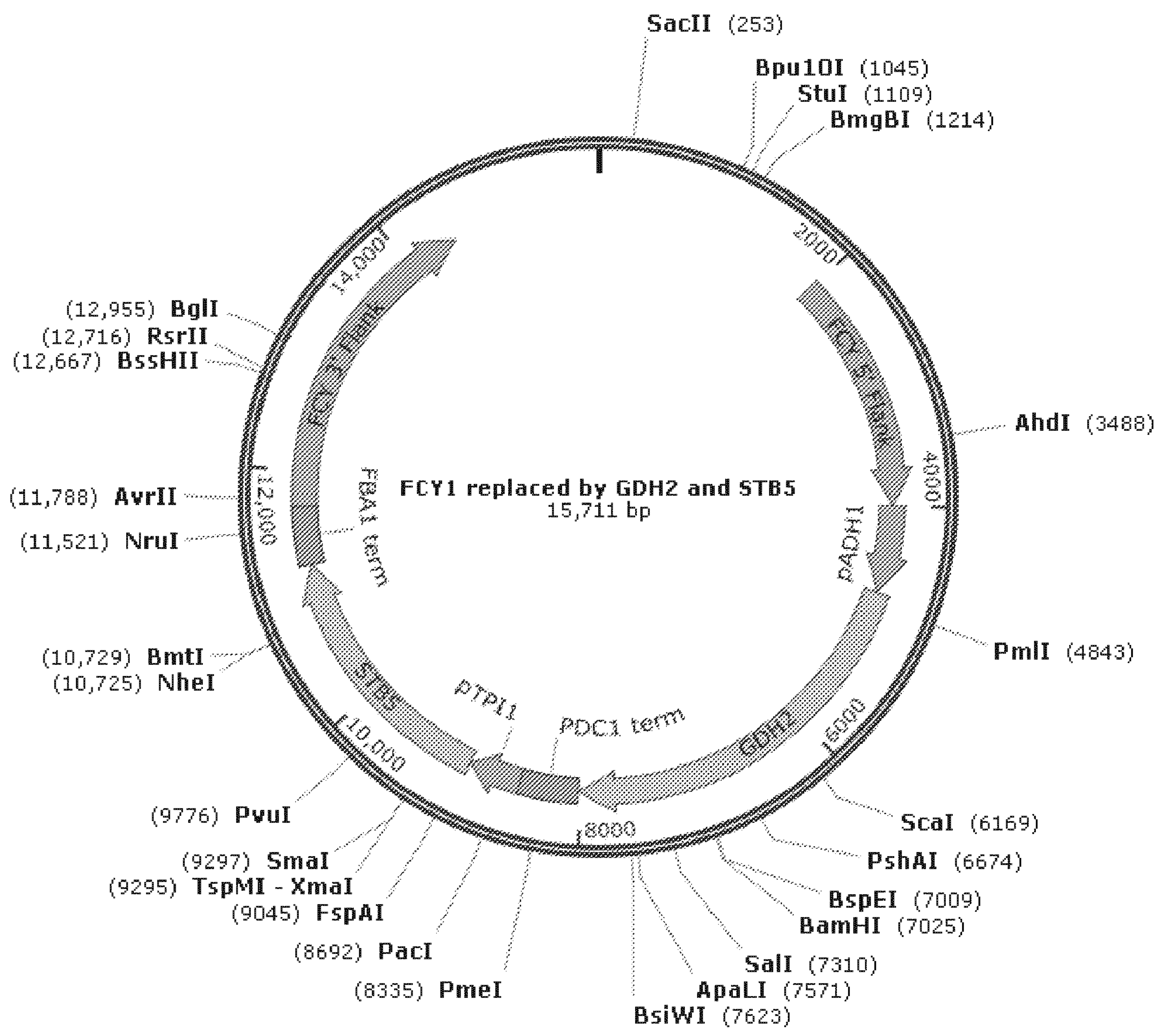
FCY1 replaced by GDH2 and STB5
15,711 bp
SacII (253)
Bpu10I (1045)
StuI (1109)
BmgBI (1214)
AhdI (3488)
PmlI (4843)
ScaI (6169)
PshAI (6674)
BspEI (7009)
BamHI (7025)
SalI (7310)
ApaLI (7571)
BsiWI (7623)
(8335) PmeI
(8692) PacI
(9045) FspAI
(9295) TspMI - XmaI
(9297) SmaI
(9776) PvuI
(10,725) NheI
(10,729) BmtI
(11,521) NruI
(11,788) AvrII
(12,667) BssHII
(12,716) RsrII
(12,955) BglI
FCY 5' Flank
pADH1
GDH2
PDC1 term
pTPI1
STB5
FBA1 term
FCY 3' Flank
2000
4000
6000
8000
10,000
12,000
14,000

Figure 19

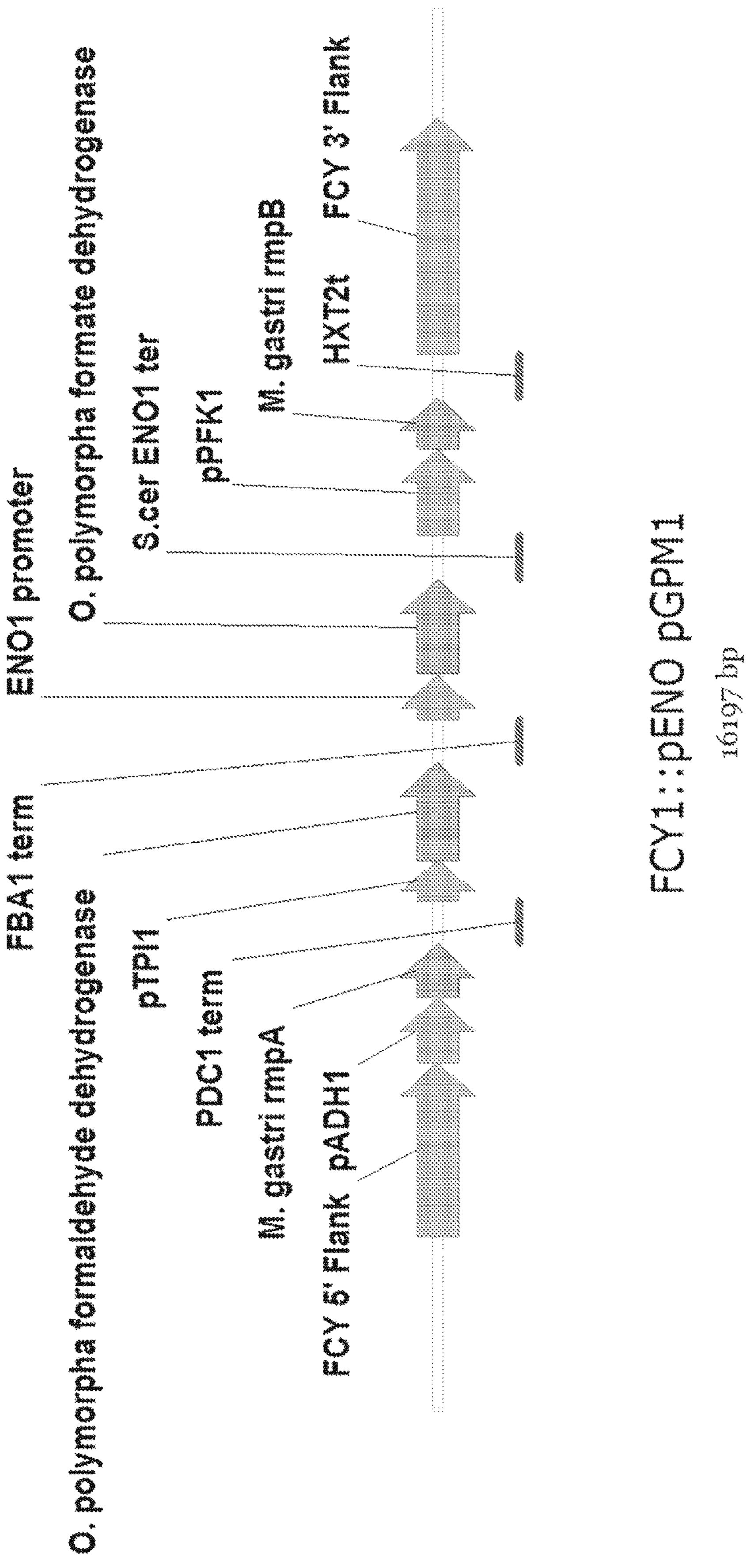
FCY 5' Flank
pADH1
M. gastri rmpA
PDC1 term
pTPI1
O. polymorpha formaldehyde dehydrogenase
FBA1 term
ENO1 promoter
O. polymorpha formate dehydrogenase
S.cer ENO1 ter
pPFK1
M. gastri rmpB
HXT2t
FCY 3' Flank
FCY1::pENO pGPM1
16197 bp

Figure 20

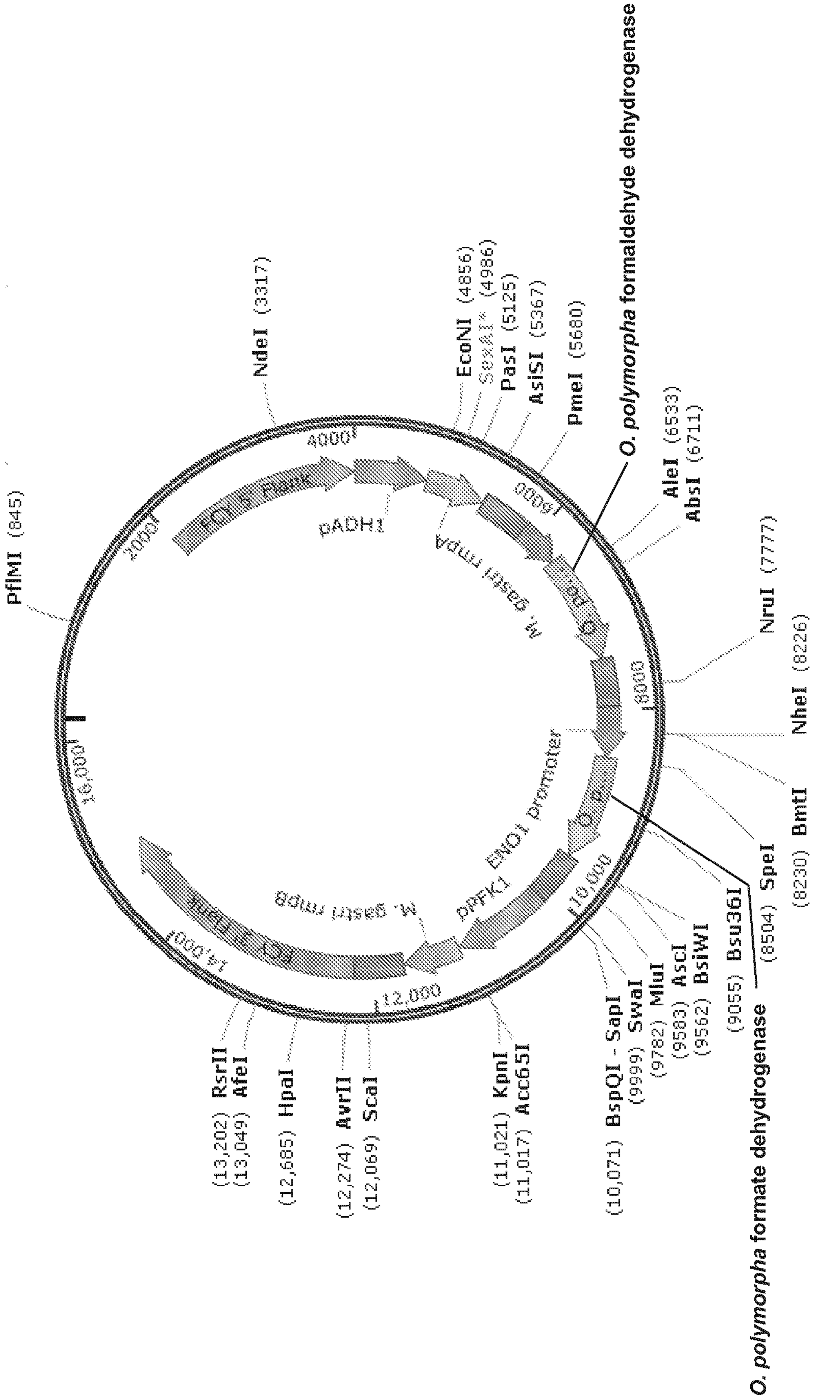


FCY1 replaced by Mg-rmpA Mg-rmpB OpFLD OpFMD
16,197 bp

Figure 21

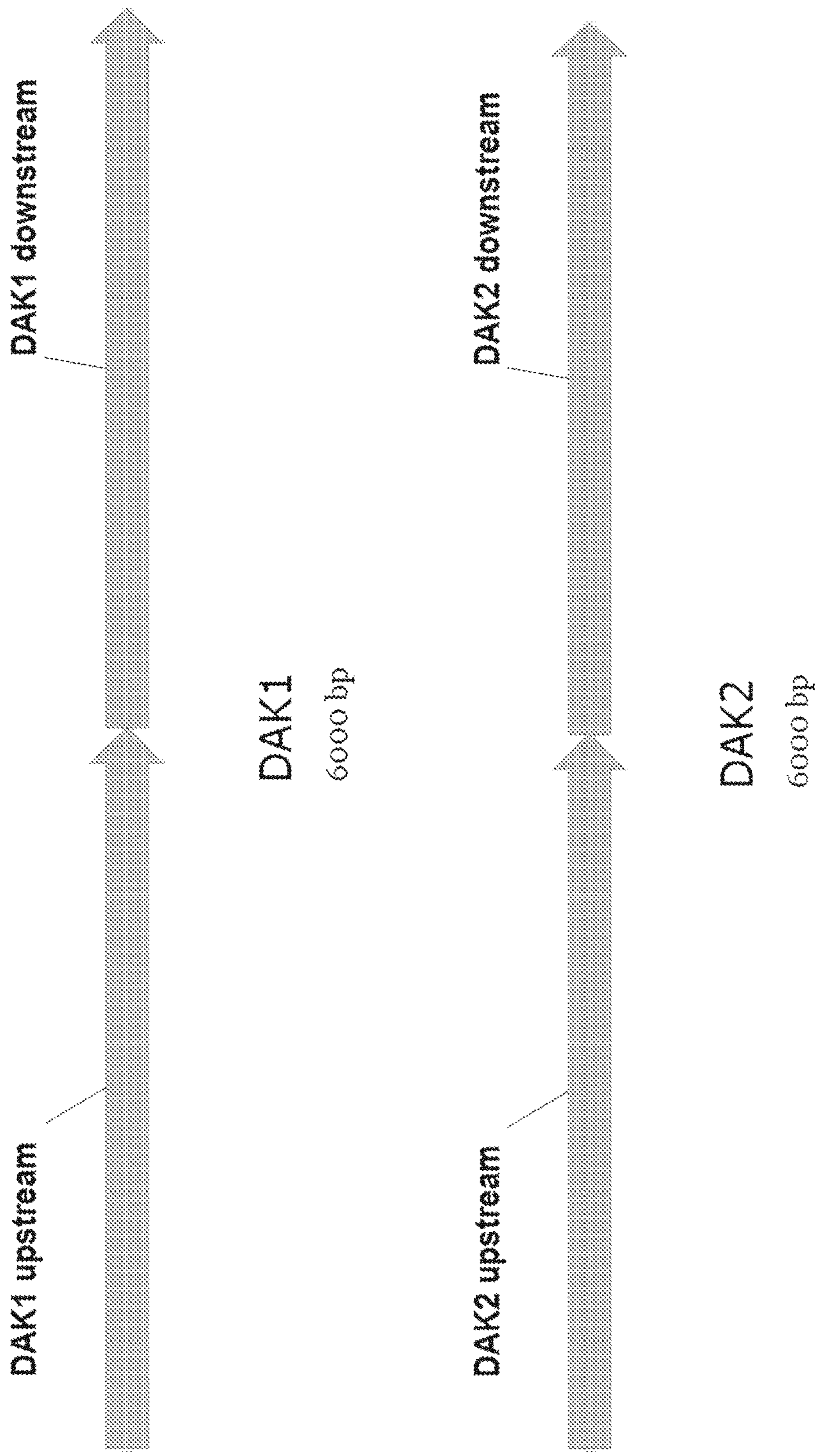
DAK1 upstream
DAK1 downstream
DAK1
6000 bp
DAK2 upstream
DAK2 downstream
DAK2
6000 bp

Figure 22

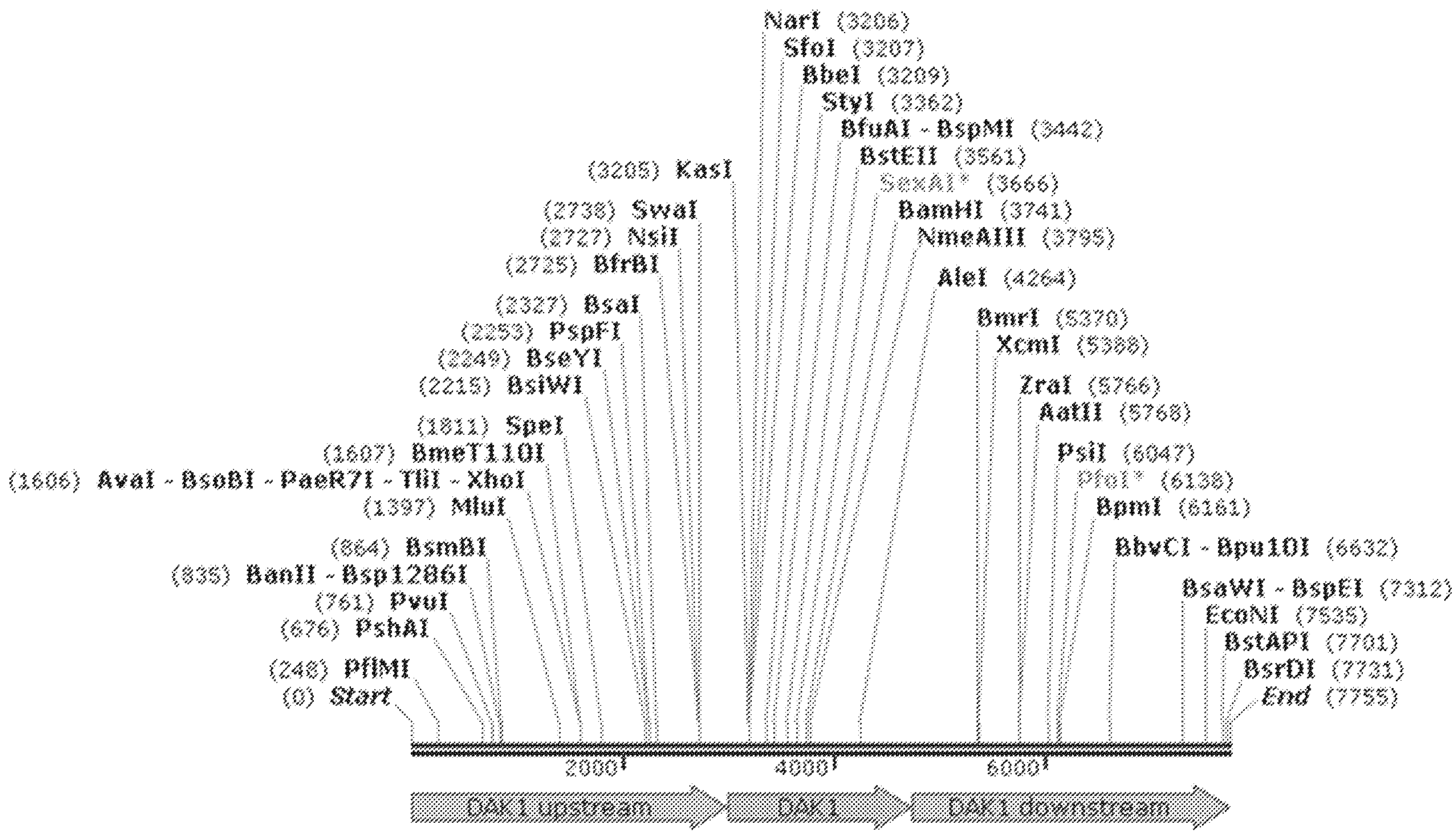


DAK1 plus-min 3 kb
7755 bp

Figure 23

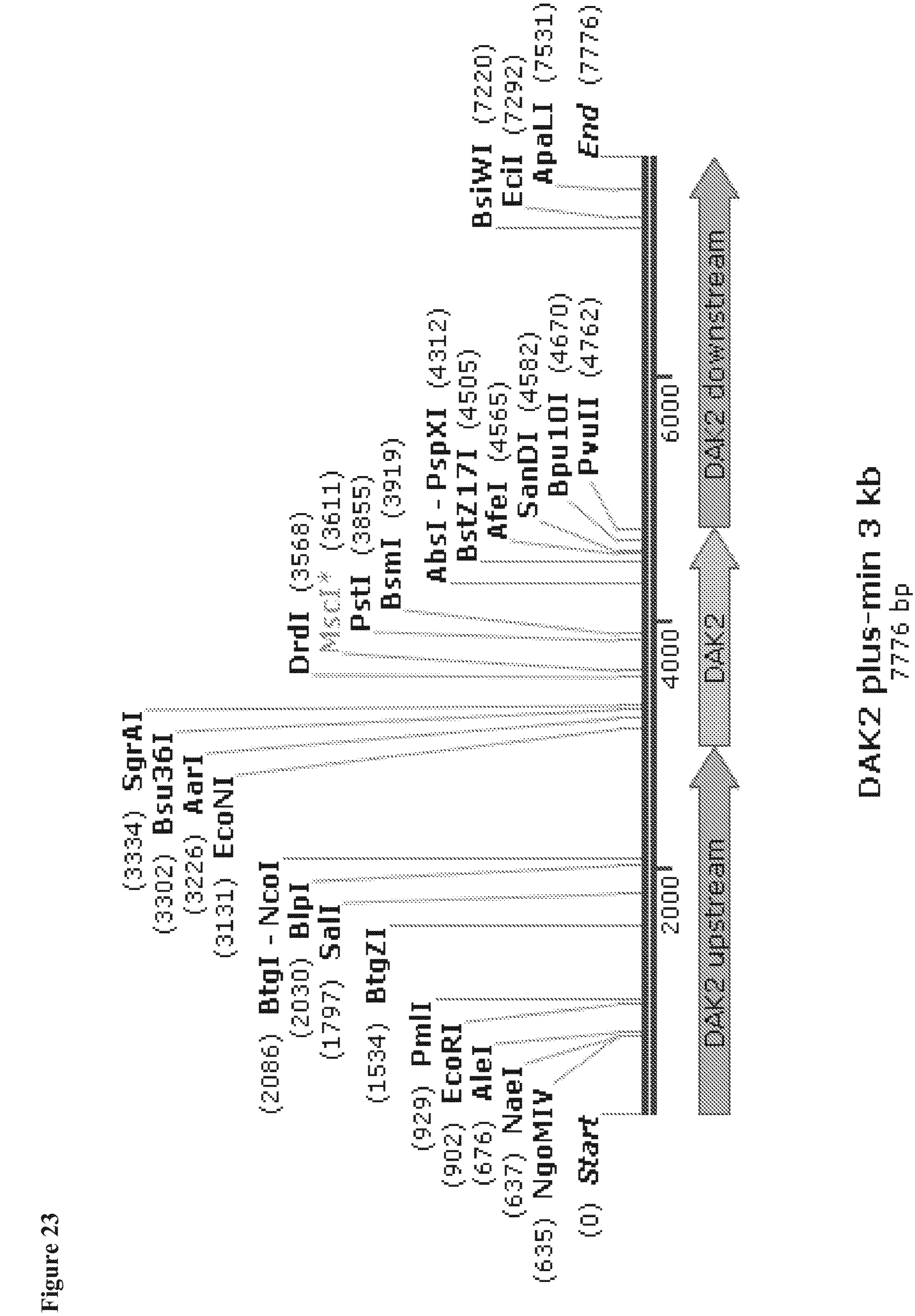
(0) Start
(635) NgoMIV
(637) NaeI
(676) AleI
(902) EcoRI
(929) PmlI
(1534) BtgZI
(1797) SalI
(2030) BlpI
(2086) BtgI - NcoI
(3131) EcoNI
(3226) AarI
(3302) Bsu36I
(3334) SgrAI
DrdI (3568)
MscI* (3611)
PstI (3855)
BsmI (3919)
AbsI - PspXI (4312)
BstZ17I (4505)
AfeI (4565)
SanDI (4582)
Bpu10I (4670)
PvuII (4762)
BsiWI (7220)
EciI (7292)
ApaLI (7531)
End (7776)
2000
4000
6000
DAK2 upstream
DAK2
DAK2 downstream
DAK2 plus-min 3 kb
7776 bp

Figure 24

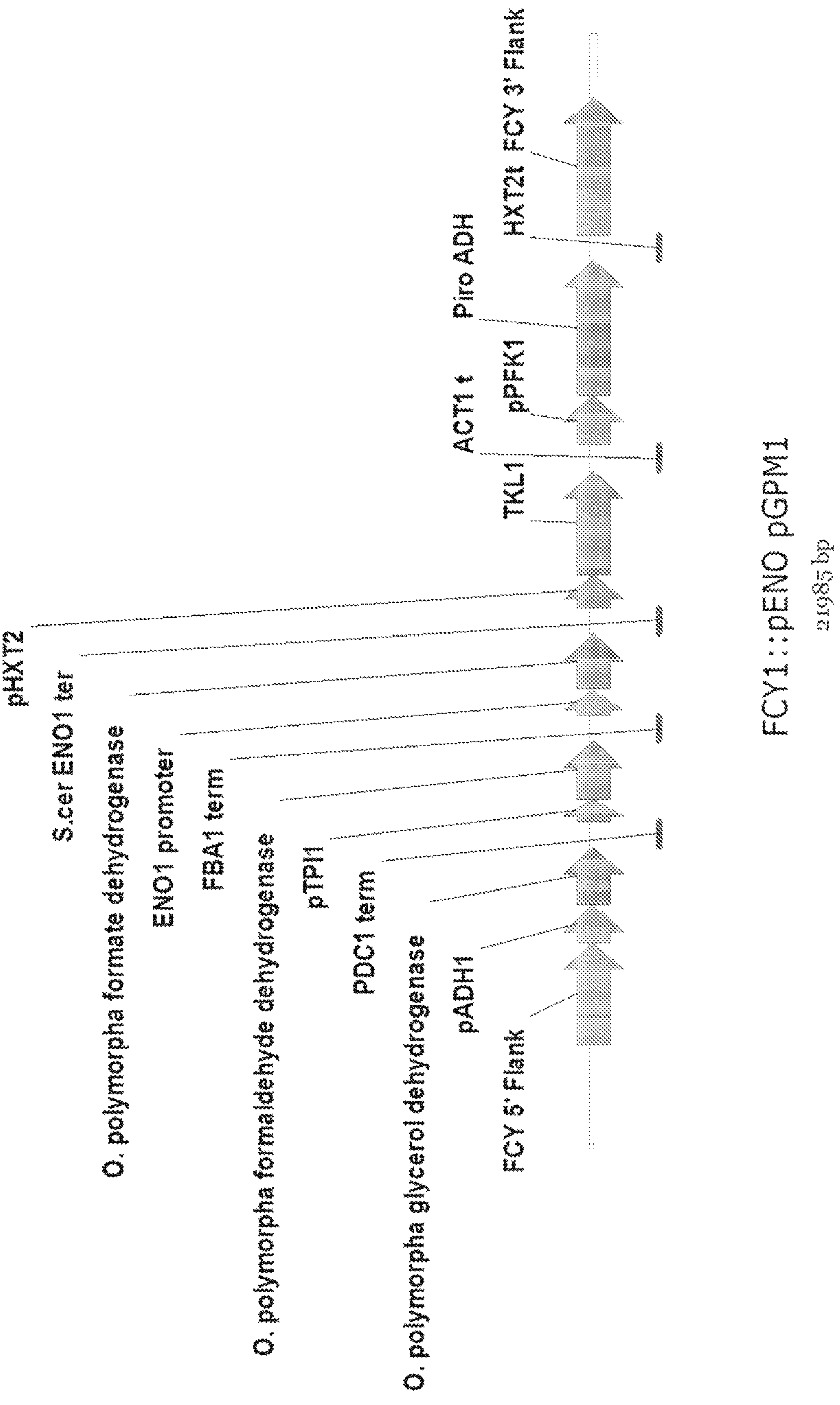
FCY 5' Flank
pADH1
O. polymorpha glycerol dehydrogenase
PDC1 term
pTPI1
O. polymorpha formaldehyde dehydrogenase
FBA1 term
ENO1 promoter
O. polymorpha formate dehydrogenase
S.cer ENO1 ter
pHXT2
TKL1
ACT1 t
pPFK1
Piro ADH
HXT2t
FCY 3' Flank
FCY1::pENO pGPM1
21985 bp

Figure 25

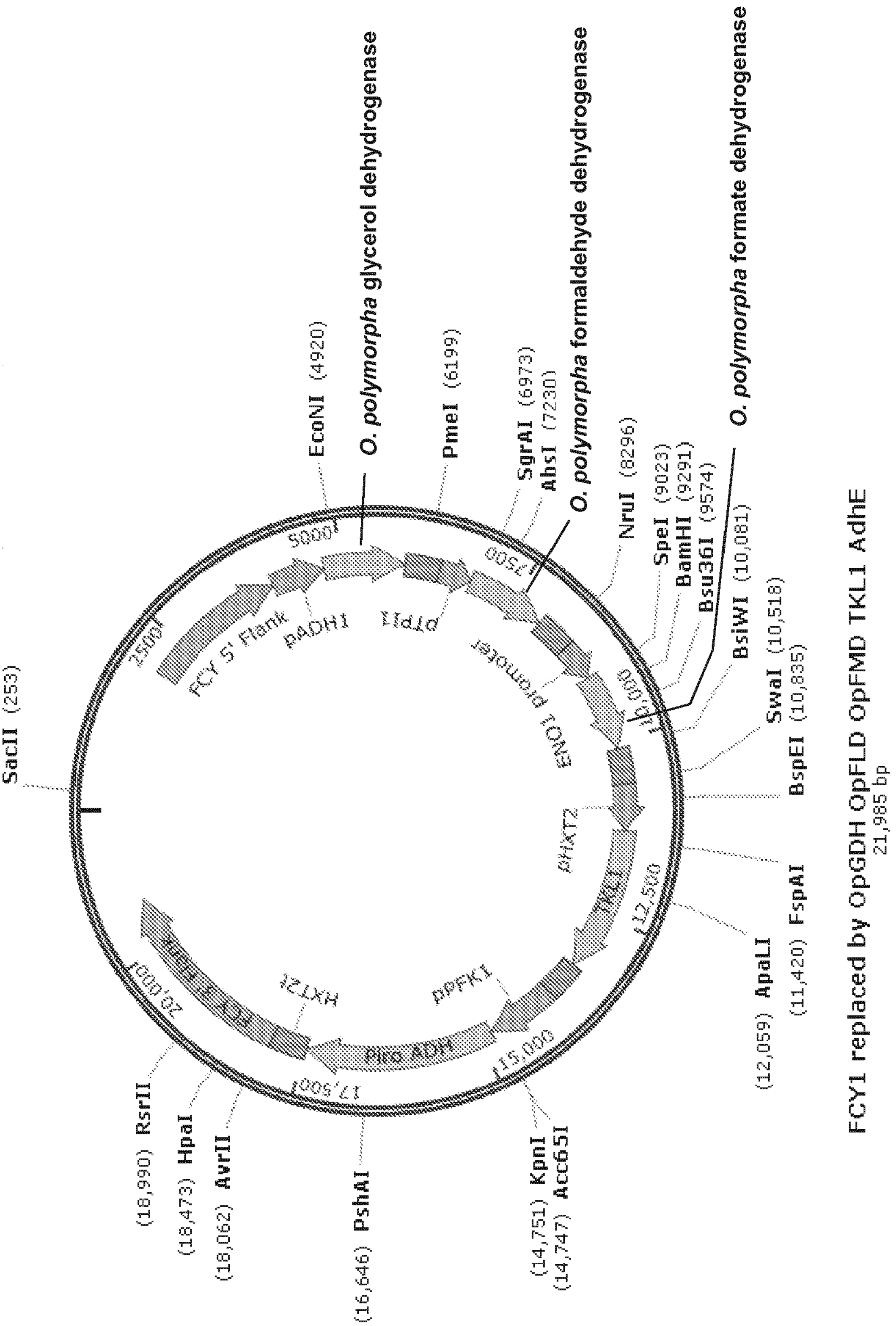
SacII (253)
EcoNI (4920)
O. polymorpha glycerol dehydrogenase
PmeI (6199)
SgrAI (6973)
AhsI (7230)
O. polymorpha formaldehyde dehydrogenase
NruI (8296)
SpeI (9023)
BamHI (9291)
Bsu36I (9574)
O. polymorpha formate dehydrogenase
BsiWI (10,081)
SwaI (10,518)
BspEI (10,835)
FspAI (11,420)
ApaLI (12,059)
(14,751) KpnI
(14,747) Acc65I
(16,646) PshAI
(18,062) AvrII
(18,473) HpaI
(18,990) RsrII
FCY 5' Flank
pADH1
pTPI1
ENO1 promoter
tHXT2
TKL1
pPFK1
Piro ADH
HXT2t
FCY 3' Flank
2500
5000
7500
10,000
12,500
15,000
17,500
20,000
FCY1 replaced by OpGDH OpFLD OpFMD TKL1 AdhE
21,985 bp

Figure 26

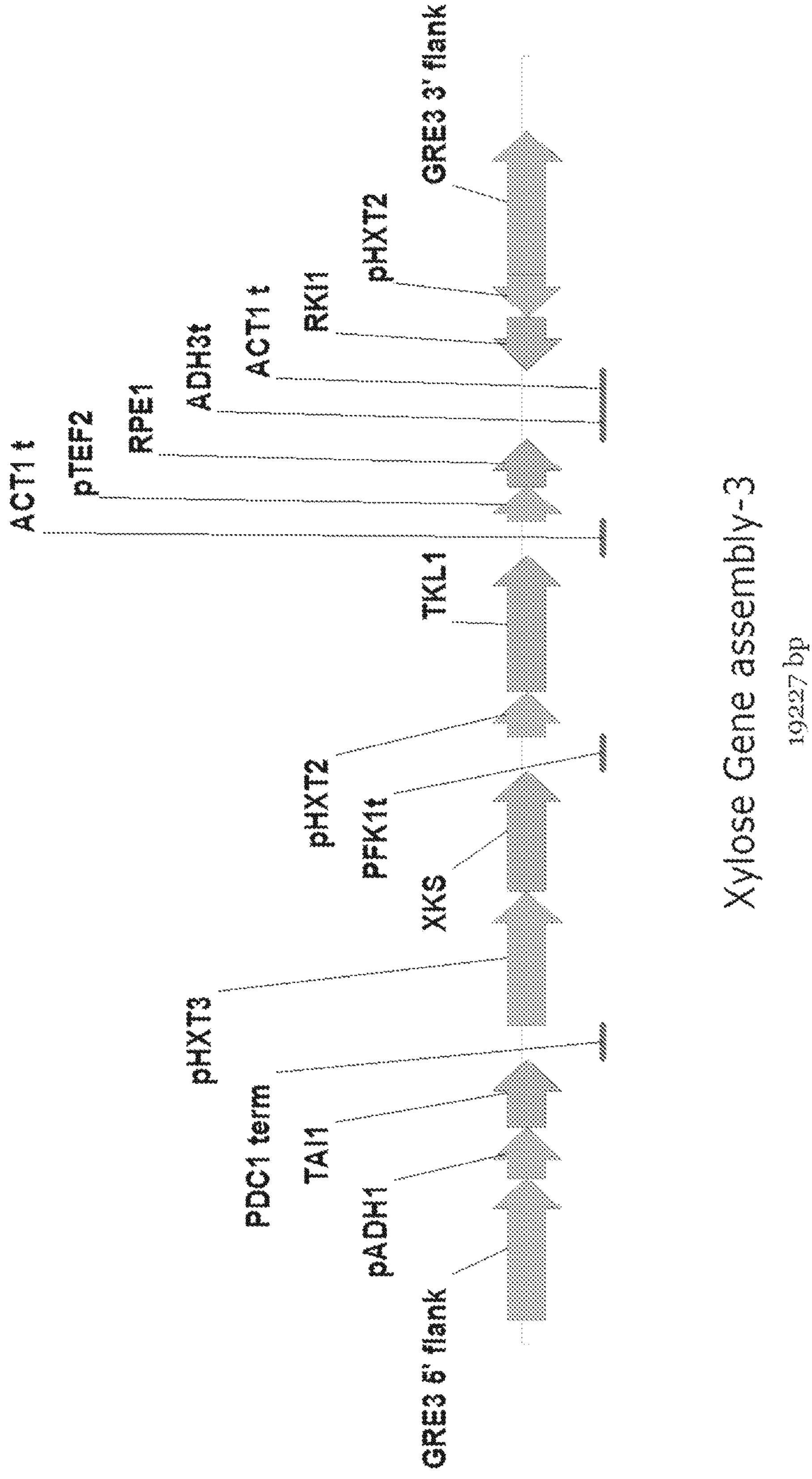
GRE3 5' flank
pADH1
PDC1 term
TAI1
pHXT3
XKS
PFK1t
pHXT2
TKL1
ACT1 t
pTEF2
RPE1
ADH3t
ACT1 t
RKI1
pHXT2
GRE3 3' flank
Xylose Gene assembly-3
19227 bp

Figure 27

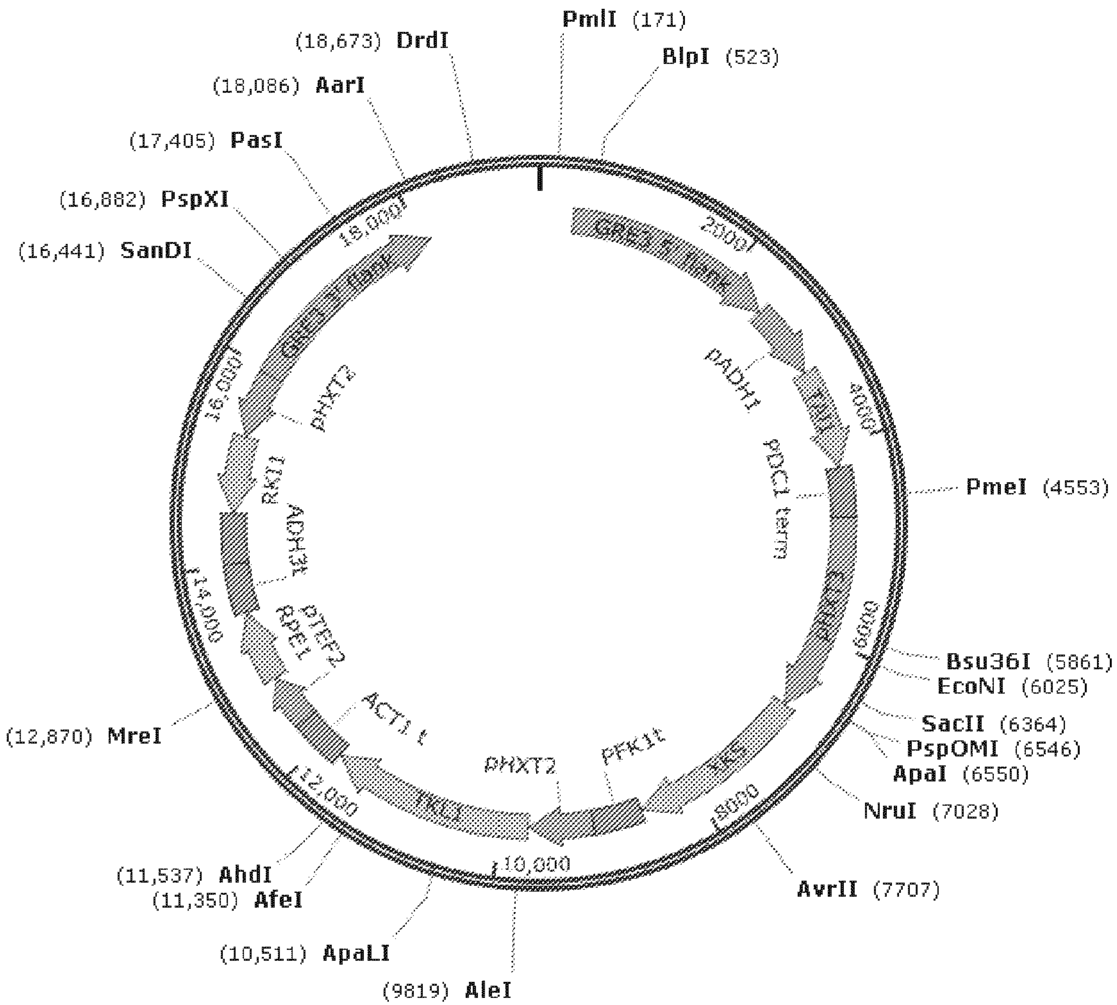


GRE3 replaced by TAL1 XKS1 TKL1 RPE1 and RKI1
19,227 bp

Figure 28

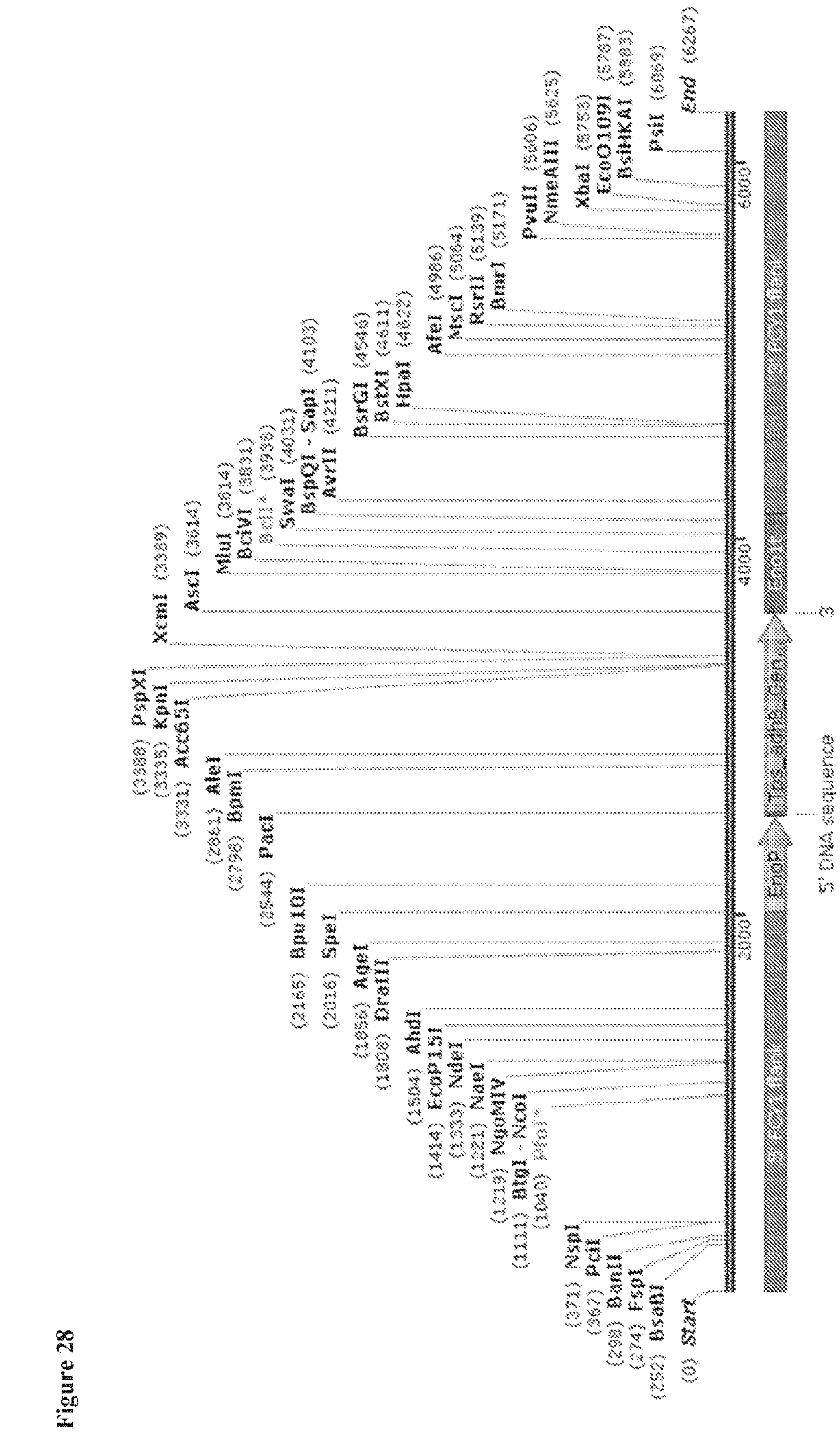


M4596 FCY1 integrated site
6267 bp

Figure 29

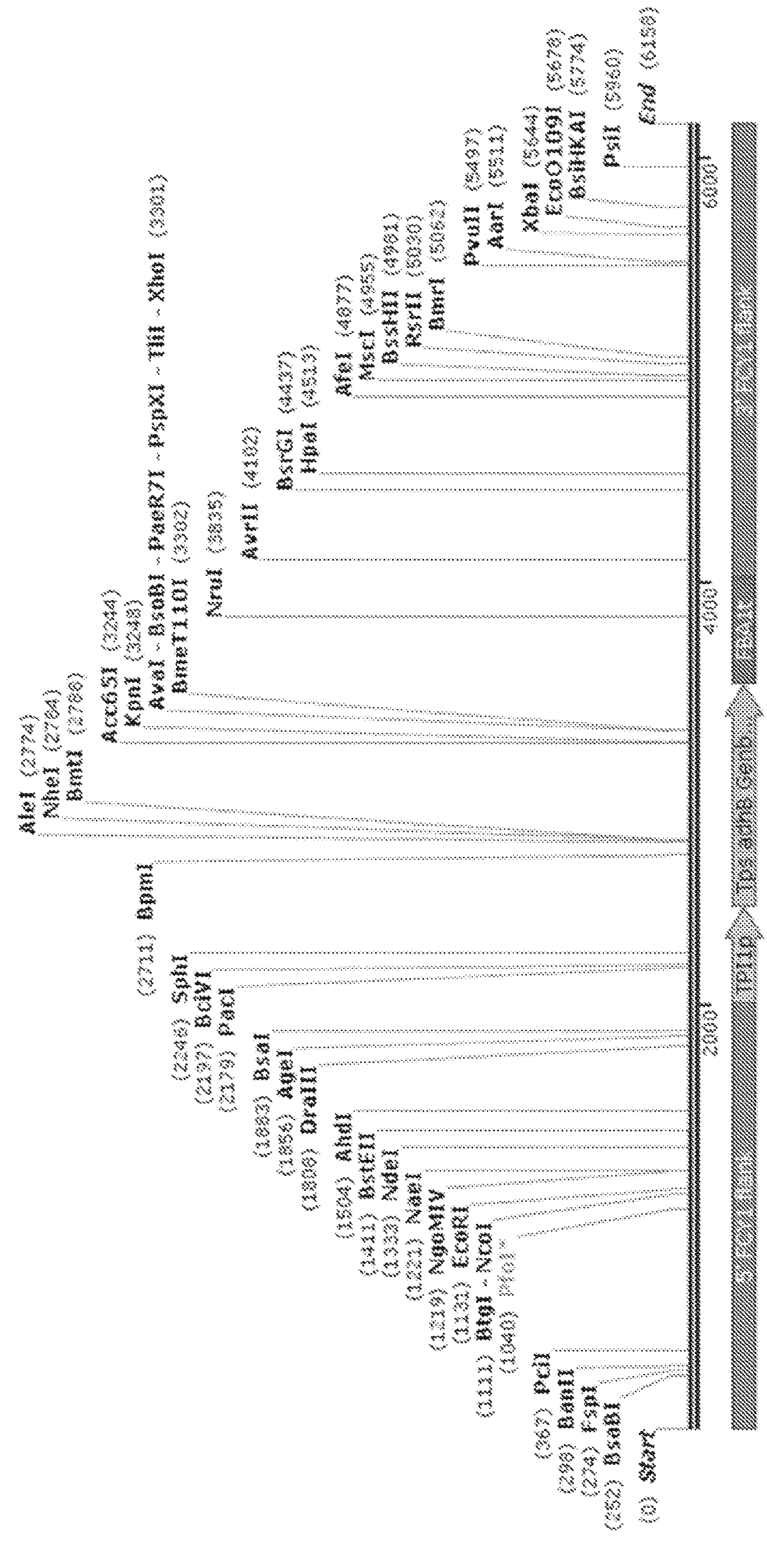


M4598 FCY1 integrated site
6158 bp

Figure 30

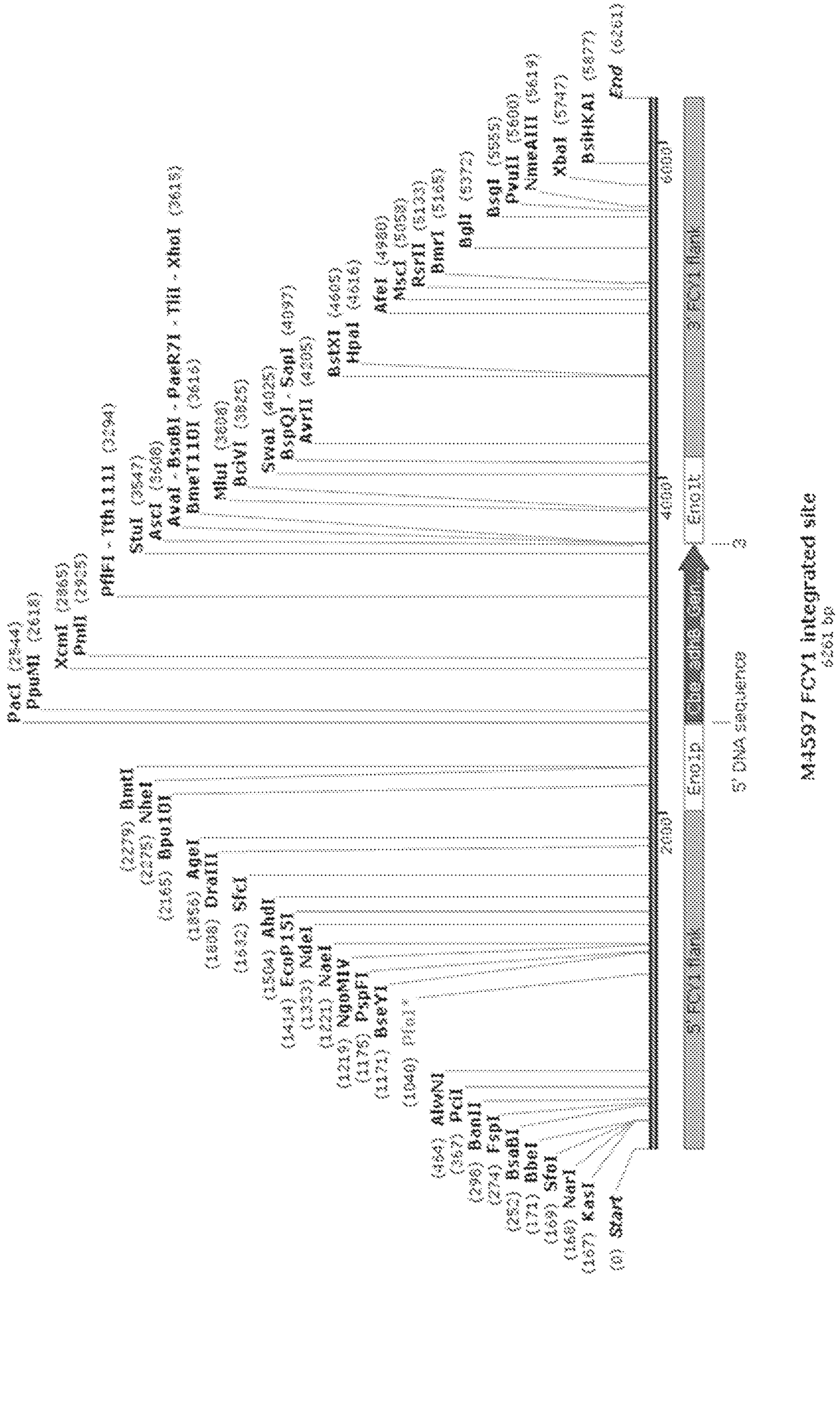
(0) Start
(167) KasI
(168) NarI
(169) SfoI
(171) BbeI
(250) BsaBI
(274) FspI
(296) BanII
(367) PciI
(464) AlwNI
(1040) PfoI*
(1171) BseYI
(1176) PspFI
(1219) NgoMIV
(1221) NaeI
(1393) NdeI
(1414) EcoP15I
(1504) AhdI
(1632) SfcI
(1808) DraIII
(1856) AgeI
(2165) Bpu10I
(2278) NheI
(2279) BmtI
PacI (2544)
PpuMI (2618)
XcmI (2865)
PmlI (2905)
PflFI - Tth111I (3294)
StuI (3547)
AscI (3606)
AvaI - BsoBI - PaeR7I - TliI - XhoI (3615)
BmeT110I (3616)
MluI (3808)
BciVI (3825)
SwaI (4025)
BspQI - SapI (4097)
AvrII (4205)
BstXI (4605)
HpaI (4616)
AfeI (4980)
MscI (5058)
RsrII (5133)
BmrI (5165)
BglI (5372)
BsgI (5555)
PvuII (5600)
NmeAIII (5619)
XbaI (5747)
BstHKAI (5877)
End (6261)
2000
4000
6000
5' FCY1 flank
Eno1p
Eno1t
3' FCY1 flank
5' DNA sequence
3
M4597 FCY1 integrated site
6261 bp

Figure 31

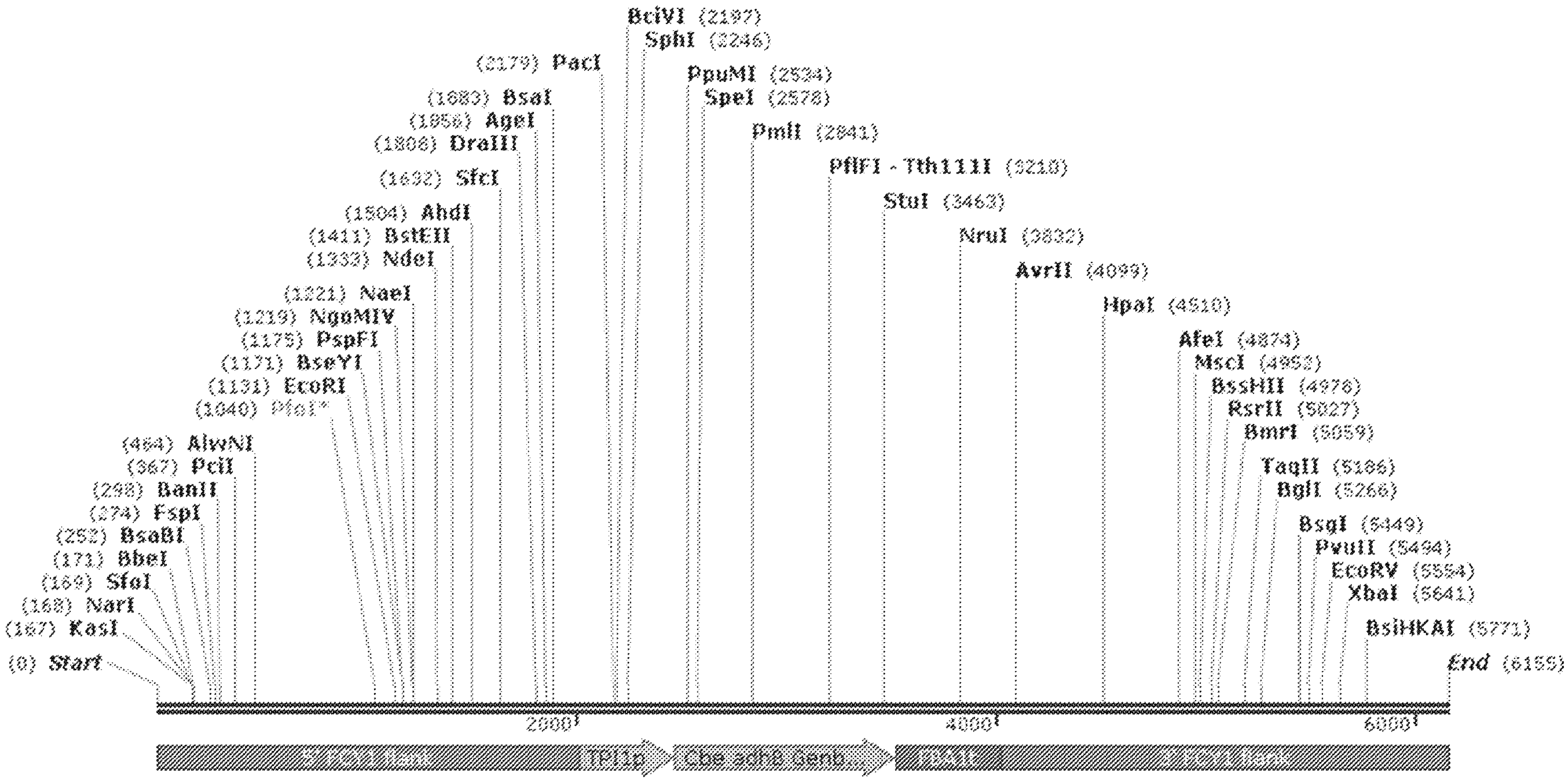


M4599 FCY1 integrated site
6155 bp

Figure 32

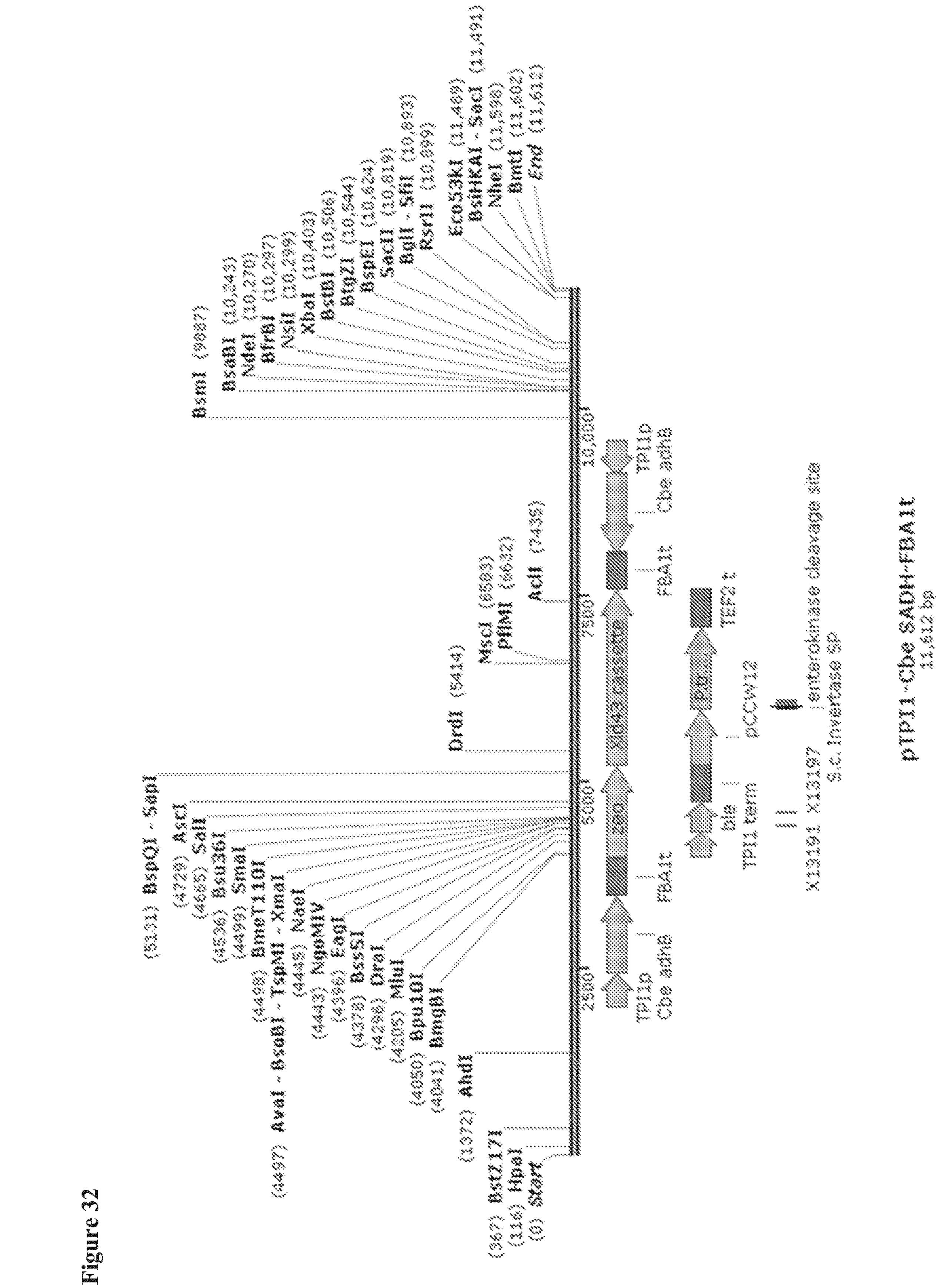

(0) Start
(116) HpaI
(367) BstZ17I
(1372) AhdI
(4041) BmgBI
(4050) Bpu10I
(4205) MluI
(4296) DraI
(4378) BssSI
(4396) EagI
(4443) NgoMIV
(4445) NaeI
(4497) AvaI - BsoBI - TspMI - XmaI
(4498) BmeT110I
(4499) SmaI
(4536) Bsu36I
(4665) SalI
(4729) AscI
(5331) BspQI - SapI
DrdI (5414)
MscI (6583)
PflMI (6632)
AclI (7435)
BsmI (9887)
BsaBI (10,243)
NdeI (10,270)
BfrBI (10,287)
NsiI (10,299)
XbaI (10,403)
BstBI (10,506)
BtgZI (10,544)
BspEI (10,624)
SacII (10,819)
BglI - SfiI (10,893)
RsrII (10,899)
Eco53kI (11,469)
BsiHKAI - SacI (11,491)
NheI (11,596)
BmtI (11,602)
End (11,612)
2500
5000
7500
10,000
zeo
X043 cassette
TPI1p
Cbe adhB
FBA1t
TPI1 term
bla
X13191
X13197
pCCW12
S.c. Invertase SP
enterokinase cleavage site
TEF2 t
pTPI1-Cbe SADH-FBA1t
11,612 bp

Figure 33

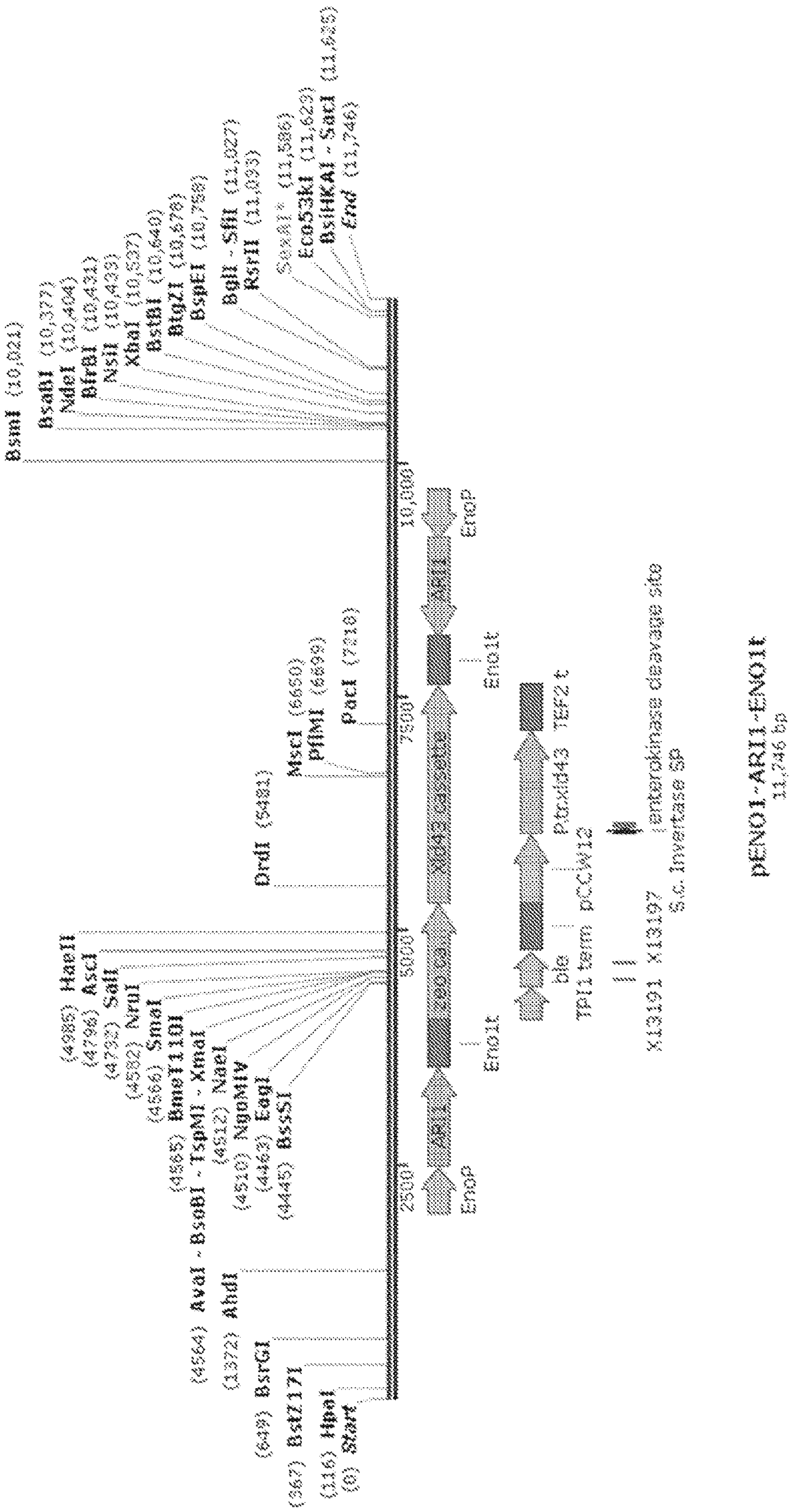

(0) Start
(116) HpaI
(367) BstZ17I
(649) BsrGI
(1372) AhdI
(4564) AvaI - BsoBI - TspMI - XmaI
(4565) BmeT110I
(4566) SmaI
(4582) NruI
(4732) SalI
(4796) AscI
(4985) HaeII
(4512) NaeI
(4510) NgoMIV
(4463) EagI
(4445) BssSI
DrdI (5481)
MscI (6650)
PflMI (6699)
PacI (7210)
BsmI (10,021)
BsaBI (10,377)
NdeI (10,404)
BfrBI (10,431)
NsiI (10,433)
XbaI (10,537)
BstBI (10,640)
BtgZI (10,676)
BspEI (10,758)
BglI - SfiI (11,027)
RsrII (11,093)
SexAI* (11,586)
Eco53kI (11,623)
BsiHKAI - SacI (11,625)
End (11,746)
2500
5000
7500
10,000
EnoP
ARI1
Eno1t
zeo cas
XID43 cassette
Eno1t
XRI1
EnoP
TPI1 term
ble
pCCW12
Ptrxid43
TEF2 t
X13191
X13197
S.c. Invertase SP
enterokinase cleavage site
pENO1-ARI1-ENO1t
11,746 bp

Figure 34

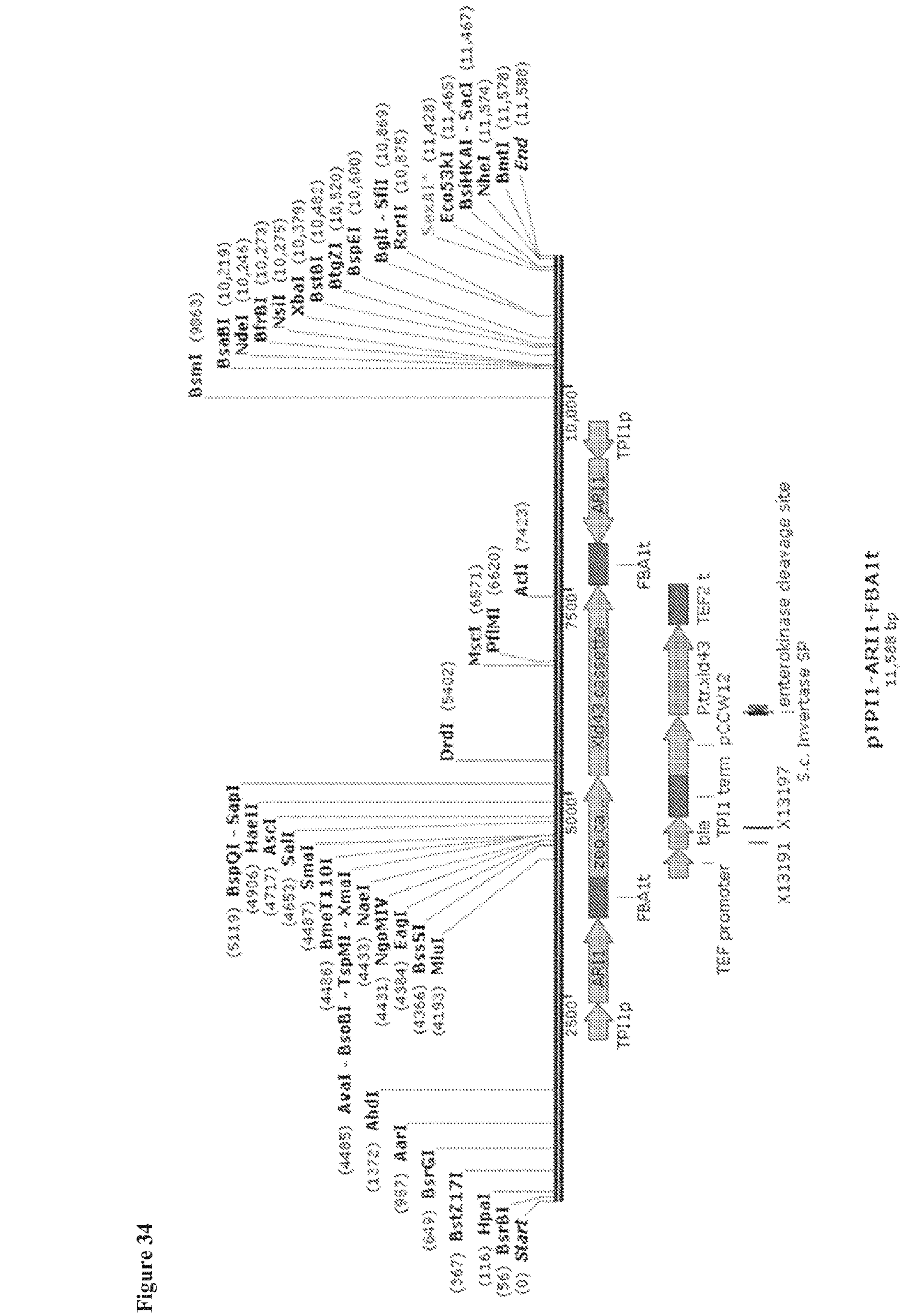
(0) Start
(56) BsrBI
(116) HpaI
(367) BstZ17I
(649) BsrGI
(957) AarI
(1372) AhdI
(4465) AvaI - BsoBI - TspMI - XmaI
(4466) BmeT110I
(4467) SmaI
(4653) SalI
(4717) AscI
(4906) HaeII
(5119) BspQI - SapI
(4433) NaeI
(4433) NgoMIV
(4384) EagI
(4366) BssSI
(4193) MluI
DrdI (5402)
MscI (6571)
PflMI (6620)
AclI (7423)
BsmI (9963)
BsaBI (10,219)
NdeI (10,246)
BfrBI (10,273)
NsiI (10,275)
XbaI (10,379)
BstBI (10,482)
BtgZI (10,520)
BspEI (10,600)
BglI - SfiI (10,849)
RsrII (10,875)
SexAI* (11,428)
Eco53kI (11,465)
BsiHKAI - SacI (11,467)
NheI (11,574)
BmtI (11,578)
End (11,588)
2500
5000
7500
10,000
TPI1p
ARI1
FBA1t
zeo cas
xld43 cassette
FBA1t
ARI1
TPI1p
TEF promoter
ble
TPI1 term
pCCW12
Ptr.xld43
TEF2 t
X13191
X13197
S.c. Invertase SP
enterokinase cleavage site
pTPI1-ARI1-FBA1t
11,588 bp

# Figure 35

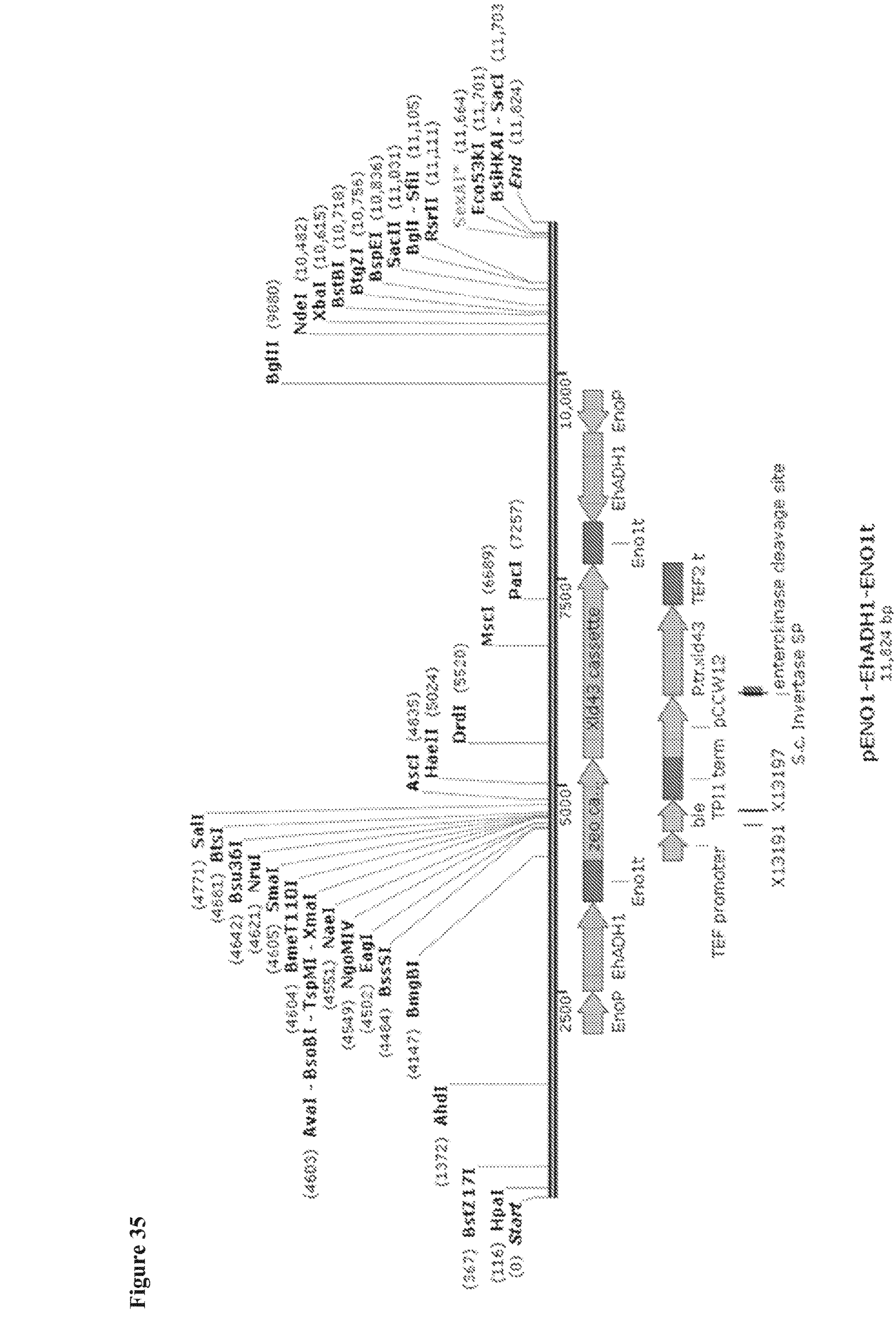

(0) Start
(116) HpaI
(367) BstZ17I
(1372) AhdI
(4147) BmgBI
(4464) BssSI
(4502) EagI
(4549) NgoMIV
(4551) NaeI
(4603) AvaI - BsoBI - TspMI - XmaI
(4604) BmeT110I
(4605) SmaI
(4621) NruI
(4642) Bsu36I
(4681) BtsI
(4771) SalI
AscI (4835)
HaeII (5024)
DrdI (5520)
MscI (6689)
PacI (7257)
BglII (9080)
NdeI (10,482)
XbaI (10,615)
BstBI (10,719)
BtgZI (10,756)
BspEI (10,836)
SacII (11,031)
BglI - SfiI (11,105)
RsrII (11,111)
SexAI* (11,664)
Eco53kI (11,701)
BsiHKAI - SacI (11,703)
End (11,824)
2500
5000
7500
10,000
EnoP
EhADH1
Eno1t
zeo CA
Xld43 cassette
Eno1t
EhADH1
EnoP
TEF promoter
ble
TP11 term
pCCW12
Ptr.xld43
TEF2 t
X13191
X13197
S.c. Invertase SP
enterokinase cleavage site
pENO1-EhADH1-ENO1t
11,824 bp

Figure 36

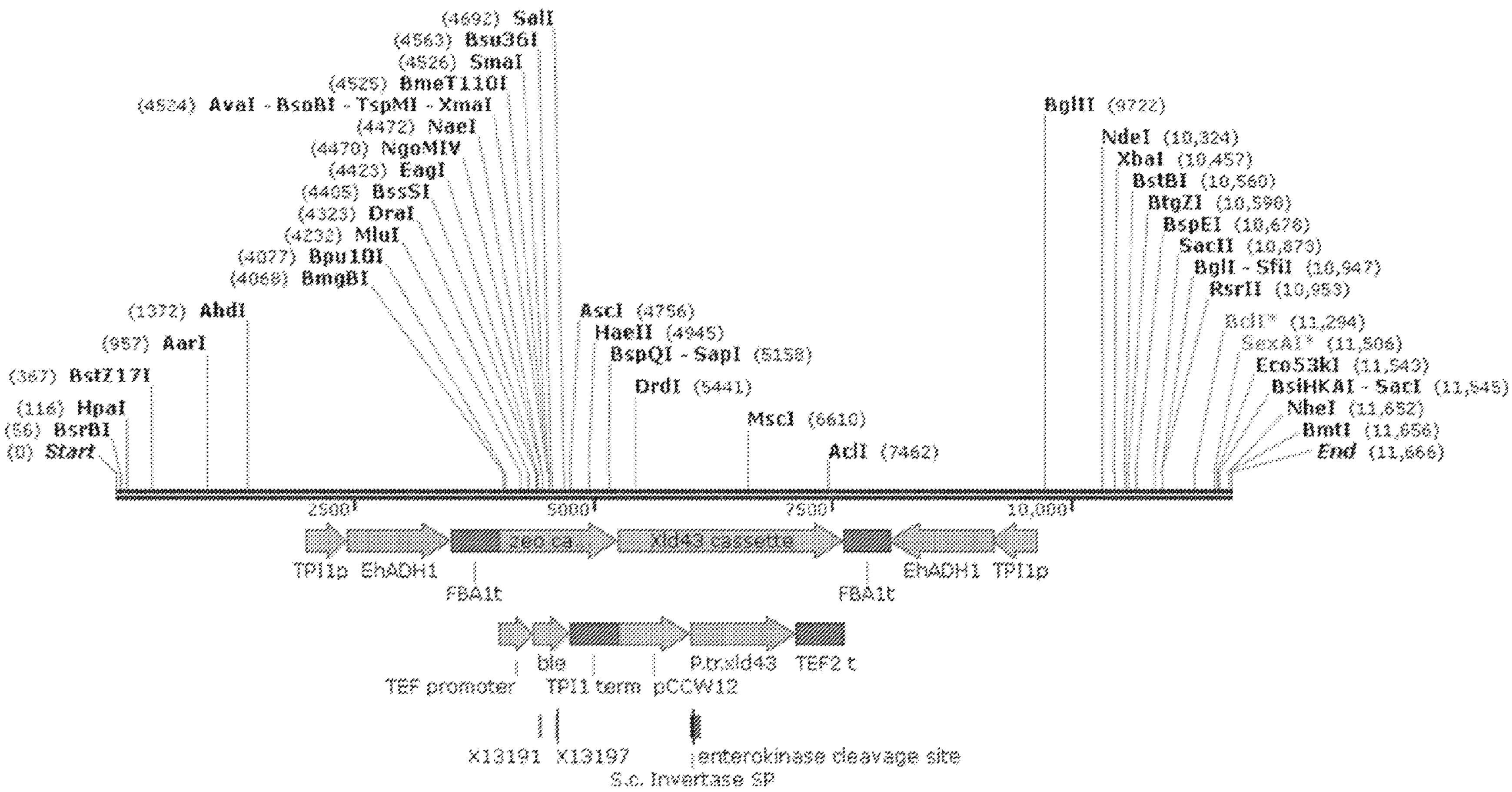


pTPI1-EhADH1-FBA1t
11,666 bp

Figure 37

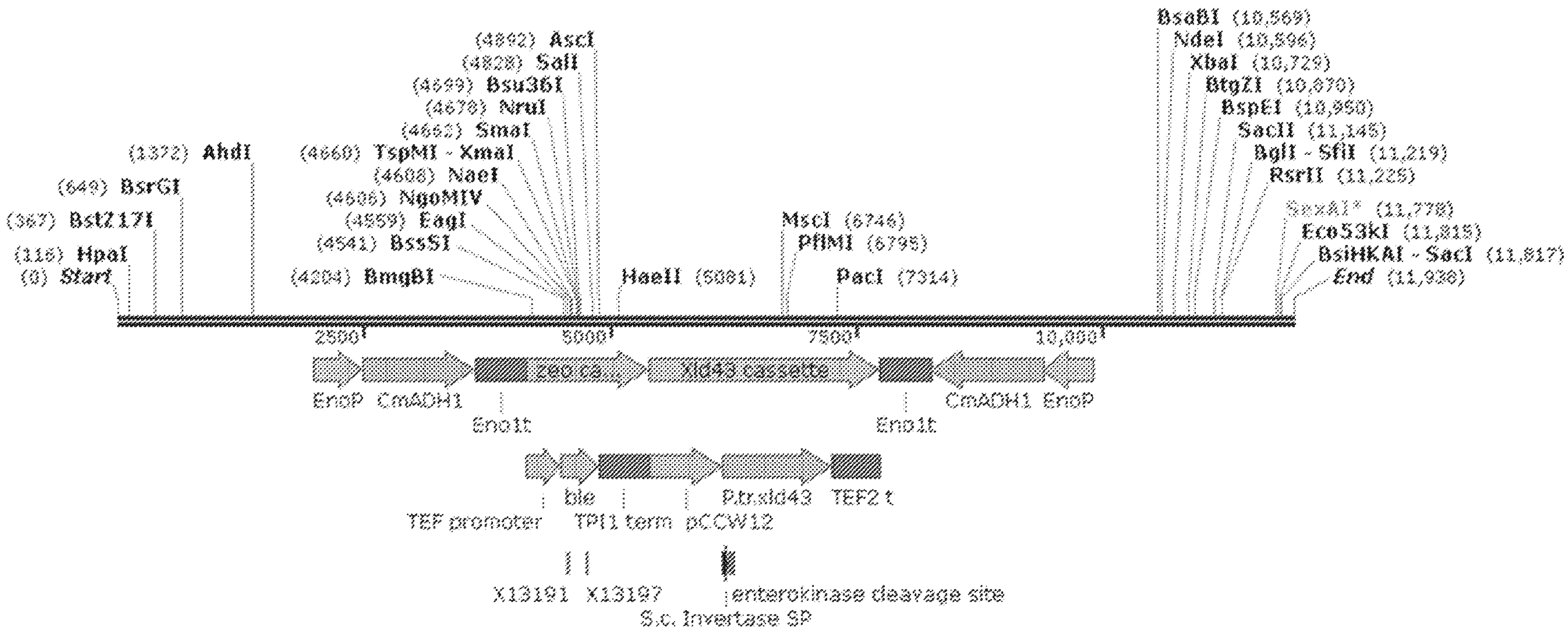


pENO1-CmADH1-ENO1t
11,936 bp

Figure 38

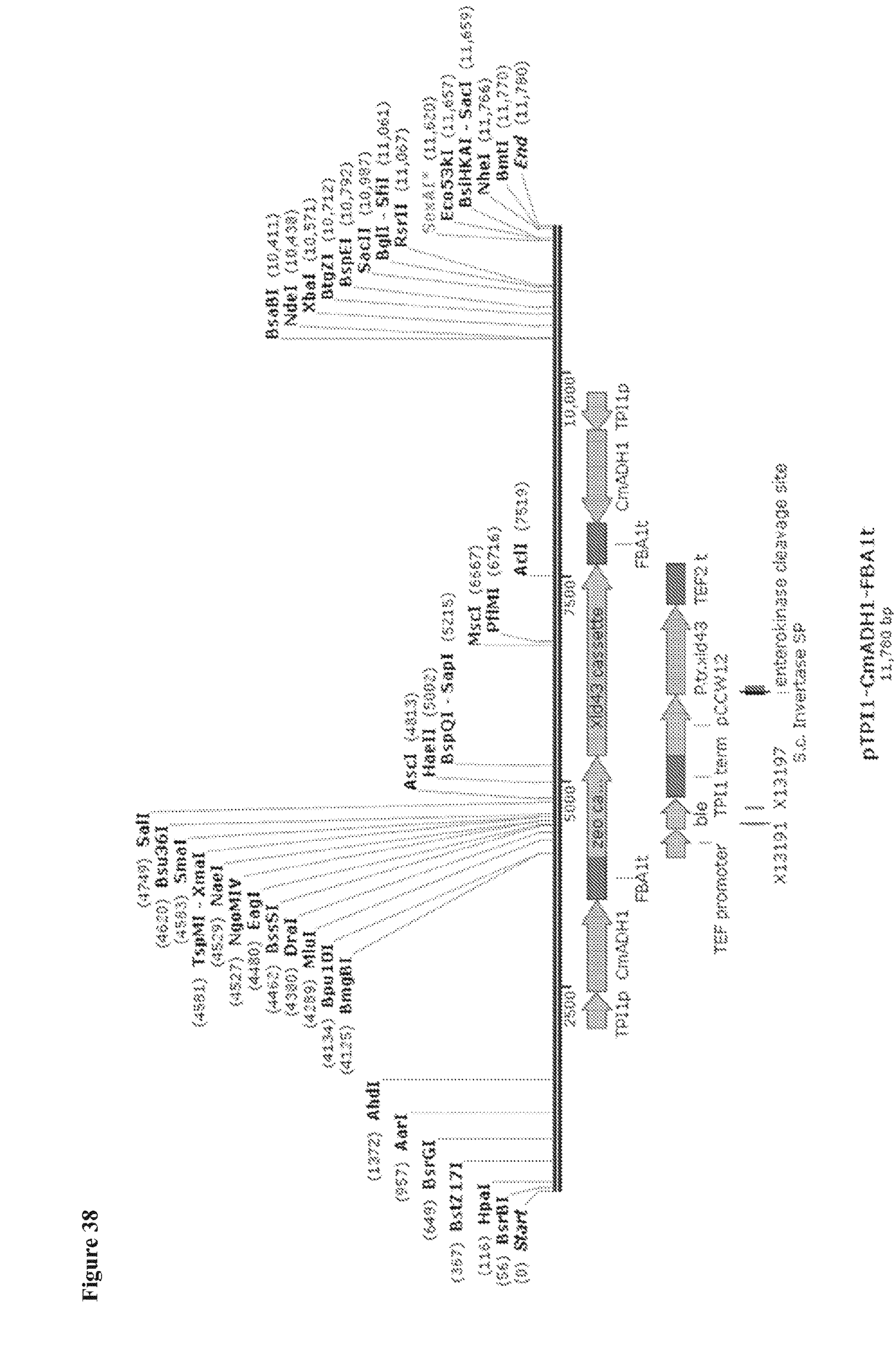

(0) Start
(56) BsrBI
(116) HpaI
(367) BstZ17I
(649) BsrGI
(957) AarI
(1372) AhdI
(4135) BmgBI
(4134) Bpu10I
(4289) MluI
(4380) DraI
(4462) BssSI
(4480) EagI
(4527) NgoMIV
(4529) NaeI
(4581) TspMI - XmaI
(4583) SmaI
(4620) Bsu36I
(4749) SalI
AscI (4813)
HaeII (5002)
BspQI - SapI (5215)
MscI (6667)
PflMI (6716)
AclI (7519)
BsaBI (10,411)
NdeI (10,438)
XbaI (10,571)
BtgZI (10,712)
BspEI (10,792)
SacII (10,987)
BglI - SfiI (11,061)
RsrII (11,067)
SexAI* (11,620)
Eco53kI (11,657)
BsiHKAI - SacI (11,659)
NheI (11,766)
BmtI (11,770)
End (11,760)
2500
5000
7500
10,000
TPI1p
CmADH1
FBA1t
zeo ca...
XD43 cassette
FBA1t
CmADH1
TPI1p
TEF promoter
ble
TPI1 term
pCCW12
Ptrxid43
TEF2 t
X13191
X13197
S.c. Invertase SP
enterokinase cleavage site
pTPI1-CmADH1-FBA1t
11,760 bp

Figure 39

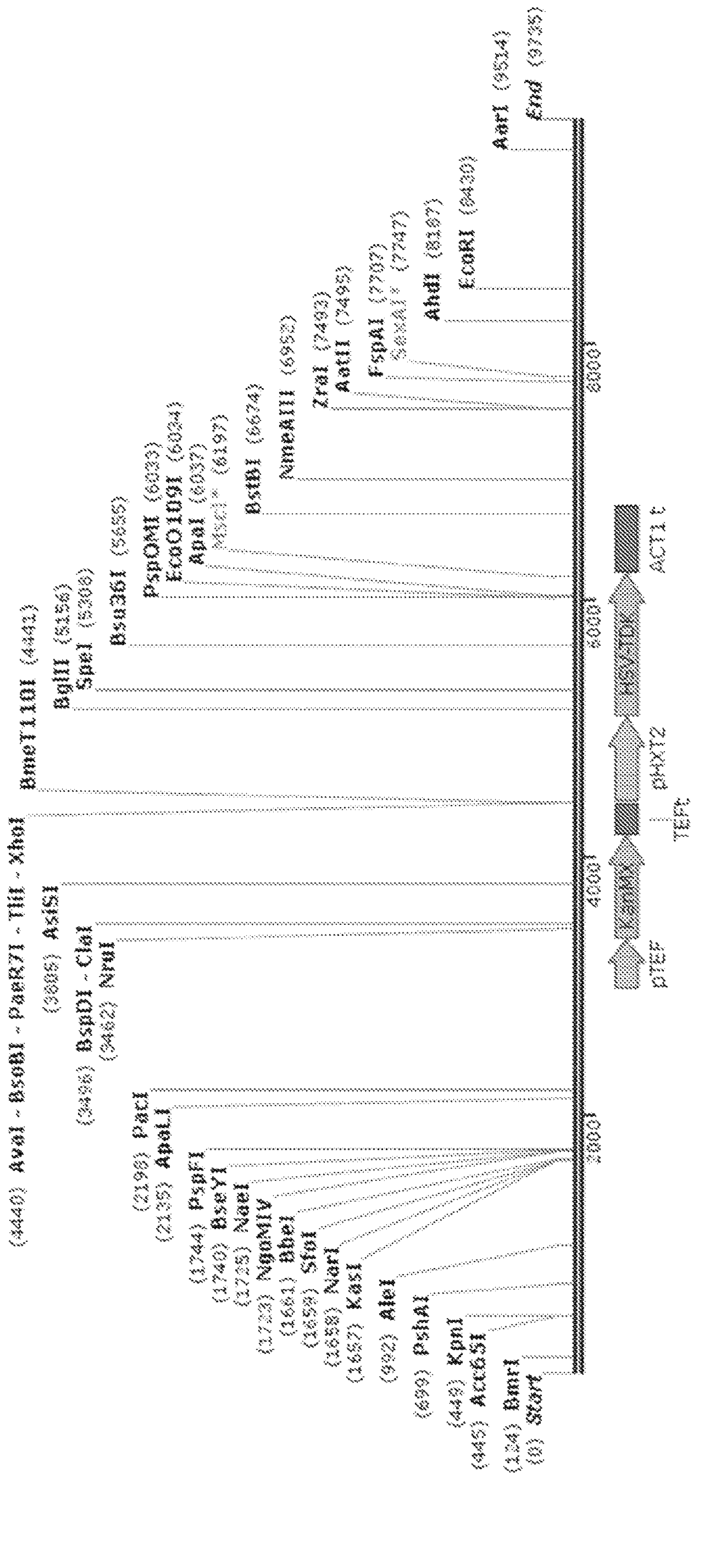


MA 668 - ADH1 deletion cassette G418
9735 bp

Figure 40

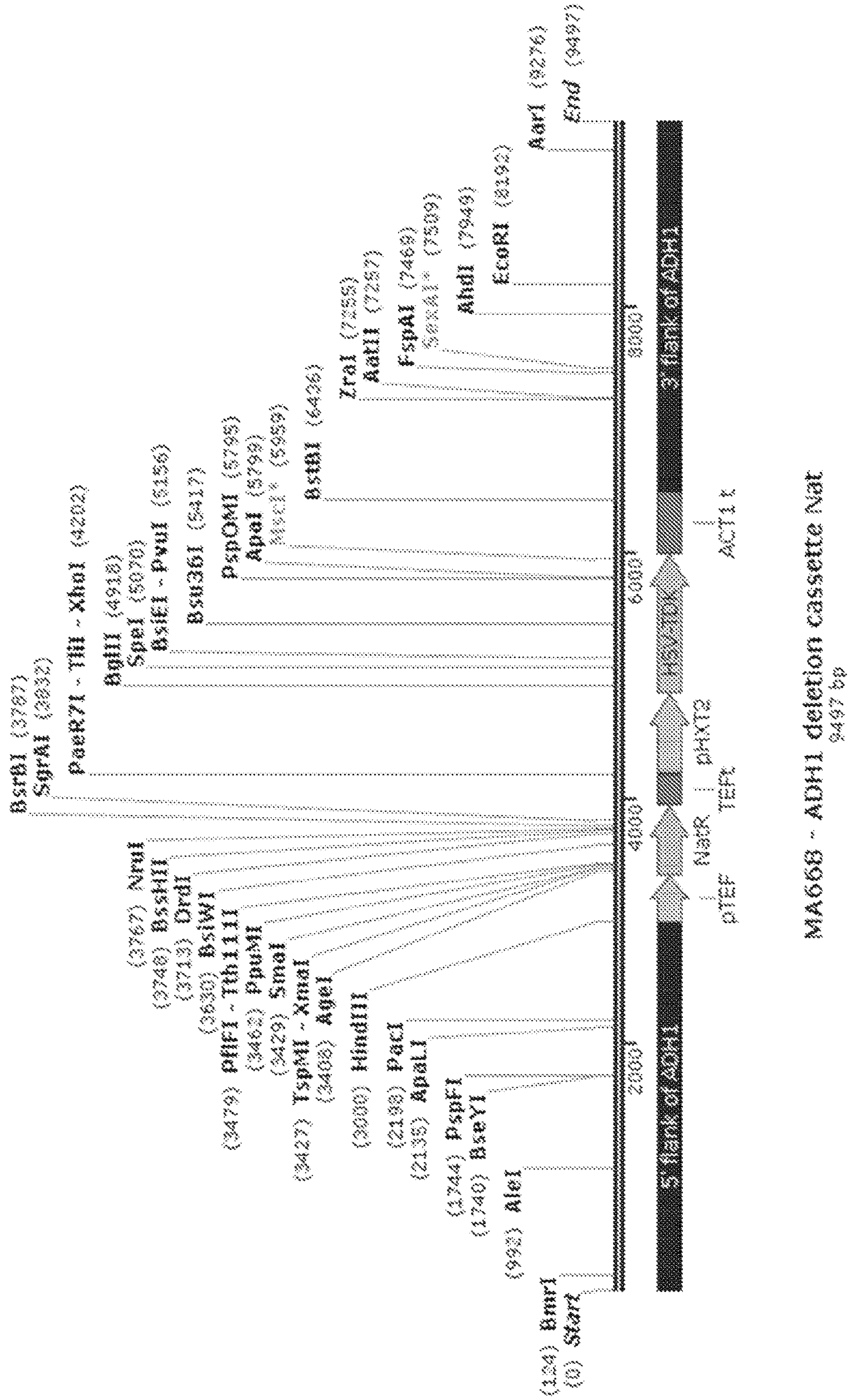
(0) Start
(134) BmrI
(993) AleI
(1740) BseYI
(1744) PspFI
(2135) ApaLI
(2198) PacI
(3000) HindIII
(3408) AgeI
(3427) TspMI - XmaI
(3429) SmaI
(3462) PpuMI
(3479) PflFI - Tth111I
(3630) BsiWI
(3713) OrdI
(3748) BssHII
(3767) NruI
BsrBI (3797)
SgrAI (3832)
PaeR7I - TliI - XhoI (4202)
BglII (4918)
SpeI (5070)
BsiEI - PvuI (5156)
Bsu36I (5417)
PspOMI (5795)
ApaI (5799)
MscI* (5959)
BstBI (6436)
ZraI (7255)
AatII (7257)
FspAI (7469)
SexAI* (7509)
AhdI (7949)
EcoRI (8192)
AarI (9276)
End (9497)
2000
4000
6000
8000
5' flank of ADH1
pTEF
NatR
TEFt
pHXT2
HSV-TDK
ACT1 t
3' flank of ADH1
MA668 - ADH1 deletion cassette Nat
9497 bp

Figure 41

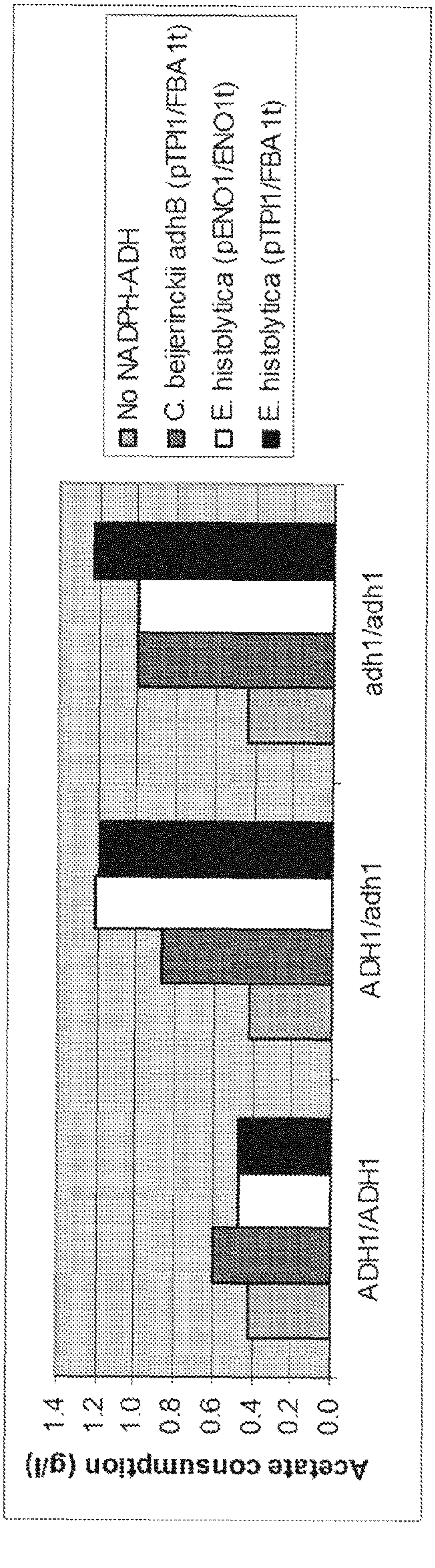
Acetate consumption (g/l)
1.4
1.2
1.0
0.8
0.6
0.4
0.2
0.0
ADH1/ADH1
ADH1/adh1
adh1/adh1
No NADPH-ADH
C. beijerinckii adhB (pTPI1/FBA1t)
E. histolytica (pENO1/ENO1t)
E. histolytica (pTPI1/FBA1t)

Figure 42

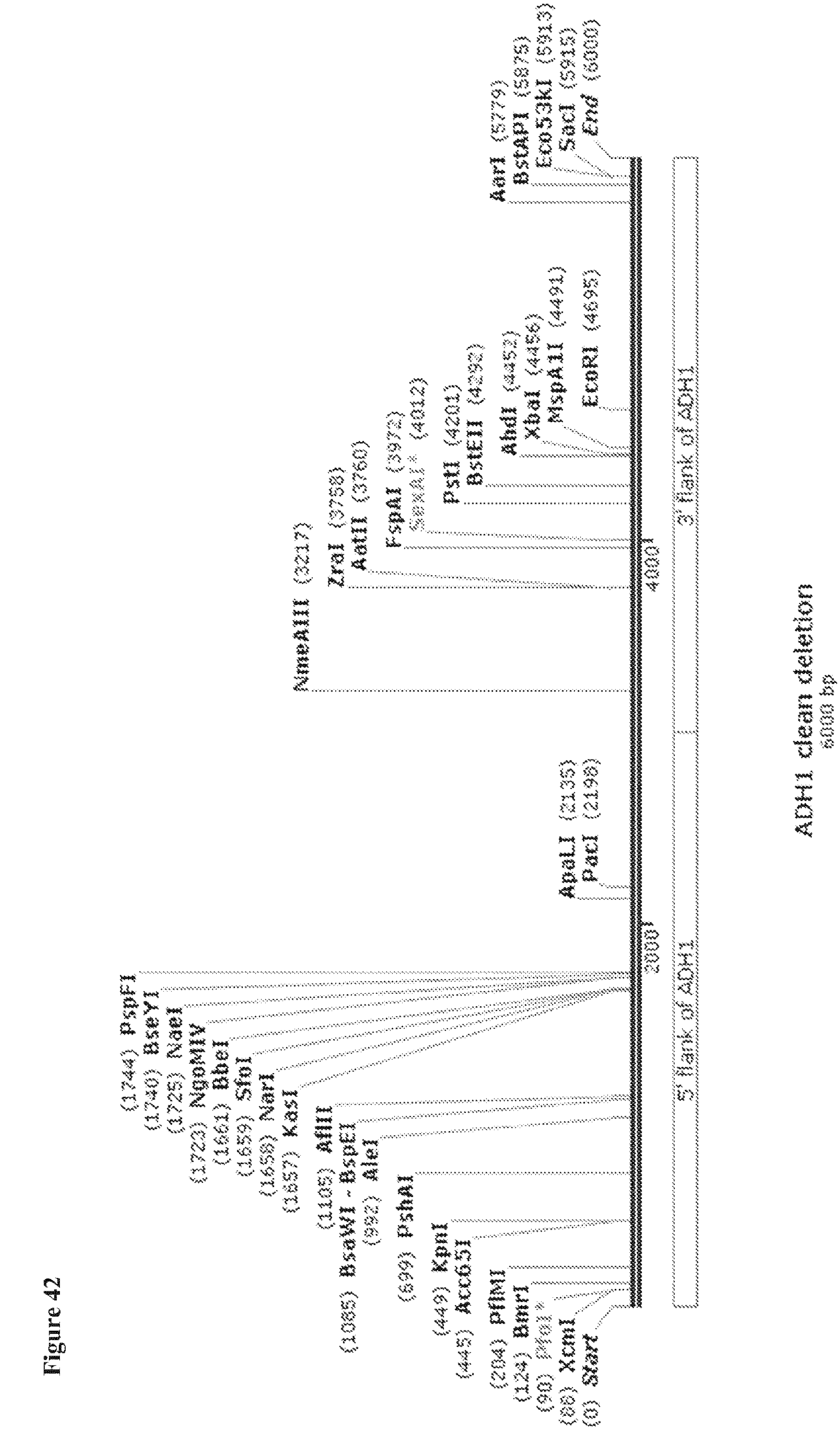
(0) Start
(88) XcmI
(90) PfoI*
(124) BmrI
(204) PflMI
(445) Acc65I
(449) KpnI
(699) PshAI
(992) AleI
(1085) BsaWI - BspEI
(1105) AflIII
(1657) KasI
(1658) NarI
(1659) SfoI
(1661) BbeI
(1723) NgoMIV
(1725) NaeI
(1740) BseYI
(1744) PspFI
ApaLI (2135)
PacI (2198)
NmeAIII (3217)
ZraI (3758)
AatII (3760)
FspAI (3972)
SexAI* (4012)
PstI (4201)
BstEII (4292)
AhdI (4452)
XbaI (4456)
MspA1I (4491)
EcoRI (4695)
AarI (5779)
BstAPI (5875)
Eco53kI (5913)
SacI (5915)
End (6000)
2000
4000
5' flank of ADH1
3' flank of ADH1
ADH1 clean deletion
6000 bp

Figure 43

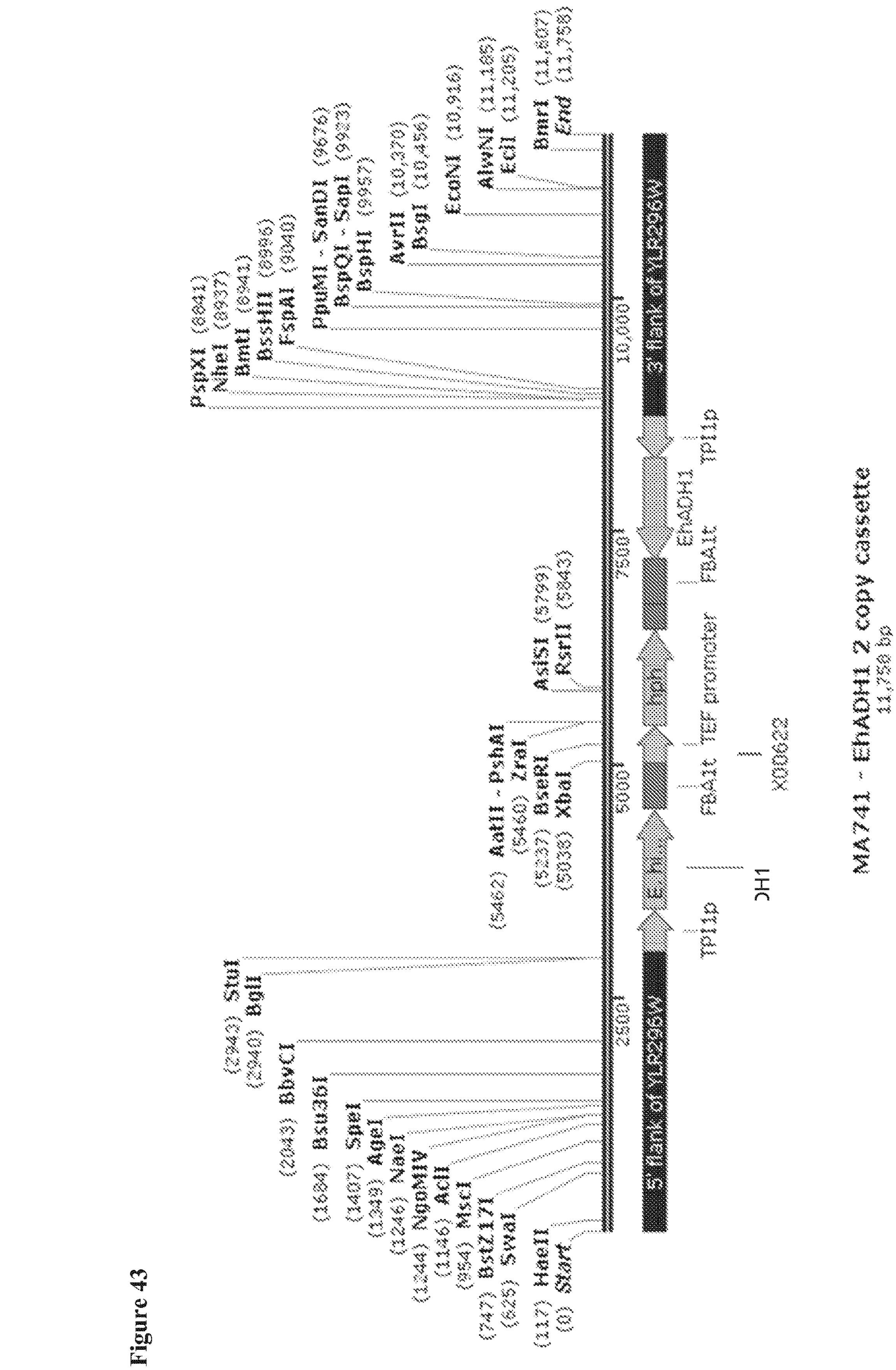

(0) Start
(117) HaeII
(625) SwaI
(747) BstZ17I
(954) MscI
(1146) AclI
(1244) NgoMIV
(1246) NaeI
(1249) AgeI
(1407) SpeI
(1684) Bsu36I
(2043) BbvCI
(2940) BglI
(2943) StuI
(5462) AatII - PshAI
(5460) ZraI
(5237) BseRI
(5036) XbaI
AsiSI (5799)
RsrII (5843)
PspXI (8841)
NheI (8937)
BmtI (8941)
BssHII (8996)
FspAI (9040)
PpuMI - SanDI (9676)
BspQI - SapI (9923)
BspHI (9957)
AvrII (10,370)
BsgI (10,456)
EcoNI (10,916)
AlwNI (11,185)
EciI (11,205)
BmrI (11,607)
End (11,758)
2500
5000
7500
10,000
5' flank of YLR296W
TP11p
FBA1t
TEF promoter
hph
X00622
FBA1t
EhADH1
TP11p
3' flank of YLR296W
MA741 - EhADH1 2 copy cassette
11,758 bp

Figure 44

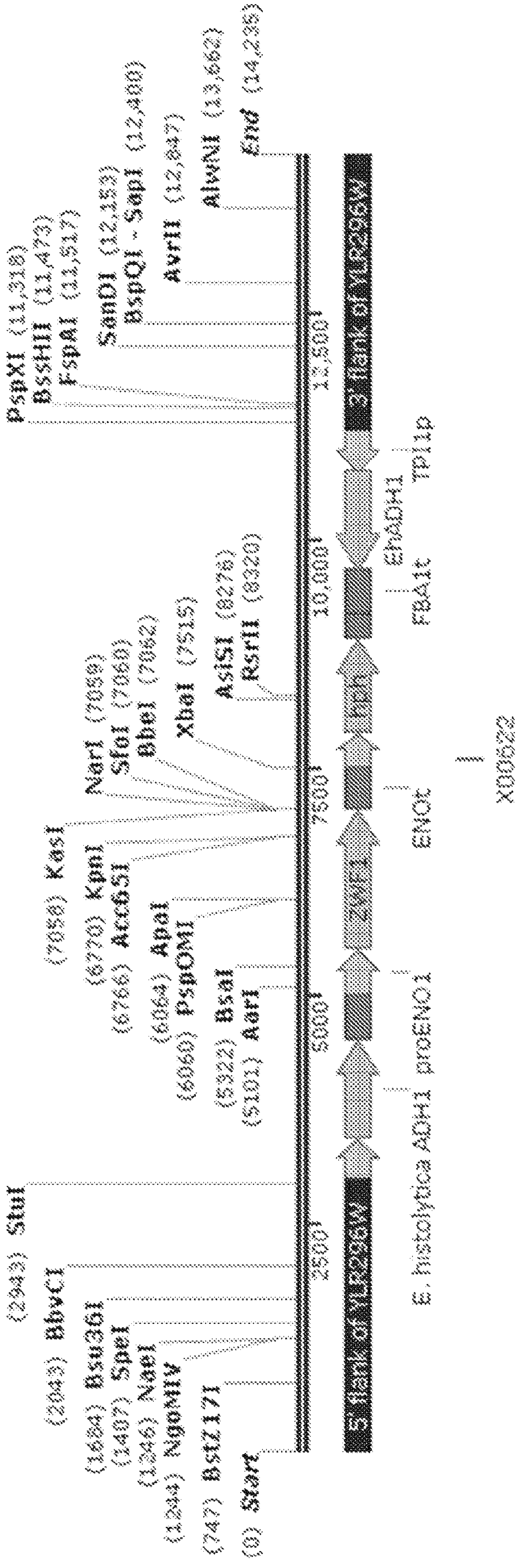


MA743 - EhADH1 2 copy cassette with ZWF1
14,235 bp

Figure 45

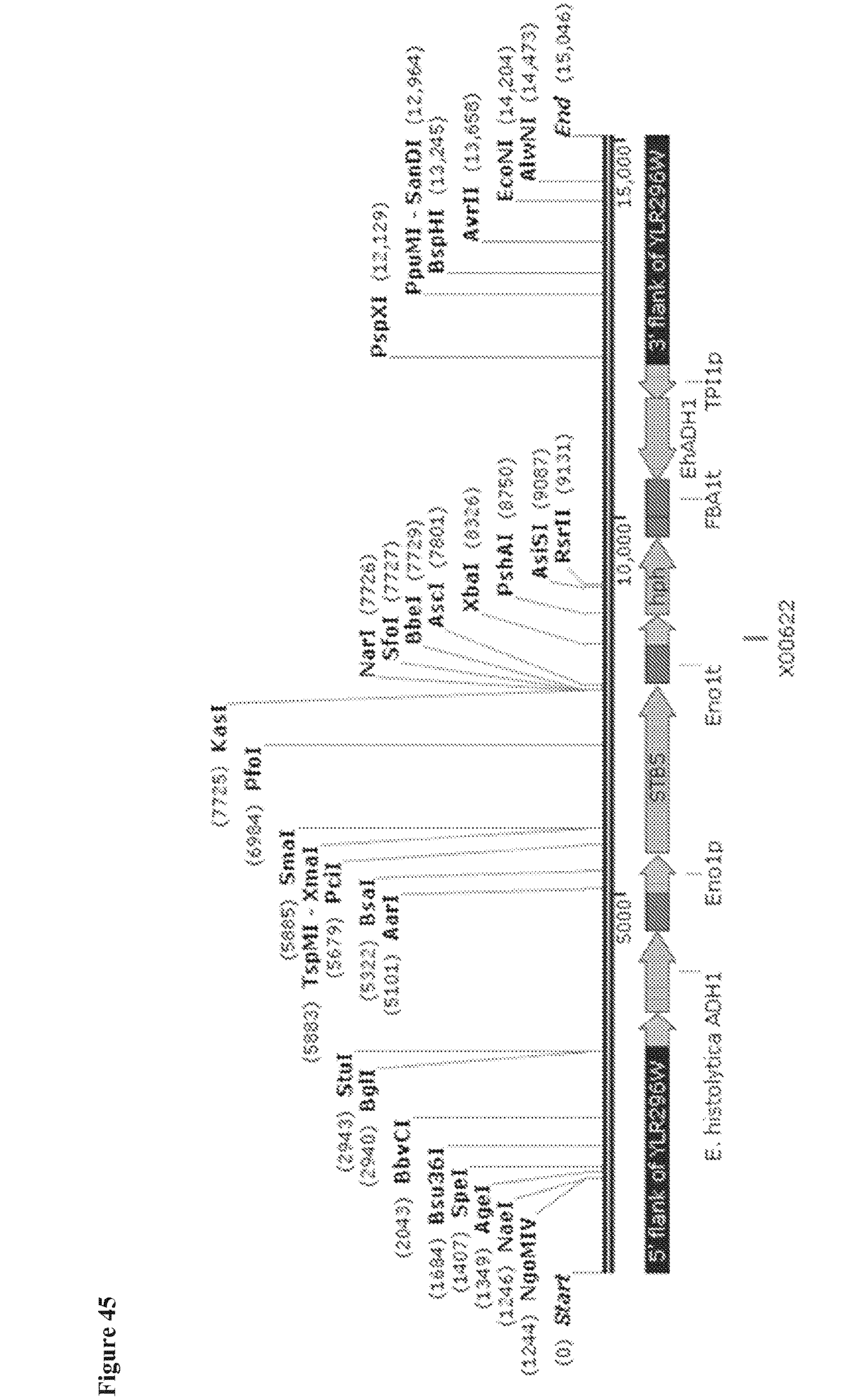


MA742 - EhADH1 2 copy cassette with STB5
15,046 bp

Figure 46

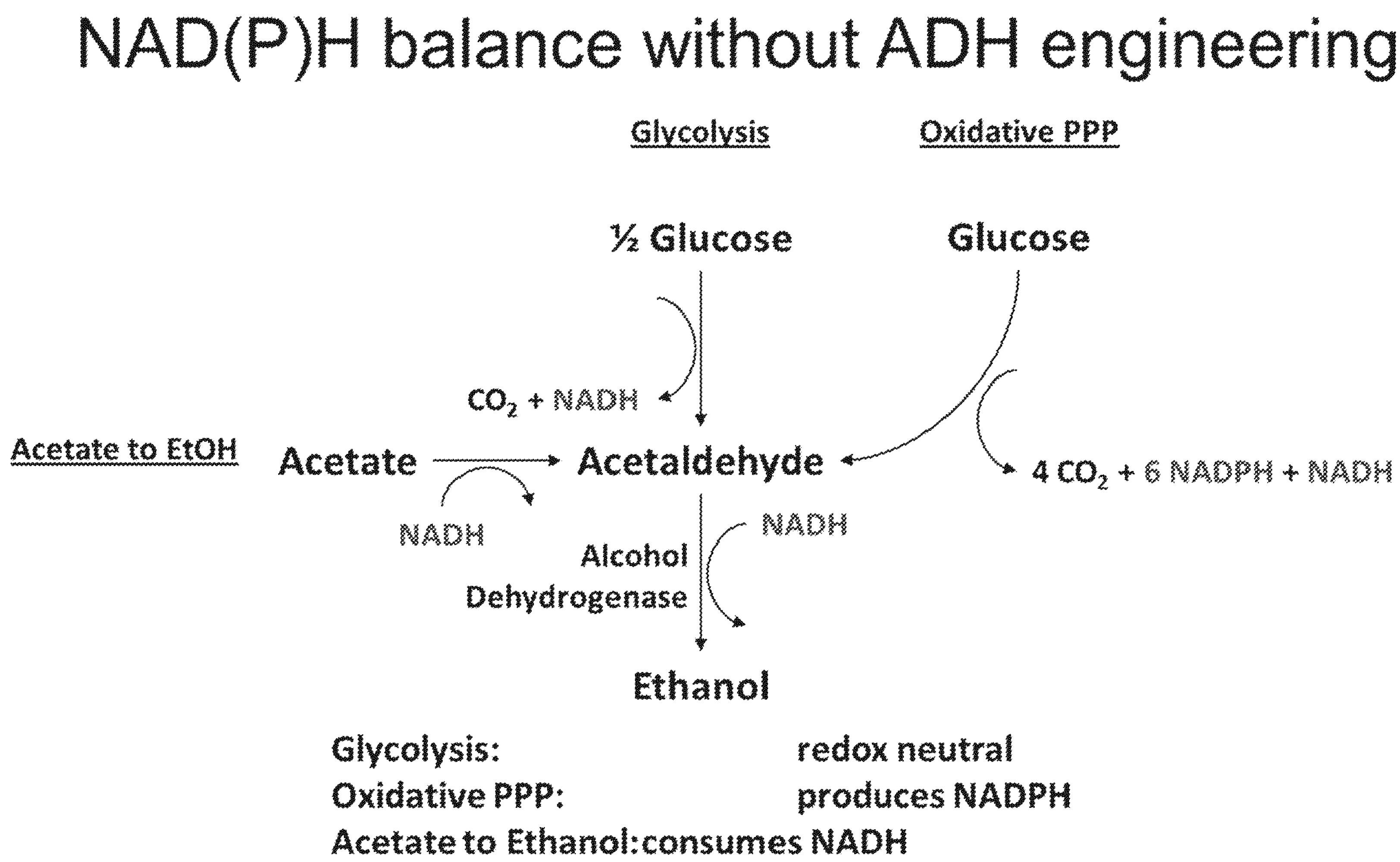
NAD(P)H balance without ADH engineering
Glycolysis
Oxidative PPP
½ Glucose
Glucose
$CO_2$ + NADH
Acetate to EtOH
Acetate
Acetaldehyde
$4\ CO_2$ + 6 NADPH + NADH
NADH
NADH
Alcohol
Dehydrogenase
Ethanol
Glycolysis: redox neutral
Oxidative PPP: produces NADPH
Acetate to Ethanol:consumes NADH

Figure 47

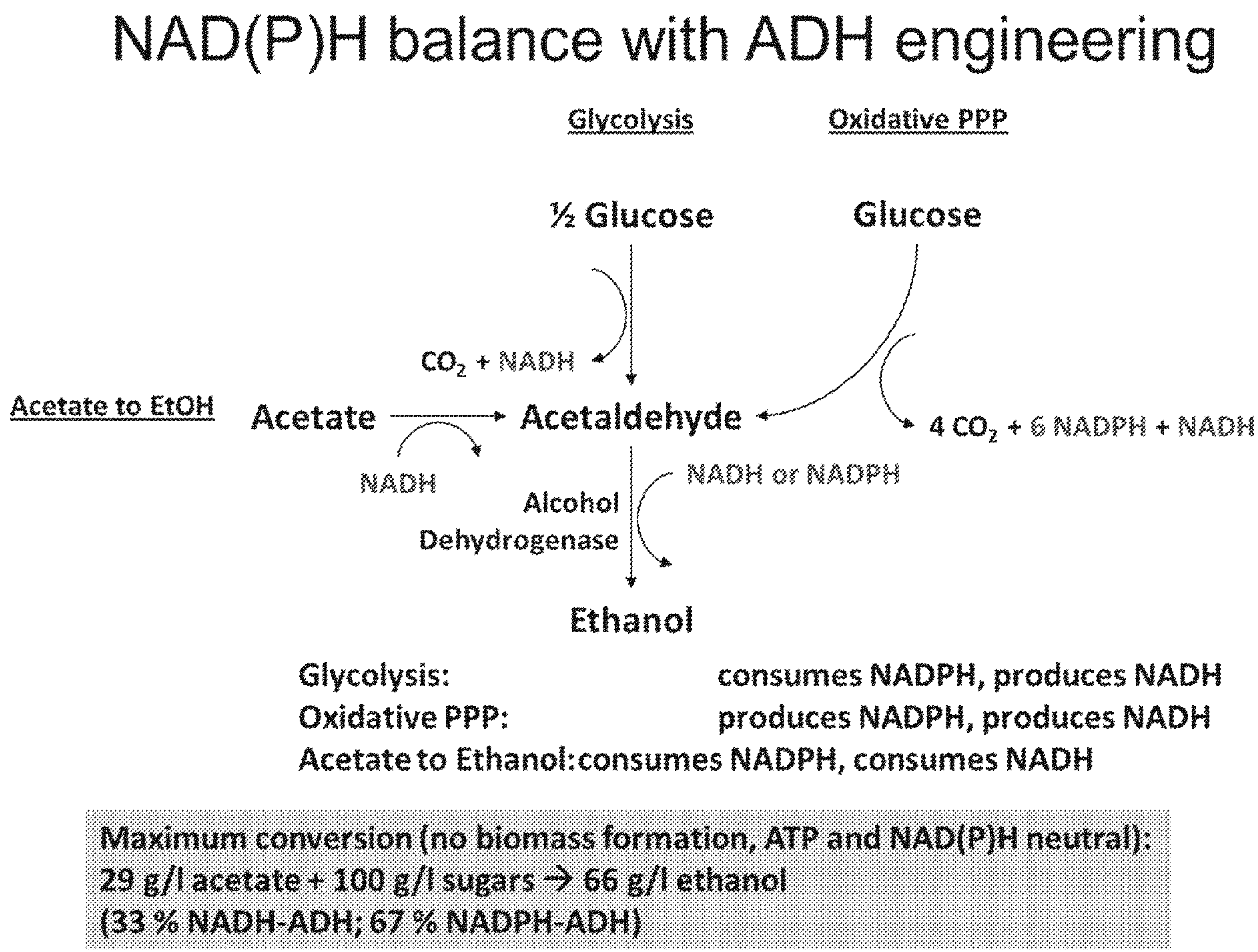
NAD(P)H balance with ADH engineering
Glycolysis
Oxidative PPP
½ Glucose
Glucose
$CO_2$ + NADH
Acetate to EtOH
Acetate
Acetaldehyde
4 $CO_2$ + 6 NADPH + NADH
NADH
NADH or NADPH
Alcohol Dehydrogenase
Ethanol
Glycolysis: consumes NADPH, produces NADH
Oxidative PPP: produces NADPH, produces NADH
Acetate to Ethanol: consumes NADPH, consumes NADH
Maximum conversion (no biomass formation, ATP and NAD(P)H neutral):
29 g/l acetate + 100 g/l sugars → 66 g/l ethanol
(33 % NADH-ADH; 67 % NADPH-ADH)

Figure 48

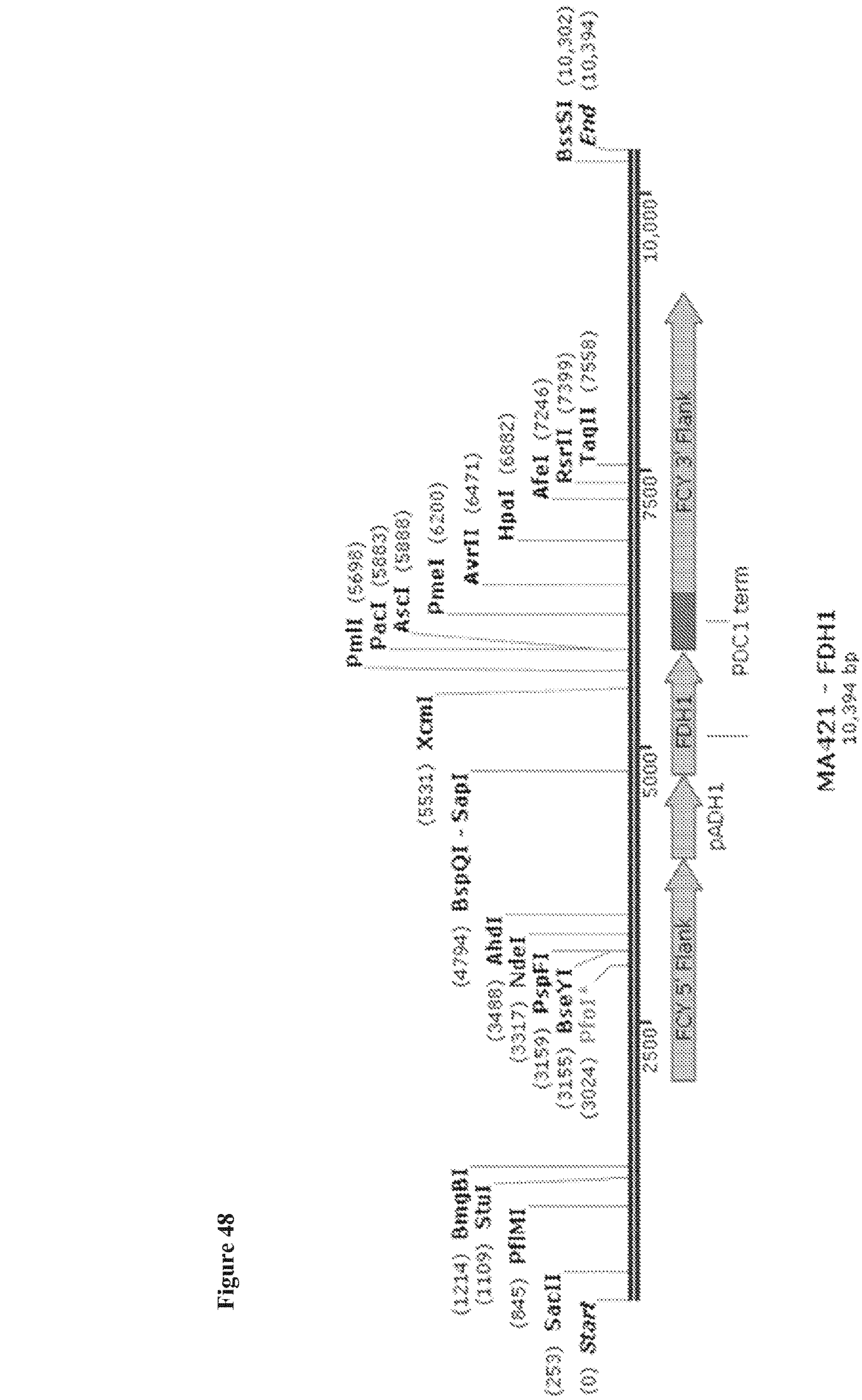

(0) Start
(253) SacII
(845) PflMI
(1109) StuI
(1214) BmgBI
(3024) PflFI
(3155) BseYI
(3159) PspFI
(3317) NdeI
(3486) AhdI
(4794) BspQI - SapI
(5531) XcmI
PmlI (5698)
PacI (5663)
AscI (5888)
PmeI (6200)
AvrII (6471)
HpaI (6882)
AfeI (7246)
RsrII (7399)
TaqII (7558)
BssSI (10,302)
End (10,394)
2500
5000
7500
10,000
FCY 5' Flank
pADH1
FDH1
PDC1 term
FCY 3' Flank
MA421 - FDH1
10,394 bp

Figure 49

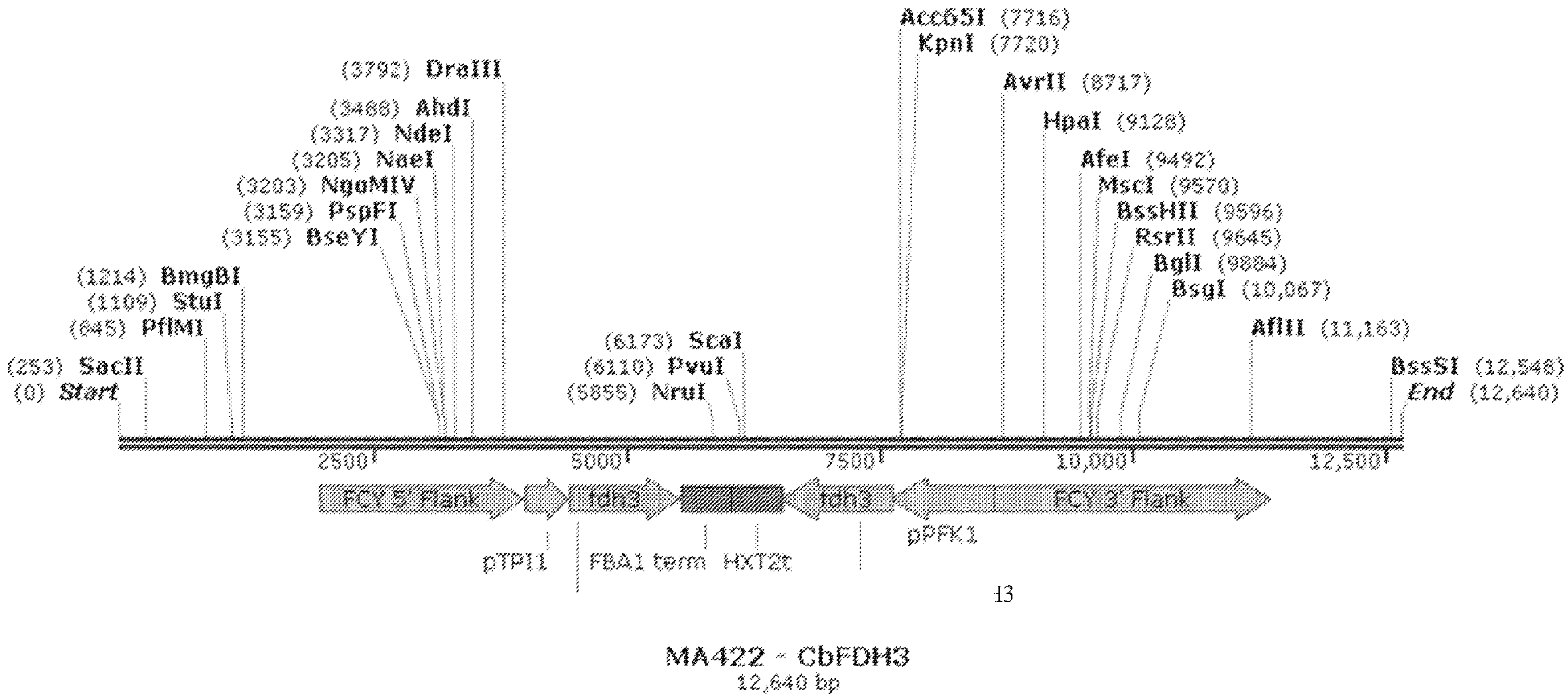
(0) Start
(253) SacII
(845) PflMI
(1109) StuI
(1214) BmgBI
(3155) BseYI
(3159) PspFI
(3203) NgoMIV
(3205) NaeI
(3317) NdeI
(3488) AhdI
(3792) DraIII
(5855) NruI
(6110) PvuI
(6173) ScaI
Acc65I (7716)
KpnI (7720)
AvrII (8717)
HpaI (9128)
AfeI (9492)
MscI (9570)
BssHII (9596)
RsrII (9645)
BglI (9884)
BsgI (10,067)
AflII (11,163)
BssSI (12,548)
End (12,640)
2500
5000
7500
10,000
12,500
FCY 5' Flank
fdh3
fdh3
FCY 3' Flank
pTPI1
FBA1 term
HXT2t
pPFK1
H3
MA422 - CbFDH3
12,640 bp

Figure 50

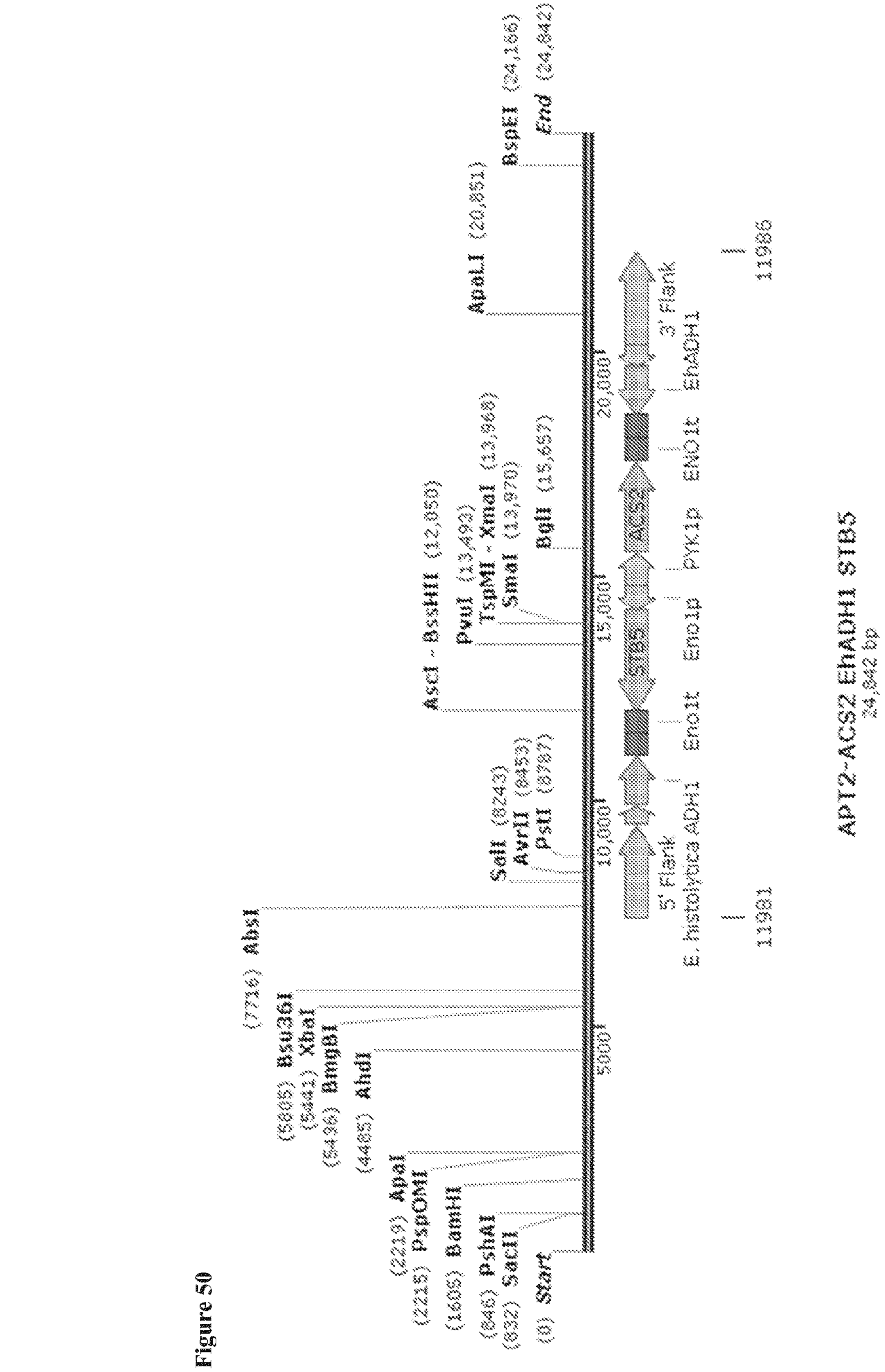
(0) Start
(832) SacII
(846) PshAI
(1605) BamHI
(2215) PspOMI
(2219) ApaI
(4485) AhdI
(5436) BmgBI
(5441) XbaI
(5605) Bsu36I
(7716) AbsI
SalI (8243)
AvrII (8453)
PstI (8787)
AscI - BssHII (12,850)
PvuI (13,493)
TspMI - XmaI (13,968)
SmaI (13,970)
BglI (15,657)
ApaLI (20,851)
BspEI (24,166)
End (24,942)
5000
10,000
15,000
20,000
5' Flank
E. histolytica ADH1
Eno1t
STB5
Eno1p
PYK1p
ACS2
ENO1t
EhADH1
3' Flank
11981
11986
APT2-ACS2 EhADH1 STB5
24,942 bp

Figure 51

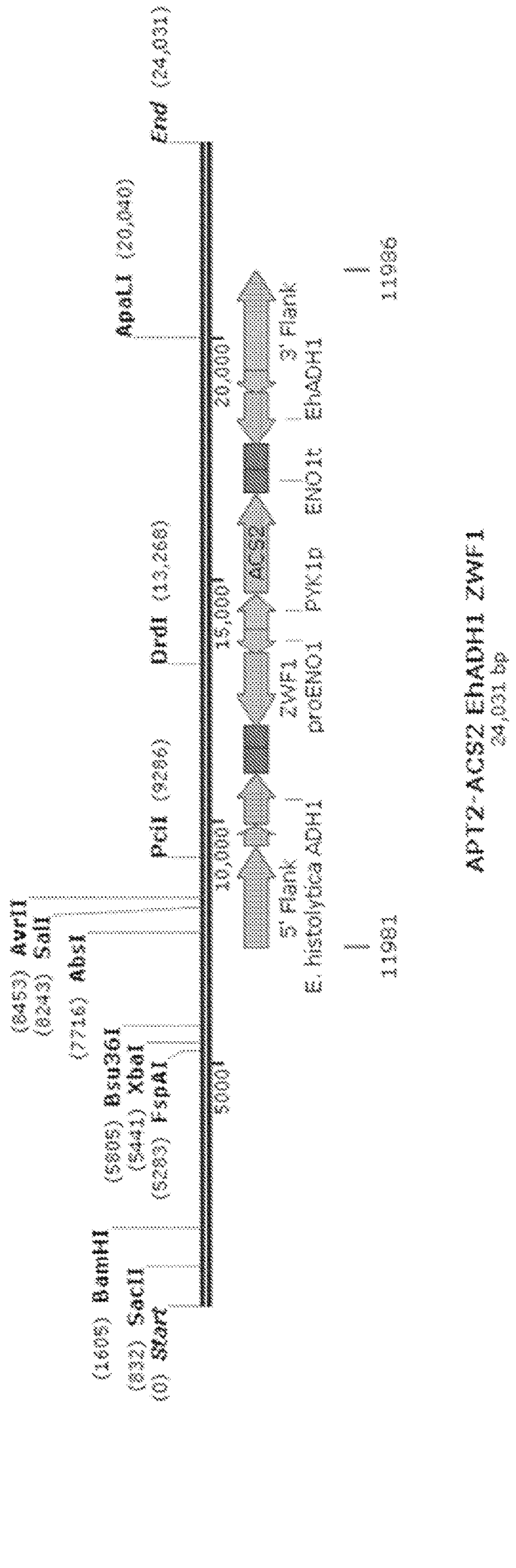
(0) Start
(832) SacII
(1605) BamHI
(5283) FspAI
(5441) XbaI
(5805) Bsu36I
(7716) AbsI
(8243) SalI
(8453) AvrII
PciI (9286)
DrdI (13,268)
ApaLI (20,040)
End (24,031)
5000
10,000
15,000
20,000
5' Flank
E. histolytica ADH1
ZWF1
proENO1
PYK1p
ACS2
ENO1t
EhADH1
3' Flank
11981
11986
APT2-ACS2 EhADH1 ZWF1
24,031 bp

Figure 52

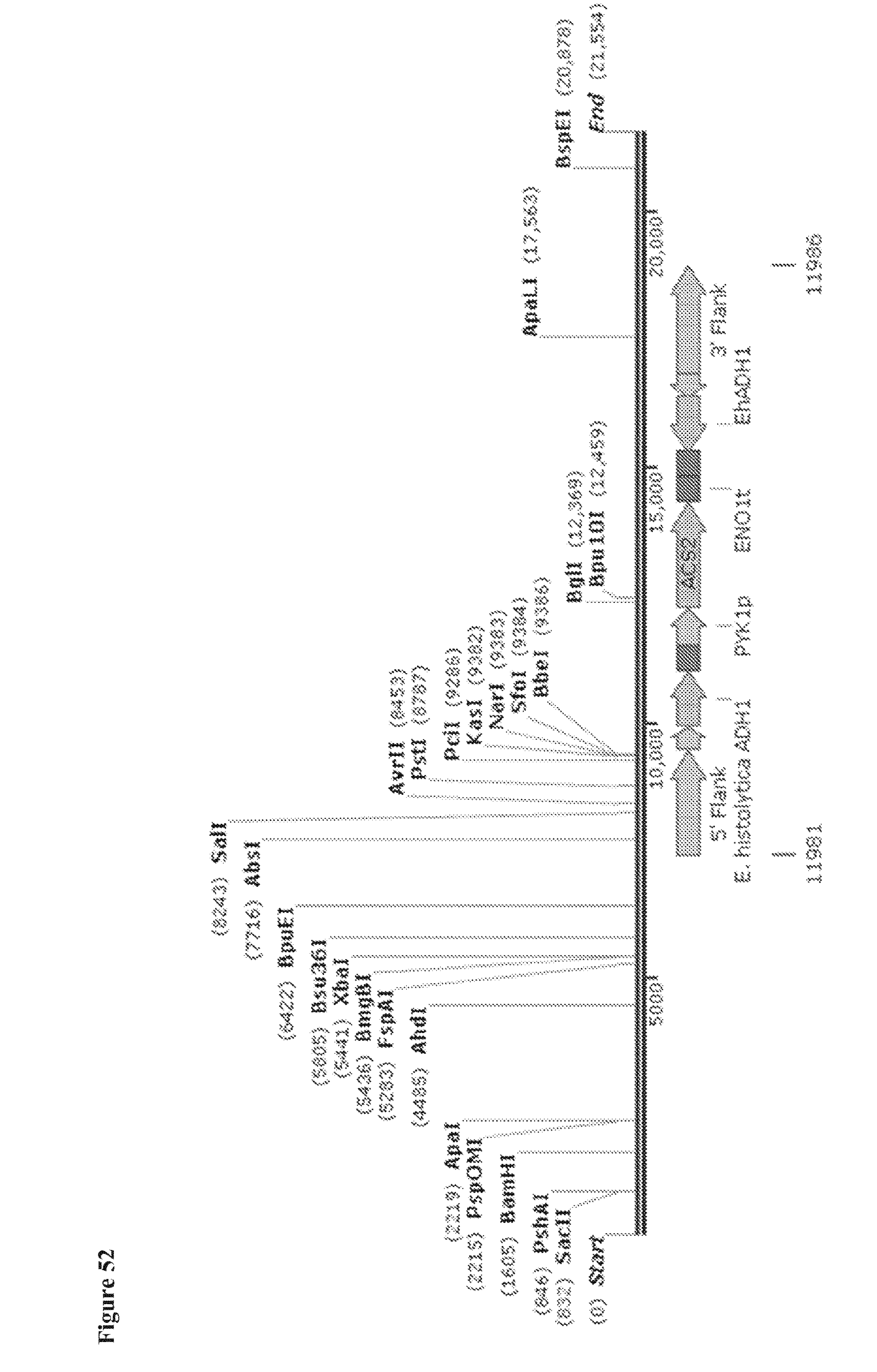


APT2-ACS2 EhADH1
21,554 bp

Figure 53

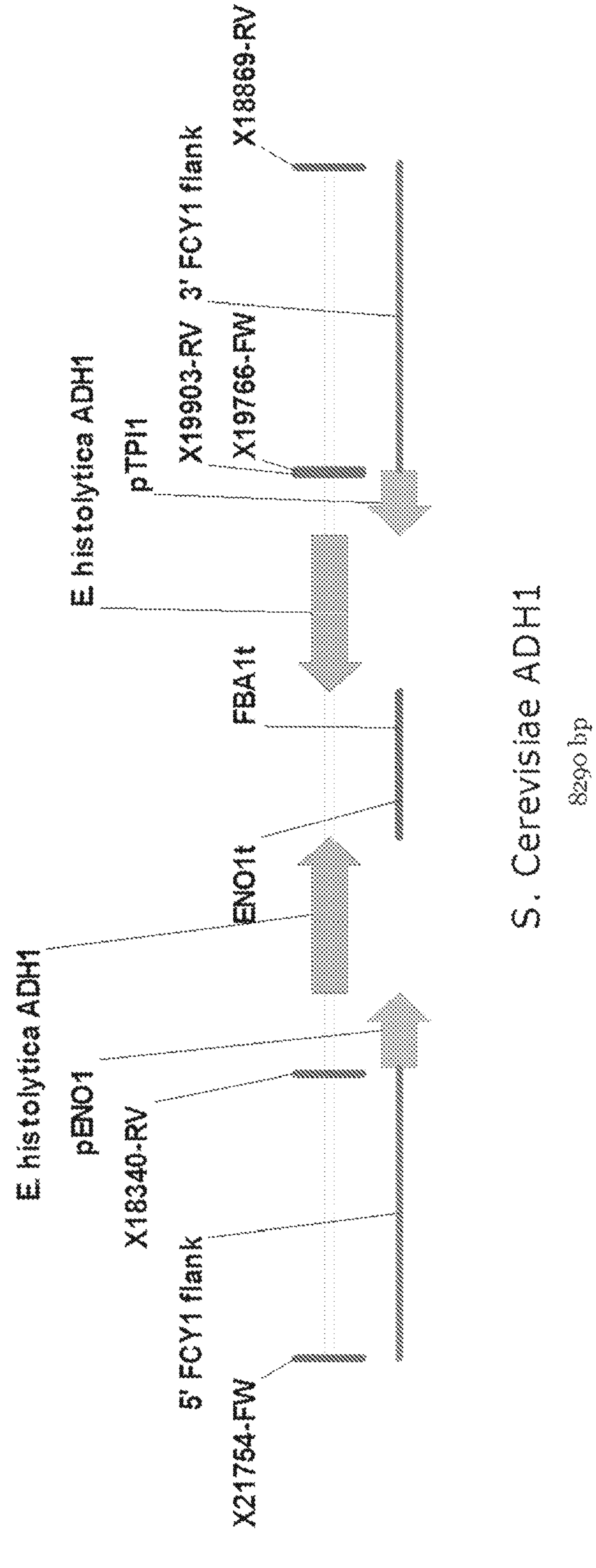
X21754-FW
5' FCY1 flank
X18340-RV
pENO1
E. histolytica ADH1
ENO1t
FBA1t
E. histolytica ADH1
pTPI1
X19903-RV
X19766-FW
3' FCY1 flank
X18869-RV
S. Cerevisiae ADH1
8290 bp

Figure 54

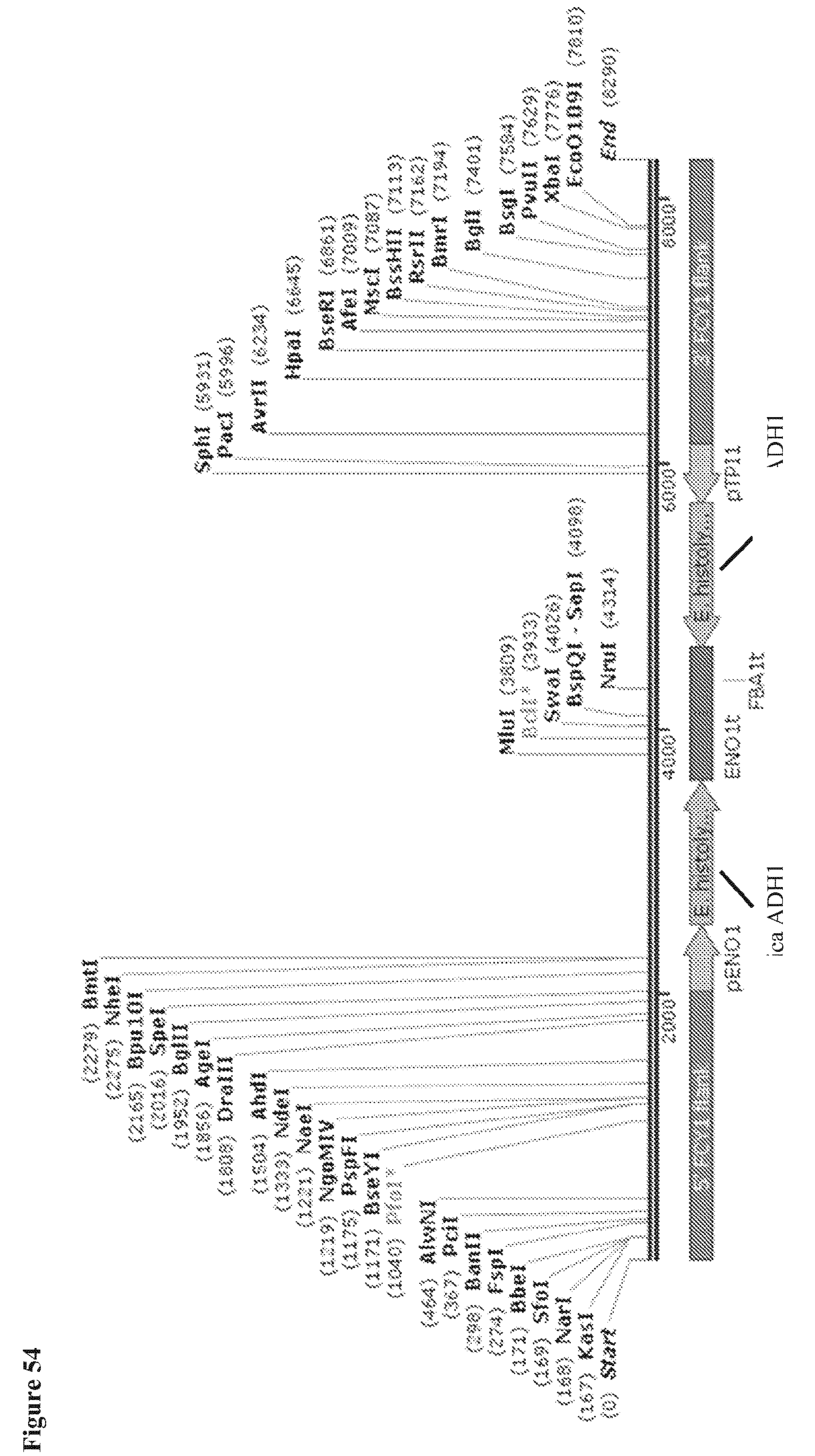


MA1181 - EhADH1 2 copy cassette at FCY1
8290 bp

Figure 55

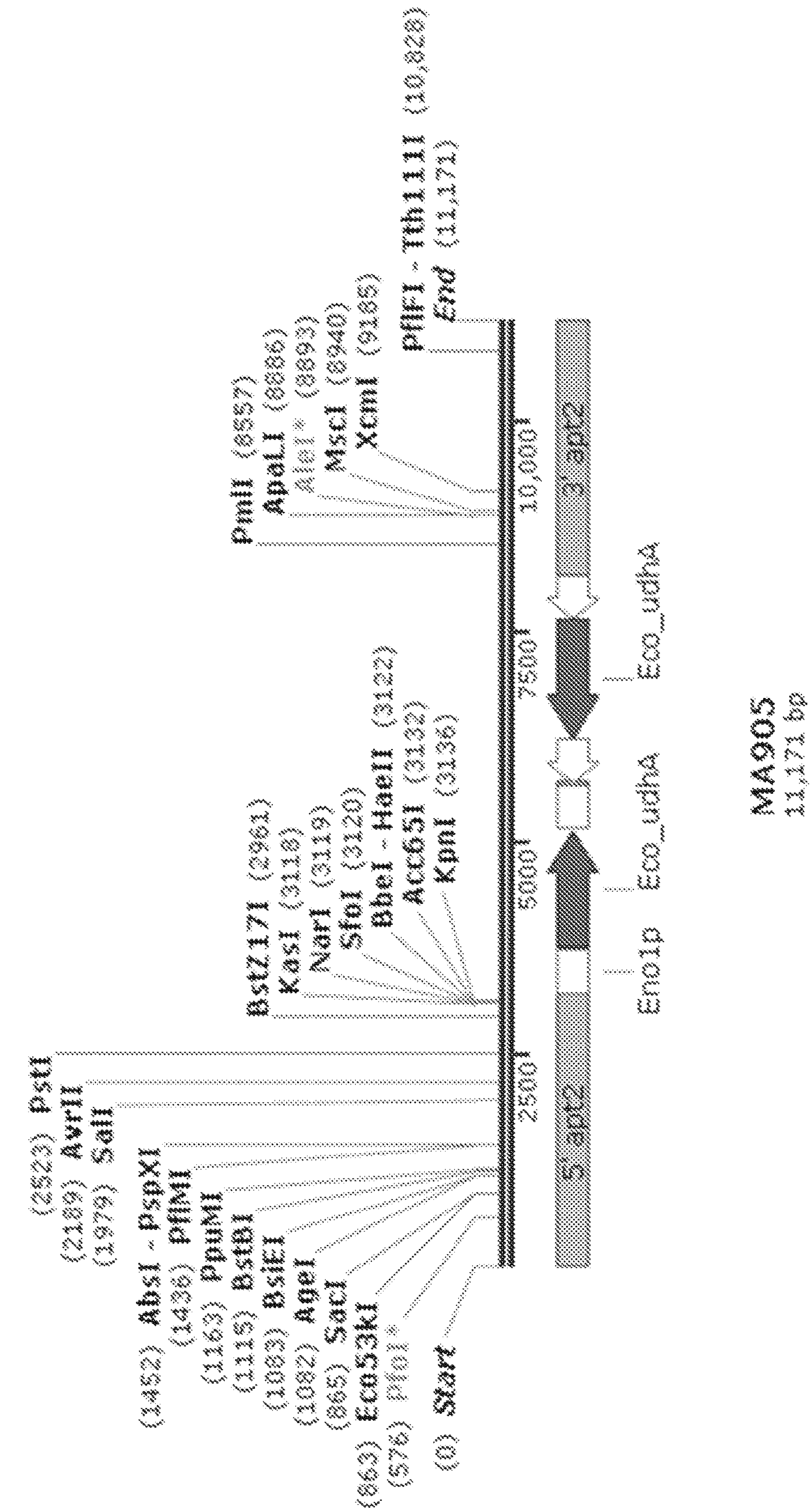
(0) Start
(576) PflI*
(863) Eco53kI
(865) SacI
(1082) AgeI
(1083) BsiEI
(1115) BstBI
(1163) PpuMI
(1436) PflMI
(1452) AbsI - PspXI
(1979) SalI
(2189) AvrII
(2523) PstI
BstZ17I (2961)
KasI (3118)
NarI (3119)
SfoI (3120)
BbeI - HaeII (3122)
Acc65I (3132)
KpnI (3136)
PmlI (6557)
ApaLI (8886)
AleI* (8893)
MscI (8940)
XcmI (9185)
PflFI - Tth111I (10,828)
End (11,171)
2500
5000
7500
10,000
5' apt2
Eno1p
Eco_udhA
Eco_udhA
3' apt2
MA905
11,171 bp

Figure 56

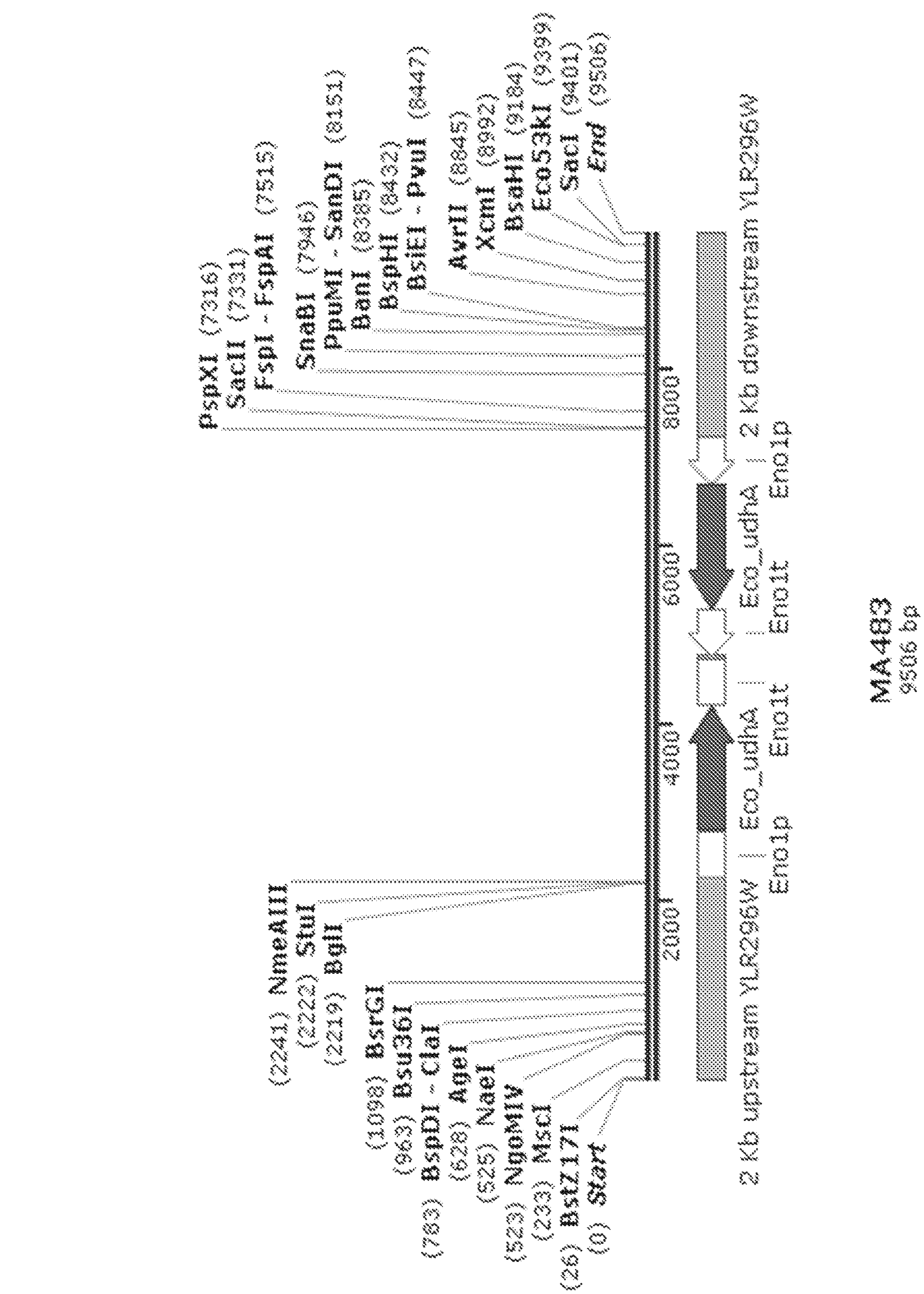
(2241) NmeAIII
(2222) StuI
(2219) BglI
(1098) BsrGI
(963) Bsu36I
(783) BspDI - ClaI
(628) AgeI
(525) NaeI
(523) NgoMIV
(233) MscI
(26) BstZ17I
(0) Start
PspXI (7316)
SacII (7331)
FspI - FspAI (7515)
SnaBI (7946)
PpuMI - SanDI (8151)
BanI (8385)
BspHI (8432)
BsiEI - PvuI (8447)
AvrII (8845)
XcmI (8992)
BsaHI (9184)
Eco53kI (9399)
SacI (9401)
End (9506)
2000
4000
6000
8000
2 Kb upstream YLR296W
Eno1p
Eco_udhA
Eno1t
Eno1t
Eco_udhA
Eno1p
2 Kb downstream YLR296W
MA483
9506 bp

Figure 57A

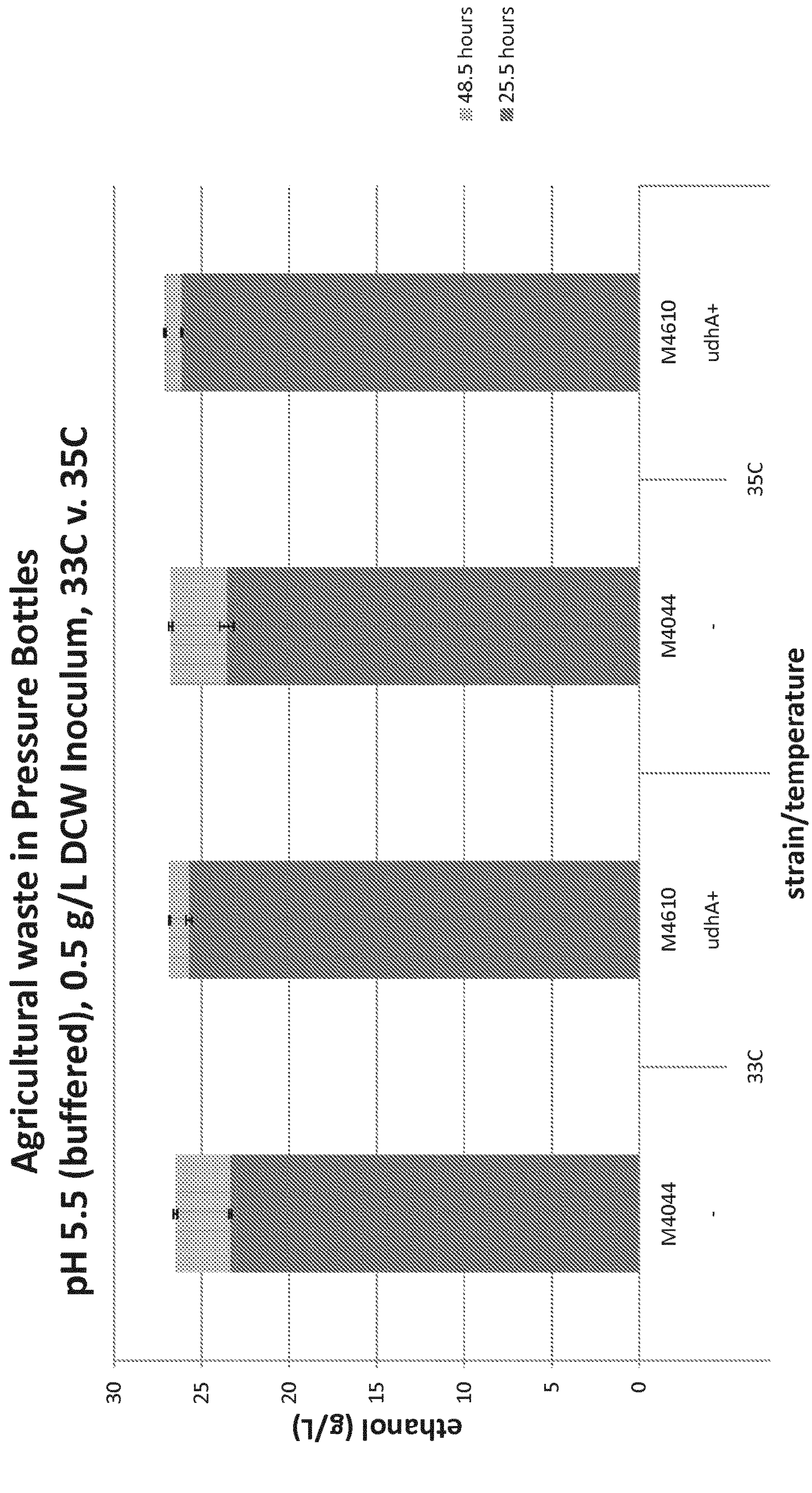
Agricultural waste in Pressure Bottles
pH 5.5 (buffered), 0.5 g/L DCW Inoculum, 33C v. 35C
ethanol (g/L)
0
5
10
15
20
25
30
M4044
-
M4610
udhA+
33C
M4044
-
M4610
udhA+
35C
strain/temperature
48.5 hours
25.5 hours

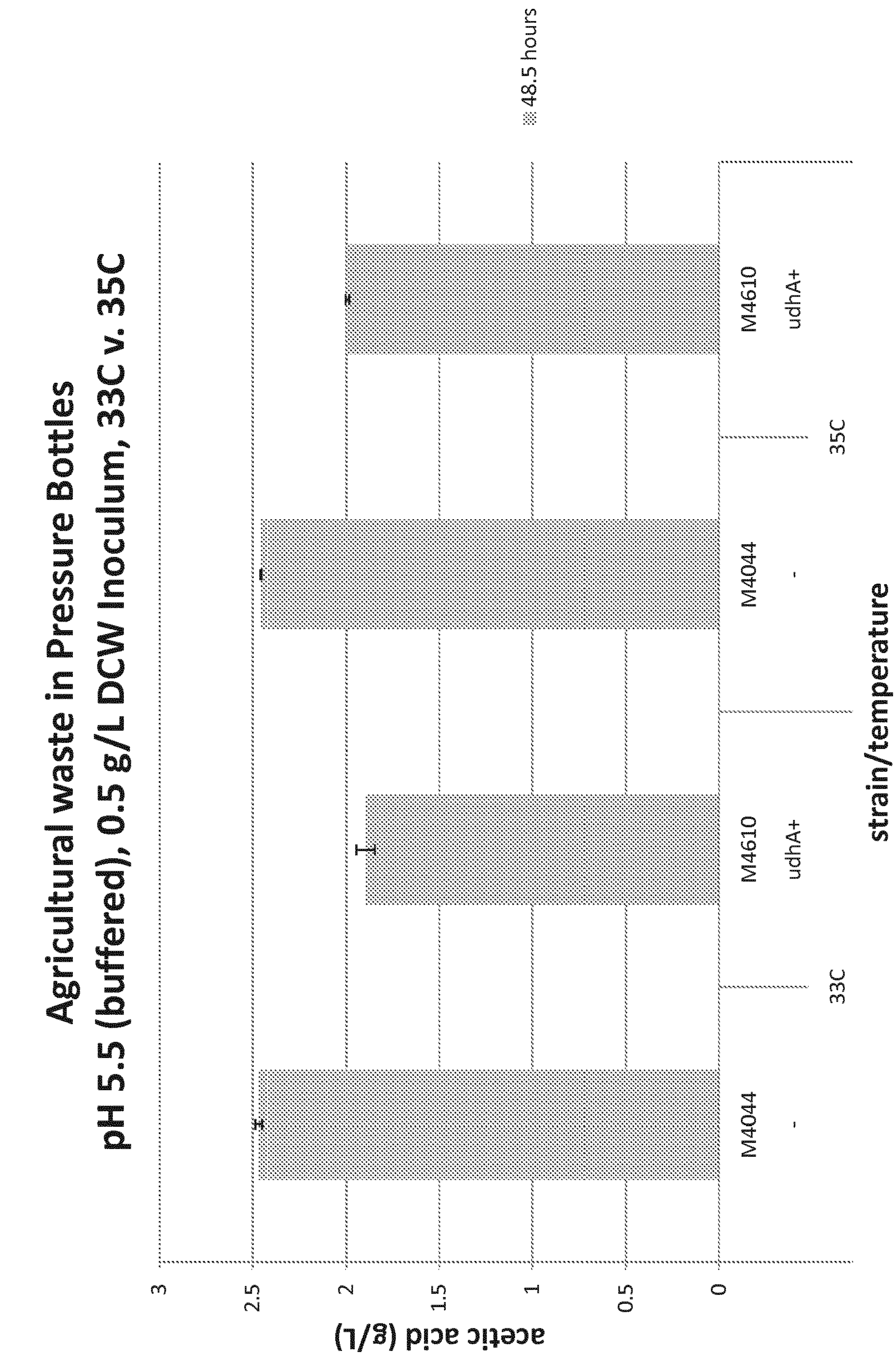
Agricultural waste in Pressure Bottles
pH 5.5 (buffered), 0.5 g/L DCW Inoculum, 33C v. 35C
48.5 hours
acetic acid (g/L)
3
2.5
2
1.5
1
0.5
0
M4044
-
M4610
udhA+
33C
M4044
-
M4610
udhA+
35C
strain/temperature

Figure 57B

Figure 57C

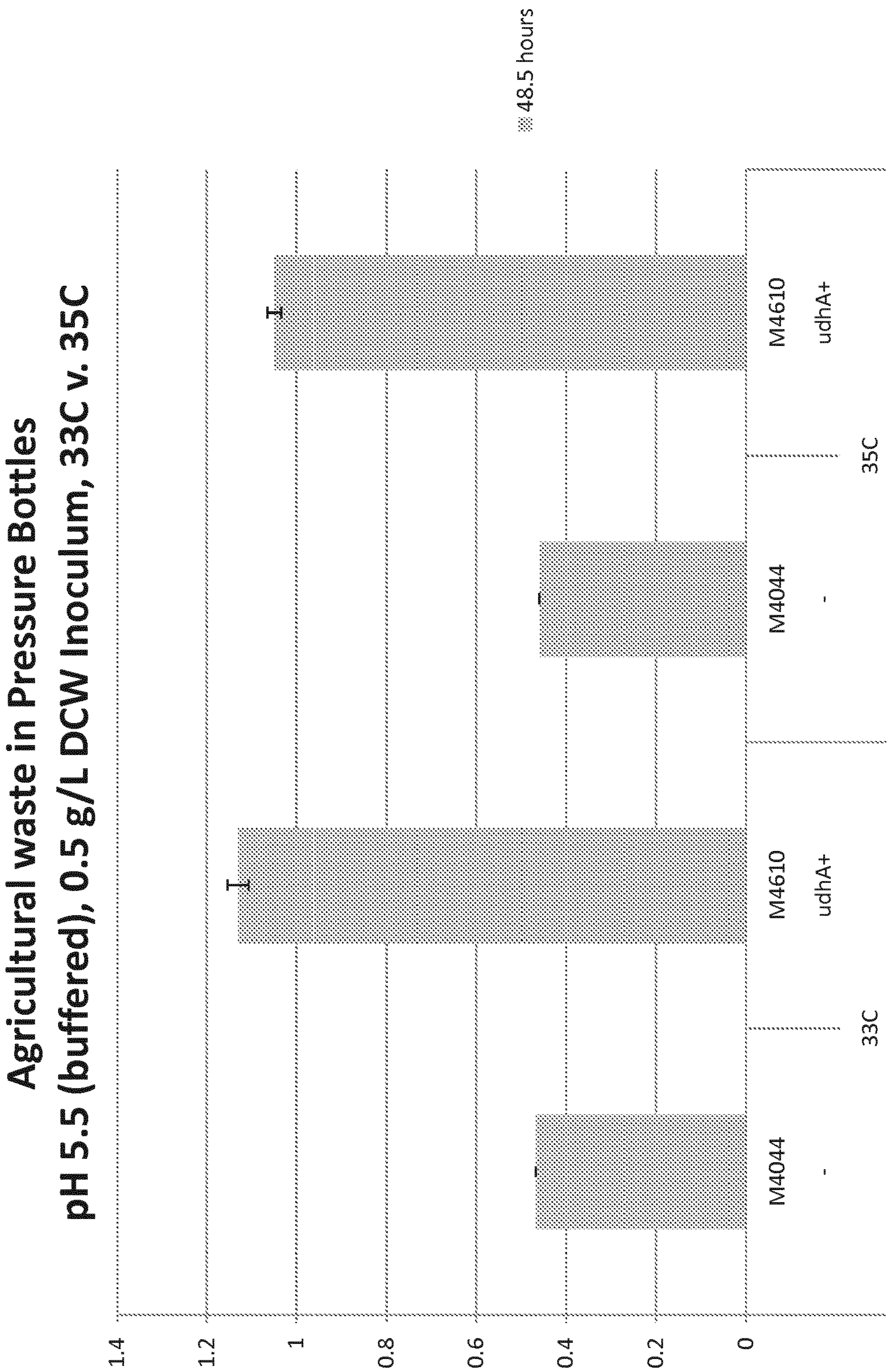
Agricultural waste in Pressure Bottles
pH 5.5 (buffered), 0.5 g/L DCW Inoculum, 33C v. 35C
glycerol (g/L)
1.4
1.2
1
0.8
0.6
0.4
0.2
0
M4044
-
M4610
udhA+
33C
M4044
-
M4610
udhA+
35C
strain/temperature
48.5 hours

Figure 58A

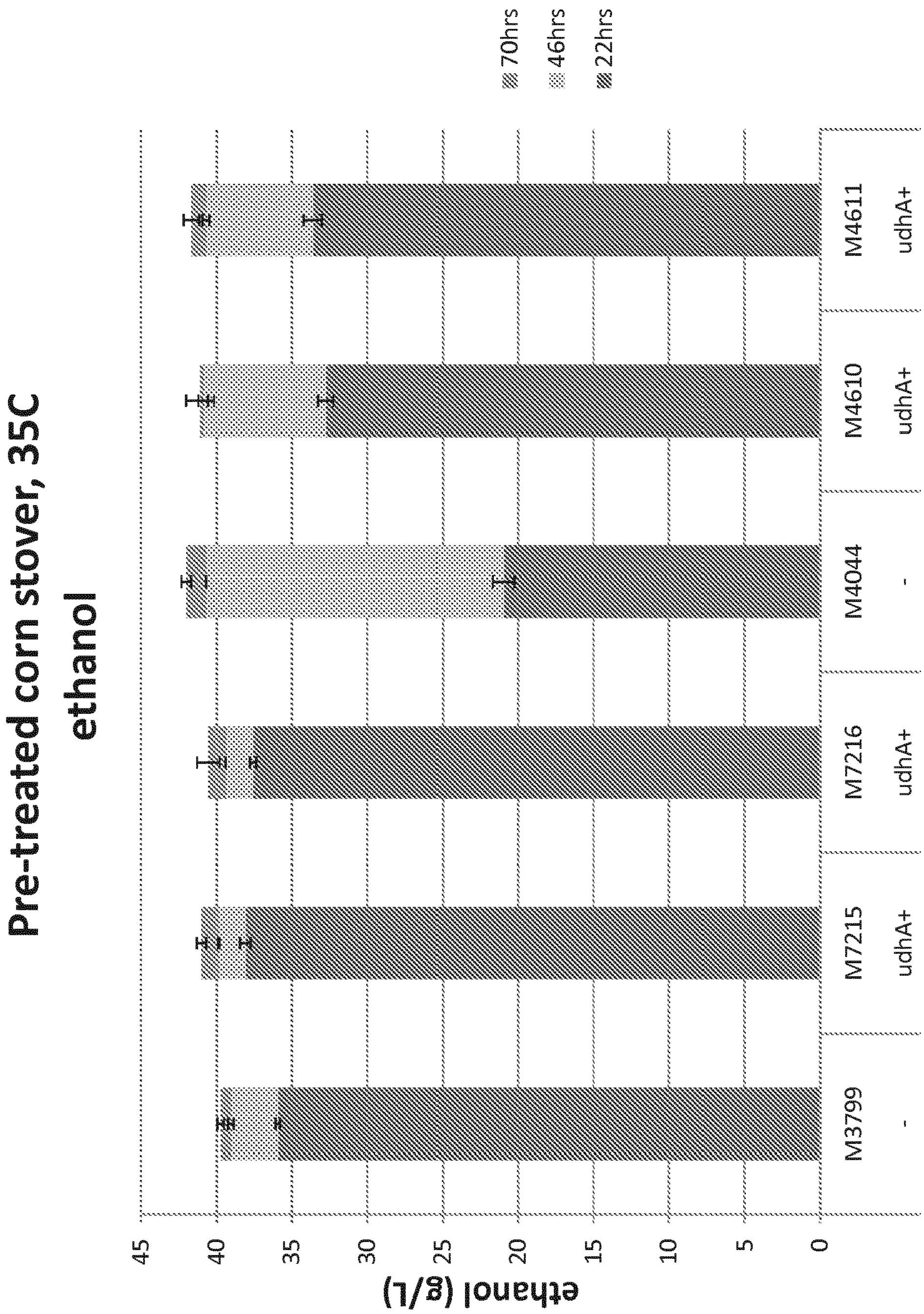
Pre-treated corn stover, 35C
ethanol
ethanol (g/L)
45
40
35
30
25
20
15
10
5
0
M3799
-
M7215
udhA+
M7216
udhA+
M4044
-
M4610
udhA+
M4611
udhA+
strain
70hrs
46hrs
22hrs

Figure 58B

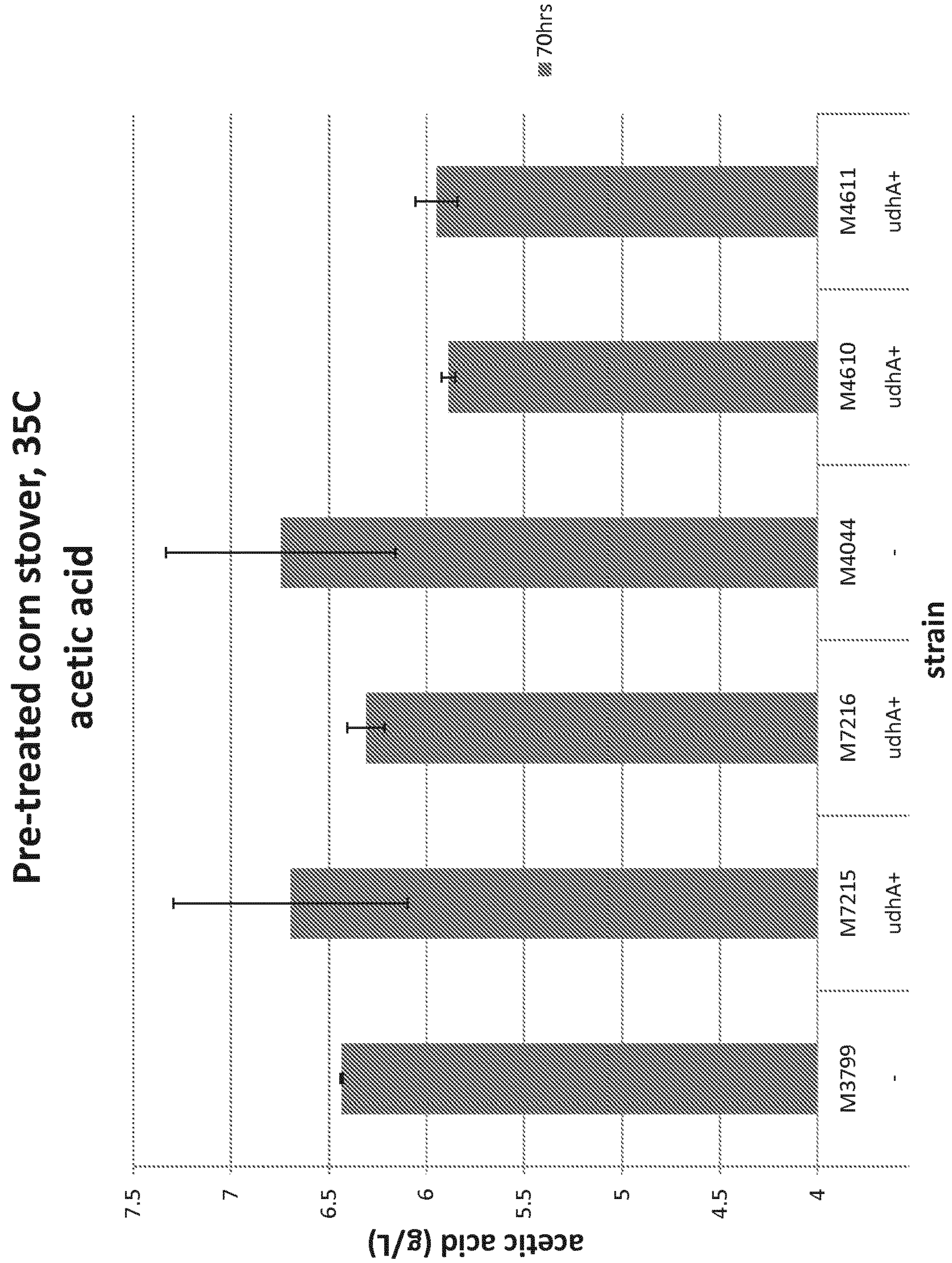
Pre-treated corn stover, 35C
acetic acid
70hrs
acetic acid (g/L)
7.5
7
6.5
6
5.5
5
4.5
4
M3799
-
M7215
udhA+
M7216
udhA+
M4044
-
M4610
udhA+
M4611
udhA+
strain

Figure 58C

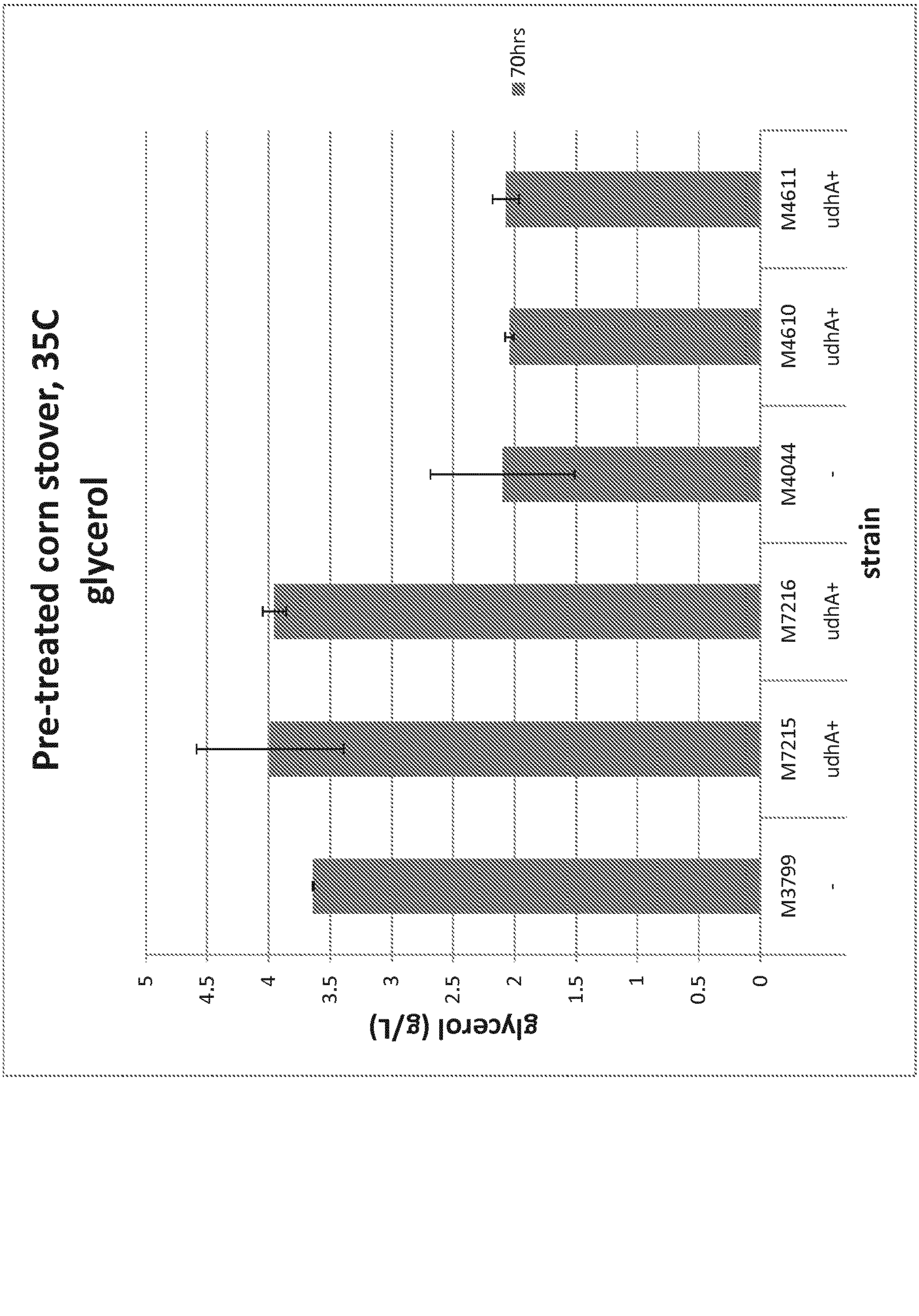
Pre-treated corn stover, 35C
glycerol
glycerol (g/L)
0
0.5
1
1.5
2
2.5
3
3.5
4
4.5
5
M3799
-
M7215
udhA+
M7216
udhA+
M4044
-
M4610
udhA+
M4611
udhA+
strain
70hrs

Figure 59

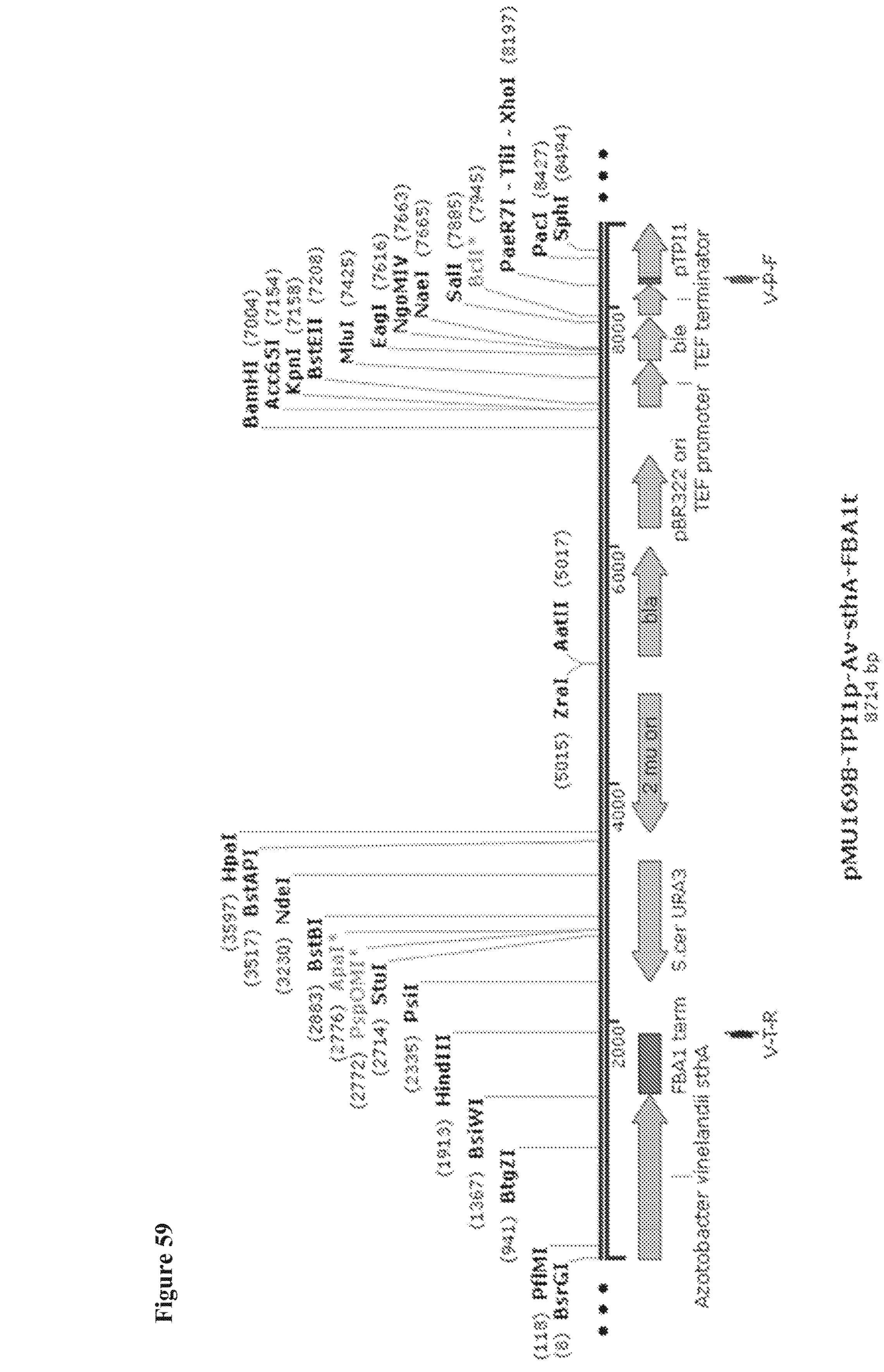
(118) PflMI
(8) BsrGI
(941) BtgZI
(1367) BsiWI
(1913) HindIII
(2335) PstI
(2714) StuI
(2772) PspOMI*
(2776) ApaI*
(2863) BstBI
(3230) NdeI
(3517) BstAPI
(3597) HpaI
(5015) ZraI
AatII (5017)
BamHI (7004)
Acc65I (7154)
KpnI (7158)
BstEII (7208)
MluI (7425)
EagI (7616)
NgoMIV (7663)
NaeI (7665)
SalI (7885)
BclI* (7945)
PaeR7I - TliI - XhoI (8197)
PacI (8427)
SphI (8494)
2000
4000
6000
8000
Azotobacter vinelandii sthA
FBA1 term
S.cer URA3
2 mu ori
bla
pBR322 ori
TEF promoter
ble
TEF terminator
pTPI1
V-T-R
V-P-F
pMU1698-TPI1p-Av-sthA-FBA1t
8714 bp

Europäisches Patentamt
European Patent Office
Office européen des brevets

# EUROPEAN SEARCH REPORT

Application Number
EP 18 17 1345

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/085627 A1 (FORSKARPATENT I SYD AB [SE]; SONDEREGGER MARCO [CH] ET AL.) 7 October 2004 (2004-10-07) * page 1 - page 3; claims 1-8 * ----- | 1-4,10, 13,14 | INV. C12N9/04 C12N9/02 C12P7/10 |
| A,D | WO 2010/056805 A2 (MASCOMA CORP [US]; HOGSETT DAVID A [US] ET AL.) 20 May 2010 (2010-05-20) * paragraph [0160] * ----- | 1-15 | |

TECHNICAL FIELDS SEARCHED (IPC)
C12N
C12P

The present search report has been drawn up for all claims

1

| Place of search | Date of completion of the search | Examiner |
|---|---|---|
| The Hague | 5 April 2019 | Devijver, Kristof |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 17 1345

This annex lists the patent family members relating to the patent documents cited in the above-mentioned European search report.
The members are as contained in the European Patent Office EDP file on
The European Patent Office is in no way liable for these particulars which are merely given for the purpose of information.

05-04-2019

| Patent document cited in search report | Publication date | Patent family member(s) | Publication date |
|---|---|---|---|
| WO 2004085627 A1 | 07-10-2004 | NONE | |
| WO 2010056805 A2 | 20-05-2010 | CA 2743505 A1 | 20-05-2010 |
| | | US 2012094343 A1 | 19-04-2012 |
| | | WO 2010056805 A2 | 20-05-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20120094343 A1
- US 20080261287 A1
- EP 2277989 A
- US 61472085 A
- US 2009002902 W
- US 2009003972 W
- US 2009003970 W
- WO 2010060056 A
- US 2009069443 W
- US 2009064128 W
- WO IB2009005881 A
- US 2011039192 W
- US 61116981 A
- US 20120129229 A1
- US 61351165 A
- US 701652 A
- US 61420142 A
- US 696207 A
- WO 2012138942 A
- US 2012032443 W
- WO 2011149353 A
- US 2013000090 W


### Non-patent literature cited in the description


- **FILBURN, C.R.** Acid Phosphatase Isozymes of Xenoupus laevis Tadpole Tails: I. Spearation and Partial Characterization. *Archives of Biochem. And Biophysics,* 1973, vol. 159, 683-93
- **JEPPSSON, M. et al.** The level of glucose-6-phosphate dehydrogenase activity strongly influences xylose fermentation and inhibitor sensitivity in recombinant Saccharomyces cerevisiae strains. *Yeast,* 2003, vol. 20, 1263-1272
- **CADIÈRE, A. et al.** The Saccharomyces cerevisiae zinc factor protein Stb5p is required as a basal regulator of the pentose phosphate pathway. *FEMS Yeast Research,* 2010, vol. 10, 819-827
- **KARHUMAA et al.** *Microb Cell Fact.,* 05 February 2007, vol. 6, 5
- **MITSUI, R. et al.** A Novel Operon Encoding Formaldehyde Fixation: the Ribulose Monophosphate Pathway in the Gram-Positive Facultative Methylotrophic Bacterium Mycobacterium gastri MB19. *Journal of Bacteriology,* 2000, vol. 182, 944-948
- **YASUEDA, H. et al.** Bacillus subtilis yckG and yckF Encode Two Key Enzymes of the Ribulose Monophosphate Pathway Used by Methylotrophs, and yckH is Required for Their Expression. *J. of Bacteriol.,* 1999, vol. 181, 7154-60
- **FERENCI, T. et al.** Purification and properties of 3-hexulose phosphate synthase and phospho-3-hexuloisomerase from Methylococcus capsulatus. *Biochem J.,* 1974, vol. 144, 477-86
- **ORITA, I. et al.** The Ribulose Monophosphate Pathway Substitutes for the Missing Pentose Phosphate Pathway in the Archaeon Thermococcus kodakaraensis. *J. Bacteriol.,* 2006, vol. 188, 4698-4704
- **NGUYEN, H.T.T. ; NEVOIGT, E.** Engineering of Saccharomyces cerevisiae for the production of dihydroxyacetone (DHA) from sugars: A proof of concept. *Metabolic Engineering,* 2009, vol. 11, 335-46
- **MOLIN, M. ; A. BLOMBERG.** Dihydroxyacetone detoxification in Saccharomyces cerevisiae involves formaldehyde dissimilation. *Mol. Microbiol.,* 2006, vol. 60, 925-938
- **YU, K. O. et al.** Engineering of glycerol utilization pathway for ethanol production by Saccharomyces cerevisiae. *Bioresource Technol.,* 2010, vol. 101, 4157-4161
- **JUNG, J.-Y. et al.** Production of 1,2-propanediol from glycerol in Saccharomyces cerevisiae. *J. Microbiol. Biotechnol.,* 2011, vol. 21, 846-853
- **NGUYEN, H. T. T. ; NEVOIGT, E.** Engineering of Saccharomyces cerevisiae for the production of dihydroxyacetone (DHA) from sugars: A proof of concept. *Metabolic Engineering,* 2009, vol. 11, 335-346
- **HORBACH, S. et al.** Enzymes involved in the formation of glycerol 3-phosphate and the by-products dihydroxyacetone and glycerol in Zymomonas mobilis. *FEMS Microbiology Letters,* 1994, vol. 120, 37-44

- **ANDERLUND, M. et al.** Expression of the Escherichia coli pntA and pntB Genes, Encoding Nicotinamide Nucleotide Transhydrogenase, in Saccharomyces cerevisiae and Its Effect on Product Formation during Anaerobic Glucose Fermentation. *Appl. Envirol. Microbiol.,* 1999, vol. 65, 2333-340
- **HEUX, S. et al.** Glucose utilization of strains lacking PGI1 and expressing a transhydrogenase suggests differences in the pentose phosphate capacity among Saccharomyces cerevisiae strains. *FEMS Yeast Research,* 2008, vol. 8, 217-224
- **JEUN, Y.-S. et al.** Expression of Azotobacter vinelandii soluble transhydrogenase perturbs xylose reductase-mediated conversion of xylose to xylitol by recombinant Saccharomyces cerevisiae. *Journal of Molecular Catalysis B: Enzymatic,* 2003, vol. 26, 251-256
- **NISSEN, T. L. et al.** Expression of a cytoplasmic transhydrogenase in Saccharomyces cerevisiae results in formation of 2-oxoglutarate due to depletion of the NADPH pool. *Yeast,* 2001, vol. 18, 19-32
- **BOLES, E. et al.** The role of the NAD-dependent glutamate dehydrogenase in restoring growth on glucose of a Saccharomyces cerevisiae phosphoglucose isomerase mutant. *European Journal of Biochemistry,* 1993, vol. 217, 469-477
- **ANSELL, R. et al.** *EMBO J.,* 1997, vol. 16, 2179-87
- **PAHLMAN, A-K. et al.** *J. Biol. Chem.,* 2001, vol. 276, 3555-63
- **GUO, Z.P. et al.** *Metab. Eng.,* 2011, vol. 13, 49-59
- **LIDÉN, G. et al.** *Appl. Env. Microbiol.,* 1996, vol. 62, 3894-96
- **MEDINA, V.G. et al.** *Appl. Env. Microbiol.,* 2010, vol. 76, 190-195
- **KUYPER M. et al.** *FEMS Yeast Res.,* 2004, vol. 4, 655-64
- **KUYPER M. et al.** *FEMS Yeast Res.,* 2005, vol. 5, 399-409
- **KUYPER M. et al.** *FEMS Yeast Res.,* 2005, vol. 5, 925-34
- **NAKAMURA, Y. et al.** Codon usage tabulated from the international DNA sequence databases: status for the year 2000. *Nucl. Acids Res.,* 2000, vol. 28, 292
- **MCLAUGHLIN et al.** *Environ. Sci. Technol.,* 2002, vol. 36, 2122
- **DESAI et al.** *Appl. Microbiol. Biotechnol.,* 2004, vol. 65, 600
- **LYND et al.** *Microbiol. Mol. Biol. Rev.,* 2002, vol. 66, 506
- **ARGYROS, D. A. et al.** High Ethanol Titers from Cellulose by Using Metabolically Engineered Thermophilic, Anaerobic Microbes. *Appl. Environ. Microbiol.,* 2011, vol. 77 (23), 8288-94
- **WATANABE, S. et al.** Ethanol production from xylose by recombinant Saccharomyces cerevisiae expressing protein engineered NADP+-dependent xylitol dehydrogenase. *J. Biotechnol.,* 2007, vol. 130, 316-19
- **MEDINA, V. G. et al.** Elimination of Glycerol Production in Anaerobic Cultures of a Saccharomyces cerevisiae Strain Engineered To Use Acetic Acid as an Electron Acceptor. *Appl. Environ. Microbiol.,* 2010, vol. 76, 190-195
- **VERDUYN et al.** Effect of benzoic acid on metabolic fluxes in yeasts: a continuous-culture study on the regulation of respiration and alcoholic fermentation. *Yeast,* 1992, vol. 8 (7), 501-17
- **CELTON, M. et al.** A constraint-based model analysis of the metabolic consequences of increased NADPH oxidation in Saccharomyces cerevisiae. *Metabolic Eng.,* 2012, vol. 14 (4), 366-79
- **BURDETTE D. ; ZEIKUS, J. G.** Purification of acetaldehyde dehydrogenase and alcohol dehydrogenases from Thermoanaerobacter ethanolicus 39E and characterization of the secondary-alcohol dehydrogenase (2° Adh) as a bifunctional alcohol dehydrogenase-acetyl-CoA reductive thioesterase. *Biochem J.,* 1994, vol. 302, 163-70
- **VAN DEN BRINK, J. et al.** Dynamics of Glycolytic Regulation during Adaptation of Saccharomyces cerevisiae to Fermentative Metabolism. *Appl. Environ. Microbiol.,* 2008, vol. 74 (18), 5710-23
- **IDA, Y. et al.** Stable disruption of ethanol production by deletion of the genes encoding alcohol dehydrogenase isozymes in Saccharomyces cerevisiae. *J. Biosci. Bioeng.,* 2012, vol. 113 (2), 192-95
- **CORDIER, H. et al.** A metabolic and genomic study of engineered Saccharomyces cerevisiae strains for high glycerol production. *Metab. Engineer.,* vol. 9 (4), 364-78
- **LIU, Z. L. ; MOON, J.** A novel NADPH-dependent aldehyde reductase gene from Saccharomyces cerevisiae NRRL Y-12632 involved in the detoxification of aldehyde inhibitors derived from lignocellulosic biomass conversion. *Gene,* 2009, vol. 446 (1), 1-10
- **KUMAR, A. et al.** Cloning and expression of an NADP(+)-dependent alcohol dehydrogenase gene of Entamoeba histolytica. *PNAS,* 1992, vol. 89 (21), 10188-92
- **QUEZ, D. et al.** Two highly divergent alcohol dehydrogenases of melon exhibit fruit ripening-specific expression and distinct biochemical characteristics. *Plant Molecular Biology,* 2006, vol. 61 (4), 675-85
- **RODRIGUES, F. et al.** The Fate of Acetic Acid during Glucose Co-Metabolism by the Spoilage Yeast Zygosaccharomyces bailii. *PLOS One,* 2012, vol. 7 (12), e52402

- **SOUSA, M.J. et al.** Mechanisms underlying the transport and intracellular metabolism of acetic acid in the presence of glucose in the yeast Zygosaccharomyces bailii. *Microbiology,* 1998, vol. 144 (3), 665-70
- **RODRIGUES, F. et al.** Isolation of an acetyl-CoA synthetase gene (ZbACS2) from Zygosaccharomyces bailii. *Yeast,* 2004, vol. 21 (4), 325-31
- **VILELA-MOURA, A. et al.** Reduction of volatile acidity of wines by selected yeast strains. *Appl. Microbiol. Biotechnol.,* 2008, vol. 80 (5), 881-90
- **O'SULLIVAN, J. ; ETTLINGER, L.** Characterization of the acetyl-CoA synthetase of Acetobacter aceti. *Biochimica et Biophysica Acta (BBA) - Lipid and Lipid Metabolism,* 1976, vol. 450 (3), 410-17
- **YURIMOTO, H. et al.** Genomic organization and biochemistry of the ribulose monophosphate pathway and its application in biotechnology. *Appl. Microbiol. Biotechnol.,* 2009, vol. 84, 407-416
- **GEERTMAN, J-M. A. et al.** Engineering NADH metabolism in Saccharomyces cerevisiae: formate as an electron donor for glycerol production by anaerobic, glucose-limited chemostat cultures. *FEMS Yeast Research,* 2006, vol. 6 (8), 1193-1203
- **BAERENDS, R. J. S. et al.** Engineering and Analysis of a Saccharomyces cerevisiae Strain That Uses Formaldehyde as an Auxiliary Substrate. *Appl. Environ. Microbiol.,* 2008, vol. 74 (1), 3182-88
- **JUNG, J-Y. et al.** Production of 1,2-propanediol from glycerol in Saccharomyces cerevisiae. *J. Microbiol. Biotechnol.,* 2011, vol. 21, 846-853
- **ANDERLUND, M. et al.** Expression of the Escherichia coli pntA and pntB Genes, Encoding Nicotinamide Nucleotide Transhydrogenase, in Saccharomyces cerevisiae and Its Effect on Product Formation during Anaerobic Glucose Fermentation. *Appl. Envirol Microbiol.,* 1999, vol. 65, 2333-2340
- **NISSEN, T.L. et al.** Expression of a cytoplasmic transhydrogenase in Saccharomyces cerevisiae results in formation of 2-oxoglutarate due to depletion of the NADPH pool. *Yeast,* 2001, vol. 18, 19-32